(19) Europäisches Patentamt / European Patent Office / Office européen des brevets

(11) **EP 0 623 168 B1**

(12) **EUROPEAN PATENT SPECIFICATION**

(45) Date of publication and mention
of the grant of the patent:
**30.09.2009 Bulletin 2009/40**

(21) Application number: **92922076.2**

(22) Date of filing: **15.10.1992**

(51) Int Cl.:
*C12N 15/12* (2006.01)   *A61K 39/35* (2006.01)
*C12N 15/62* (2006.01)   *G01N 33/53* (2006.01)

(86) International application number:
**PCT/US1992/008637**

(87) International publication number:
**WO 1993/008279 (29.04.1993 Gazette 1993/11)**

(54) **T CELL EPITOPES OF THE MAJOR ALLERGENS FROM DERMATOPHAGOIDES (HOUSE DUST MITE)**

T-ZELL EPITOPEN VON DEN WICHTIGSTEN ALLERGENE VON DERMATOPHAGOIDES (HAUSMILBE)

EPITOPES DE LYMPHOCYTES T DES ALLERGENES PRINCIPAUX DES DERMATOPHAGOIDES (ACARIENS DE LA POUSSIERE)

(84) Designated Contracting States:
**AT BE CH DE DK ES FR GB GR IE IT LI LU MC NL SE**

(30) Priority: **16.10.1991 US 777859**
**08.05.1992 US 881396**

(43) Date of publication of application:
**09.11.1994 Bulletin 1994/45**

(73) Proprietor: **Merck Patent GmbH**
**64293 Darmstadt (DE)**

(72) Inventors:
• **GARMAN, Richard, D.**
**Arlington, MA 02174 (US)**
• **GREENSTEIN, Julia, L.**
**West Newton, MA 02165 (US)**
• **KUO, Mei-Chang**
**Winchester, MA 01890 (US)**
• **ROGERS, Bruce, L.**
**Belmont, MA 02178 (US)**

(56) References cited:
EP-A1- 0 410 848        EP-A1- 0 445 971
WO-A1-88/10297          WO-A1-91/06571
WO-A1-92/04445          FR-A1- 2 437 837

• ANNUAL REVIEW OF IMMUNOLOGY vol. 9, 1991, PALO ALTO, CA US pages 67 - 95 O'HEHIR, R.E. ET AL. 'The specificity and regulation of T-cell responsiveness to allergens' cited in the application
• CLINICAL AND EXPERIMENTAL ALLERGY vol. 21, January 1991, pages 25 - 32 DILWORTH, R.J. ET AL. 'Sequence analysis of cDNA coding for a major house dust mite allergen, Der f I' cited in the application
• JOURNAL OF IMMUNOLOGY vol. 148, no. 3, 1 February 1992, BALTIMORE US pages 738 - 745 YSSEL, H. ET AL. 'T cell activation-inducing epitopes of the house dust mite allergen Der p I' cited in the application
• MCCALL ET AL VETERINARY IMMUNOLOGY IMMUNOPATHOLOGY vol. 78, 2001, pages 231 - 247
• KAWAMOTO ET AL FEBS LETTERS vol. 516, 2002, pages 234 - 238
• O'NEIL ET AL CLINICAL AND EXPERIMENTAL ALLERGY vol. 36, 2006, pages 831 - 839
• KHLGATYAN ET AL BIOKHIMIYA vol. 60, no. 2, 1995, pages 218 - 237

**Description**

**Related Applications**

This application is a continuation-in-part of U.S.S.N. 07/881,396 entitled "T Cell Epitopes of the Major Allergens From Dermatophagoides (House Dust Mite), filed May 8, 1992, which is a continuation-in-part of U.S.S.N. 07/777,859 entitled "T Cell Epitopes of the Major Allergens From Dermatophagoides (House Dust Mite)", filed October 16, 1991, the teachings of which are incorporated herein by reference.

**Background of the Invention**

Recent reports have documented the importance of responses to the Group I (e.g., Der p I and Der f I) and Group II (e.g., Der p II and Deaf II) protein allergens in house dust mite allergy. For example, it has been documented that over 60% of patients have at least 50% of their anti-mite antibodies directed towards these proteins (e.g., Lind, P. et al., Allergy. 39:259-274 (1984); van der Zee, J.S. et al.. Journal Allergy and Clinical Immunology, 81:884-896 (1988)). It is possible that children show a greater degree of reactivity to the Group I and Group II allergens (Thompson, P.J., et al., Immunology. 64:301-314 (1988)). Allergy to mites of the genus Dermatophagoides D.) is associated with conditions such as asthma, rhinitis and ectopic dermatitis. Two species, D. pteronyssinus and D. farinae, predominate and, as a result, considerable effort has been expended in trying to identify the allergens produced by these two species.

A concerted effort has been made to characterize by gene cloning the major allergens from both D. pteronyssinus and D. farinae. Consequently, several publications have reported the complete nucleotide sequences of several allergens including Der p I (Thomas, W.R., et al., International Archives of Allergy and Applied Immunology, 85:127-129 (1988); and Chua, K.Y.. et al., Journal of Experimental Medicine, 167:175-182 (1988)), Der p II (Chua, K.Y., et al., International Archives of Allergy and Applied Immunology, 91:118-123 (1990)), Der f I (Dilworth, R.J., et al., Clinical and Experimental Allergy 21:25-32 (1891)), Der f II (Yuuki, T., et al., Japan Journal Allergol., 39:557-461 (1990); and Trudinger, M., et al., Clinical and Experimental Allergy, 21:33-37 (1991)) and a low molecular weight allergen (Ovey, E.R., et al, Journal of Experimental Medicine, 170:1457-1462 (1989)).

The published nucleotide sequences of cDNAs encoding Der p I and Der f I demonstrate that these two proteins are highly homologous at the amino acid level (81% identity) and that the mature protein products are comprised of 222 and 223 residues, respectively (Chua, K.Y., et al., Journal of Experimental Medicine, 167: 175-182 (1988); and Dilworth, R.J., et al., supra)). The protein allergens Der p II and Der f II are both comprised of 129 residues, and are also highly homologous (88% identity) in amino acid sequence (Trudinger, M., et al. supra; Yuuki, T., et al. supra); Chua, KY., et al. International Archives of Allergy and Applied Immunology 91:118-123 (1990)).

The isolation of cDNAs clones encoding Der p I and Der p II has permitted antibody binding studies on the recombinant antigens (Green. W.K., et al., International Archives of Allergy and Applied Immunology, 93:30-38 (1990); Chua, K.Y.. et al., International Archives of Allergy and Applied Immunology, 91:124-129 (1990)). Complementary DNA fragments of Der p I have been expressed in E. coli and IgE binding studies with pooled human mite allergic IgE sera have demonstrated binding and non-binding regions throughout the molecule (Thomas, W.R., et al., In: Epitopes of Atopic Allergens, Proceedings of Workshop from XIV Congress of the European Academy of Allergy and Clinical Immunology, Berlin, Sept 1989. pp 77-82). T cell epitopes of Der p I have been reported (O'Hehir, R.E., et aL, Annual Review Immunology, 9:67-95 (1991); Stewart, G.A., et al., In: Epitopes of Atopic Allergens, Proceedings of Workshop from XIV Congress of the European Academy of Allergy and Clinical Immunology. Berlin, Sept. 1989. pp 41-47; Yessel, H., et al, In: T cell Activation in Health and Disease: Discrimination Between Immunity and Tolerance, Conference 22-26 Sept, 1990, Trinity College, Oxford, U.K. and Hessel, H., et al., Journal of Immunology, 148 (3): 738-745 (Feb. 1, 1992).

**Summary of the Invention**

The present invention provides isolated peptides of the Dermatophagoides group I or II protein allergens. Peptides within the scope of the invention comprise at least one T cell epitope, preferably at least two T cell epitopes of a protein allergen selected from the allergens Der p I, Der p II, Per f I, or Der f II. The invention further provides peptides comprising at least two regions, each region comprising at least one T cell epitope of a mite protein allergen. The regions are derived from the same or from different Dermatophagoides group I or II protein allergens.

The invention also provides modified peptides having similar or enhanced therapeutic properties as the corresponding, naturally-occurring allergen or portion thereof, but having reduced side effects, as well as modified peptides having improved properties such as increased solubility and stability. Peptides of the invention are capable of modifying, in a house dust mite-sensitive individual to whom they are administered, the allergic response of the individual to a house dust mite allergen or an allergen immunologically corss-reactive with house dust mite allergen. Methods of treatment or of diagnosis of sensitivity to house dust mite in an individual and therapeutic compositions comprising one or more peptides of the invention are also provided.

## Brief Description of the Drawings

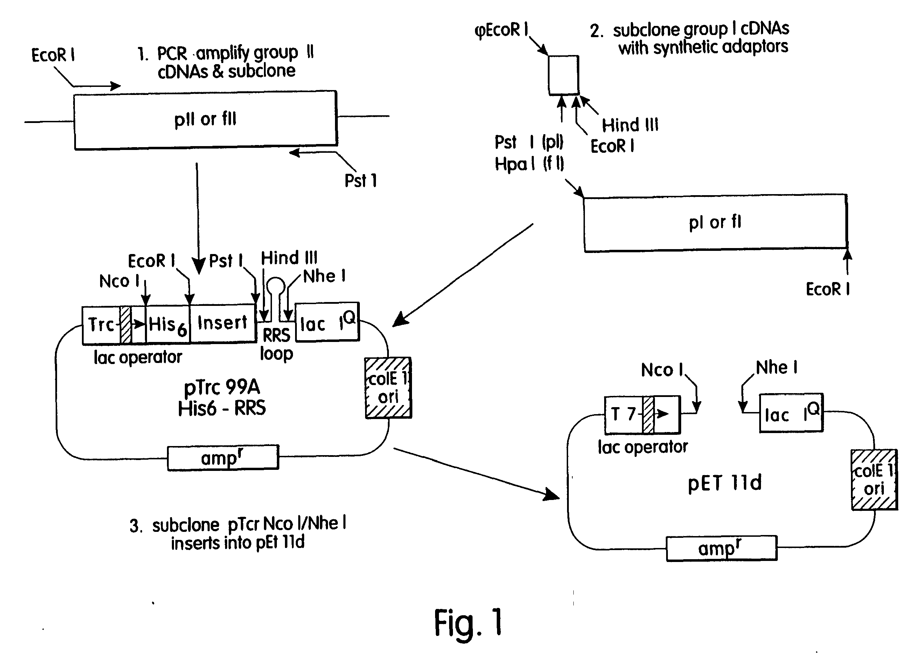

Fig. 1 shows the subcloning and expression of Group I and Group II allergens from <u>D. pteronyssinus</u> and <u>D. farinae.</u>

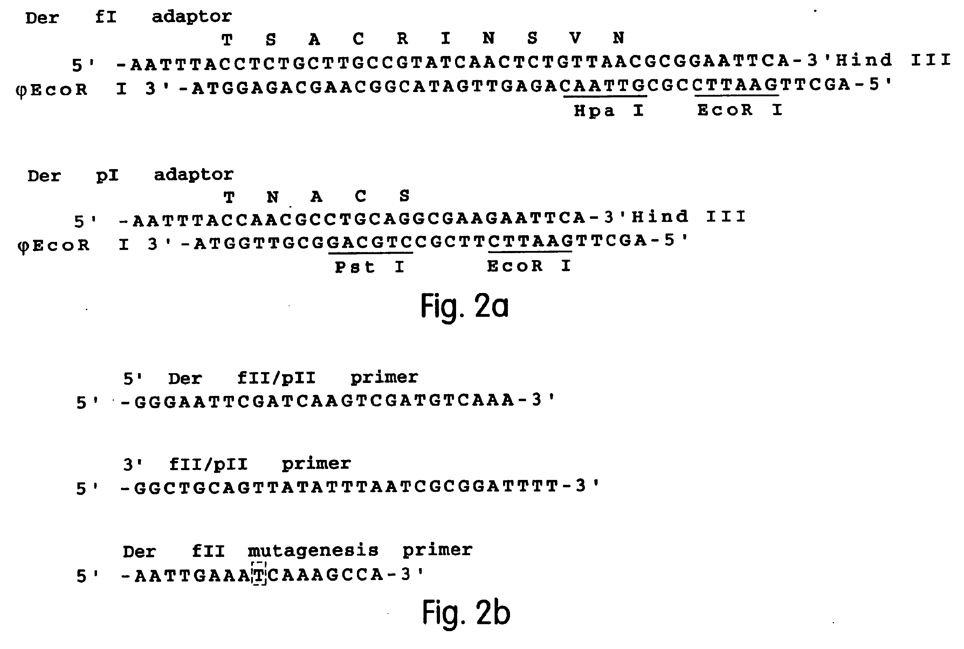

Fig. 2a shows adaptors used in the expression of <u>Der p</u> I and <u>Der f</u> I and Fig. 2b shows primers for amplification of <u>Der f</u> II and <u>Der p</u> II and a <u>Der f</u> II mutagenesis primer.

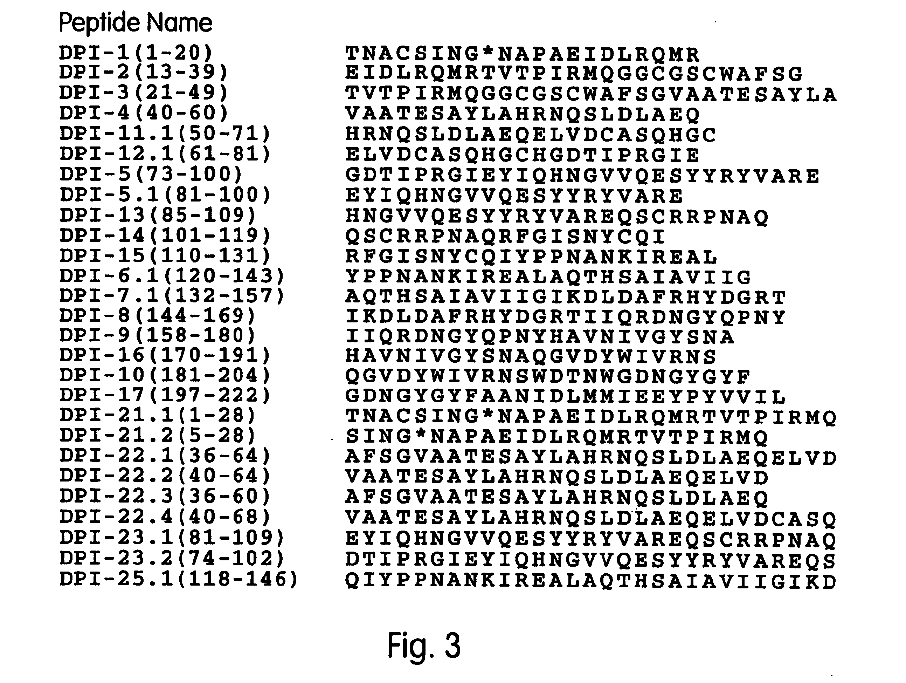

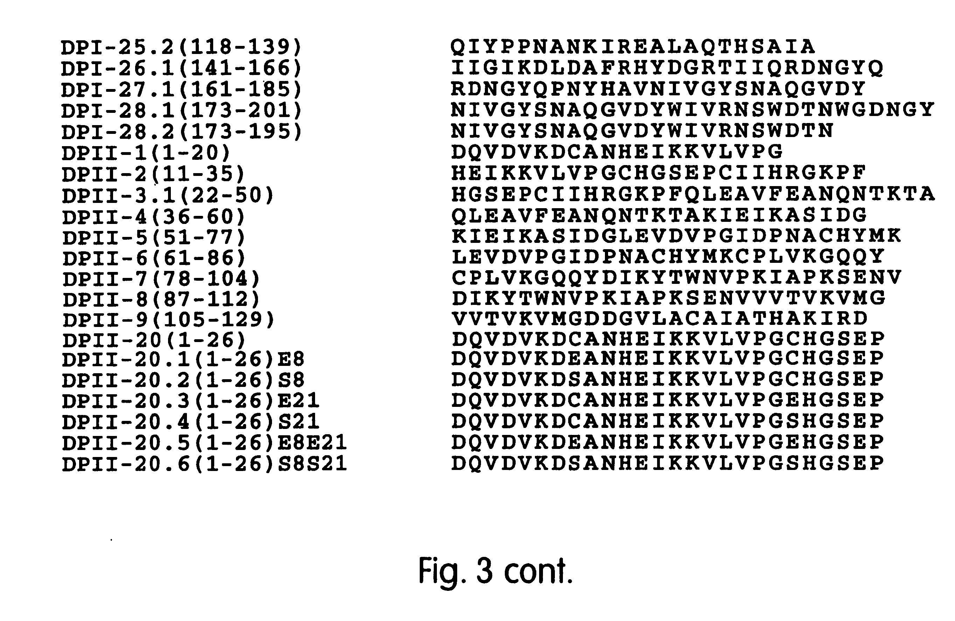

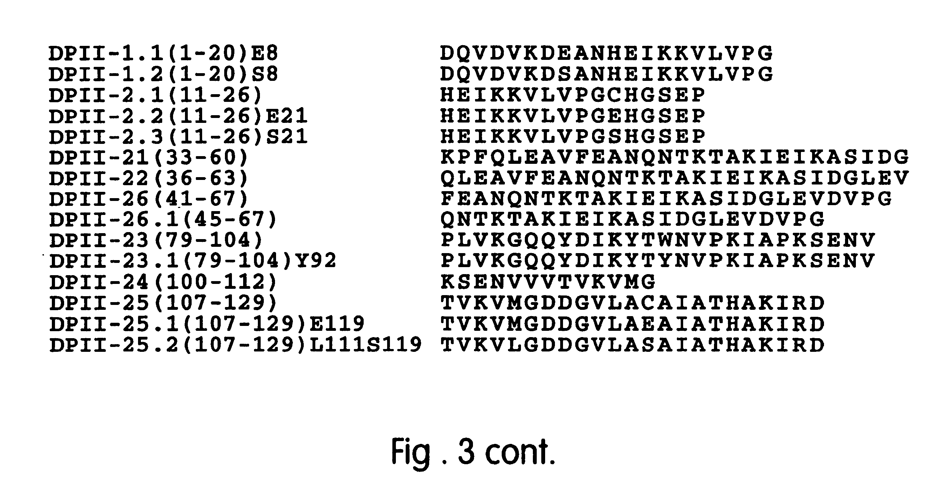

Fig. 3 shows various peptides of desired lengths derived from the <u>Der p</u> I and <u>Der p</u> II protein allergens.

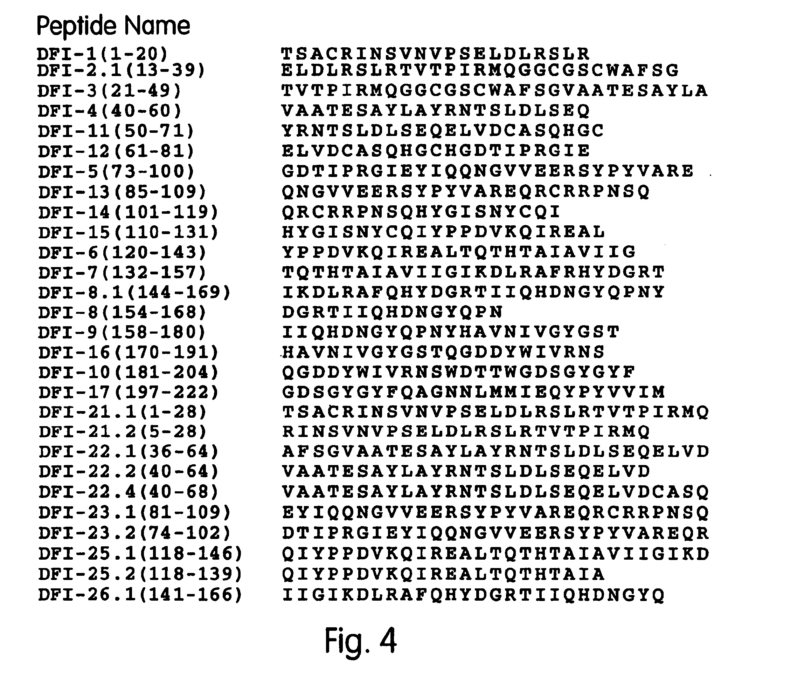

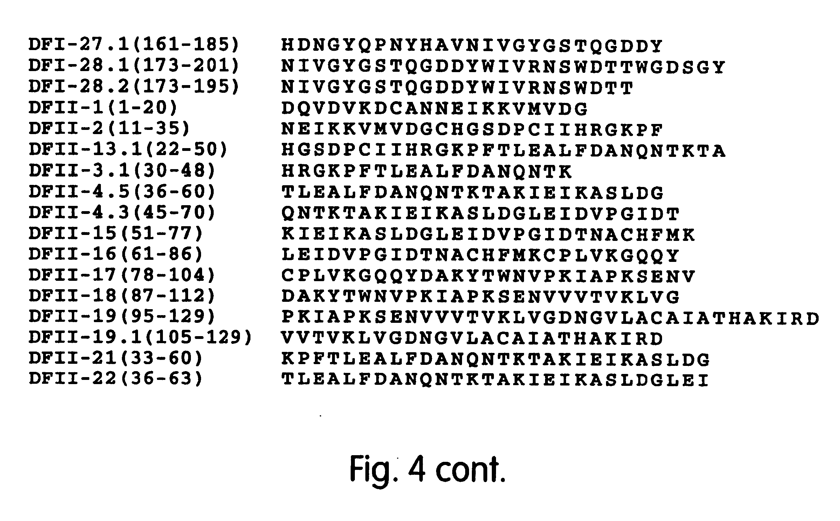

Fig. 4 shows various peptides of desired lengths derived from the <u>Der f</u> I and <u>Der f</u> II protein allergens.

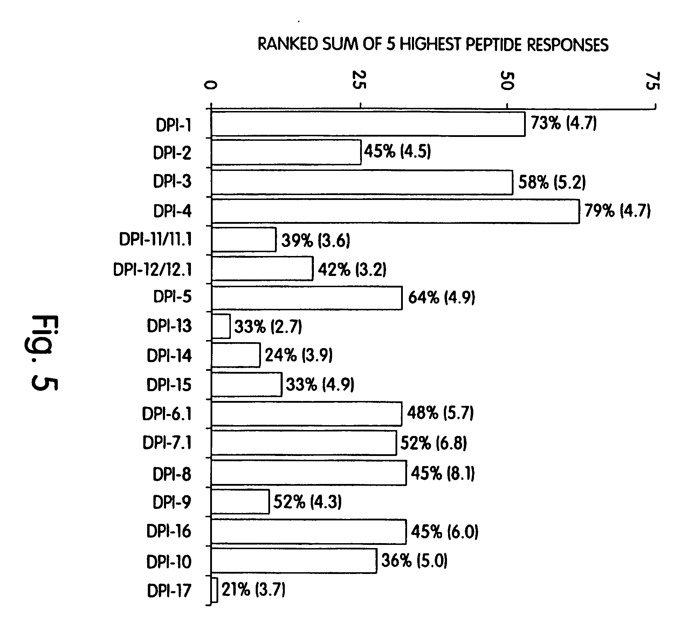

Fig. 5 is a graphic representation depicting the responses of T cell lines from 33 patients primed <u>in vitro</u> to either purified native (N) or recombinant (R) <u>Der p</u> I protein and analyzed for response to various overlapping <u>Der p</u> I peptides by percent of responses with a T cell Stimulation Index (S.I.) of at least 2 within the individuals tested, the mean T cell stimulation index of positive responses for the peptide and the ranked sum of Peptide responses.

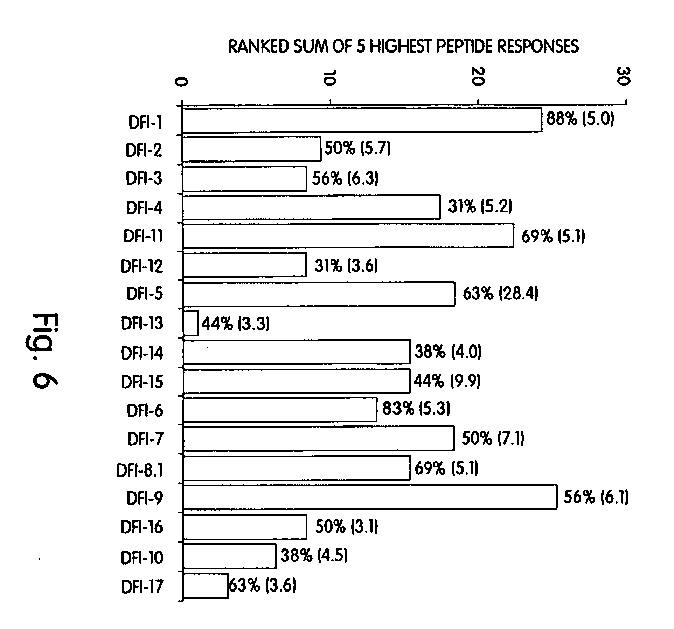

Fig. 6 is a graphic representation depicting the responses of T cell lines from 16 patients primed <u>in vitro</u> to the <u>Der f</u> I protein and analyzed for response to various overlapping <u>Der f</u> I peptides by percent of responses with an (S.I.) of at least 2 within the individuals tested, the mean T cell stimulation index of positive responses for the peptide and the ranked sum of peptide. responses.

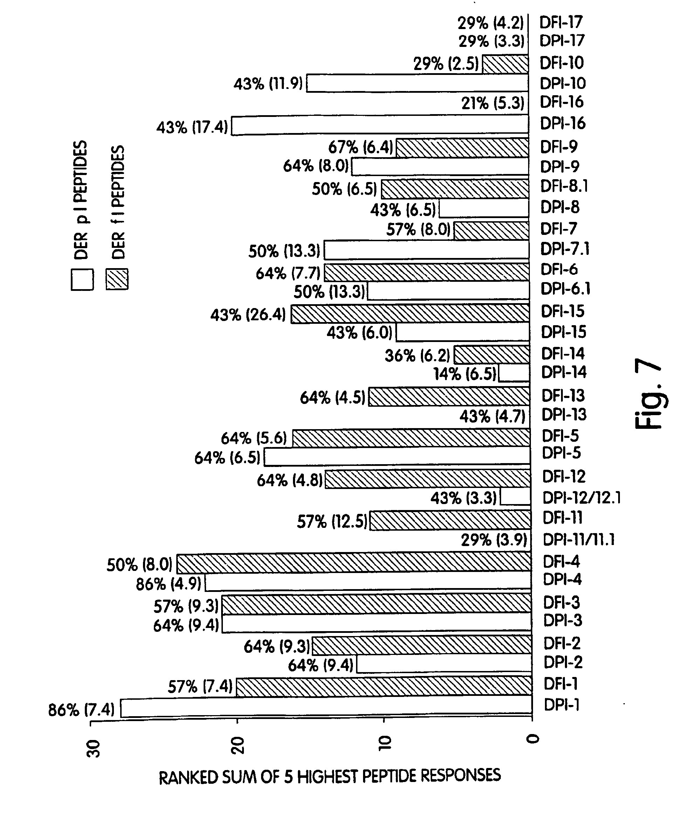

Fig. 7 is a graphic representation depicting the responses of T cell lines from 14 patients primed <u>in vitro</u> to the <u>Der p</u> I protein and analyzed for response to various overlapping <u>Der p</u> I peptides and substantially matching <u>Der f</u> I peptides by percent of responses with an S.I. of at least 2 within the individuals tested, the mean T cell stimulation index of positive responses for the peptide and the ranked sum of peptide responses.

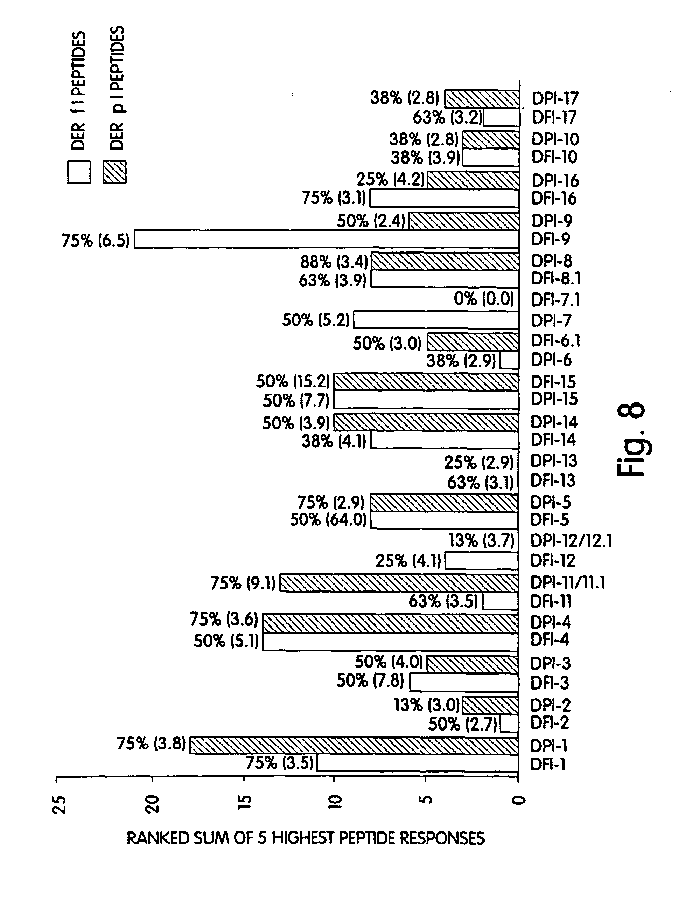

Fig. 8 is a graphic representation depicting the responses of T cell lines from 8 patients primed <u>in vitro</u> to the <u>Der f</u> I protein and analyzed for response to various overlapping <u>Der f</u> I peptides and substantially matching <u>Der p</u> I peptides by percent of responses with an S.I. of at least 2 within the individuals tested, the mean T cell stimulation index of positive responses for the peptide and the ranked sum of peptide responses.

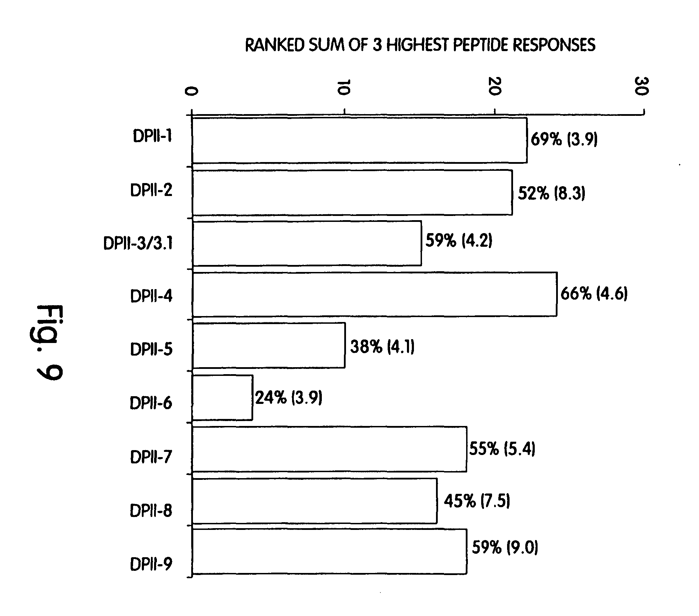

Fig. 9 is a graphic representation depicting the responses of T cell lines from 29 patients primed <u>in vitro</u> to the <u>Der p</u> II protein and analyzed for response to various overlapping <u>Der p</u> II peptides by percent of responses with an S.I. of at least 2 within the individuals tested, the mean T cell stimulation index of positive responses for the peptide and the ranked sum of peptide responses.

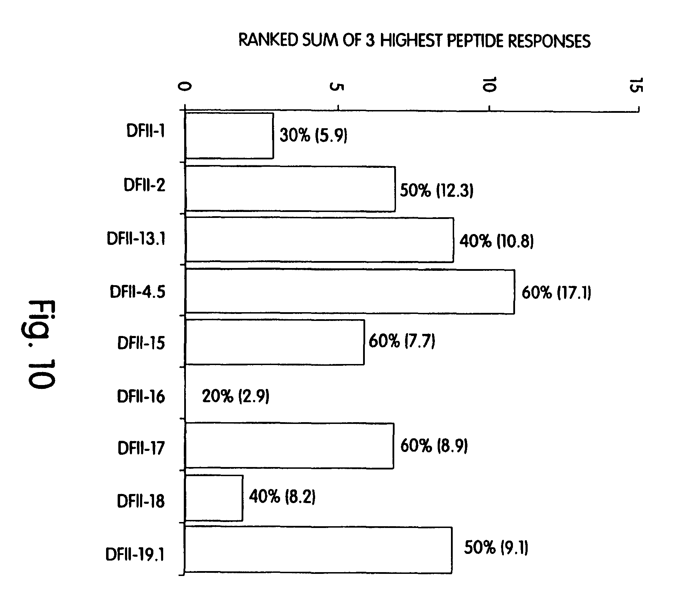

Fig. 10 is a graphic representation depicting the responses of T cell lines from 10 patients primed <u>in vitro</u> to the <u>Der f</u> II protein and analyzed for response to various overlapping <u>Der f</u> II peptides by percent of responses with an S.I. of at least 2 within the individuals tested, the mean T cell stimulation index of positive responses for the peptide and the ranked sum of peptide responses.

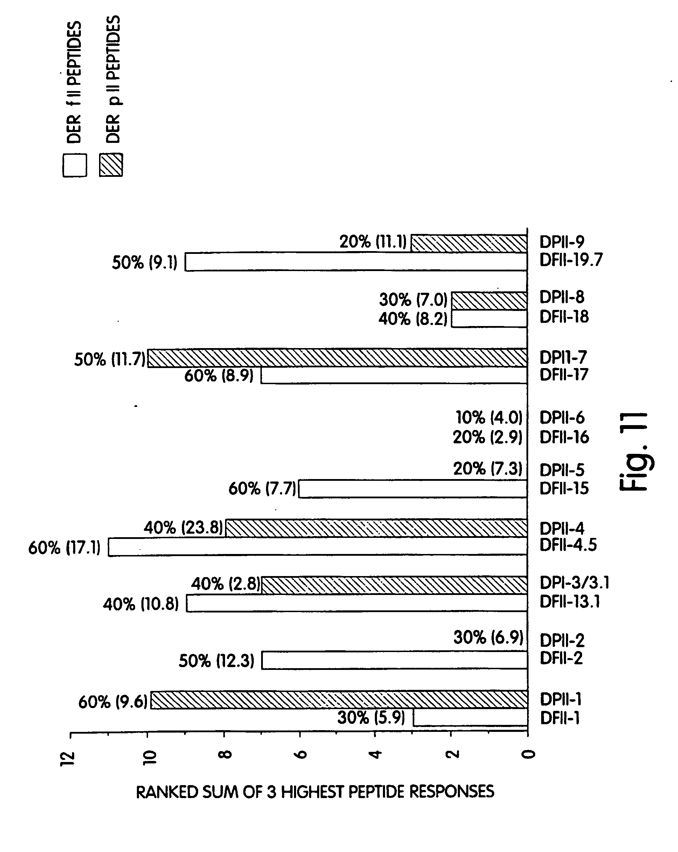

Fig. 11 is a graphic representation depicting the responses of T cell lines from 10 patients primed <u>in vitro</u> to the <u>Der f</u> II protein and analyzed for response to various overlapping <u>Der f</u> II peptides and substantially matching <u>Der p</u> II peptides by percent of responses with an S.L of at least 2 within the individuals tested, the mean T cell stimulation index of positive responses for the peptide and the ranked sum of peptide responses.

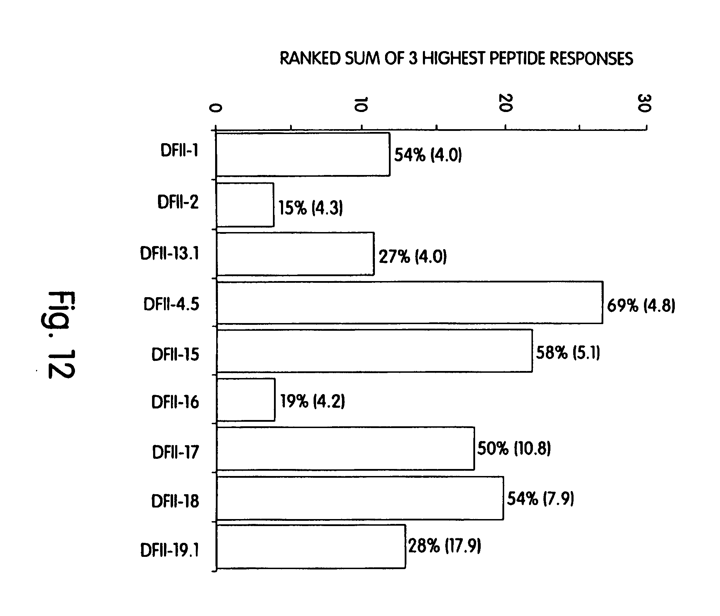

Fig. 12 is a graphic representation depicting the responses of T cell lines from 26 patients primed <u>in vitro</u> to the <u>Der p</u> II protein and analyzed for response to various overlapping <u>Der f</u> II peptides by percent of responses with an S.I. of at least 2 within the individuals tested, the mean T cell stimulation index of positive responses for the peptide and the ranked sum of peptide responses.

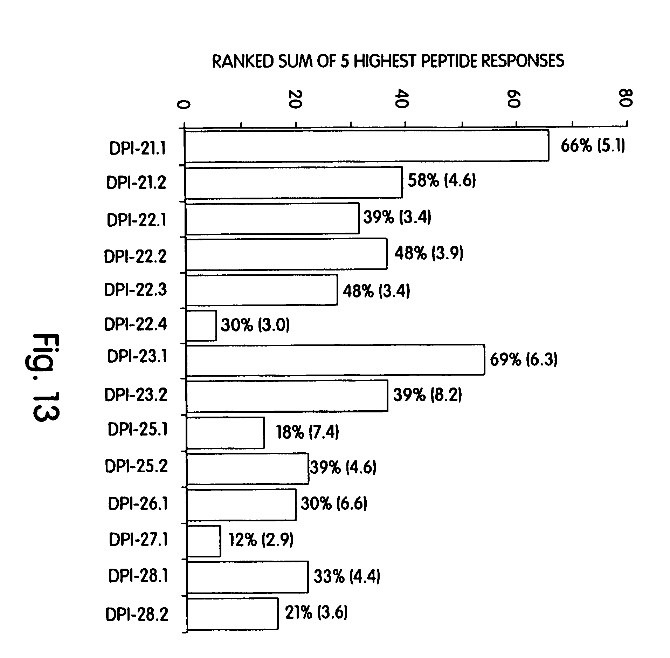

Fig. 13 is a graphic representation depicting the responses of T cell lines from 33 patients primed <u>in vitro</u> to the <u>Der p</u> I protein and analyzed for response to selected peptides of desired lengths derived from the <u>Der p</u> I protein allergen by percent of responses with an S.I. of at least 2 within the individuals tested, the mean T cell stimulation index of positive responses for the peptide and the ranked sum of peptide responses.

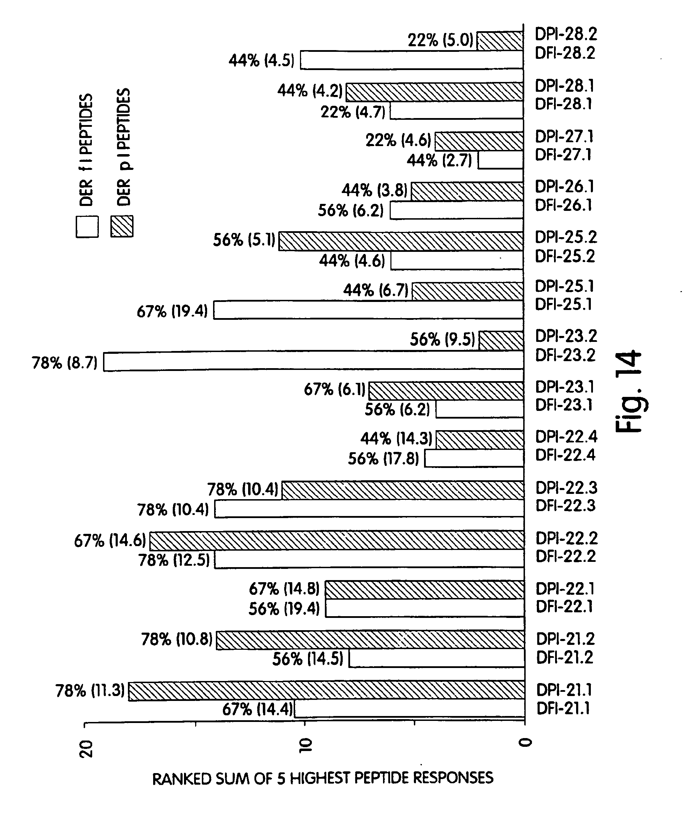

Fig. 14 is a graphic representation depicting the responses of T cell lines from 9 patients primed <u>in vitro</u> to the <u>Der f</u> I protein and analyzed for response to selected peptides of desired lengths derived from the <u>Der f</u> I protein allergen, by percent of responses with an S.I. of at least 2 within the individuals tested and the mean T cell stimulation index of positive responses for the peptide and the ranked sum of peptide responses.

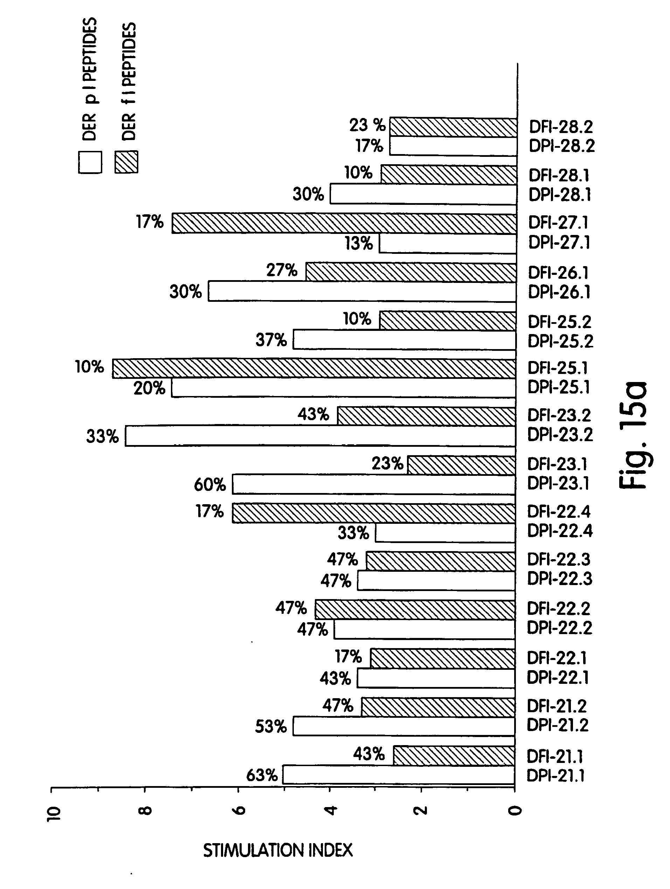

Fig. 15a is a graphic representation depicting the responses of T cell lines from 30 matched patients primed <u>in vitro</u> to the <u>Der p</u> I protein and analyzed for response to selected peptides of desired lengths derived from the <u>Der p</u> I and the <u>Der f</u> I protein allergens, by percent of responses with an S.I. of at least 2 within the individuals tested and the mean T cell stimulation index of positive responses for the peptide.

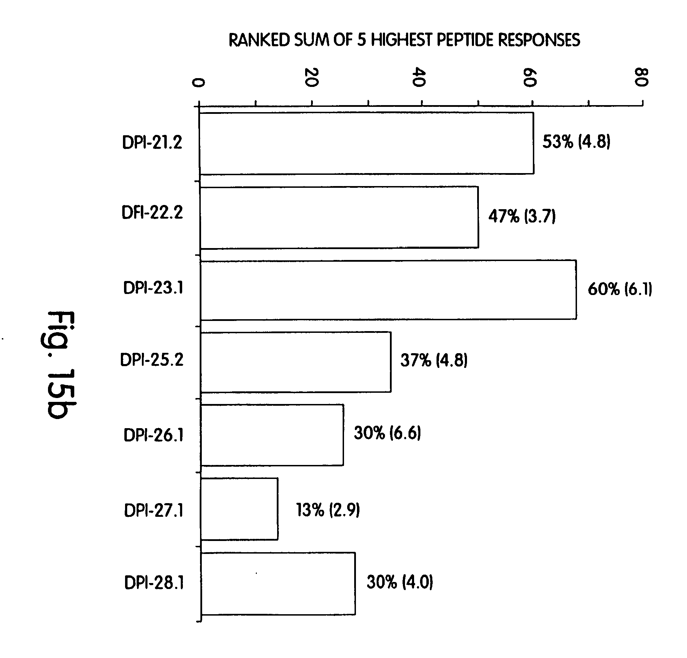

Fig. 15b is a graphic representation derived from the same data shown in Fig. 15a showing the response of <u>Der p</u> I primed T cells to preferred <u>Der p</u> I peptides analyzed by percent of response with an S.I. of at least two within the individuals tested (above each bar), the mean T cell stimulation index (above each bar in parenthesis) and the ranked sum of peptide responses.

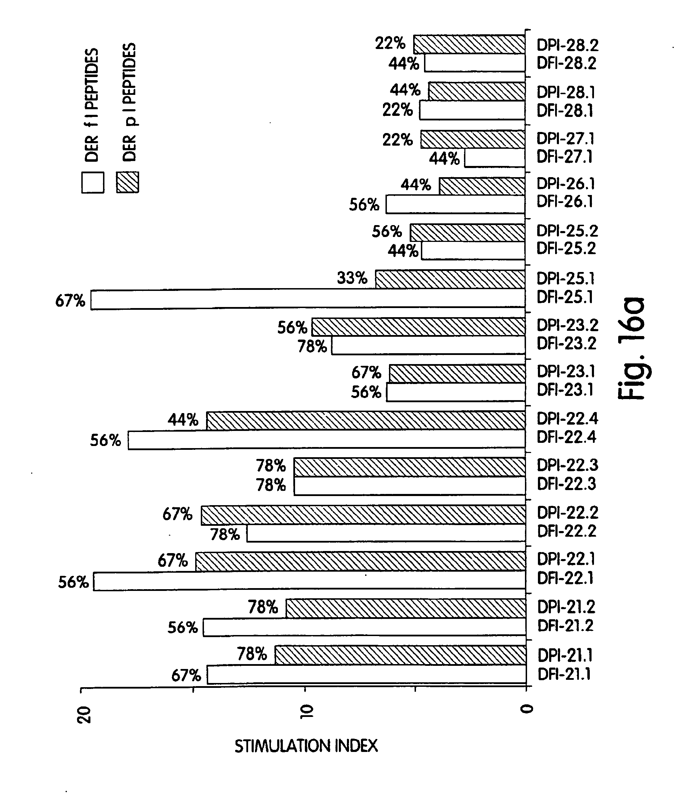

Fig. 16a is a graphic representation depicting the responses of T cell lines from 9 patients primed in vitro to the Der f I protein and analyzed for response to selected peptides of desired lengths derived from the Der f I and Der p I protein allergens, by percent of responses with an S.I. of at least 2 within the individuals tested and the mean T cell stimulation index of responses with an S.I. of at least 2 for the peptide.

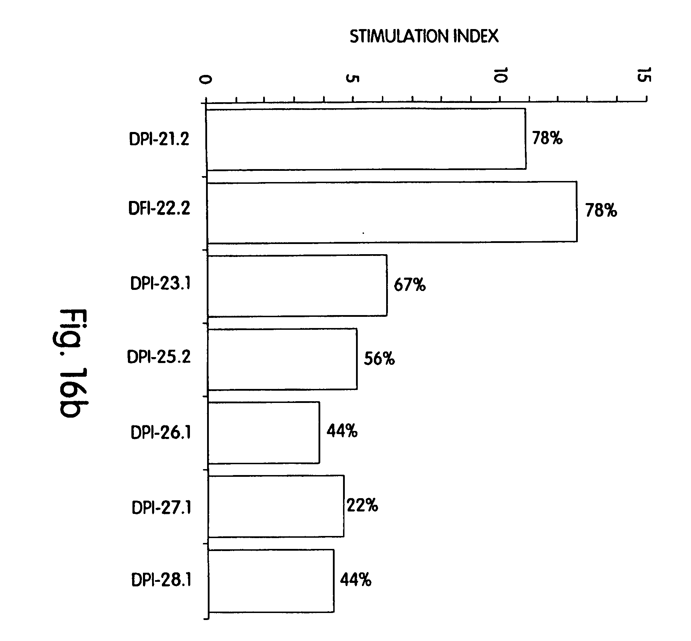

Fig. 16b is a graphic representation derived from the same data shown in Fig. 16a showing the response of Der p I primed T cell lines to preferred Der f I and Der p I peptides by percent of responses with an S.I. of at least 2 within the individuals tested and the mean T cell stimulation index of responses with an S.I. of at least 2 for the peptide.

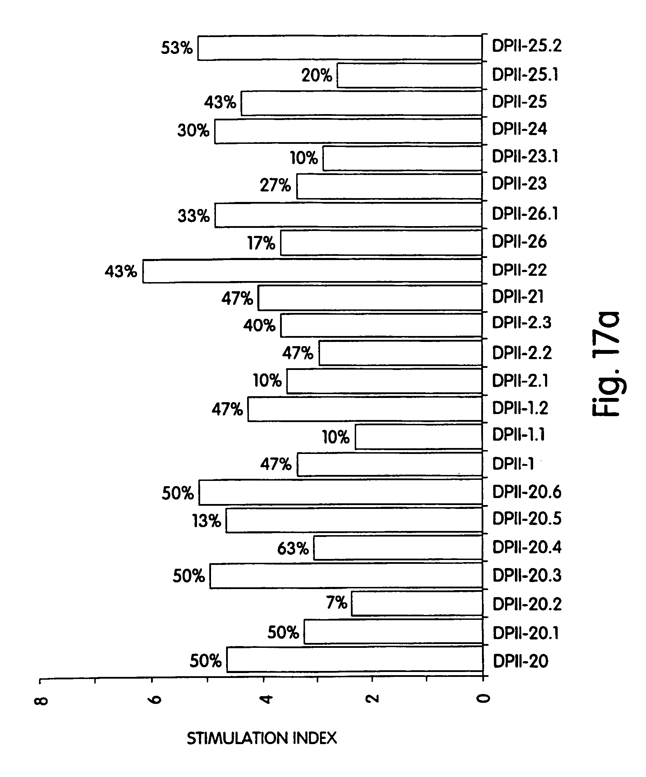

Fig. 17a is a graphic representation depicting the response of T cells from 29 patients primed in vitro to the Der p II protein and analyzed for response to selected peptides of desired lengths derived from the Der p II protein, by the T cell stimulation index of a response with an S.I. of at least 2 for the peptide.

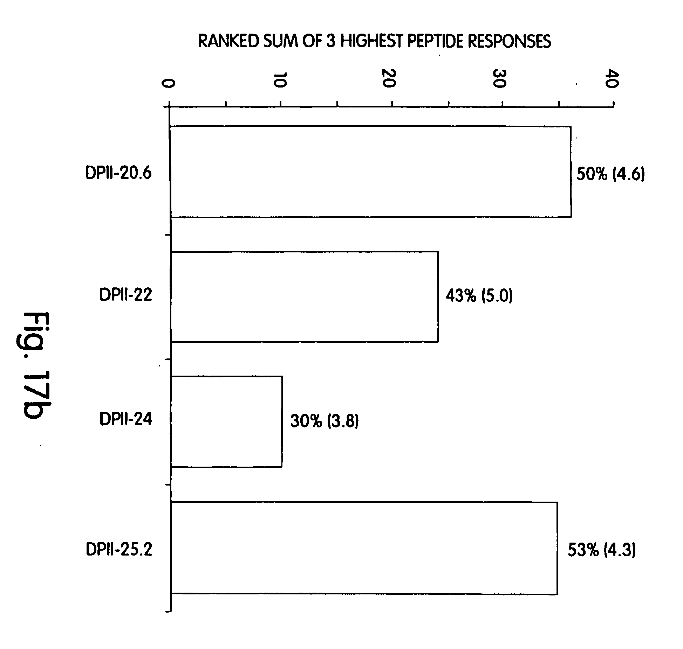

Fig. 17b is a graphic representation depicting the response of 30 patients primed in vitro to the Der p II protein and analyzed for response to selected peptides derived from the Der p II protein by percent of responses with an S.I. of at least 2 within the individuals tested (above each bar), the mean T cell stimulation index (above each bar in parenthesis) and the ranked sum of peptide response (X-axis).

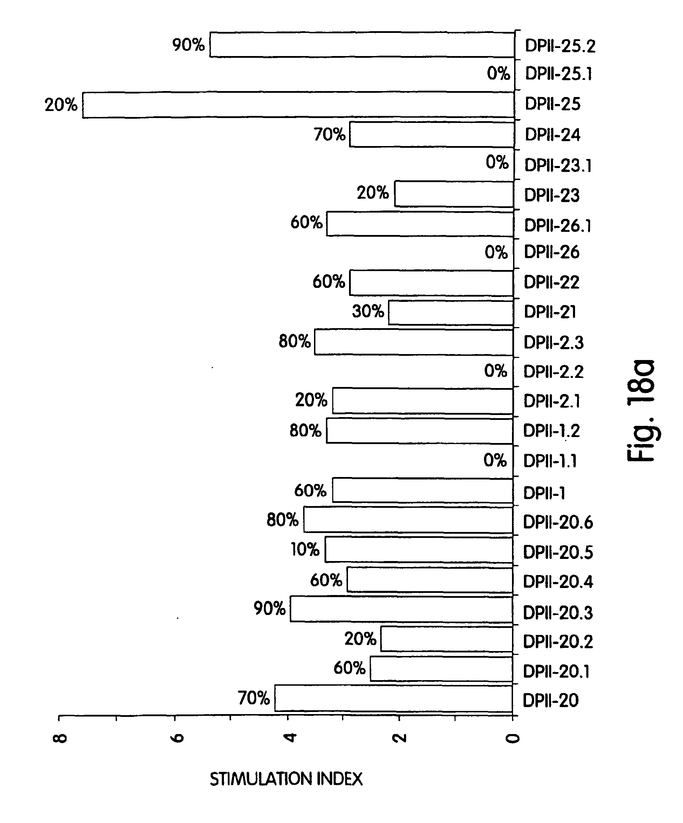

Fig. 18a is a graphic representation depicting the response of T cells from 10 patients primed in vitro to the Der f II protein and analyzed for response to selected peptides of desired lengths derived from the Der p II and Der f II protein, by the T cell stimulation index of a response with an S.I. of at least 2 for the peptide.

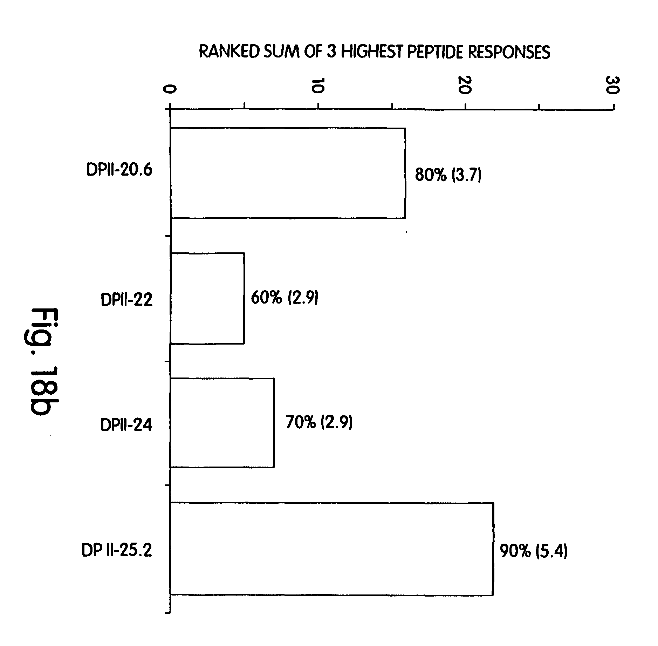

Fig. 18b is a graphic representation derived from the same data shown in Fig. 18a showing the response of Der f II primed T cell lines to preferred Der p II peptides analyzed by percent responses with an S.I. of at least 2 within the individuals tested (above each bar), the mean T cell stimulation index (above each bar in parenthesis) and the ranked sum of peptide responses (X-axis).

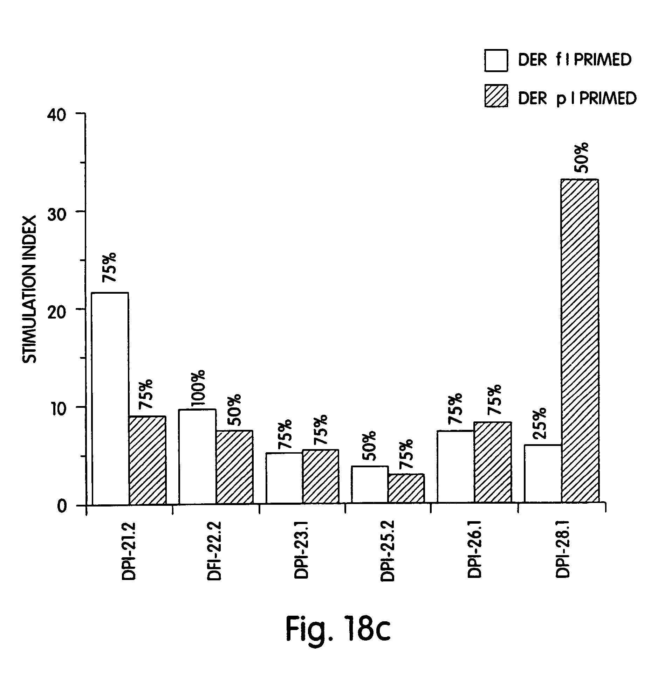

Fig. 18c is a graphic representation depicting the responses of T cell lines from 4 matched patients primed in vitro with mite group I allergen and analyzed for response to preferred Der p I and Der p I peptides by percent of responses with an S.I. of at least 2 within the individuals tested and the mean T cell stimulation index of positive responses for the peptide.

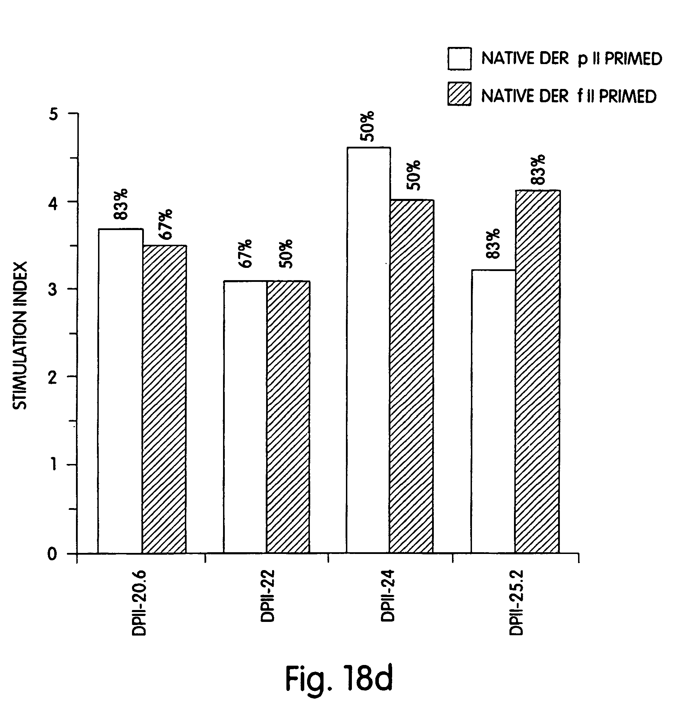

Fig. 18d is a graphic representation depicting the responses of T cell lines from 6 matched patients primed in vitro with mite group II allergen and analyzed for response to preferred Der p II peptides by percent of responses with an S.I. of at least 2 within the individuals tested and the mean stimulation index of positive responses for the peptide.

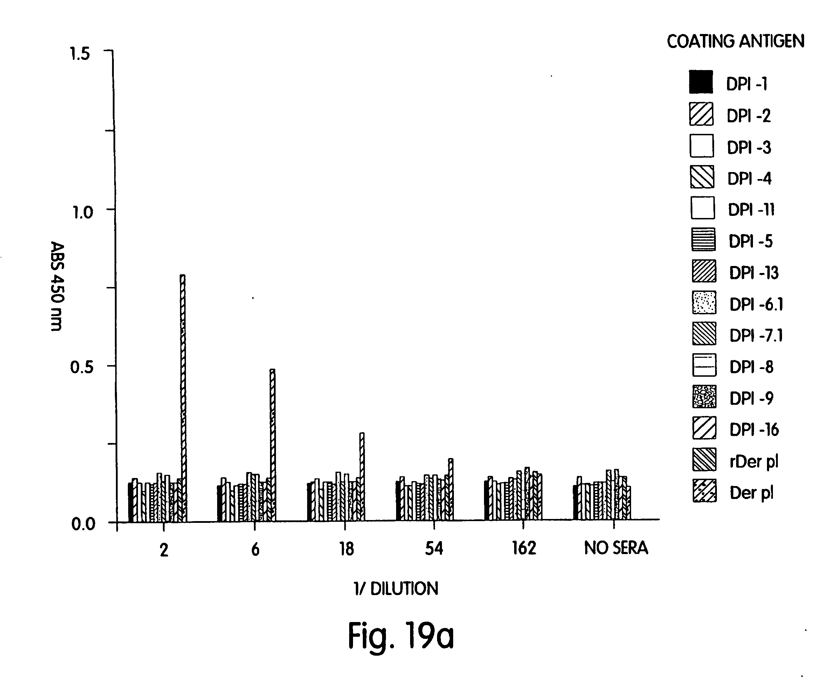

Fig. 19a-19b are graphic representations of the results of a direct binding assay of IgE to affinity purified and recombinant Der p I and Der p II proteins and certain Der p I and Der p II overlapping peptides.

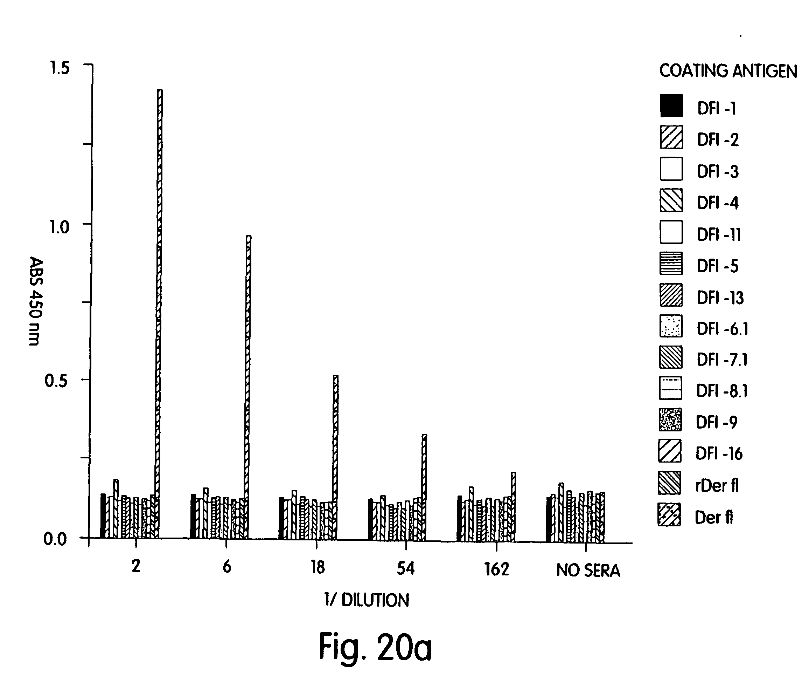

Fig. 20a-20b are graphic representations of the results of a direct binding assay of IgE to affinity purified and recombinant Der f I and Der f II proteins and certain Der f I and Der f II overlapping peptides.

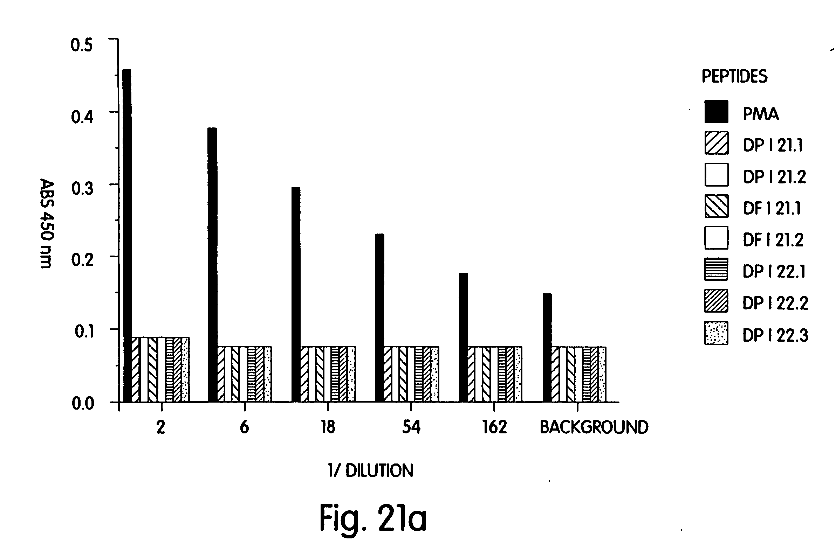

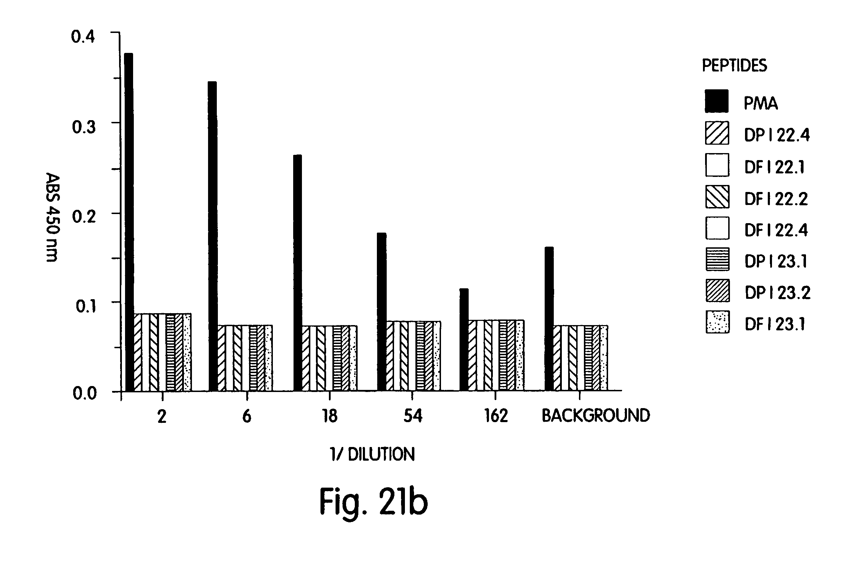

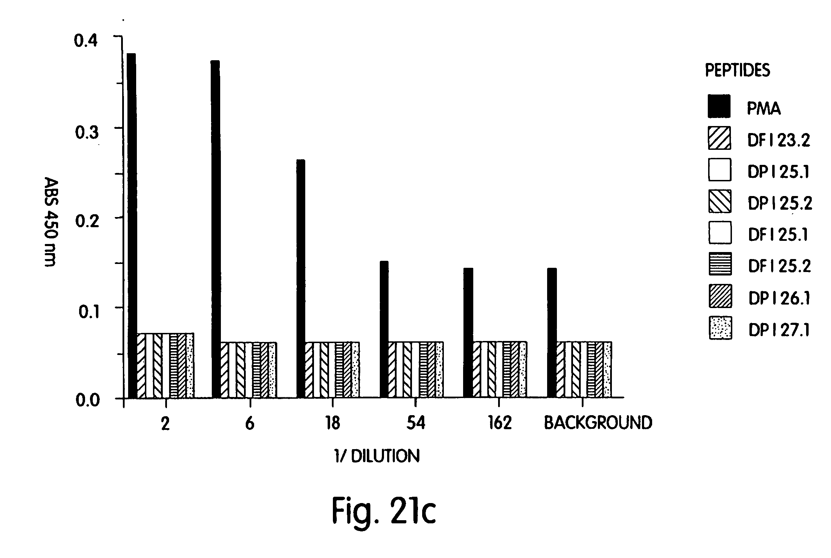

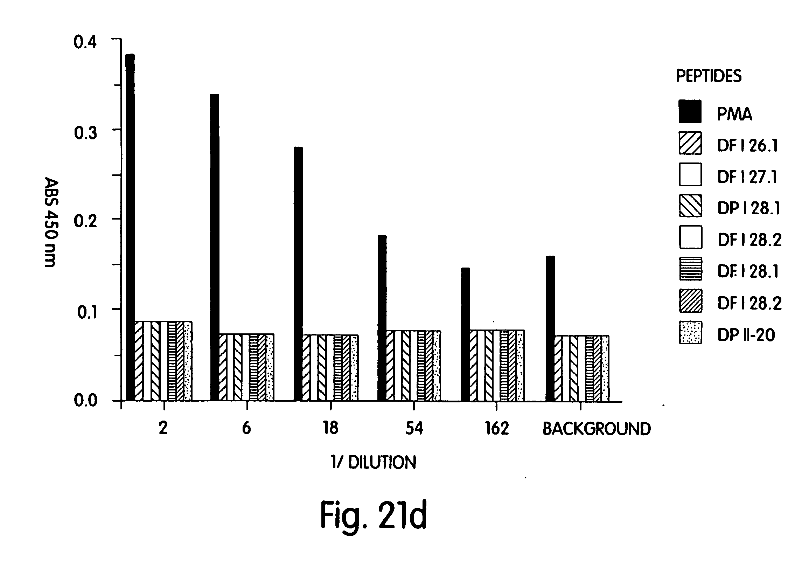

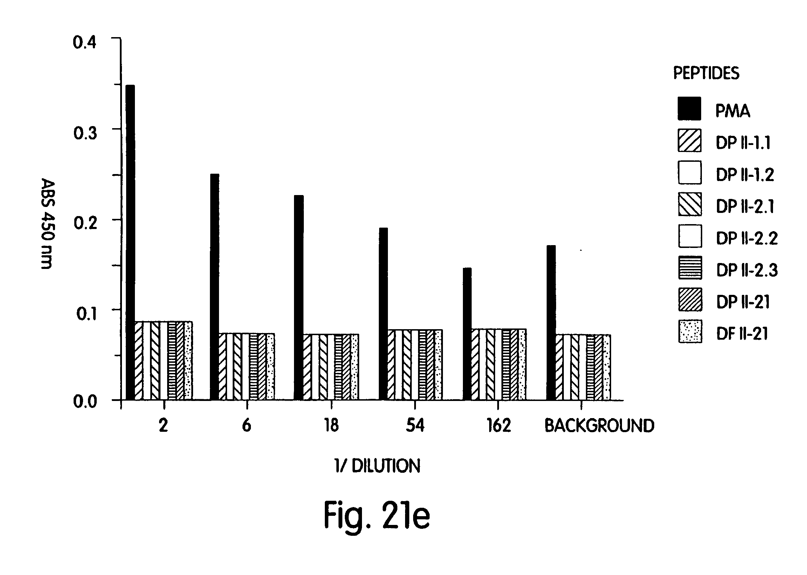

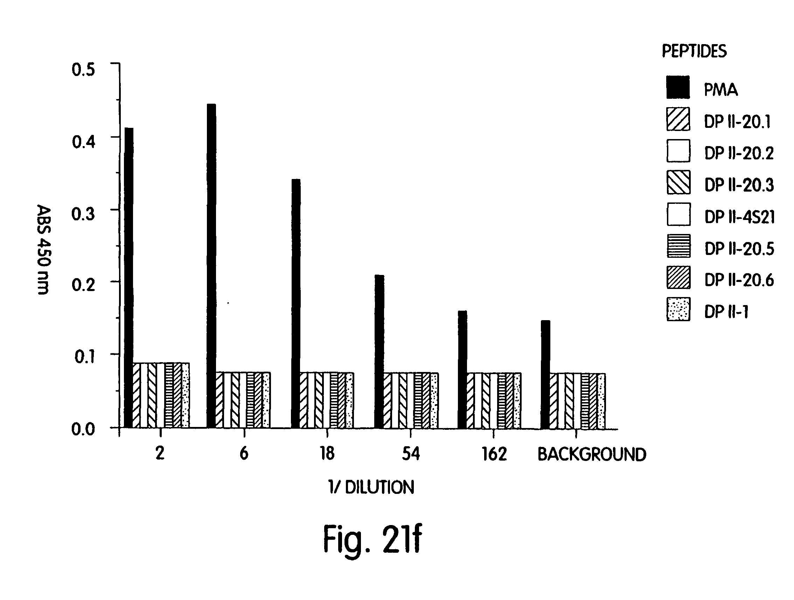

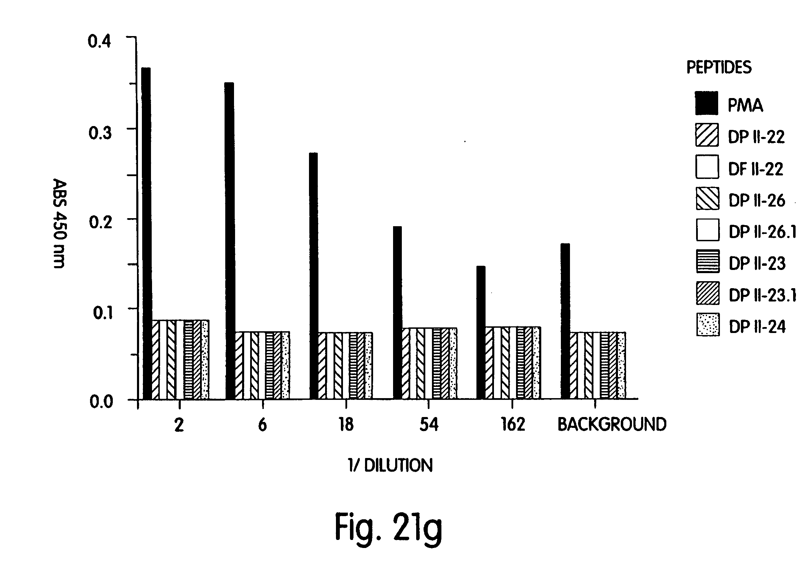

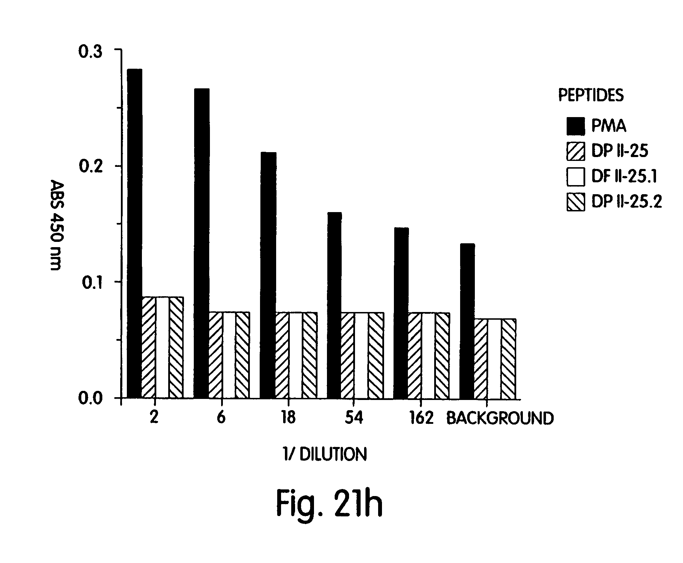

Fig. 21a-21h are graphic representations of the results of a direct binding assay of IgE to a mixture of biochemically purified mite allergens (PMA) and various peptides derived from Der p I, Der f I, Der p II and Der f II.

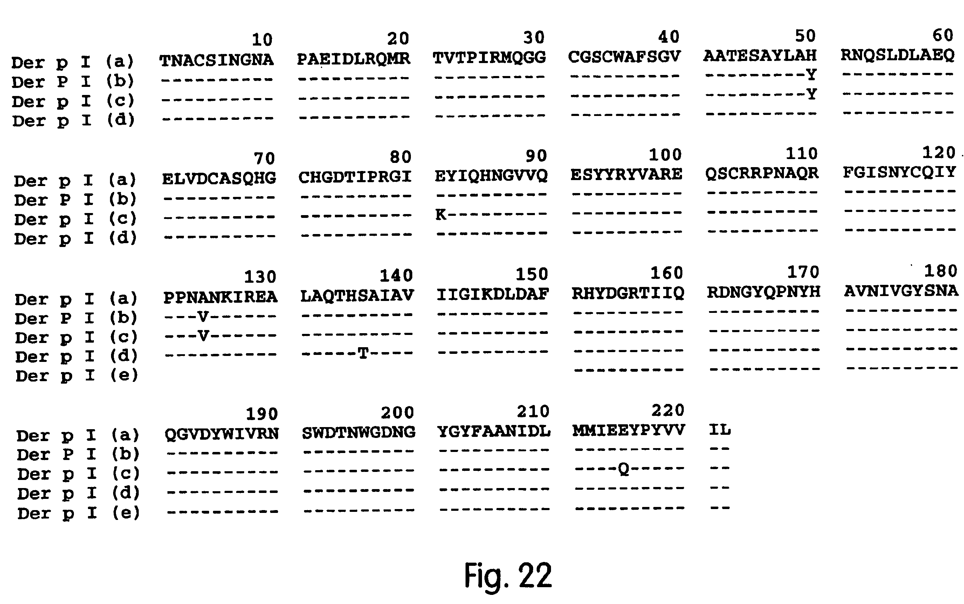

Fig. 22 is a composite alignment of the amino acid sequences of five Der p I clones (a)-(e) which illustrates polymorphism in the Der p I protein. The numbering refers to the sequence of the Der p I(a) clone. The symbol (-) is used to indicate that the amino acid residue of a Der p I clone is identical to the corresponding amino acid sequences of Der p I(a) at that position. The amino acid sequences of these clones indicate that there may be significant variation in Der p I, with five polymorphic amino acid residues found in the five sequences.

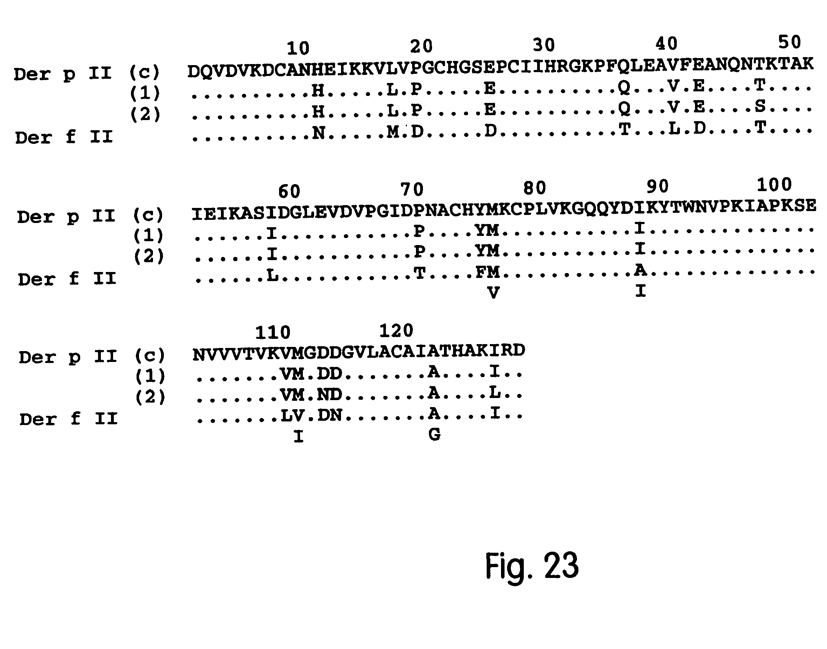

Fig. 23 is a composite alignment of the amino acid sequences of three Der p II clones (c), (1) and (2) which illustrates polymorphism in the Der p II protein. The numbering refers to the sequence of the Der p II(c) clone. The symbol (.) is used to indicate that the amino acid residue of a Der p II clone is identical to the corresponding amino acid residue of Der p II (c) at that position.

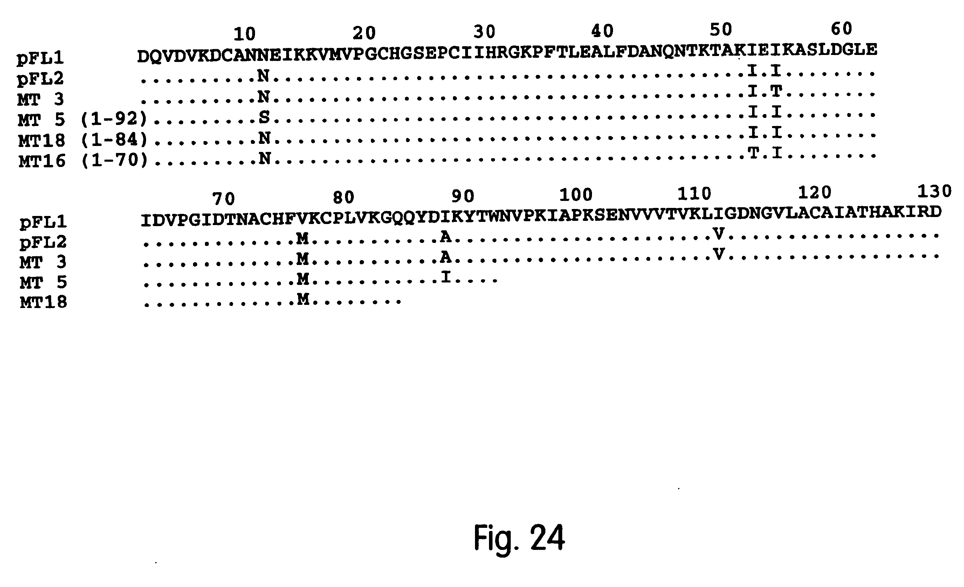

Fig. 24 is a composite alignment of the amino acid sequences of six Der f II clones (i.e., pFL1, pFL2, MT3, MT5, MT18 and MT16) which illustrates polymorphism is the Der f II protein. The numbering refers to the sequences of the Der f pLF1 clone. The symbol (.) is used to indicate that the amino acid residue of a Der f II clone is identical to the corresponding amino acid residue of Der f II pFL1 at that position.

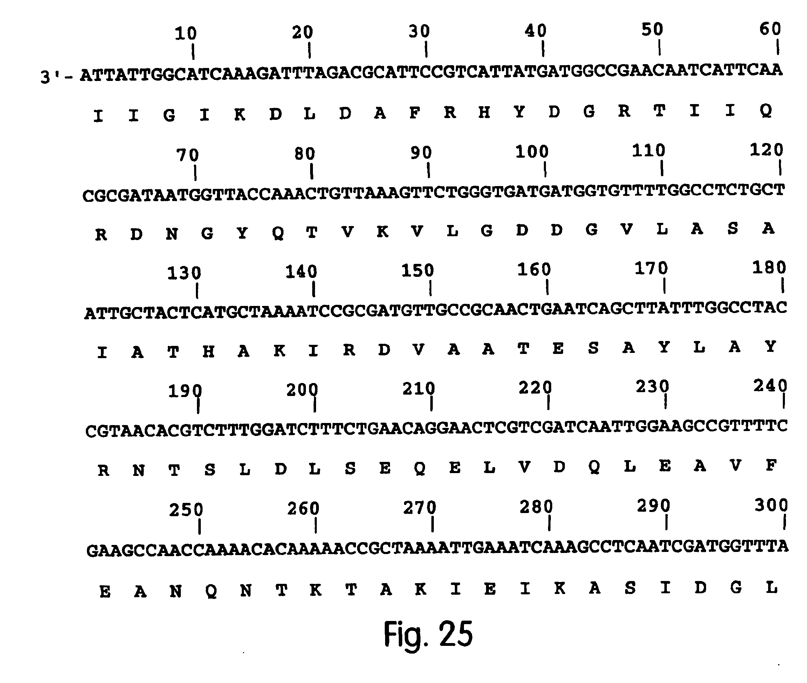

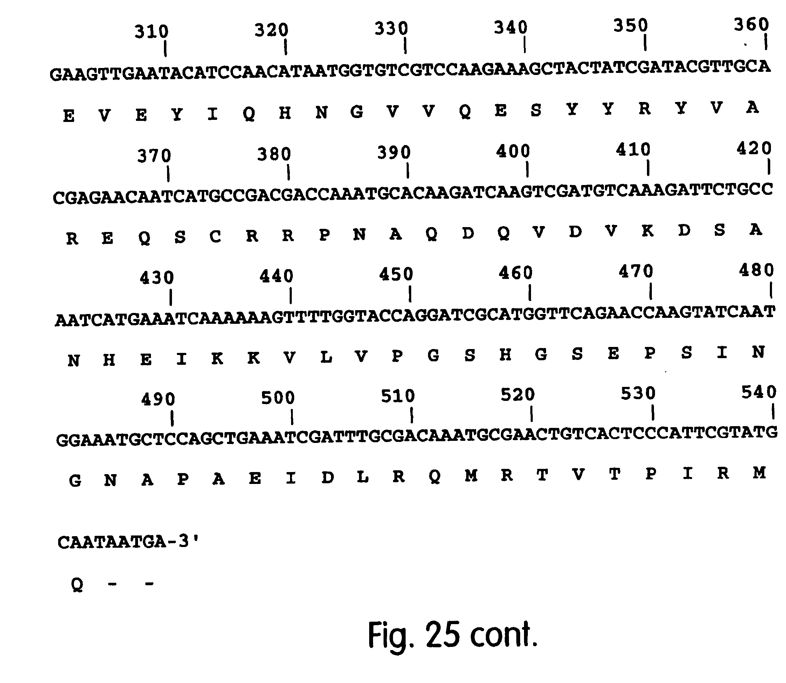

Fig. 25 shows the nucleotide and amino acid sequences of a selected peptide which comprises various regions derived from Der p I, Der p II and Der f I protein allergens.

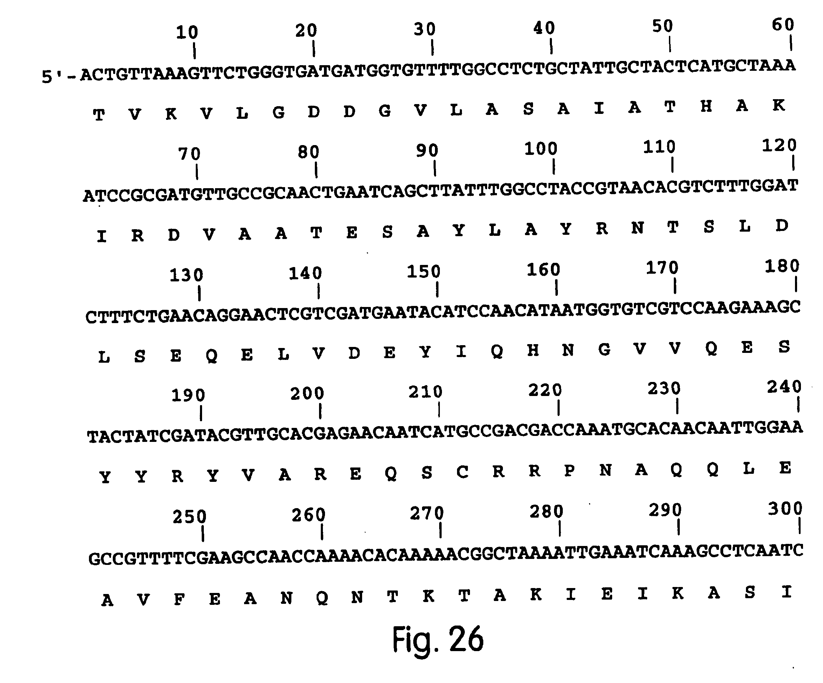

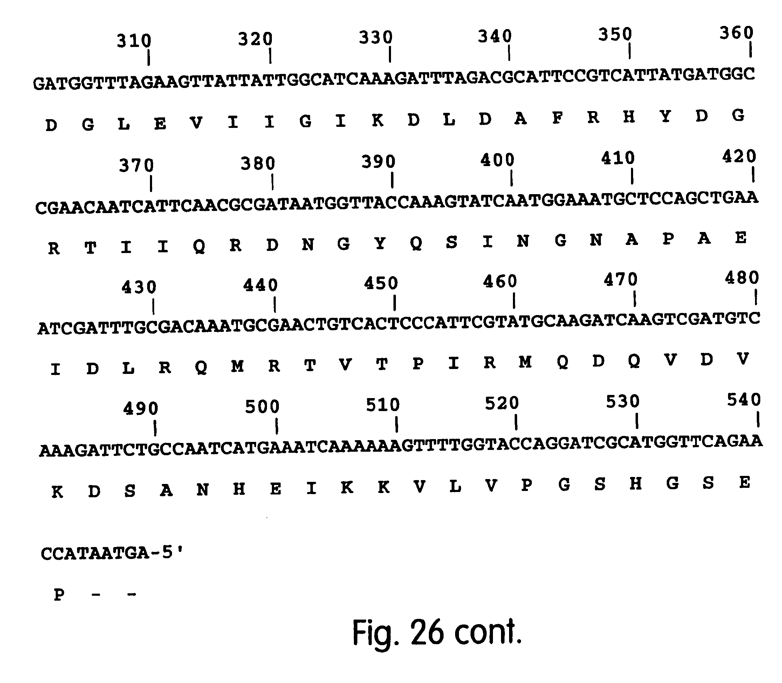

Fig. 26 shows the nucleotide and amino acid sequences of a selected peptide which comprises various regions derived from Der p I, Der p II and Der f I protein allergens.

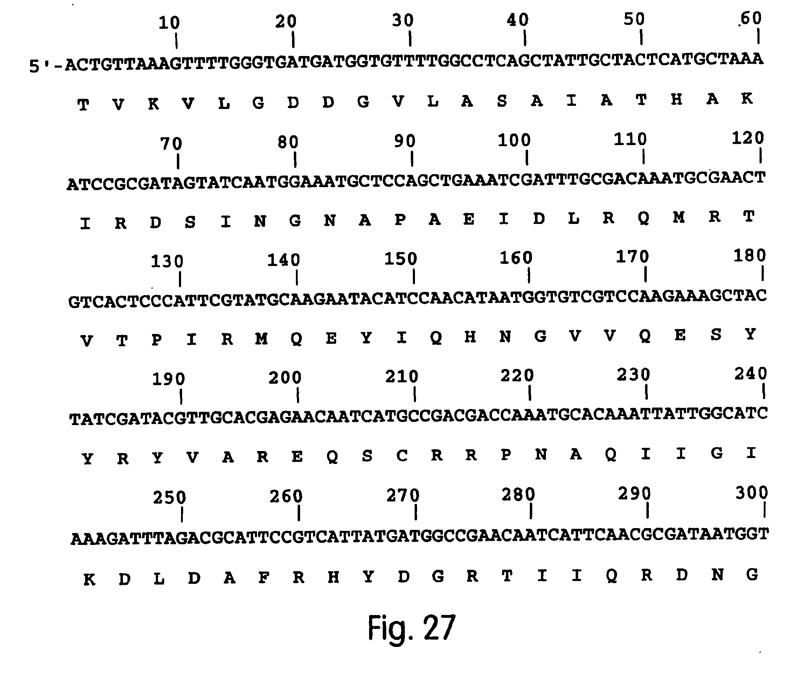

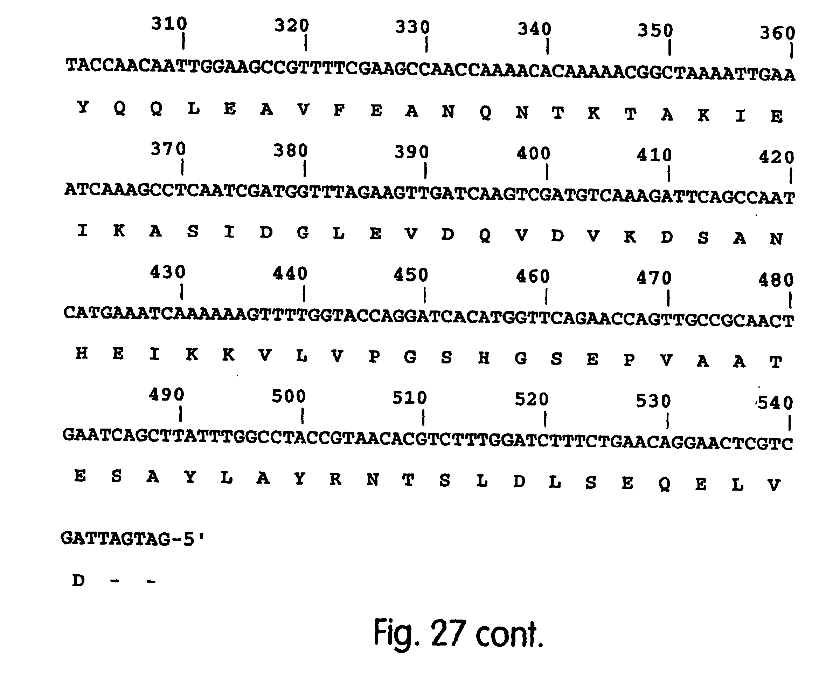

Fig. 27 shows the nucleotide and amino acid sequences of a selected peptide which comprises various regions derived from Der p I, Der p II and Der f I protein allergens.

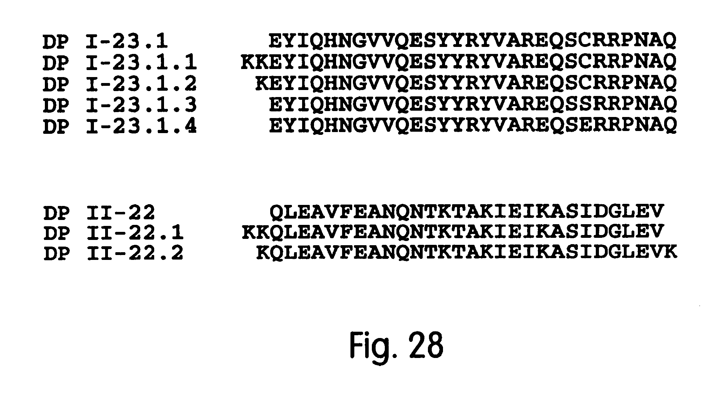

Fig. 28 shows the amino acid sequences of modified peptides in accordance with the invention.

**Detailed Description of the Invention**

The present invention provides isolated peptides derived from the Dermatophagoides group I or II protein allergens. As used herein, a peptide or fragment of a protein refers to an amino acid sequence having fewer amino acid residues than the entire amino acid sequence of the protein. Peptides of the invention include peptides derived from <u>Der p</u> I (SEQ ID NO: 1 and 2), <u>Der p</u> II (SEQ ID NO: 3 and 4), <u>Der f</u> I (SEQ ID NO: 5 and 6) and <u>Der f</u> II ( SEQ ID :7 (SEQ ID NO: 3 and 4), <u>Der f</u> I (SEQ ID NO: 5 and 6) and <u>Der f</u> II (SEQ ID NO: 7 and 8) which comprise at least one T cell epitope of the allergen.

Peptides comprising at least two regions, each region comprising at least one T cell epitope of a Dermatophagoides groups I or II protein allergen within the scope of the invention. Each region of such peptides is derived from the same or from different mite allergens. Isolated peptides or regions of isolated peptides, each comprising at least two T cell epitopes of a mite protein allergen are particularly desirable for increased therapeutic effectiveness. Peptides which are immunologically related (e.g., by antibody or T cell cross-reactivity) to peptides of the present invention are also within the scope of the invention. Peptides immunologically related by antibody cross-reactivity are bound by antibodies specific for a peptide of a Dermatophagoides groups I or II protein allergen. Peptides immunologically related by T cell cross-reactivity are capable of reacting with the same T cells as a peptide of the invention.

Isolated peptides of the invention can be produced by recombinant DNA techniques in a host cell transformed with a nucleic acid having a sequence encoding such peptide. The isolated peptides of the invention can also be produced by chemical synthesis. In certain limited situations, isolated peptides can be produced by chemical cleavage of the protein allergen. When a peptide is produced by recombinant techniques, host cells transformed with a nucleic acid having a sequence encoding the peptide or the functional equivalent of the nucleic acid sequence are cultured in a medium suitable for the cells and peptides can be purified from cell culture medium, host cells, or both using techniques known in the art for purifying peptides and proteins including ion-exchange chromatography, gel filtration chromatography, ultrafiltration; electrophoresis or immunopurification with antibodies specific for the peptide, the Dermatophagoides groups I or II protein allergen from which the peptide is derived, or a portion thereof. Isolated peptides of the invention are substantially free of cellular material or culture medium when produced by recombinant DNA techniques, or substantially free of chemical precursors or other chemicals when synthesized chemically.

The present invention provides expression vectors and host cells transformed to express the nucleic acid sequences of the invention. A nucleic acid sequence coding for a mite allergen, or at least one fragment thereof may be expressed in bacterial cells such as *E. coli,* insect cells (baculovirus), yeast, or mammalian cells such as Chinese hamster ovary cells (CHO). Suitable expression vectors, promoters, enhancers, and other expression control elements may be found in Sambrook et al. Molecular Cloning: A Laboratory Manual, second edition, Cold Spring Harbor Laboratory Press, Cold Spring Harbor, New York (1989). Other suitable expression vectors, promoters, enhancers, and other expression elements are known to those skilled in the art. Expression in mammalian, yeast or insect cells leads to partial or complete glycosylation of the recombinant material and formation of any inter- or intra-chain disulfide bonds. Suitable vectors for expression in yeast include YepSec 1 (Baldari et al. (1987) Embo J.6: 229-234); pMFa (Kurjan and Herskowitz (1982) Cell 30: 933-943); JRY88 (Schultz et al. (1987) Gene 54: 113-123) and pYES2 (Invitrogen Corporation, San Diego, CA). These vectors are freely available. Baculovirus and mammalian expression systems are also available. For example, a baculovirus system is commercially available (PharMingen, San Diego, CA) for expression in insect cells while the pMSG vector is commerically available (Pharmacia, Piscataway, NJ) for expression in mammalian cells.

For expression in *E. coli,* suitable expression vectors include, among others, pTRC (Amann et al. (1988) Gene 69: 301-315); pGEX (Amrad Corp., Melbourne, Australia); pMAL (N.E. Biolabs, Beverly, MA); pRIT5 (Pharmacia, Piscataway, NJ); pET-11d (Novagen, Madison, WI) Jameel et al., (1990) J. Virol. 64:3963-3966; and pSEM (Knapp et al. (1990) BioTechniques 8: 280-281). The use of pTRC, and pET-11d, for example, will lead to the expression of unfused protein. The use of pMAL, pRIT5 pSEM and pGEX will lead to the expression of allergen fused to maltose E binding protein (pMAL), protein A (pRIT5), truncated B-galactosidase (PSEM), or glutathione S-transferase (pGEX). When a mite protein allergen, fragment, or fragments thereof is expressed as a fusion protein, it is particularly advantageous to introduce an enzymatic cleavage site at the fusion junction between the carrier protein and a mite protein allergen or fragment thereof. A mite protein allergen or fragment thereof may then be recovered from the fusion protein through enzymatic cleavage at the enzymatic site and biochemical purification using conventional techniques for purification of proteins and peptides. Suitable enzymatic cleavage sites include those for blood clotting Factor Xa or thrombin for which the appropriate enzymes and protocols for cleavage are commercially available from, for example, Sigma Chemical Company, St. Louis, MO and N.E. Biolabs, Beverly, MA. The different vectors also have different promoter regions allowing constitutive or inducible expression with, for example, IPTG induction (PRTC, Amann et al., (1988) *supra;* pET-11d, Novagen, Madison, WI) or temperature induction (pRIT5, Pharmacia. Piscataway, NJ).

To obtain isolated peptides of the present invention, a mite allergen is divided into non-overlapping peptides of desired length or overlapping peptides of desired lengths as discussed in Example III which can be produced recombinantly, synthetically, or in certain limited situations, by chemical cleavage of the allergen. Peptides comprising at least one T cell epitope are capable of eliciting a T cell response, such as stimulation (i.e. proliferation or lymphokine secretion) and/or are capable of inducing T cell anergy (i.e., tolerization). To determine peptides comprising at least one T cell epitope, isolated peptides are tested by, for example, T cell biology techniques, to determine whether the peptides elicit a T cell response or induce T cell anergy. Those peptides found to elicit a T cell response or induce T cell anergy are defined as having T cell stimulating activity.

As discussed in the Examples, human T cell stimulating activity can be tested by culturing T cells obtained from an individual sensitive to a mite allergen, (i.e., an individual who has an IgE mediated immune response to a mite allergen) with a peptide derived from the allergen and determining whether proliferation of T cells occurs in response to the peptide as measured, e.g., by cellular uptake of tritiated thymidine. Stimulation indices for responses by T cells to peptides can be calculated as the maximum CPM in response to a peptide divided by the control CPM. A T cell stimulation index (S.I.) equal to or greater than two times the background level is considered "positive." Positive results are used to calculate the mean stimulation index for each peptide for the group of peptides tested. Preferred peptides of this invention comprise at least one T cell epitope and have a mean T cell stimulation index of greater than or equal to 2.0. A peptide having a T cell stimulation index of greater than or equal to 2.0 is considered useful as a therapeutic agent. Preferred peptides have a mean T cell stimulation index of at least 2.5, more preferably at least 3.5, even more preferably at least 4.0, and most preferably at least 5.0.

In addition, preferred peptides have a positivity index (P.I.) of at least about 100, more preferably at least 150, even more preferably at least about 200 and most preferably at least about 250. The positivity index for a peptide is determined by multiplying the mean T cell stimulation index by the percent of individuals, in a population of individuals sensitive to house dust mite (e.g., preferably at least 9 individuals, more preferably at least 16 individuals or more, more preferably at least 29 individuals or more, or even more preferably at least 30 individuals or more), who have T cells that respond to the peptide. Thus, the positivity index represents both the strength of a T cell response to a peptide (S.I.) and the frequency of a T cell response to a peptide in a population of individuals sensitive to house dust mite. For example, as shown in Fig. 5, peptide DP I-1 has a mean S.I. of 4.7 and 73% of positive responses in the group of individuals tested resulting in a positivity index of 343.1. Peptides of Der p I having a positivity index of at least about 150 and a mean T cell stimulation index of at least about 4 include: DP I-1 (SEQ ID NO: 9); DP I-21.1 (SEQ ID NO: 27); DP 1-21.2 (SEQ ID NO: 28); DP I-23.I (SEQ ID NO: 33): DP 1-23.2 (SEQ ID NO: 34); DP 1-25.2 (SEQ ID NO: 36); and DP I-26.1 (SEQ ID NO: 37).

In order to determine precise T cell epitopes by, for example, fine mapping techniques, a peptide having T cell stimulating activity and thus comprising at least one T cell epitope as determined by T cell biology techniques is modified by addition or deletion of amino acid residues at either the amino or carboxy terminus of the peptide and tested to determine a change in T cell reactivity to the modified peptide. If two or more peptides which share an area of overlap in the native protein sequence are found to have human T cell stimulating activity, as determined by T cell biology techniques, additional peptides can be produced comprising all or a portion of such peptides and these additional peptides can be tested by a similar procedure. Following this technique, peptides are selected and produced recombinantly or synthetically. Peptides are selected based on various factors, including the strength of the T cell response to the peptide (e.g., stimulation index), the frequency of the T cell response to the peptide in a population of individuals sensitive to house dust mite, and the potential cross-reactivity of the peptide with other allergens of the genus Dermatophagoides. The physical and chemical properties of these selected peptides (e.g., solubility, stability) are examined to determine whether the peptides are suitable for use in therapeutic compositions or whether the peptides require modification as described herein. The ability of the selected peptides or selected modified peptides to stimulate human T cells (e.g., induce proliferation, lymphokine secretion) is determined.

Additionally, preferred peptides of the invention do not bind immunoglobulin E (IgE) or bind IgE to a substantially lesser extent than the protein allergen from which the peptide is derived binds IgE. The major complications of standard immunotherapy are IgE-mediated responses such as anaphylaxis. Immunoglobulin E is a mediator of anaphylactic reactions which result from the binding and crosslinking of antigen to IgE on mast cells or basophils and the release of mediators (e.g., histamine, serotonin, eosinophil chemotacic factors). Thus, anaphylaxis in a substantial percentage of a population of individuals sensitive to house dust mite could be avoided by the use in immunotherapy of a peptide or peptides which do not bind IgE in a substantial percentage (e.g., at least about 75%) of a population of individuals sensitive to a house dust mite allergen, or if the peptide binds IgE, such binding does not result in the release of mediators from mast cells or basophils. The risk of anaphylaxis could be reduced by the use in immunotherapy of a peptide or peptides which have reduced IgE binding. Moreover, peptides which have minimal IgE stimulating activity are desirable for therapeutic effectiveness. Minimal IgE stimulating activity refers to IgE production that is less than the amount of IgE production and/or IL-4 production stimulated by the native protein allergen (e.g., Der p I).

A peptide of the invention, when administered to a house-dust mite-sensitive individual, is capable of modifying the allergic response of the individual to the allergen. Particularly, peptides of the invention comprising at least one T cell epitope of a mite allergen or at least two regions derived from a mite allergen, each comprising at least one T cell epitope, when administered to an individual sensitive to house dust mite are capable of modifying T cell response of the individual to the allergen. As used herein, modification of the allergic response of an individual to a house dust mite allergen can be defined as non-responsiveness or diminution in symptoms upon exposure to a mite allergen, as determined by standard clinical procedures (see e.g., Varney et al., British Medical Journal 302: 265-269 (1990)) including dimunition in mite protein allergen induced asthmatic symptoms. As referred to herein, a diminunition in symptoms includes any reduction in allergic response of an individual to the allergen after the individual has completed a treatment regimen with a peptide or protein of the invention. This dimunition may be subjective (i.e. the patient feels more comfortable in the presence of the allergen). Dimunition in symptoms can be deterined clinically as well, using standard skin tests as is known in the art.

As a result of the work described herein, peptides derived from mite allergens comprising at least one T cell epitope have been produced. T cell epitopes are believed to be involved in initiation and perpetuation of the immune responses to mite allergen(s) which are responsible for the clinical symptoms of mite allergy. These T cell epitopes are thought to trigger early events at the level of the T helper cell by binding to an appropriate HLA molecule on the surface of an antigen presenting cell and stimulating the relevant T cell subpopulation. These events lead to T cell proliferation, lymphokine secretion, local inflammatory reactions, the recruitment of additional immune cells to the site, and activation of the B cell cascade leading to production of antibodies. One isotype of these antibodies, IgE, is fundamentally important in the development of allergic symptoms and its production is influenced early in the cascade of events, at the level of the T helper cell, by the nature of the lymphokines secreted. A T cell epitope is the basic element or smallest unit of recognition by a T cell receptor where the epitope comprises amino acid residues essential to receptor recognition which may be contiguous and/or non-contiguous in the amino acid sequence of the protein. Amino acid sequences which mimic those of T cell epitopes and which modify the allergic response to protein allergens of the genus Dermatophagoides are within the scope of this invention.

Exposure of mite allergic patients to peptides of the present invention may tolerize or anergize appropriate T cell subpopulations such that they become unresponsive to mite allergen(s) and do not participate in mounting an immune response upon such exposure. In addition, administration of a peptide of the present invention may modify the lymphokine secretion profile as compared with exposure to the naturally-occurring mite protein allergen or portion thereof (e.g., result in a decrease of IL-4 and/or an increase in IL-2). Furthermore, exposure to a peptide of the invention may influence T cell subpopulations which normality participate in the response to mite allergen(s) such that these T cells are drawn away from the site(s) of normal exposure to the allergen (e.g., nasal mucosa, skin, and lung) towards the site(s) of therapeutic administration of the peptide. This redistribution of T cell subpopulations may ameliorate or reduce the ability of an individual's immune system to mount an immune response at the site of normal exposure to the mite allergen(s), resulting in a diminution in allergic symptoms.

Isolated peptides of the invention comprise at least one T cell epitope of a Dermatophagoides groups I or II protein allergen and accordingly, the peptide comprises at least approximately seven amino acid residues of the protein allergen. For purposes of therapeutic effectiveness, therapeutic compositions of the invention preferably comprise at least two T cell epitopes of a mite allergen. Accordingly, isolated peptides of the invention preferably comprise at least two T cell epitopes and accordingly, the peptide comprises at least approximately eight amino acid residues, and preferably fifteen amino acid residues. Additionally, therapeutic compositions of the invention preferably comprise a sufficient percentage of the T cell epitopes of the entire protein allergen such that a therapeutic regimen of administration of the composition to an individual sensitive to house dust mite, results in T cells of the individual being tolerized to the protein allergen. Synthetically produced peptides of the invention comprising up to approximately forty-five amino acid residues in length, and most preferably up to approximately thirty amino acid residues in length are particularly desirable as increases in length may result in difficulty in peptide synthesis as well as retention of an undesirable property (e.g., immunoglobulin binding or enzymatic activity) due to maintenance of conformational similarity between the peptide and the protein allergen from which it is derived. All of the peptides shown in Fig. 3 and Fig. 4 were found to . have human T cell stimulating activity.

Preferred peptides comprise the areas of major T cell reactivity within the Der p I, Der f I, Der p II and Der f II protein allergens, i.e., Region 1, Region 2, Region 3, Region 4, Region 5. Region 6a, Region 6b, Region 7, Region 8. Region 9 and Region 10. Each area is defined as follows: Region 1 comprises amino acid residues 1-28 of the Der p I and Der f I protein allergens; Region 2 comprises amino acid residues 36-68 of the Der p I and Der f I protein allergens; Region 3 comprises amino acid residues 74-109 of the Der p I and Der f I protein allergens; Region 4 comprises amino acid residues 118-139 of the Der p I and Der f I protein allergens; Region 5 comprises amino acid residues 141-166 of the Der p I and Der f I protein allergens; Region 6a comprises amino acid residues 161-185 of the Der p I and Der f I protein allergens; Region 6b comprises amino acid 173-201 of the Der p I and Der f I allergens; Region 7 comprises amino acid residues 1-26 of the Der p II and Der f II protein allergens; Region 8 comprises amino acid residues 33-67 of the Der p II and Der f II protein allergens; Region 9 comprises amino acid residues 79-104 of the Der p II and Der f II protein allergens and Region 10 comprises amino acid residues 107-129 of the Der p II and Der f II protein allergens.

Preferred peptides derived from the Der p I protein comprise the following peptides: DP I-1 (SEQ ID NO: 9); DP I-2 (SEQ ID NO: 10); DP I-3 (SEQ ID NO: 11); DP I-4 (SEQ ID NO: 12); DP I-11.1 (SEQ ID NO: 13); DP I-12.1 (SEQ ID NO: 14); DP I-5 (SEQ ID NO: 15); DP I-13- (SEQ ID NO: 17); DP I-14 (SEQ ID NO: 18); DP I-15 (SEQ ID NO: 19); DP I-6.1 (SEQ ID NO: 20); DP I-7.I (SEQ ID NO: 21); DP I-8 (SEQ ID NO: 22); DP I-9 (SEQ ID NO: 23); DP I-16 (SEQ ID NO: 24); DP I-10 (SEQ ID NO: 25); DP I-17 (SEQ ID NO: 26); DP I-21.1 (SEQ ID NO: 27); DP I-21.2 (SEQ ID NO: 28); DP I-22.1 (SEQ ID NO: 29); DP I-22.2 (SEQ ID NO: 30); DP I-22.3 (SEQ ID NO: 31); DP I-22.4 (SEQ ID NO: 32); DP I-23.1 (SEQ ID NO: 33); DP I-23.2 (SEQ ID NO: 34); DP I-25.1 (SEQ ID NO: 35); DP I-25.2 (SEQ ID NO: 36); DP I-26.1 (SEQ ID NO: 37); DP I-27.1 (SEQ ID NO: 38); DP I-28.1 (SEQ ID NO: 39); and DP I-28.2 (SEQ ID NO: 40), More preferably, peptides derived from the Der p I protein comprise the following peptides: DP I-212, DPI-22.2. DPI-23.1, DP 1-25.2. DPI-26.1, DPI-27.1 and DP I-28.1, and most preferably, peptides derived from the Der p I protein comprises DP I-21.2, DP I-23.1 and DP I-26.1.

Preferred peptides derived from the Der f I, the Der p II and the Der f II proteins include: DF I-1 (SEQ ID NO: 72); DF I-2.1 (SEQ ID NO: 73); DF 1-3 (SEQ ID NO: 74); DF 1-4 (SEQ ID NO: 75); DF I-11 (SEQ ID NO: 76); DF I-12 (SEQ ID NO: 77); DF I-5 (SEQ ID NO: 78); DF I-13 (SEQ ID NO: 79); DF I-14 (SEQ ID NO: 80); DF 1-15 (SEQ ID NO: 81); DF 1-6 (SEQ ID NO: 82); DF 1-7 (SEQ ID NO: 83); DF I-8.1 (SEQ ID NO: 84); DF I-8 (SEQ ID NO: 85); DF I-9 (SEQ ID NO: 86); DF I-16 (SEQ ID NO: 87); DF I-10 (SEQ ID NO: 88); DF I-17 (SEQ ID NO: 89); DF I-21.1 (SEQ ID NO: 90); DF 1-21.2 (SEQ ID NO: 91); DF I-22.1 (SEQ ID NO: 92); DF 1-22.2 (SEQ ID NO: 93); DF 1-22.4 (SEQ ID NO: 94); DF I-23.1 (SEQ ID NO: 95); DF 1-23.2 (SEQ ID NO: 96); DF I-25.1 (SEQ ID NO: 97); DF 1-25.2 (SEQ ID NO: 98); DF I-26.1 (SEQ ID NO: 99); DF I-27.1 (SEQ ID NO: 100); DF I-28.1 (SEQ ID NO: 101); DF I-28.2 (SEQ ID NO: 102); DP II-20 (SEQ ID NO: 50); DP II-20.1 (SEQ ID NO: 51); DP II-20.2 (SEQ ID NO: 52); DP II-20.3 (SEQ ID NO: 53); DP II-20.4 (SEQ ID NO: 54); DP II-20.5 (SEQ ID NO: 55); DP II 20.6 (SEQ ID NO: 56); DP II-1 (SEQ ID NO: 41); DP II-2 (SEQ ID NO: 42); DP II-3.1 (SEQ ID NO: 43); DP II-4 (SEQ ID NO: 44); DP II-5 (SEQ ID NO: 45); DP II-6 (SEQ ID NO: 46); DP II-7 (SEQ ID NO: 47); DP II-8 (SEQ ID NO: 48); DP II-9 (SEQ ID NO: 49); DP II-1.1 (SEQ ID NO: 57); DP II-1.2 (SEQ ID NO: 58); DP II-2.1 (SEQ ID NO: 59); DP II-2.2 (SEQ ID NO: 60); DP II-2.3 (SEQ ID NO: 61); DP II-21 (SEQ ID NO: 62); DP II-22 (SEQ ID NO: 63); DP II-26 (SEQ ID NO: 64); DP II-26.1 (SEQ ID NO: 65); DP II-23 (SEQ ID NO: 66); DP II-23.1 (SEQ ID NO: 67); DP II-24 (SEQ ID NO: 68); DP II-25 (SEQ ID NO: 69); DP II-25.1 (SEQ ID NO: 70); DP II-25.2 (SEQ ID NO: 71); DF II-1 (SEQ ID NO: 103); DF II-2 (SEQ ID NO: 104); DF II-13.1 (SEQ ID NO: 105); DF II-3.1 (SEQ ID NO: 106); DF II-4.5 (SEQ ID NO: 107); DF II-4.3 (SEQ ID NO: 108); DF II-15 (SEQ ID NO: 109); DF II-16 (SEQ ID NO: 110); DF II-17 (SEQ ID NO: 111); DF II-18 (SEQ ID NO: 112); DF II-19 (SEQ ID NO: 113); DF II-19.1 (SEQ ID NO: 114); DF II-21 (SEQ ID NO: 115); and DF II-22 (SEQ ID NO: 116) comprising at least one T cell epitope. More preferably, peptides derived from the Der p II and Der f I proteins comprise of the following peptides: DF I-22.2, DP II-20.6, DP II-22, DP II-24 and DP II-25.2.

Another embodiment of the present invention provides peptides comprising at least two regions, each region comprising at least one T cell epitope of a Dermatophagoides group I or II protein allergen and accordingly, each region comprises at least approximately seven amino acid residues). These peptides comprising at least two regions can comprise as many amino acid residues as desired and preferably comprise at least about 14, even more preferably about 30, and most preferably at least about 40 amino acid residues: of a mite allergen. Each region of such peptide preferably comprises up to 45 amino acid residues in length, more preferably up to 40 residues in length and most preferably up to 30 amino acid residues in length as increases in length of a region may result in difficulty in peptide synthesis as well as retention of an undesirable property (e.g., immunoglobulin binding or enzymatic activity) due to maintenance of conformational similarity between the peptide and the protein allergen from which it is derived. If desired, the amino acid sequences of the regions can be produced and joined by a linker to increase sensitivity to processing by antigen-presenting cells. Such linker can be any non-epitope amino acid sequence or other appropriate linking or joining agent. To obtain preferred peptides comprising at least two regions, each comprising at least one T cell epitope, the regions are arranged in a configuration different from a naturally-occurring configuration of the regions in the allergen or a combination of different mite protein allergens. For example, the regions containing T cell epitope(s) can be arranged in a noncontiguous configuration and can preferably be derived from the same protein allergen or a combination of protein allergens. Noncontiguous is defined as an arrangement of regions containing T cell epitope(s) which is different than that of an amino acid sequence present in the protein allergen from which the regions are derived. Furthermore, the noncontiguous regions containing T cell epitopes can be arranged in a nonsequential order (e.g., in an order different from the order of the amino acids of the native protein allergen from which the region containing T cell epitope(s) are derived in which amino acids are arranged from an amino terminus to a carboxy terminus).

The individual peptide regions can be produced and tested to determine which regions bind immunoglobulin E specific for a mite allergen and which of such regions would cause the release of mediators (e.g., histamine) from mast cells or basophils. Those peptide regions found to bind immunoglobulin E and cause the release of mediators from mast cells or basophils in greater than approximately 10-15% of the allergic sera tested are preferably not included in the peptide regions arranged to form peptides of the invention.

Preferred peptides of the invention comprise two or more regions derived from the same or from different mite allergens (e.g. Der p I, Der p II, Der f I and DEr f II). For example, one region can be derived from Der p I and one region can be derived from Der p II; one region can be derived from Der p I and one region can be derived from Der f I; one region can be derived from <u>Der p</u> II and one region can be derived from <u>Der f</u> I; one region can be derived from <u>Der p</u> II and one region can be derived from <u>Der f</u> II; one region can be derived from <u>Der p</u> I and one region can be derived from <u>Der f</u> II; and one region can be derived from <u>Der f</u> I and one region can be derived from <u>Der f</u> II. In addition, the regions can be derived from the same protein allergen, e.g., <u>Der p</u> I and <u>Der p</u> I, etc.

Regions of a peptide of the invention preferably comprise the above discussed preferred areas of major T cell reactivity within each mite allergen (i.e., Regions 1-6a-6b of <u>Der p</u> I and <u>Der f</u> I and Regions 7-10 of <u>Der p</u> II and <u>Der f</u> II). For example, one region can comprise Region 1 (amino acid residues 1-28 of <u>Der p</u> I or <u>Der f</u> I) and one region can comprise Region 2 (amino acid residues 36-68 of <u>Der p</u> I or <u>Der f</u> I). Peptides of the invention can comprise two or more of these Regions (i.e., Regions 1-10) and preferred resulting peptides do not bind IgE and cause the release of mediators from mast cells or basophils. Preferred peptides derived from <u>Der p</u> I and <u>Der f</u> I comprise Region 1, Region 2, Region 3 and optionally Region 4. Preferred peptides derived from <u>Der p</u> II and <u>Der f</u> II comprise Region 7 and Region 8 and, Region 10. Further, if one of these Regions is found to bind IgE and cause the release of mediators from mast cells or basophils, then it is preferred that the peptide not comprise such Regions but rather comprises various Regions derived from such Regions which do not bind IgE or cause release of mediators from mast cells or basophils.

Examples of preferred regions include following amino acid sequences: DP I 21.1 (SEQ ID NO: 27); DP I-21.2 (SEQ ID NO: 28); DP I-22.1 (SEQ ID NO: 29); DP I-22.2 (SEQ ID NO: 30); DP I-22.3 (SEQ ID NO: 31); DP I-22.4 (SEQ ID NO: 32); DP I-23.1 (SEQ ID NO: 33); DP 1-23.2 (SEQ ID NO: 34); DP I-25.1 (SEQ ID NO: 35); DP I-25.2 (SEQ ID NO: 36); DP I-26.1 (SEQ ID NO: 37); DP I-27.1 (SEQ ID NO: 38); DP I-28.1 (SEQ ID NO: 39); DP I-28.2 (SEQ ID NO: 40); DP I- 1 (SEQ ID NO: 9); DF I- 1 (SEQ ID NO: 72); DF I-21.1 (SEQ ID NO: 90); DF I-21.2 (SEQ ID NO: 91); DF I-22.1 (SEQ ID NO: 92); DF I-22.2 (SEQ ID NO: 93); DF I-22.4 (SEQ ID NO: 94); DF I-23.1 (SEQ ID NO: 95); DF I-23.2 (SEQ ID NO: 96); DF I-25.1 (SEQ ID NO: 97); DF I-25.2 (SEQ ID NO: 98); DF I-26.1 (SEQ ID NO: 99); DF I-27.1 (SEQ ID NO: 100); DF I-28.1 (SEQ ID NO: 101); DF I-28.2 (SEQ ID NO: 102); DP II-20 (SEQ ID NO: 50); DP II-20.1 (SEQ ID NO: 51); DP II-20.2 (SEQ ID NO: 52); DP II-20.3 (SEQ ID NO: 53); DP II-20.4 (SEQ ID NO: 54); DP II-20.5 (SEQ ID NO: 55); DP II 20.6 (SEQ ID NO: 56); DP II-1 (SEQ ID NO: 41); DP II-1.1 (SEQ ID NO: 57); DP II-1.2 (SEQ ID NO: 58); DP II-2.1 (SEQ ID NO: 59); DP II-2.2 (SEQ ID NO: 60); DP II-2.3 (SEQ ID NO: 61); DP II-21 (SEQ ID NO: 62); DP II-22 (SEQ ID NO: 63); DP II-26 (SEQ ID NO: 64); DP II-26.1 (SEQ ID NO: 65); DP II-23 (SEQ ID NO: 66); DP II-23.1 (SEQ ID NO: 67); DP II-24 (SEQ ID NO: 68); DP II-25 (SEQ ID NO: 69); DP II-25.1 (SEQ ID NO: 70); DP II-25.2 (SEQ ID NO: 71); DF II-1 (SEQ ID NO: 103) DF II-2 (SEQ ID NO: 104); DF II-13.1 (SEQ ID NO: 105); DF II-3.1 (SEQ ID NO: 106); DF II-4.5 (SEQ ID NO: 107); DF II-4.3 (SEQ ID NO: 108); DF II-15 (SEQ ID NO: 109); DF II-16 (SEQ ID NO: 110); DF II-17 (SEQ ID NO: 111): DF II-18 (SEQ ID NO: 112); DF II-19 (SEQ ID NO: 113); DF II-19.1 (SEQ ID NO: 114); DF II-21 (SEQ ID NO: 115); and DF II-22 (SEQ ID NO: 116), the amino acid sequences of such regions being shown in Fig. 3 and Fig. 4, comprising at least one T cell epitopes.

Preferred peptides comprise various combinations of two or more regions, each region comprising the above-discussed preferred areas of major T cell reactivity. Preferred peptides comprise a combination of two or more regions (each region having an amino acid sequence as shown in Fig. 3 and Fig. 4) including: DP I-22.1 (SEQ ID NO: 29) and DP 1-25.1 (SEQ ID NO: 35); DP I-21.1 (SEQ ID NO: 27) and DP 1-25.2 (SEQ ID NO: 36); DP I-22.1 (SEQ ID NO: 29) and DP I-1 (SEQ ID NO: 9); DP I-21.1 (SEQ ID NO: 27), DP I-22.1 (SEQ ID NO: 29), and DP 1-25.2 (SEQ ID NO: 36); DP I-21.2 (SEQ ID NO: 28), DP I-22.1 (SEQ ID NO: 29), and DP I-23.1 (SEQ ID NO: 39); DP I-1 (SEQ ID NO: 9), DP I-22.1 (SEQ ID NO: 29), and DP I-23.1 (SEQ ID NO: 33); DP I-1 (SEQ ID NO: 9), DP I-22.1 (SEQ ID NO: 29), and DP 1-25.2 (SEQ ID NO: 36); DP I-21.1 (SEQ ID NO: 27), DP I-22.1 (SEQ ID NO: 29), DP I-23.1 (SEQ ID NO: 33), and DP 1-25.2 (SEQ ID NO: 36); DP 1-21.2 (SEQ ID NO: 28), DP I-22.1 (SEQ ID NO: 29), and DP 1-25.2 (SEQ ID NO: 36); DP I-21.2 (SEQ ID NO: 27), DP I-22.1 (SEQ ID NO: 29), DP 1-25.2 (SEQ ID NO: 36), and DP I-26.1 (SEQ ID NO: 37); DF I-21.2 (SEQ ID NO: 91) and DF I-22.1 (SEQ ID NO: 92); DF I-21.1 (SEQ ID NO: 90), DF I-22.1 (SEQ ID NO: 92), and DF I-25.1 (SEQ ID NO: 97); DF I-21.2 (SEQ ID NO: 91), DF I-22.1 (SEQ ID NO: 92), and DF I-25.1 (SEQ ID NO: 97); DF I-1 (SEQ ID NO: 72) and DF I-22.1 (SEQ ID NO: 92); DF I-1 (SEQ ID NO: 72), DF I-22.1 (SEQ ID NO: 92), and DF I-25.1 (SEQ ID NO: 97); DF I-22.1 (SEQ ID NO: 29), and DF I-25.1 (SEQ ID NO: 35); DF I-21.1 (SEQ ID NO: 90), DF I-22.1 (SEQ ID NO: 92), and DF I-23.1 (SEQ ID NO: 95); DP I-21.1 (SEQ ID NO: 27), and DF I-22.1 (SEQ ID NO: 92); DP I-1 (SEQ ID NO: 9), DP I-23.1 (SEQ ID NO: 33), DP I-25.1 (SEQ ID NO: 35), and DF I-1 (SEQ ID NO: 72); t) DP I-1 (SEQ ID NO: 9), DP I-25.1 (SEQ ID NO: 35), DP I-23.1 (SEQ ID NO: 33), and DF I-21.2 (SEQ ID NO: 91); DP I-1 (SEQ ID NO: 9), DP I-25.1 (SEQ ID NO: 35), DP I-23.1 (SEQ ID NO: 33), and DF I-21.1 (SEQ ID NO: 90); DP II-22 (SEQ ID NO: 63), and DP II-25.2 (SEQ ID NO: 71); DP II-22 (SEQ ID NO: 63), DP II-25.2 (SEQ ID NO: 71), and DP I-21.1 (SEQ ID NO: 27) and DP I-22.1 (SEQ ID NO: 29); DP II-22 (SEQ ID NO: 63), DP II-25.2 (SEQ ID NO: 71), DP II-20.6 (SEQ ID NO: 56), DP I-22.1 (SEQ ID NO: 29), DP I-21.1 (SEQ ID NO: 27), and DP I-23.1 (SEQ ID NO: 33); DP II-22 (SEQ ID NO: 63), DP II-25.2 (SEQ ID NO: 71), DP II-20.6 (SEQ ID NO: 56), DP I-21.1 (SEQ ID NO: 27), DP I-22.1 (SEQ ID NO: 29), and DP I-25.2 (SEQ ID NO: 36); DP II-22 (SEQ ID NO: 63), DP II-25.2 (SEQ ID NO: 71), DP I-21.1 (SEQ ID NO: 27), DP I-22.1 (SEQ ID NO: 29), and DP 1-25.2 (SEQ ID NO: 36); DP II-22 (SEQ ID NO: 63), DP II-25.2 (SEQ ID NO: 71), DP I-21.1 (SEQ ID NO: 27), DP I-22.1 (SEQ ID NO: 29), and DP I-23.1 (SEQ ID NO: 33); DP II-22 (SEQ ID NO: 63), DP II-25.2 (SEQ ID NO: 71), DP I-1 (SEQ ID NO: 9), and DP I-22.1 (SEQ ID NO: 29); DF II-4.5 (SEQ ID NO:107) and DF II-2 (SEQ ID NO: 104); DF II-4.5 (SEQ ID NO: 107) and DF II-19.1 (SEQ ID NO: 114); DF II-4.5 (SEQ ID NO: 107), DF II-2 (SEQ ID NO: 104), and DF II-19.1 (SEQ ID NO: 114); DF II 4.5 (SEQ ID NO: 107), DF II-2 (SEQ ID NO: 104), and DF II-9 (SEQ ID NO: 86); DF II-4.5 (SEQ ID NO:107); and DF I-21.1 (SEQ ID NO: 90); DF II-4.5 (SEQ ID NO: 107), DP II-22 (SEQ ID NO: 63), and DP II-25.2 (SEQ ID NO: 71); and i') DF II-4.5 (SEQ ID NO: 107), DF II-2 (SEQ ID NO: 104), and DP II-22 (SEQ ID NO: 63).

Additional preferred peptides comprising a combination of two or more Regions and including the following combinations: DP I-21.2, DP I-23.1, DP I-26.1, DP II-20.6, DP II-22, DP II-25.2 and DF II-22.2; DP I-21.2. DF II-22.2; DP I-21.2, DF I-22.2, DP I-23.1, DP I-25.2, DP I-26.1, DP I-27.1, DP II-20.6, DP II-22, DP II-24, and DP II-25.2; DP I-23.1, DP I-21.2, DP I-22. DF I-22.2, DP II-20.6, and DP II-25.2; DP I-23.1, DP I-21.2, DF I-22.2. DP II-20.6, and DP II-25.2; DP I-23.1, DF I-22 and DP II-20.6; DP I-26.1, DF I-22.2 and DP II-25.2; DP I-21.2, DF I-22.2 and DP II-22; and DP I-21.2 and DP II-22.

The most preferred peptides comprise a combination of two or more regions (each region having an amino acid sequence as shown in Fig. 3 and Fig. 4) derived from mite allergens <u>Der p</u> I, <u>Der p</u> II, and <u>Der f</u> I each of said preferred peptides having the following specific sequential arrangement of amino acid sequences as shown in Figs. 25-27: DPI-26.1, DPII-25.2, DFI-22, DPII-20.6 and DPI-21.2 respectively; DPII-25.2, DFI-22.2, DPI-23.1, DPII-22, DPI-21.2 and DPII-20.6 respectively; and DPII-25.2, DPI-21.2, DPI-23:1, DPI-26.1, DPII-22, DPII-20.6 and DFI-22.2 respectively. The nucleic acid and amino acid sequences of the above peptides are shown in Figs. 25, 26 and 27 respectively.

Peptides of Dermatophagoides group I or II protein allergens within the scope of the invention can be used in methods of treating and preventing allergic reactions to mite allergens. Thus, one aspect of the present invention provides therapeutic compositions comprising a peptide of <u>Der p</u> I, <u>Der p</u> II, <u>Der f</u> I, or <u>Der f</u> II including at least one T cell epitope, or preferably at least two T cell epitopes, and a pharmaceutically acceptable carrier or diluent. In another aspect, the therapeutic composition comprises a pharmaceutically acceptable carrier or diluent and a peptide comprising at least two regions, each region comprising at least one T cell epitope of a mite allergen and derived from the same or from different mite protein allergens.

Preferred therapeutic compositions comprise at least one peptide of at least one Dermatophagoides group I or II protein allergen. The composition comprises at least one T cell epitope of at least one protein allergen such that a therapeutic regimen of administration of the composition to an individual sensitive to a house dust mite allergen resulting in T cells of the individual being tolerized to the protein allergen. Preferably, the composition comprises at least one T cell epitope of one or more protein allergens such that at least about 40%, and more preferably at least about 60% of the T cell reactivity of each protein is included in the composition. Such compositions can be administered to an individual to treat or prevent sensitivity to house dust mite or to an allergen which is immunologically cross-reactive with house dust mite.

Administration of the therapeutic compositions of the present invention to desensitive an individual can be carried out using known techniques. For example, a peptide derived from a mite allergen comprising at least one T cell epitope can be administered in combination with an appropriate diluent, a carrier, and/or an adjuvant. To induce T cell anergy in an individual, the terapeutic composition is preferably administered in non-immunogenic form, e.g. it does not contain adjuvant Pharmaceutically acceptable diluents include saline and aqueous buffer solutions. Pharmaceutically acceptable carriers include polyethylene glycol (Wie et al. International Archives of Allergy and Applied Immunology 64: 84-99 (1981)) and liposomes (Strejan et al., Journal of Neuroimmunology 7: 27 (1984)). Such compositions will generally be administered by injection, (subcutaneous, intravenous, etc.) oral administration, (e.g. as in the form of a capsule), inhalation, transdermal application or rectal administration. The therapeutic compositions of the invention are administered to individuals sensitive to house dust mite at dosages and for lengths of time effective to reduce sensitivity (i.e., reduce the allergic response) of the individual to a house dust mite allergen. A therapeutically effective amount of one or more of the same or of different therapeutic compositions can be administered simultaneously or sequentially to an individual sensitive to house dust mite. Effective amounts of the therapeutic compositions will vary according to factors such as the degree of sensitivity of the individual to house dust mites, the age, sex, and weight of the individual, and the ability of the peptide to stimulate a T cell response in the individual.

In yet another aspect of the present invention, a composition is provided comprising at least two peptides (e.g., a physical mixture of at least two peptides), each comprising at least one T cell epitope of a protein allergen of the genus <u>Dermatophagoides</u>. The peptides are derived from the same or from different mite allergens. Such compositions can be administered in the form of a therapeutic composition with a pharmaceutically acceptable carrier of diluent. A therapeutically effective amount of one or more of such compositions can be administered simultaneously or sequentially to an individual sensitive to house dust mite.

Preferred compositions and preferred combinations of peptides which can be administered simultaneously or sequentially (comprising peptides having amino acid sequences shown in Fig. 3 and Fig. 4) include the following combinations: DP I-22.1 (SEQ ID NO: 29) and DP I-25.1 (SEQ ID NO: 35); DP I-21.1 (SEQ ID NO: 27) and DP I-25.2 (SEQ ID NO: 36); DP I-22.1 (SEQ ID NO: 29) and DP I-1 I (SEQ ID NO: 9); DP 1-21.1 (SEQ ID NO: 27), DP I-22.1 (SEQ ID NO: 29), and DP 1-25.2 (SEQ ID NO: 36); DP I-21.2 (SEQ ID NO: 28), DP I-22.1 (SEQ ID NO: 29), and DP I-23.1 (SEQ ID NO: 39); DP I-1 (SEQ ID NO: 9), DP I-22.1 (SEQ ID NO: 29), and DP I-23.1 (SEQ ID NO: 33); DP I-1 (SEQ ID NO: 9), DP I-22.1 (SEQ ID NO: 29), and DP 1-25.2 (SEQ ID NO: 36); DP I-21.1 (SEQ ID NO: 27), DP I-22.1 (SEQ ID NO: 29), DP I-23.1 (SEQ ID NO: 33), and DP 1-25.2 (SEQ ID NO: 36); DP I-21.2 (SEQ ID NO: 28), DP I-22.1 (SEQ ID NO: 29), and DP 1-25.2 (SEQ ID NO: 36); DP I-21.2 (SEQ ID NO: 27), DP I-22.1 (SEQ ID NO: 29), DP 1-25.2 (SEQ ID NO: 36), and DP I-26.1 (SEQ ID NO: 37); DF I-21.2 (SEQ ID NO: 91) and DF I-22.1 (SEQ ID NO: 92); DF I-21.1 (SEQ ID NO: 90), DF I-22.1 (SEQ ID NO: 92), and DF I-25.1 (SEQ ID NO: 97); DF I-21.2 (SEQ ID NO: 91), DF I-22.1 (SEQ ID NO: 92), and DF I-25.1 (SEQ ID NO: 97); DF I-1 (SEQ ID NO: 72) and DF I-22.1 (SEQ ID NO: 92); DF I-1 (SEQ ID NO: 72), DF I-22.1 (SEQ ID NO: 92), and DF I-25.1 (SEQ ID NO: 97); DF I-22.1 (SEQ ID NO: 29), and DF 1-25.1 (SEQ ID NO: 35); DF I-2I.1 (SEQ ID NO: 90), DF I-22.1 (SEQ ID NO: 92), and DF I-23.1 (SEQ ID NO: 95); DP I-21.1 (SEQ ID NO: 27), and DF I-22.1 (SEQ ID NO: 92); DP I-1 (SEQ ID NO: 9), DP I-23.1 (SEQ ID NO: 33), DP I-25.1 (SEQ ID NO: 35), and DF I-1 (SEQ ID NO: 72); DP I-1 (SEQ ID NO: 9), DP I-25.1 (SEQ ID NO: 35), DP I-23.1 (SEQ ID NO: 33), and DF I-21.2 (SEQ ID NO: 91); DP I-1 (SEQ ID NO: 9), DP I-25.1 (SEQ ID NO: 35), DP I-23.1 (SEQ ID NO: 33), and DF I-21.1 (SEQ ID NO: 90); DP II-22 (SEQ ID NO: 63), and DP II-25.2 (SEQ ID NO: 71); DP II.22 (SEQ ID NO: 63), DP II.25.2 (SEQ ID NO: 71), and DP I-21.1 (SEQ ID NO: 27) and DP I-22.1 (SEQ ID NO: 29); DP II-22 (SEQ ID NO: 63), DP II-25.2 (SEQ ID NO: 71), DP II-20.6 (SEQ ID NO: 56), DP I-22.1 (SEQ ID NO: 29), DP I-21.1 (SEQ ID NO: 27), and DP I-23.1 (SEQ ID NO: 33); DP II-22 (SEQ ID NO: 63), DP II-25.2 (SEQ ID NO: 71). DP 11-20.6 (SEQ ID NO: 56), DP I-21.1 (SEQ ID NO: 27), DP I-22.1 (SEQ ID NO: 29), and DP 1-25.2 (SEQ ID NO: 36); DP II-22 (SEQ ID NO: 63), DP II-25.2 (SEQ ID NO: 71), DP I-21.1 (SEQ ID NO: 27), DP I-22.1 (SEQ ID NO: 29), and DP I-25.2 (SEQ ID NO: 36); DP II-22 (SEQ ID NO: 63), DP II-25.2 (SEQ ID NO: 71), DP 1-21.1 (SEQ ID NO: 27), DP 1-22.1 (SEQ ID NO: 29), and DP I-23.1 (SEQ ID NO: 33); DP II-22 (SEQ ID NO: 63), DP II-25.2 (SEQ LID NO: 71), DP I-1 (SEQ ID NO: 9), and DP I-22.1 (SEQ ID NO: 29); DF II-4.5 (SEQ ID NO: 107) and DF II-2 (SEQ ID NO: 104); DF II-4.5 (SEQ ID NO: 107) and DF U-19.1 (SEQ ID NO: 114); DF II-4.5 (SEQ ID NO: 107), DF II-2 (SEQ ID NO: 104), and DF II-19.1 (SEQ ID NO: 114): DF II-4.5 (SEQ ID NO: 107), DF II.2 (SEQ ID NO: 104), and DF II-9 (SEQ ID NO: 86); DF II-4.5 (SEQ ID NO: 107); and DF I-21.1 (SEQ ID NO: 90); DF II-4.5 (SEQ ID NO: 107), DP II-22 (SEQ ID NO: 63), and DP II-25.2 (SEQ ID NO: 71); and DF II-4.5 (SEQ ID NO: 107), DF II-2 (SEQ ID NO: 104), and DP II-22 (SEQ ID NO: 63); and DP I-26.1, DP II-25.2, DF I-22, DP II-20.6 and DP I-21.2.

The present invention also provides methods of detecting sensitivity in individuals to house dust mite allergens comprising combining a blood sample obtained from the individual with a peptide of the present invention, under conditions appropriate for binding of blood components with the peptide and determining the extent to which such binding occurs. The extent to which binding occurs is determined by assessing T cell function, T cell proliferation or a combination thereof.

The present invention also provides nucleic acids having sequences encoding peptides of the invention. Nucleic acid sequences used in any embodiment of this invention can be cDNA as described herein, or alternatively, can be any oligodeoxynucleotide sequence having a sequence represented herein, or their functional equivalents. Such oligodeoxynucleotide sequences can be produced chemically or mechanically, using known techniques. A functional equivalent of an oligonucleotide sequence is one which is 1) a sequence capable of hybridizing to a complementary oligonucleotide to which the sequence (or corresponding sequence portions) of SEQ ID NO:1, SEQ IF7 NO: 3, SEQ ID NO: 5 and SEQ ID NO: 7, or fragments thereof hybridizes, or 2) the sequence (or corresponding sequence portion) complementary to SEQ ID NO: 1. SEQ ID NO: 3, SEQ ID NO: 5 and SEQ ID NO: 7 and/or 3 a sequence which encodes a product (e.g., a polypeptide or peptide) having the same functional characteristics of the product encoded by the sequence (or corresponding sequence portion) of SEQ ID NO: 1. SEQ ID NO: 3, SEQ ID NO: 5 and SEQ ID NO: 7. Whether a functional equivalent must meet one or more criteria will depend on its use (e.g., if it is to be used only as an oligoprobe, it need meet only the first or second criteria and if it is to be used to produce a peptide of the present invention, it need only meet the third criterion).

This invention is further illustrated by the following non-limiting examples.

### Example I Native Mite Allergen Purification

What follows is a description of the work done to purify the group I and group II allergens of the house dust mites _Dermatophagoides_ _pteronyssinus_ and _Dermatophagoides_ _farinae_ in their native form as primary antigens for human T-cell epitope mapping.

All four protein allergens, _Der f_ I, _Der p_ I, _Der f_ II, and _Der p_ II, were immunoaffinity purified from spent mite culture media obtained from either Commonwealth Sera Laboratories Melbourne, Australia or Dr. Larry G. Arlian at Wright State University Dayton, Ohio. A 10% (wt./vol.) aqueous extract of dessicated spent mite culture media was prepared in PBS (phosphate Buffered Saline) with stirring overnight at 4°C. Insoluble material was removed by centrifugation at 10,000 X g for 1 hour at 4°C. The supernatant was then filtered on a vacuum manifold through Whatman #1 paper, and re-centrifuged at 15,000 X g for 1 hour at 4°C. A final filtration was carried out through a 0.45 m cellulose acetate filter.

Monoclonal antibodies 4C1 and 6D6 (University of Virginia, NC) were used for immunoaffinity purification of group I or group II mite allergens respectively (Chapman et al., J. Allergy Clin. Immunol. 80: 1479-1484 (1987); Heymann et al., J. Allergy Clin. Immunol. 83: 1055-1067 (1989)). The 4C1 monoclonal antibody reacts with an epitope shared by both the Der I and Der p I proteins; similarly, the 6D6 monoclonal antibody reacts with both the Der f II and Der p II proteins.

For each monoclonal antibody, ascites fluid was cut in 50% ammonium sulfate and the antibodies coupled to CNBr-activated Sepharose 4B (Pharmacia) in 100 mM $NaHCO_3$, 500 mM NaCl, pH 8.3, overnight at 4°C.

Monoclonal antibody columns were equilibrated in PBS and the filtered extracts loaded at 15 ml per hour. The column was then washed in 20 volumes of PBS, after which a more stringent wash of 20 column volumes was carried out with PBS supplemented with 500 mM NaCl. Proteins were eluted in 500 mM NaCl, 100 mM glycine pH 11.0, and fractions evaluated for protein content by spectrophotometric absorbance at 280 nm. Peak fractions were pooled and dialyzed extensively against PBS, concentrated with a negative pressure dialysis device (obtained from Amicon, Beverly MA) and were used in T-cell epitope mapping studies of the mite group I and group II allergens. Recovered proteins were obtained at purities ranging from 80% (group II) to 90% (group I).

Reverse Phase HPLC chromatography was applied to further purify the immunoaffinity purified group II mite protein allergen according to the conditions in Heymann et al., J. Allergy Clin. Immunol. 83: 1055-1067 (1989). Briefly, immunoaffinity purified protein was applied to a 5 $\mu$m 300 A C-8 column (Applied Biosystems Inc.) in 0.1 % vol./vol. $TFA/H_2O$. The flow rate for the column was 1 ml/min. with a gradient of 0-60% acetonitrile/0.1% TFA over 60 minutes. Group II proteins eluted around 45% acetonitrile/0.1 % TFA. Fractions were analyzed for purity by SDS-polyacrylamide gel electrophoresis followed by densitometric scanning. Those fractions with group II protein of purity greater than 90% were subsequently utilized for human T-cell mapping of the allergen.

## Example II Recombinant Mite Allergens Expression.

What follows is a description of the work done to produce the group I and group II allergens of the house dust mites Dermatophagoides pteronyssinus and Dermatophagoides farinae as recombinant proteins in E.coli.

All four protein allergens, Der f I, Der p I, Der f II, and Der p II, were fused at their mature amino termini with the leader sequence MGHHHHHHEF (where amino acids EF are encoded by the EcoR I restriction site GAATTC). Since the $H_6$ stretch of amino acids coordinates $Ni^{++}$ ions on NTA agarose columns (Diagen GmbH), this activity was exploited in the purification of the recombinant proteins (Hochuli et al., Biotechnology 86; 1321-1325 (1988)). Ultimately, all four allergens were subcloned into the expression vector pET 11d (Studier et al., Methods In Enzymology 185: 60-88 (1990)) under the transcriptional control of the phage T7 gn 10 promoter.

The cDNAs encoding the group I allergens from D. pteronyssinus and D. farinae were obtained from Dr. Wayne Thomas in plasmid form as subclones from λgt11 (Chua et al., J. Exp. Med. 167:175-182 (1988); Dilworth et al., Clin. Exp. Allergy 21: 25-32 (1991)). The Der p I cDNA had been subcloned from an M13 RF plasmid as an EcoRI fragment into puck18 by Dr. Roland Buelow at ImmuLogic (Palo Alto), while the Der f I cDNA was manipulated directly from the M13mp19 RF plasmid Df I(1).

Initially the cDNAs were subcloned into the expression vector pTrc99A $His_6$ Insert RRRS which is a modified version of pTrc99A (Amann et al., Gene 69: 301-315 (1988)). The original vector was modified by the addition of a synthetic adaptor (consisting of two complimentary oligonucleotides) encoding the leader sequence MGHHHHHHEF between its NcoI (MG) and EcoRI (EF) sites at the 5' end of the polylinker, and an RRS (Retro Regulatory Sequence) into its Hind III site at the polylinker's 3' end. The leader sequence was used as a purification aid as described above, while the RRS was added to enhance recombinant message stability and thereby increase recombinant protein yield (Skoglund et al., Gene 88: 1-5 (1990)). Lastly, an insert, in this case an Amb a 1.1 cDNA encoding the major allergen of the short ragweed (Rafnar et al., J. Biol. Chem. 226: 1229-1236 (1991)) was subcloned in frame with the $H_6$ leader as an EcoRI to PstI fragment.

To express the mite group I allergens, the Amb a I.1 cDNA was excised by EcoRI/Hind III digestion and replaced by adaptors with φEcoRI/Hind III overhangs (composed of a pair of complimentary oligonucleotides). These adaptors (Fig. 2a) encoded the first 5 amino acids, up to the Pst I site, of the mature Der p I $NH_2$ terminus, and the first 10 amino acids, up to the HpaI site, of the mature Der f INH2 terminus. Upon ligation of the EcoRI site of the vector and the φEcoRI site in the adaptors, the EcoRI site in the vector was destroyed, leaving the EcoRI site encoded internal to the adaptor as the sole EcoRI site in the intermediate constructs pTrc99A $His_6$ 5' pI RRS and pTrc99A $His_6$ 5' fI RRS. The Der p I cDNA was inserted as a PstI/EcoRI fragment into PstI/EcoRI digested pTrc99A $His_6$ 5' pI RRS, while the Der f I cDNA was inserted as a HpaI/EcoRI fragment into HpaI/EcoRI digested pTrc99A $His_6$ 5' fI RRS. The sequence of the 5' end of each construct, pTrc99A $His_6$ 5' pI RRS and pTrc99A $His_6$ fI RRS, was verified by dideoxy chain termination DNA sequencing using a Sequenase™ II kit (U.S. Biochemicals). Both the $H_6$ pI and $H_6$ fI coding cassettes were excised by NcoI/NheI digestion (an NheI site existed at the 3' end of the RRS adaptor) and inserted into NcoI/NheI digested pET11d. These two constructs, pET11d $His_6$ pI RRS and pET11d $His_6$ fI RRS, were transformed into competent BL21 [DE3] bacteria for expression of the recombinant proteins. BL21 [DE3] contains a recombinant phage λ lysogen, DE 3, with a phage T7 RNA polymerase gene under the transcriptional control of the lac UV5 promoter. T7 RNA polymerase gene expression is induced by the addition of IPTG (Isopropyl-B-D-thiogalactopyrano- side), which in turn leads to high level expression of the recombinant gene subcloned 3' of the pET vector's T7 gn 10 promoter.

The cDNAs encoding the group II allergens from D. pteronyssinus and D. farinae were also obtained from Dr. Wayne Thomas in plasmid form as subclones from λgt11 (Chua et al., Int Arch. Appl. Immun. 91:118-123 (1990); Trudinger et al., Clin. Exp. Allergy 21: 33-37 (1991)). The original Der p II cDNA was subcloned from an M13RF plasmid into the pCA and pGEX vectors by Dr. Roland Buelow at ImmuLogic (Palo Alto). Dr. Thomas' group had supplied the Der f II cDNA as a subclone in pGEX. Both pGEX plasmids harboring group II cDNAs were used as templates for PCR amplification with the same 5' sense/3' antisense primer pair (Fig. 2b). The primers were designed to fuse an EcoRI site (GAATTC encoding the amino acids EF) in frame with the $NH_2$ terminus of the mature group II proteins and place a PstI site 3' of the group II coding region. An MJ Research Thermal Controller with a program of 30 X (94°C 1 min./55° 1 min. 30 sec./72°C 2 min.) was used in conjunction with reagents from a Perkin Elmer - Cetus Gene Amp kit for PCR amplification. The PCR products were EcoRI/PstI digested and subcloned into EcoRI/PstI digested M13mp19RF. DNA sequence analysis was performed to verify the sequence of the PCR products. Correct M 13RF were EcoRI/PstI digested, their group II cDNA inserts isolated, and subcloned into EcoRI/PstI digested pTrc99A $His_6$ Amb a I.1 RRS (which served to exchange the group II cDNAs with the ragweed cDNA (Fig. 1)).

The Der f II cDNA possessed a sequence polymorphism at position 54 (i.e., threonine residue in place of isoleucine). To alter this polymorphism, site directed mutagenesis of the $T_{54}$ residue in the Der f II cDNA was performed using a Muta-Gene kit from Bio-Rad Laboratories, based on the method of Kunkel, Proc. Natl. Acad. Sci. USA. 82: 488-492 (1985). The original M13mp19RF with the $T_{54}$ Der f II cDNA was transformed into CJ236 bacteria, which tolerate the incorporation of uracil in place of thymidine during DNA replication, and single stranded phage DNA prepared as template. A mutagenic 17 base pair primer (Fig. 2b) was annealed to the phage template DNA. T4 DNA polymerase was used to copy the template DNA primed by the mutagenic oligonucleotide, and T4 DNA ligase served to seal gaps in the DNA strand. The reaction was transformed into MV 1190 bacteria, which are wild type for the editing of uracil residues from DNA and therefore selectively replicate the mutagenized (non-uracil containing) strand, and single stranded phage DNA prepared from recombinant (white) plaques. Several recombinants were subjected to DNA sequence analysis and Der f II cDNAs with the corrected $I_{54}$ sequence subcloned as EcoRI/PstI fragments into EcoRI/PstI digested pTrc99A $His_6$ Amb a I.1 RRS.

Since one previous work had shown that the pET11d vector was, in most cases, capable of expressing recombinant proteins at higher levels than the pTrc99A vector, the two mite group II cDNAs were subcloned as $H_6$ fusion proteins into T7 vector. pTrc ppA $His_6$ f II RRS and pTrc99A $His_6$ p II RRS were Nco I/NheI digested, the cDNA inserts isolated, and subcloned into NcoI/NheI digested pET11d $His_6$ pl (Fig. 1). Recombinant plasmids were transformed into BL 21 [DE3] bacteria for expression.

BL21 DE3 host bacteria harboring the pET11d mite allergen expression constructs were freshly streaked onto a BHI agar plate (3.7% wt./vol. Difco Brain Heart Infusion; 1.5% wt./vol. Difco agar) supplemented with 200 μg/ml ampicillin and incubated overnight at 37°C. A single colony was innoculated into a 2 ml of 200 μg/ml ampicillin/BH1 media (3.7% wt./vol. Difco Brain Heart Infusion) and shaken at 300 rpm at 37°C until turbid but not saturated. The 2 ml culture was then added to 100 ml of 200 μg/ml ampicillin/BH1 media, shaken at 300 rpm at 37°C until turbid but not saturated, at which point the culture was divided into 18 X 500 ml (9 litres) of 200 μg/ml ampicillin/BH1 media and shaken at 300 rpm at 37°C. When the $OD_{595}$ of the culture reached 1.0, expression of the recombinant molecules was induced by the addition of IPTG to 400 μM, and allowed to continue for two hours.

Bacteria were harvested by centrifugation at 10,000 X g for 15 minutes, and resuspended in 1/20th volume of lysis buffer (0.2 mg/ml lysozyme, 100 mM $NaPO_4$ pH 8.0, 50 mM NaCl), incubated on ice for 30 minutes, and frozen at -70°C. The frozen bacteria were fractured by a quick thaw at 37°C and then sonicated 5 times for 20 seconds at 30 second intervals. The sonicated samples were centrifuged at 15,000 X g to separate soluble and particulate bacterial proteins. The soluble proteins were poured off, and the pelleted protein resuspended in 6 M guanidine HCl, 100 mM 2- mercaptoethanol, 100 mM $NaPO_4$, 10 mM Tris pH 8.0. This suspension was subjected to centrifugation at 15,000 X g, and the supernatant removed, adjusted to pH 8.0 with 10 N NaOH, and applied to an NTA agarose column that had been equilibrated in 6 M guanidine HCl, 100 mM $NaPO_4$, 10 mM Tris pH 8.0. The column was washed in 6 M guanidine HCl, 100 mM $NaPO_4$, 10 mM Tris pH 8.0 until the $OD_{280}$ of the effluent reached background. The column buffer was then switched to 8 M urea, 100 mM $NaPO_4$, 10 mM Tris pH 8.0. After equilibration, a more stringent wash was performed in 8 M urea, 100 mM NaOAc, 10 mM Tris pH 6.3 until the $OD_{280}$ of the effluent reached background. Recombinant mite protein (as an $H_6$ fusion) was then eluted in 8 M urea, 100 mM NaOAc, 10 mM Tris pH 4.5 and collected in aliquots whose $OD_{280}$ profile was monitored. The protein peak was dialyzed 3 times into 500 volumes of PBS for human T-cell analysis. Yield ranged from 10 to 70 mg of recombinant protein per liter with purity (as determined by densitometric scanning) ranging from 80 (Der f II) to 95%.

To further purify the recombinant Der f II protein allergen, Reverse Phase HPLC chromatography was applied. Approximately 100 mg of $His_6$-Der f II protein was reduced in 20 mM DTT at 36°C for 30 minutes and then applied to a Pharmacia PRO RPC HR 10/10 column in 0.1 % vol./vol. TFA/$H_2O$. The flow rate for the column was 1.5 ml/min. with a gradient of 0-70% acetonitrile in 0.1% TFA over 40 minutes, followed by a gradient of 70-100% acetonitrile in 0.1 % TFA. His$_6$-Der f II protein eluted between 54-96% acetonitrile. Fractions detected at 214 nm and 280 nm were analyzed for purity by SDS-polyacrylamide gel electrophoresis/densitometric scanning. Those fractions with His6-Der f II protein of purity greater than 95% were subsequently utilized for human T-cell mapping of the allergen. Yield from the preparative Reverse Phase HPLC was approximately 69%.

Determination of Nucleotide Sequence Polymorphisms in the Der p I, Der p II and Der f II Allergens

It was expected that there were sequence polymorphisms in the nucleic acid sequence coding for Der p I, Der p II, Der f I and Der f II, due to natural allelic variation among individual mites. Several nucleotide and resulting amino acid sequence polymorphisms have been discovered during the sequencing of different Der p I, II and Der f II clones. The amino acid sequence polymorphisms are shown in Figs. 22, 23 and 24.

**Examples III Synthesis of Overlapping Peptides**

Der p I, Der f I, Der p II, and Der f II overlapping peptides as shown in Figs. 3 and 4 were synthesized using standard Fmoc/tBoc synthetic chemistry and purified by dialysis or Reverse Phase HPLC. The amino acid residues of the synthesized peptides are in brackets by the peptide name and the amino acid sequence (in single letter code) is next to the peptide name. The peptide names are consistent throughout the Figures. The Der p I, Der f I, Der p II and Der f II proteins were divided into overlapping peptides in such a way that the overlapping peptides of Der p I and Der f I as well as for Der p II and Der f II have corresponding amino acid residue numbers, e.g. DP I-1 and DF I-1 both contain amino acid residues 1-20 of the Der p I and Der f I allergens, respectively. This correspondence in amino acid position between the Der p and Der f peptides was done purposefully in order to best test for cross-reactivity of Der p and Der f T cell epitopes and, thus, determine peptides which, upon administration to an individual sensitive to dust mite, would treat sensitivity to both Der p and Der f allergens. In the design of the overlapping peptides the relationship of the Group I and Group II allergens at the level of T cell cross-reactivity was considered. In addition, the function of the Group I allergens as serine proteases was considered and the amino acid sequences of other known serine proteases, e.g., papain, actinidin, were examined to identify similar conserved and variable regions within the Group I allergens. It was expected that conserved regions within the Group I allergens would contain "shared" T cell epitopes.

**Example IV Mite Allergic Patient Primary T Cell Responses to Der p I and Der p II Proteins and Peptids**

Peripheral blood mononuclear cells (PBMC) were purified by Ficoll-Hypaque centrifugation of a peripheral blood specimen from mite-allergic patient RB. and were assayed for proliferation in response to various antigens, i.e., affinity-purified Der p I, affinity purified Der p II, various Der p I and Der p II peptides. For assay, 5 X 10$^4$ PBMC were cultured in triplicate microwells for 7 days at 37°C in the presence of various concentrations of antigen in 200 $\mu$l RPMI-1640 containing 5% human AB serum. Each well then received 1 $\mu$Ci tritiated thymidine for 16 hours. The counts incorporated were collected onto glass fiber filters and processed for liquid scintillation counting. Table I shows the results of this assay. The CPM +/- standard deviation are shown. The stimulation index of each response (S.I.) is the ratio of the $^3$H-thymidine CPM incorporated by the cells in response to antigen divided by the $^3$H-thymidine CPM incorporated by cells in medium only. The results indicate that this patient responds with an S.L of at least 2.0 to peptides DP I-1, DP 1-3, DP 1-8, DP I-10, DP 1-5.2, DP II-4 and DP II-9. Thus, these peptides contain Der p I or Der p II T cell epitopes recognized by T cells from this particular allergic patient.

TABLE I

| Antigen | Concentration $\mu$g/ml | CMP $\pm$ S.D. | S.I. |
| --- | --- | --- | --- |
| Medium | -- | 1071$\pm$30 | -- |
| Der p I | 10 | 920$\pm$15 | 0.9 |
|  | 30 | 2122$\pm$93 | 2.0 |
|  | 100 | 1492$\pm$13 | 1.4 |
| DP I-1 | 10 | 1099$\pm$48 | 1.0 |
|  | 30 | 3527$\pm$73 | 3.3 |
|  | 100 | 2746$\pm$81 | 2.6 |

| (continued) | | | |
|---|---|---|---|
| Antigen | Concentration μg/ml | CMP ± S.D. | S.I. |
| DP 1-2 | 10 | 1395±47 | 1.3 |
|  | 30 | 1283±34 | 1.5 |
|  | 100 | 1486±38 | 1.4 |
| DP 1-3 | 10 | 2608±52 | 2.4 |
|  | 30 | 1561±13 | 1.5 |
|  | 100 | 5252±67 | 4.9 |
| DP 1-8 | 10 | 1439±32 | 1.3 |
|  | 30 | 1045±32 | 1.0 |
|  | 100 | 2272±40 | 2.1 |
| DP 1-10 | 10 | 1936±50 | 1.8 |
|  | 30 | 3042±89 | 2.8 |
|  | 100 | 2644±63 | 2.5 |
| DP 1-5.2 | 10 | 1374±20 | 1.3 |
|  | 30 | 2241±87 | 2.1 |
|  | 100 | 3132±111 | 2.9 |
| Der p II | 10 | 1113±35 | 1.0 |
|  | 30 | 2104±43 | 2.0 |
|  | 100 | 1057±27 | 1.0 |
| DP II-4 | 10 | 2126±25 | 2.0 |
|  | 30 | 1979±94 | 1.8 |
|  | 100 | 2314±116 | 2.2 |
| DP II-9 | 10 | 3970±87 | 3.7 |
|  | 30 | 4464±86 | 4.2 |
|  | 100 | 2237±53 | 2.1 |

### Example V T Cell Epitope Studies with Der p I

Peripheral blood mononuclear cells (PBMC) were purified by Ficoll-Hypaque centrifugation of 60 ml of heparinized peripheral blood from house dust mite-allergic individuals who exhibited clinical symptoms of mite allergy and who were skin test positive for house dust mite.

$10^7$ PBMC from individual 543 were cultured in 10 ml RPMI-1640 containing 5% pooled human AB serum and supplemented with glutamine, penicillin, streptomycin and HEPES buffer in the presence of 20 μg/ml purified native Der p I/ml at 37°C for 7 days. Viable cells were then purified by Ficoll-Hypaque centrifugation and cultured for 2 additional weeks in RPMI-1640/5% AB serum containing 5 units recombinant human IL-2/ml and 5 units recombinant human IL-4/ml. The resting T cells were then tested in a secondary proliferation assay to assess T cell responses to various house dust mite proteins and peptides. For assay, 2 X $10^4$ resting T cells were cultured in 200 μl of RPMI-1640/5% AB serum for 3 days at 37°C in the presence of 2 X $10^4$ autologous Epstein-Barr virus transformed B cells (20,000 Rads) as antigen presenting cells with various concentrations of purified native Der p I or synthetic Der p I peptides. Each well then received 1 μCi tritiated thymidine for 16 hours. The counts incorporated were collected onto glass fiber filters and processed for liquid scintillation counting. Medium alone, acting as negative control, contained no allergen or peptide. The results of this experiment indicate that this particular patient responds with an S.I. of at least 2.0 to several peptides derived from the Der p I protein, including DP I-1, DP I-2, DP I-4, DP I-11, DP I-5, DP I-13, DP I-15, DP I-6.1, DP I-8, DP I-9, DP I-16, DP I-10 and DP I-17 (data not shown).

The above procedure was followed with a number of other house dust mite allergic individuals except a) in individual cases, the length of time of cultivation with IL-2 and IL-4 varied; b) in individual cases, the T cells were primed with either purified native (N) or recombinant (R) Der p I protein at either 20 μg/ml or 10 μg/ml; and c) in individual cases, x-irradiated (3500 Rads) autologous PBMC were used as antigen presenting cells in the secondary proliferation assay. In addition, three peptides (DP I-11 (SEQ ID NO: 117), DP I-12 (SEQ ID NO: 118), and DP II-3 (SEQ ID NO: 119)) were found to contain a low number of conservative changes from the native sequence in their amino acid sequence. Three additional peptides (DP I-11.1 (SEQ ID NO: 13), DP I-12.1 (SEQ ID NO: 14) and DP II-3.1 (SEQ ID NO: 43)) were synthesized with no changes from the native sequence. Some T cell analysis was done with the original peptides (i.e., DP I-11, DP I-12 and DP II-3). Following T cell analysis conducted with the additional peptides (i.e., DP I-11.1, DP I-12.1 and DP II-3.1) no significant difference in mean S.I. or percentage of positive responses between the original peptides and the additional peptides was detected. Thus, the data from both groups of peptides was pooled.

Individual results were considered positive and used if the individual responded to the Der p I protein and at least one peptide derived from Der p I at an S.I. of 2.0 or greater. A summary of the results of 33 positive experiments is shown in Fig. 5. The resting T cell lines primed with recombinant or native Der p I-stimulated PBMC were tested for reactivity to synthetic Der p I peptides. The maximum response for each peptide in a titration of the antigen is expressed as the T cell stimulation index (S.I.). The S.I. is the counts-per-minute (CPM) incorporated by cells in response to the peptide divided by the CPM incorporated by cells in medium only. An S.I. value greater than the background level indicates that the peptide contains a T cell epitope. However, only individual S.I. values greater than or equal to 2.0 (a response two-fold or greater over background), referred to herein as "positive" results, were used in calculating mean T cell stimulation indices for each peptide for the patient or group of patients tested. In parentheses above each bar on the histogram are the mean T cell stimulation indices calculated after discarding the highest and lowest positive responses for each peptide to minimize the effect of extreme outliers. The T cell stimulation index is calculated by:

$$\frac{\text{(CPM of T cell+APC+Antigen)}}{\text{CPM of T cell +APC+Control}}$$

The bar represents the cumulative rank of the peptide response in the group of patients. To determine the cumulative rank, the 5 peptides with the highest S.L in each individual were determined and assigned a numerical rank in descending order, with 5 representing the strongest response. The ranks for each peptide were then summed in the group of patients to determine the cumulative rank for the peptide. Above each bar is the percent of positive responses with an S.I. of at least 2 to the peptide in the group of patients tested. Given the percent positive and the mean T cell stimulation index, the positivity index (P.I.) for each peptide can be calculated. The P.I. for each individual is determined by multiplying the mean S.I. by the percent of individuals, in a population of individuals sensitive to house dust mite (e.g. preferably at least 15 individuals, more preferably at least 30 individuals or more) who responded with an S.I. of at least 2.0 to that peptide (e.g., for DP I-1 in Fig. 5, the P.I. would be about 343 (73% x 4.7). The P.I. therefore represents both the strength of a T cell response to a peptide (S.I.) and the frequency of a T cell response to a peptide in a population of individuals sensitive to house dust mite. Fig. 5 demonstrates that peptides DP I-1, DP I-2, DP I-3, DP I-4, DP I-5, DP I-6.1, DP I-7.1, DP I-8, DP 1-9. DP I-16, and DP I-10 contain significant regions of T cell reactivity in this panel of patients.

### Example VI T Cell Epitope Studies with Der f I

Experiments similar to those of Example V were performed to determine the T cell-reactive areas of the Der f I protein. For example, PBMC from house dust mite-allergic patient 783 were isolated as described in Example V and were stimulated in vitro with recombinant purified Der f I at 20 µg/ml. The results of the proliferation assay with Der f I peptides using x-irradiated (3500 Rads) autologous PBMC as antigen presenting cells indicate that T cells from this patient respond to the peptides DF I-8.1, DF 1-9, DF I-6, DF I-10, DF I-2.1, DF I-3, DF I-11, DF 1-5, DF I-1, and DF I-17 (data not shown).

The above procedure was followed in a number of patients except in individual cases, T cell lines were primed with affinity purified Der f I at 20µg/ml or at 10 µg/ml and x-irradiated (25,000 Rads) autologous Epstein-Barr virus transformed B cells were used as antigen presenting cells. A summary of the results of 16 positive experiments is shown in Fig. 6. The data was analyzed as described in Example V. The data indicate that significant areas of T cell reactivity in the Der f I protein are found in the peptides DF I-1, DF I-2, DF 1-3, DF I-4, DF I-11, DF I-5, DF I-6, DF I-7, DF I-14, DF I-15, DF I-8.1 and DF 1-9.

### Example VII A Sturdy Indicating the Cross-reactivity of Der p I and Der f I T Cell Epitopes

The Der p I and Der f I proteins are very homologous (81 % identity). Thus, experiments, similar to those of Example V, were carried out to determine the T cell responses of Der p I primed T cell lines when challenged with various Der p I peptides and substantially matching Der f II peptides. T cell lines were primed in vitro as described in Example V and tested for response to a set of substantially matching Der p I and Der f I peptides (e.g., DP I-1 (amino acid residues 1-20 of Der p I) or DF I-1 (amino acid residues 1-20 of Der f I). The data was analyzed as described in Example V except the highest and lowest S.I. values of the positive responses to each peptide were not omitted from the mean S.I. calculations. A summary of a number of such experiments is shown in Fig. 7. The results of 14 positive experiments indicate that Der p I-primed T cells respond to various Der f I peptides indicating cross-reactivity within the Group I allergens. Der p I primed T cells respond significantly to peptides DF I-1, DF I-2, DF I-3, DF I-4, DF I-11, DF I-12, DF I-15, DF I-8, DF I-9, DF 1-15 and DF 1-6. In some patients, the Der f I peptide was a more potent stimulator of Der p I-primed T cells than the corresponding Der p I peptide. Fig. 8 shows the results of inverse experiments in which T cells from a number of patients were primed in vitro to the Der I protein and analyzed for response to various Der p I peptides and a set of substantially matching Der f I peptides. The results of 8 positive experiments indicate that Der I primed T cells respond significantly to peptides DP I-1, DP I-3, DP I-4, DP I-11/11.1, DP I-14, DP I-5, DP I-15, and DP 1-8.

### Example VIII T Cell Epitope Studies with Der p II.

Experiments similar to those of Example V were performed to determine the T cell-reactive areas of the Der p II protein. For example, PBMC from house dust mite-allergic patient 348 were isolated as described in Example V and were stimulated in vitro with 20 $\mu$g/ml purified native Der p II. The results of a proliferation assay using x-irradiated (25.000 Rads) Epstein-Barr virus transformed autologous B cells as antigen-presenting cells with various Der p II peptides demonstrate that this particular patient responds well to peptides DP II-1, DP II-7, DP II-8, DP II-2, and DP II-9 (data not shown).

The above procedure was followed with a number of patients except in individual cases, T cell lines were primed with recombinant Der p II at 20 $\mu$g/ml or at 3 $\mu$g/ml and x-irradiated (3500 Rads) autologous PBMC were used as antigen-presenting cells. A summary of the results of 26 positive experiments is shown in Fig. 9. The data was analyzed as described in Example V, except the ranked sum of peptide responses was analyzed assigning a value of 3, 2 or 1 to the three highest S. I. responses. Areas of significant T cell reactivity within the Der p II protein for this panel of patients are found in peptides DP II-1, DP II-2, DP II-3, DP II-4, DP II-7, DP II-8 and DP II-9.

### Example IX T Cell Epitopes Studies with Der f II

Experiments similar to those of Example V were performed to determine the T cell-reactive areas of the Der f II protein. For example, PBMC from house dust mite-allergic patient 384 were stimulated in vitro with purified recombinant Der f II and the T cell line was then challenged in the presence of x-irradiated (25,000 Rads) autologous Epstein-Barr virus transformed B cells as antigen presenting cells with various overlapping Der f II peptides. The results of this proliferation assay indicate that T cells from this particular patient respond well to peptides DF II-1, DF II-2, DF II-3.1, DF II-4.5, DF II-15, DF II-16. and DF II-19.1 (data not shown).

The above procedure was followed with 10 patients, except in individual cases, T cell lines were primed by stimulating the patient PBMC with 20 $\mu$g/ml or 3 $\mu$g/ml purified native Der f II, and were assayed in the presence of x-irradiated (3500 Rads) autologous PBMC as antigen-presenting cells. A summary of the results of 10 positive experiments is shown in Fig. 10. The data was analyzed as detailed in Example IX, except the highest and lowest S.I. values of the positive responses to each peptide were not omitted from the calculations. The data indicate that significant areas of T cell reactivity within the Der f II protein are found in peptides DF II-1, DF II-2, DF II-13.1, DF II-4.5, DF II-15, DF II-17, and DF II-19.1.

### Example X A Study Indicating the Cross-Reactivity of Der p II and Der f II T Cell Epitopes

A study similar to that described in Example VII was carried out to determine the T cell cross-reactivity of the Der p II and Der f II proteins. T cells primed with the Der f II protein were challenged with various Der f II peptides and a set of substantially matching Der p II peptides. A summary of the results of 10 positive experiments is shown in Fig. 11. The results indicate that Der f II primed T cells respond significantly to peptides DP II-1, DP II-3/3.1, DP II-4, and DP II-7. Fig. 12 shows the results of inverse experiments in which T cells from a number of patients were primed in vitro to the Der p II protein and analyzed for response to various Der f II peptides. The results of 26 positive experiments indicate that Der p II primed T cells respond significantly to peptides DF II-1, DF II-4.5, DF II-15, DF II-17, DF II-18 and DF II-19.1.

### Example XI Syntheses of Dominant Peptids

Based on the analyses described in Examples V-X, major areas of T cell reactivity within Der p I, Der p II, Der f I and Der f II were identified. In each study, all of the patients tested responded to the protein allergen (e.g., Der p I) and at least one peptide derived from a major area of T cell reactivity within the protein. Seven regions (Region 1, Region 2, Region 3, Region 4, Region 5, Region 6a and Region 6b) of major T cell reactivity were identified in the Der p I and Der f I proteins. These regions are defined as follows: Region 1, amino acid residues 1-28 of the Der p I and Der f I proteins; Region 2, amino acid residues 36-68 of the Der p I and Der f I proteins; Region 3, amino acid residues 74-109 of the Der p I and Der f I proteins; Region 4, amino acid residues 118-139 of the Der p I and Der f I proteins; Region 5, amino acid residues 141-166 of the Der p I and Der f I proteins; and Region 6a, amino acid residues 161-185 of the Der p I and Der f I proteins and Region 6b, amino acid residues 173-201 of the Der p I and Der f I proteins.

Similarly, four regions of major T cell reactivity (Region 7, Region 8, Region 9, and Region 10) were identified in the Der p II and Der f II proteins. These regions are defined as follows: Region 7, amino acid residues 1-26 of the Der p II and Der f II proteins; Region 8, amino acid residues 33-67 of the Der p II and Der f II proteins; Region 9, amino acid residues 79-104 of the Der p II and Der f II proteins; and Region 10, amino acid residues 107-129 of the Der p II and Der f II proteins. Based in part on the T cell reactivity described in Examples V-X, peptides derived from Der p I, Der f I, Der p II, and Der f II were selected and modified by addition or deletion of amino acid residues at either the 5' or 3' end of the peptide. In designing these selected peptides, various factors were considered, including the ranked sum of the overlapping peptides, the percentage of responses with an S.I. of at least 2.0 to the peptides, the potential cross-reactivity of the peptides, the difficulty of manufacture of the peptides, etc. T cell studies similar to those described in Examples V-X were performed using these selected peptides to more precisely define the major areas of T cell reactivity within Regions 1-6a and 6b of the Der p I and Der f I protein and Regions 7-10 of the Der p II and Der f II protein.

The results of T cell studies using selected peptides derived from the Der p I, the Der f I, the Der p II and the Der f II proteins are shown in Figs. 13-18a-d. The procedure described in Example V was followed with T cell lines from a number of patients primed in vitro to the Der p I protein then analyzed for response to selected peptides derived from the Der p I sequence. The results of 33 positive experiments shown in Fig. 13 indicate that the Der p I primed T cells respond significantly to peptides found in peptides DP I-21.1, DP I-21.2, DP I-22.2, DP 1-25.2, DP I-22.1, DP I-23.1, DP 1-23.2, DP I-26.1, and DP I-28.1.

Similarly, the procedure described in Example VI was followed with T cell lines from 9 patients primed in vitro to the Der f I protein then analyzed for response to selected peptides derived from the protein. The data was analyzed as described in Example VL The results of the 9 patients shown in Fig. 14 demonstrate T cell reactivity to selected peptides from Der f I.

In another experiment, the T cell lines from a number of patients were primed in vitro to the Der p I protein and analyzed for response to selected peptides derived from the Der p I protein and a set of substantially matching peptides derived from the Der f I protein. The data from 30 positive experiments was analyzed as described in Example V. As shown in Fig. 15a, the Der p I primed T cells respond significantly to peptides DF I-21.1, DF I-21.2, DF I-23.1, DF 1-22.2, DF 1-22.3, DF I-22.4, DF 1-23.2, DF I-25.1, DF I-26.1 and DF I-27.1. Fig. 15b is a subset of the data shown in Fig 15a and shows the response of Der p I primed T cells to peptides analyzed by ranked sum. Fig. 15b shows that DPI-23.1 has the highest ranked sum of this group of peptides in this study. Fig. 16a shows the results of the inverse experiment in which T cells from 9 patients were primed in vitro to the Der f I protein and challenged with selected Der f I peptides and a set of substantially matching Der p I peptides. The results indicate that Der f I primed T cells from 9 patients respond to selected peptides from Der p I. Fig. 16a shows that DF I-22.1 and DF I-25.1 have the highest stimulation indexes of this group of peptides. Fig. 16b is a subset of the same data as shown in Fig. 16a and shows the response of preferred Der f I and Der p I peptides is shown. Fig 16b shows that DF1-22.2 has the highest stimulation index of this group of preferred peptides in this experiment.

Another experiment following the procedure described in Example VIII analyzed the response of 29 patients primed in vitro to the Der p II protein and challenged with selected peptides derived from the Der p II sequence. Fig. 17a shows that Der p II primed T cells from one patient respond to selected peptides from Der p II. Fig. 17b shows the results from an experiment similar to that shown in Fig. 17a with a set of 30 patients and with the high and low omitted from the mean. Fig. 17b shows that DPII-20.6 has the highest ranked sum of this group of preferred peptides in this study. Fig. 18a shows the inverse experiment in which T cells from 1 patient were primed in vitro to the Der f II protein and challenged with selected peptides derived from the Der p II sequence. Fig. 18a shows that Der f II primed T cells from 10 patients respond to selected peptides from Der p II. As shown in Fig. 18a DP II-25 has the highest stimulation index. Fig. 18b is a subset of the same data shown in Fig. 18a and shows the response of native Der f II primed T cell lines to preferred Der p II peptides analyzed by ranked sum. As is shown in Fig. 18b, DPII-25.2 has the highest ranked sum of the preferred peptides in this study.

**Example XII Study Indicating Cross-reactivity of Selected Group I and Group II Epitopes**

A study similar to that described in Example VII was carried out with T cells from 4 matched patients primed in vitro with Group I proteins from Der f and Der p then analyzed for response to selected preferred peptides from Der p I and Der f I. The results in Fig. 18c demonstrate that T cell reactivity to a number of the selected preferred Der p I peptides was essentially equivalent for their Der f I counterpart and vice-versa.

Fig. 18d shows the results of a similar study with T cells from 6 matched patients primed in vitro with Group II proteins from Der p and Der f, then analyzed for response to selected preferred Der p II and Der f II peptides. Similar to the results in Fig. 18c, T cell reactivity to a number of the selected preferred peptides of Der p II are essentially equivalent to their Der f II counterparts and vice-versa.

## Example XIII Direct Binding Assay of IgE to Mite Allergen Proteins and Peptides

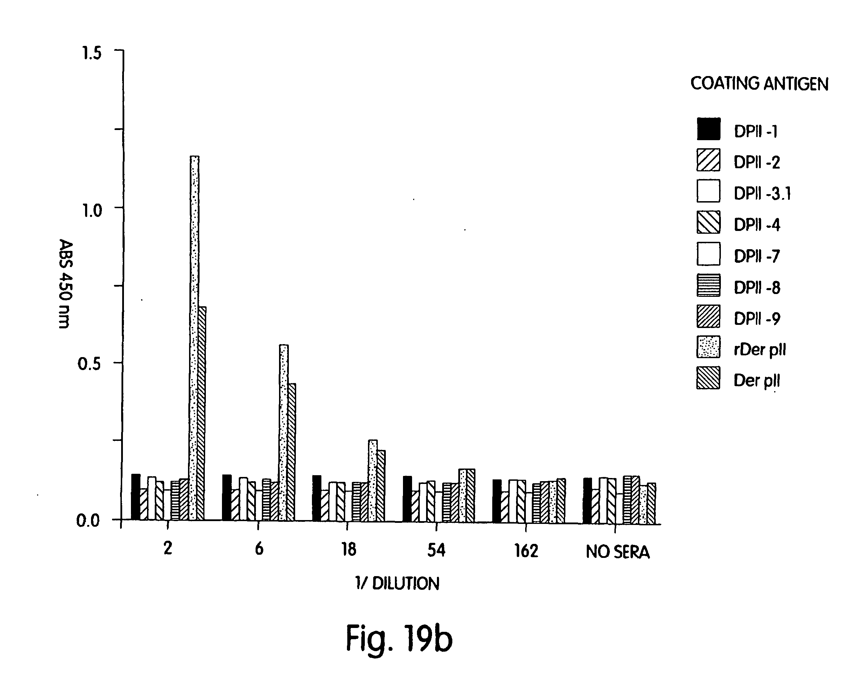

Coming assay plates (#25882-96) were coated with 5 $\mu$g/ml of each coating antigen listed in Figs. 19, 20 and 21 at 50 $\mu$l/100ml/well and incubated overnight at 4°C. The coating antigens were removed and the wells are blocked with 0.5% gelatin in PBS, 300 $\mu$l/well for 2 hours at room temperature. Pooled human plasma (a mix of plasma samples from 20 patients that were skin test positive for commerical mite extract) was serially diluted with PBS-Tween 20 (PBS with 0.05% nonionic detergent Tween-20 Sigma, St. Louis, MO) and 100 $\mu$l/well a\was added and incubated overnight at 4°C (plasma dilutions were tested in duplicate). The second antibody (biotinylated goat anti-Human IgE, 1:1000, Kirkegaard & Perry Laboratories Inc, Gaithersburg, MD), was added at 100 $\mu$l/well for one hour at room temperature. This solution was removed and streptavidin-HRPO, 1:10000, (Southern Biotechnology Associates, Inc., Birmingham, AL) was then added at 100 $\mu$l/well for one hour at room temperature (all wells are washed three times with PBS-Tween between each incubation step). TMB Membrane Peroxidase Substrate system (Kirkegaard & Perry Laboratories) was freshly miced, and added at 100ml/well. The color was allowed to develop for 2-5 minutes. The reaction was stopped by the addition of 100ml/well of 1M phosphoric acid. Plates were read on a Microplate IL310 Autoreader (Biotech Instruments, Winooski, VT) with a 450 nm filter. The absorbance levels of duplicate wells were averaged. The graphed results (log of the dilution versus absorbance) of the ELISA assays are shown in Figs. 19a-b, 20a-b and 21a-h. The order of coating antigens listed vertically in these figure legends corresponds in order from left to right to the coating antigens listed for each histogram.

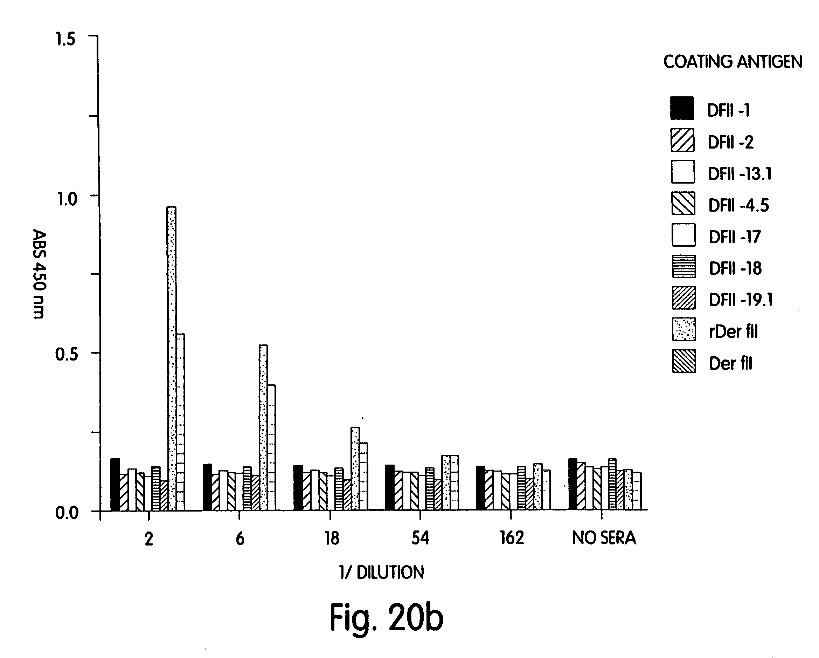

The results of the ELISA assay shown in Fig. 19b demonstrate good binding of both biochemically purified Der p II and recombinant Der p II (rDer p II) with human IgE and no detectable binding to the Der p II peptides. The IgE binding to the Der p set of peptides and proteins (Figs. 20a and 20b) shows the same pattern of reactivity as the Der f set. That is, no detectable binding to Der f I or Der f II peptides or recombinant Der f I with binding to only biochemically purified Der f I and recombinant and biochemically purified Der f II. In both cases there appears to be better binding to recombinant Der p II or Der f II than to the biochemically purified forms. All the conclusions derived from the above ELISA assay data were corroborated by another assay method, dot blots on nitrocellulose paper, using the same set of antibody and antigen reagents.

The antigen preparation that was used as a positive control was a mixture of the four major biochemically purified mite allergens (term PMA for Purified Mite Allergen); Der f I, Der f II, Der p I and Der p II. The stock was generated at a concentration of 100 $\mu$gs of each protein per millimeter or 400 $\mu$gs total protein/ml. This preparation was used on each coated ELISA plate. The results from these ELISA assays are shown in Fig. 21a-h. There is clear binding to either the purified or recombinant protein or the PMA antigen preparation on each plate indicating good IgE reactivity. However, the PMA antigen preparation, does exhibit a high degree of non-specific reactivity shown in the background dilution where no first antibody solution was added. This non-specific reactivity occurs between the PMA antigen and the biotinylated second antibody and does not compromise the finding of specific IgE reactivity to the antigen. Using a quantitative value of two-fold over background at the highest plasma concentration as a positive reading, there is no detectable IgE reactivity to any one of the 56 peptides screened by this assay method.

Although the invention has been described with reference to its preferred embodiments, other embodiments can achieve the same results. Variations and modifications to the present invention will be obvious to those skilled in the art and is intended to cover in the appended claims all such modifications and equivalents that follow in the true spirit and scope of the invention.

**Claims**

1. An isolated peptide of a Dermatophagoides group I or II protein allergen, said peptide comprising at least one T cell epitope of said protein allergen, optionally at least two T cell epitopes, said peptide having a positivity index of at least about 100 and a mean T cell stimulation index of at least about 2.0 determined in a population of individuals sensitive to said protein allergen, optionally the population being at least 9 individuals, for example at least 29 individuals

2. An isolated peptide of claim 1 which has a positivity index of at least about 150 and a mean T cell stimulation index of at least about 4.0 determined in a population of individuals sensitive to said protein allergen.

3. An isolated peptide of claim 1 or 2 selected from either:

(a) DP I-1 (SEQ ID NO: 9); DP I-21.1 (SEQ ID NO: 27); DP I-21.2 (SEQ ID NO: 28); DP I-23.1 (SEQ ID NO: 33); DP I-23.2 (SEQ ID NO: 34); DP I-25.2 (SEQ ID NO: 36); and DP I-26.1 (SEQ ID NO: 37); or
(b) DP I-21.2, DP I-23.1, DP I-26.1, DP II-20.6, DP II-22, DP II-25.2 and DF I-22.2 as shown in Figs 3 and 4.

4. An isolated peptide of any one of claim 1 to 3 which either:

(a) does not bind immunoglobulin E specific for a protein allergen of the genus *Dermatophagoides* in at least 75 % of individuals sensitive to the protein allergen, or if binding of the peptide to said immunoglobulin E occurs, such binding does not result in release of mediators from mast cells or basophils in at least 75% of individuals sensitive to the protein allergen; or
(b) binds immunoglobulin E to a lesser extent than the protein allergen from which the peptide is derived binds said immunoglobulin E.

5. An isolated peptide of any one of claims 1 to 4, said peptide selected from:

a) DP I-1 (SEQ ID NO: 9);
b) DP I-2 (SEQ ID NO: 10);
c) DP 1-3 (SEQ ID NO: 11);
d) DP 1-4 (SEQ ID NO: 12):
e) DP I-11.1 (SEQ ID NO: 13);
f) DP I-12.1 (SEQ ID NO: 14);
h) DP 1-13 (SEQ ID NO: 17);
i) DP I-14 (SEQ ID NO: 18);
j) DP I-15 (SEQ ID NO: 19);
k) DP I-6.1 (SEQ ID NO: 20);
l) DP I-7.1 (SEQ ID NO: 21);
m) DP 1-8 (SEQ ID NO: 22);
n) DP I-9 (SEQ ID NO: 23);
o) DP I-16 (SEQ ID NO: 24);
p) DP I-10 (SEQ ID NO: 25);
q) DP I-17 (SEQ ID NO: 26);
r) DP I-21.1 (SEQ ID NO: 27);
s) DP I-21.2 (SEQ ID NO: 28);
t) DP I-22.1 (SEQ ID NO: 29);
u) DP I-22.2 (SEQ ID NO: 30);
v) DP I-22.3 (SEQ ID NO: 31);
w) DP 1-22.4 (SEQ ID NO: 32);
x) DP 1-23.1 (SEQ ID NO:33);
y) DP I-23.2 (SEQ ID NO: 34);
z) DP I-25.1 (SEQ ID NO: 35);
a') DP I-25.2 (SEQ ID NO: 36);
b') DP I-26.1 (SEQ ID NO: 37);
c') DP I-27.1 (SEQ ID NO: 38);
d') DP I.28.1 (SEQ ID NO: 39): and
c') DP I-28.2 (SEQ ID NO: 40),

wherein said peptide has a mean T cell stimulation index equivalent to or greater than the mean T cell stimulation index of said peptide as shown in Fig 5 and Fig 13, optionally a mean T cell stimulation index of at least 4.0.

6. An isolated peptide according to any one of claims 1 to 5 having an amino acid sequence corresponding to an amino acid sequence of a peptide selected from the following :

a) DP I-1 (SEQ ID NO: 9);
b) DP I-2 (SEQ ID NO: 10);
c) DP 1-3 (SEQ ID NO: 11);
d) DP 1-4 (SEQ ID NO: 12);

e) DP I-11.1 (SEQ ID NO: 13);
f) DP I-12.1 (SEQ ID NO: 14);
g)
h) DP I-13 (SEQ ID NO: 17);
i) DP I-14 (SEQ ID NO: 18);
j) DP I-15 (SEQ ID NO: 19);
k) DP I-6.1 (SEQ ID NO: 20);
l) DP I-7.1 (SEQ ID NO: 21);
m) DP I-8 (SEQ ID NO: 22);
n) DP I-9 (SEQ ID NO: 23);
o) DP I-16 (SEQ ID NO: 24);
p) DP I-10 (SEQ ID NO: 25);
q) DP I-17 (SEQ ID NO: 26);
r) DP I-21.1 (SEQ ID NO: 27);
s) DP I-21.2 (SEQ ID NO: 28);
t) DP I-22.1 (SEQ ID NO: 29);
u) DP I-22.2 (SEQ ID NO: 30);
v) DP 1-22.3 (SEQ ID NO: 31);
w) DP I-22.4 (SEQ ID NO: 32);
x) DP I-23.1 (SEQ ID NO: 33);
y) DP I-23.2 (SEQ ID NO: 34);
z) DP I.25.1 (SEQ ID NO: 35);
a'), DP I-25.2 (SEQ ID NO: 36);
b') DP I-26.1 (SEQ ID NO: 37):
c') DP I-27.1 (SEQ ID NO: 38);
d') DP I-28.1 (SEQ ID NO: 39);
e') DP 1-28.2 (SEQ ID NO: 40);
f') DP I-5.1 (SEQ ID NO: 16); and
g') DP I-23 1.1,
h') DP I-23 1.2;
i') DP I-23. 1.3 ; and
j') DP 1-23 1.4

as shown in Fig 28.

7. A peptide of any one of claims 1 to 6 which does not bind immunoglobulin E specific for Der pI in at least 75 % of individuals sensitive to Der pI, or if binding of the peptide to said immunoglobulin E occurs, such binding does not result in release of mediators from mast cells or basophils in at least 75% of individuals sensitive to Der pI.

8. An isolated peptide of an one of claims 1 to 7 comprising at least two regions, each region comprising at least one T cell epitope of a Dermatophagoides group I or II protein allergen, said regions derived from the same or from different Dermatophagoides group I or II protein allergens, said regions each comprising an amino acid sequence selected from:

a) DP I-21.1 (SEQ ID NO: 27);
b) DP I-21.2 (SEQ ID NO: 28);
c) DP I-22.1 (SEQ ID NO: 29);
d) DP I-22.2 (SEQ ID NO: 30);
e) DP 1-22.3 (SEQ ID NO: 31);
f) DP 1-22.4 (SEQ ID NO: 32);
g) DP I-23.1 (SEQ ID NO: 33);
h) DP I-23.2 (SEQ ID NO: 34);
i) DP I-25.1 (SEQ ID NO: 35);
j) DP 1-25.2 (SEQ ID NO: 36);
k) DP I-26.1 (SEQ ID NO: 37);
l) DP I-27.1 (SEQ ID NO: 38);
m) DP I-28.1 (SEQ ID NO: 39);
n) DP I-28.2 (SEQ ID NO: 40);

70

o) DP I-1 (SEQ ID NO: 9);

p) DF I-1 (SEQ ID NO: 72);

q) DF I-21.1 (SEQ ID NO: 90);

r) DF I-21.2 (SEQ ID NO: 91);

s) DF I-22.1 (SEQ ID NO: 92);

t) DF 1-22.2 (SEQ ID NO: 93);

u) DF I-23.4 (SEQ ID NO: 94);

v) DF I-23.1 (SEQ ID NO: 95);

w) DF I-23.2 (SEQ ID NO: 96);

x) DF I-25.1 (SEQ ID NO: 97);

y) DF I-25.2 (SEQ ID NO: 98);

z) DF I-26.1 (SEQ ID NO: 99);

a') DF I-27.1 (SEQ ID NO: 100);

b') DF I-38.1 (SEQ ID NO: 101);

c') DF I-21.2 (SEQ ID NO: 102);

d') DP II-20 (SEQ ID NO: 50);

e') DP II-20.1 (SEQ ID NO: 51);

f') DP II-20.2 (SEQ ID NO: 52);

g') DP II-20.3 (SEQ ID NO: 53);

h') DP II-20.4 (SEQ ID NO: 54);

i') DP II-20.5 (SEQ ID NO: 55);

j') DP II 20.6 (SEQ ID NO: 56);

k') DP II-1 (SEQ ID NO: 41);

l') DP II-1.1 (SEQ ID NO: 57);

m') DP II-1.2 (SEQ ID NO: 58);

n') DP II-2.1 (SEQ ID NO: 59);

o') DP II-2.2 (SEQ ID. NO: 60);

p') DP II-2.3 (SEQ ID NO: 61);

q') DP II-21 (SEQ ID NO: 62);

r') DP II-22 (SEQ ID NO: 63);

s') DP II-26 (SEQ ID NO: 64);

t') DP II-26.1 (SEQ ID NO: 65);

u') DP II-23 (SEQ ID NO: 66);

v') DP II-23.1 (SEQ ID NO: 67);

w') DP II-24 (SEQ ID NO: 68);

x') DP II-25 (SEQ ID NO: 69);

y') DP II-25.1 (SEQ ID NO: 70);

z') DP II-25.2 (SEQ ID NO: 71);

a") DF II-1 (SEQ ID NO: 103)

b") DF II-2 (SEQ ID NO: 104);

c") DF II-13.1 (SEQ ID NO: 105);

d") DF II-3.1 (SEQ ID NO: 106);

e") DF II-4.5 (SEQ ID NO: 107);

f") DF II-4.3 (SEQ ID NO: 108);

g") DF II-15 (SEQ ID NO: 109);

h") DF II-16 (SEQ ID NO: 110);

i") DF II-17 (SEQ ID NO: 111);

j") DF II-18 (SEQ ID NO: 111);

k") DF II-19 (SEQ ID NO: 113);

l") DF II-19.1 (SEQ ID NO: 114);

m") DF II-21 (SEQ ID NO: 115); and

n") DF II-22 (SEQ ID NO: 116).

9. An isolated peptide of claim 8 wherein either:

(i) said regions comprise an amino acid sequence selected from:

a) DP I-21.2 (SEQ ID NO: 27);

b) DP I-23.1 (SEQ ID NO: 33);
c) DP I-26.1 (SEQ ID NO: 37);
d) DP II-20.6 (SEQ ID NO: 56);
e) DP II-22 (SEQ ID NO: 63);
f) DP II-25.2 (SEQ ID NO: 71); and
g) DF 1-22.2 (SEQ ID NO: 93);
all as shown in Figs 3 and 4; or

(ii) said peptide comprises a combination of regions selected from:

a) DP I-22.1 (SEQ ID NO: 29) and DP I-25.1 (SEQ ID NO: 35);
b) DP I-21.1 (SEQ ID NO: 27) and DP I-25.2 (SEQ ID NO: 36);
c) DP I-22.1 (SEQ ID NO: 29) and DP I-1 (SEQ ID NO: 9);
d) DP I-21.1 (SEQ ID NO: 27), DP I-22.1 (SEQ ID NO: 29), and DP I-25.2 (SEQ ID NO: 36):
e) DP I-21.2 (SEQ ID NO: 28). DP I-22.1 (SEQ ID NO: 29), and DP I-23.1 (SEQ ID NO:39);
f) DP I-1 (SEQ ID NO: 9), DP I-22.1 (SEQ ID NO: 29), and DP I-23.1 (SEQ ID NO: 33);
g) DP I-1 (SEQ ID NO: 9), DP I-23.1 (SEQ ID NO: 29), and DP I-25.2 (SEQ ID NO: 36);
h) DP I-21.1 (SEQ ID NO: 27). DP I-32.1 (SEQ ID NO: 29). DP I-23.1 (SEQ ID NO: 33), and DP I-25.2 (SEQ ID NO: 36):
i) DP I-21.2 (SEQ ID NO: 28). DP I-22.1 (SEQ ID NO: 29), and DP I-25.2 (SEQ ID NO: 36):
j) DP I-21.2 (SEQ ID NO: 27). DP I-22.1 (SEQ ID NO: 29). DP I-25.2 (SEQ ID NO: 36), and DP I-26. (SEQ ID NO: 37);
k) DF I-21.2 (SEQ ID NO: 91) and DF I-22.1 (SEQ ID NO: 92);
l) DF I.21.1 (SEQ ID NO: 90). DF I-22.1 (SEQ ID NO: 92), and DF I-25.1 (SEQ ID NO: 97);
m) DF I-21.2 (SEQ ID NO: 91), DF I-22.1 (SEQ ID NO: 92), and DF I-25.1 (SEQ ID NO: 97);
n) DF I-1 (SEQ ID NO: 72) and DF I-22.1 (SEQ ID NO: 92);
o) DF I-1 (SEQ ID NO: 72), DF I-22.1 (SEQ ID NO: 92), and DF I-25.1 (SEQ ID NO: 97):
p) DF I-22.1 (SEQ ID NO: 29), and DF I-25.1 (SEQ ID NO: 35):
q) DF I-21.1 (SEQ ID NO: 90), DF I-22.1 (SEQ ID NO: 92), and DF I-23.1 (SEQ ID NO:95);
r) DP I-21.1 (SEQ ID NO: 27), and DF I-22.1 (SEQ ID NO: 92);
s) DP I-1 (SEQ ID NO: 9). DP I-23.1 (SEQ ID NO: 33), DP I-25.1 (SEQ ID NO: 35), and DF I-1 (SEQ ID NO: 72):
t) DP I-1 (SEQ ID NO: 9), DP I-25.1 (SEQ ID NO: 35), DP I-23.1 (SEQ ID NO: 33), and DF I-31.2 (SEQ ID NO:91):
u) DP I.1 (SEQ ID NO: 9). DP I-25.1 (SEQ ID NO: 35). DP I-23.1 (SEQ ID NO: 33), and DF I-21.1 (SEQ ID NO: 90);
v) DP II-23 (SEQ ID NO: 63), and DP II-25.2 (SEQ ID NO:71):
w) DP II-22 (SEQ ID NO: 63), DP II-25.2 (SEQ ID NO: 71), and DP I-21.1 (SEQ ID NO: 27) and DP I-22.1 (SEQ ID NO: 29);
x) DP II-22 (SEQ ID NO: 63). DP II-25.2 (SEQ ID NO: 71), DP II-20.6 (SEQ ID NO: 56), DP I-22.1 (SEQ ID NO: 29), DP I-21.1 (SEQ ID NO: 27), and DP I-23.1 (SEQ ID NO: 33);
y) DP II-22 (SEQ ID NO: 63), DP II-25.2 (SEQ ID NO: 71), DP II-20.6 (SEQ ID NO: 56), DP I-21.1 (SEQ ID NO: 27), DP I-22.1 (SEQ ID NO: 29), and DP I-25.2 (SEQ ID NO:36):
z) DP II-22 (SEQ ID NO: 63), DP II-25.2 (SEQ ID NO: 71), DP I-21.1 (SEQ ID NO: 27), DP I-22.1 (SEQ ID NO: 29), and DP I-25.2 (SEQ ID NO: 36);
a') DP II.22 (SEQ ID NO: 63), DP II-25.2 (SEQ ID NO: 71), DP I-21.1 (SEQ ID NO: 27), DP I-22.1 (SEQ ID NO: 29), and DP I-23.1 (SEQ ID NO: 33);
b') DP II-22 (SEQ ID NO: 63), DP 11-25.2 (SEQ ID NO: 71), DP I-1 (SEQ ID NO: 9), and DP I-22.1 (SEQ ID NO: 29);
c') DF II-4.5 (SEQ ID NO: 107) and DF II-2 (SEQ ID NO: 104);
d') DF II-4.5 (SEQ ID NO: 107) and DF II-19.1 (SEQ ID NO: 114);
e') DF II-4.5 (SEQ ID NO: 107), DF II-2 (SEQ ID NO: 104), and DF II-19.1 (SEQ ID NO: 114);
f') DF II-4.5 (SEQ ID NO: 107). DF II-2 (SEQ ID NO: 104), and DF II-9 (SEQ ID NO: 86):
g') DF II-4.5 (SEQ ID NO: 107); and DF I-21.1 (SEQ ID NO: 90);
h') DF II-4.5 (SEQ ID NO: 107), DP II-22 (SEQ ID NO: 63), and DP II-25.2 (SEQ ID NO: 71); and
i') DF II-4.5 (SEQ ID NO: 107), DF II-2 (SEQ ID NO: 104), and DP II-22 (SEQ ID NO: 63) ; or

(iii) said peptide comprises the following combination of regions: DP I-21.2 (SEQ ID NO: 27); DP I-23.1 (SEQ ID NO: 33); DP I-26.1 (SEQ ID NO: 37); DP II-20.6 (SEQ ID NO: 56); DP II-22 (SEQ ID NO: 63); DP II-25.2

72

(SEQ ID NO: 71); and DF 1-22.2 (SEQ ID NO: 93); or

(iv) said peptide has a specific sequential arrangement of amino acid sequences, said arrangement of amino acid sequences being selected from:

    a) DP I-26.1, DP II-25.2, DF I-22, DP II-20.6 and DP I-21.2 respectively as shown in Fig. 25;

    b) DP II-25.2, DF I-22.2, DP I-23.1, DP II-22, DP I-26.1, DP I-21.2 and DP II-20.6 respectively as shown in Fig. 26; and

    c) DP II-25.2, DP I-21.1, DP I-26.1, DP II-22, DP II-20.2 and DF 1-22.2 respectively as shown in Fig. 27.

10. An isolated peptide of a Dermatophagoides group I or II protein allergen said peptide comprising at least one T cell epitope of said protein allergen, said peptide comprising an amino acid sequence selected from one of the following :

    a) DF I-1 (SEQ ID NO: 72):
    b) DF I-2.1 (SEQ ID NO: 73):
    c) DF I-3 (SEQ ID NO: 74);
    d) DF I-4 (SEQ ID NO: 75);
    e) DF I-11 (SEQ ID NO: 76);
    f) DF I-12 (SEQ ID NO: 77):
    g) DF I-5 (SEQ ID NO: 78);
    h) DF I-13 (SEQ ID NO: 79);
    i) DF I-14 (SEQ ID NO: 80):
    j) DF I-15 (SEQ ID NO: 81):
    k) DF I-6 (SEQ ID NO: 82);
    l) DF I-7 (SEQ ID NO: 83);
    m) DF I-8.1 (SEQ ID NO: 84);
    n) DF I-8 (SEQ ID NO: 85);
    o) DF I-9 (SEQ ID NO: 86);
    p) DF I-16 (SEQ ID NO: 87);
    q) DF I-10 (SEQ ID NO: 88);
    r) DF I-17 (SEQ ID NO: 89);
    s) DF I-21.1 (SEQ ID NO: 90);
    t) DF I-21.2 (SEQ ID NO: 91);
    u) DF I-22.1 (SEQ ID NO: 92);
    v) DF I-22.2 (SEQ ID NO: 93);
    w) DF I-22.4 (SEQ ID NO: 94):
    x) DF I-23.1 (SEQ ID NO: 95);
    y) DF 1-23.2 (SEQ ID NO: 96);
    z) DF I-25.1 (SEQ ID NO: 97);
    a') DF I-25.2 (SEQ ID NO: 98);
    b') DF I-26.1 (SEQ ID NO: 99);
    c') DF I-27.1 (SEQ ID NO: 100);
    d') DF I-28.1 (SEQ ID NO: 101);
    e') DF I-28.2 (SEQ ID NO: 102);
    f') DP II-20 (SEQ ID NO: 50);
    g') DP II-20.1 (SEQ ID NO: 51);
    h') DP II-20.2 (SEQ ID NO: 52);
    i-) DP II-20.3 (SEQ ID NO: 53);
    j') DP II-20.4 (SEQ ID NO: 54);
    k') DP II-20.5 (SEQ ID NO: 55);
    l') DP II 20.6 (SEQ ID NO:56);
    m') DP II-1 (SEQ ID NO: 41);
    o') DP II-2 (SEQ ID NO: 42);
    p') DP II-3.1 (SEQ ID NO: 43);
    q') DP II-4 (SEQ ID NO: 44);
    r') DP II-5 (SEQ ID NO: 45);
    s') DP II-6 (SEQ ID NO: 46);
    t') DP II-7 (SEQ ID NO: 47);
    u') DP II-8 (SEQ ID NO: 48);

v') DP II-9 (SEQ ID NO: 49);
w') DP II-1.1 (SEQ ID NO: 57);
x') DP II-1.2 (SEQ ID NO: 58);
y') DP II-2.1 (SEQ ID NO: 59);
z') DP II-2.2 (SEQ ID NO: 60);
a") DP II-23 (SEQ ID NO: 61);
b") DP II-21 (SEQ ID NO: 62);
c") DP II-22 (SEQ ID NO: 63);
d") DP II-25 (SEQ ID NO: 64);
e") DP II-26.1 (SEQ ID NO: 65);
f") DP II-23 (SEQ ID N.O: 66);
g") DP II-23.1 (SEQ ID NO: 67);
h") DP II-24 (SEQ ID NO: 68);
i") DP II.25 (SEQ ID NO: 69);
j") DP II-25.1 (SEQ ID NO: 70);
k") DP II-25.2 (SEQ ID NO: 71):
l") DF II-1 (SEQ ID NO: 103);
m") DF II-2 (SEQ ID NO: 104);
n") DF II-13.1 (SEQ ID NO: 105);
o") DF II-3.1 (SEQ ID NO: 106);
p") DF II-4.5 (SEQ ID NO: 107);
q") DF II-4.3 (SEQ ID NO: 108);
r") DF II-15 (SEQ ID NO: 109):
s") DF II-16 (SEQ ID NO: 110);
t") DF II-17 (SEQ ID NO: 111):
u") DF II-18 (SEQ ID NO: 112);
v") DF II-19 (SEQ ID NO: 113):
w") DF II-19.1 (SEQ ID NO: 114);
x") DF II-21 (SEQ ID NO: 115); and
y") DF II-22 (SEQ ID NO: 116).

optionally said peptide having immunoglobulin E production that is less than the amount of IgE production and/or IL/4 production stimulated by the native protein allergens.

11. An isolated peptide which is immunologically reactive with antibodies specific for or T cells reactive with a peptide of claim 10.

12. An isolated peptide of a Dermatophagoides group I or II protein allergen, said peptide selected from:

(i)

a) DF I-1 (SEQ ID NO: 72);
b) DF I-21.1 (SEQ ID NO: 90);
c) DF I-22.1 (SEQ ID NO: 92);
d) DF I-25.1 (SEQ ID NO: 97);
e) DF I-23.1 (SEQ ID NO: 95);
f) DF I-9 (SEQ ID NO: 86);
g) DF I-21.2 (SEQ ID NO: 91);
h) DP II-1 (SEQ ID NO: 41);
i) DP II-1.2 (SEQ ID NO: 58);
j) DP II-22 (SEQ ID NO: 63);
k) DP II-25.1 (SEQ ID NO: 70);
l) DP II-21 (SEQ ID NO: 62);
m) DP II-25 (SEQ ID NO: 69);
n) DP II-20.6 (SEQ ID NO: 56);
o) DP II-20 (SEQ ID NO: 50);
p) DF II-4.5 (SEQ ID NO: 107);
q) DF II-2 (SEQ ID NO: 104);

r) DF II-19.1 (SEQ ID NO:114);
s) DF II-17 (SEQ ID NO: 111);
t) DF II-15 (SEQ ID NO: 109).
u) DP II-35.2 (SEQ ID NO: 71); and
v) DF I-22.2 (SEQ ID NO: 92)

or (ii).

a) DF I-1 (SEQ ID NO: 72);
b) DF-I-2.1 (SEQ ID NO: 73);
c) DF I.3 (SEQ ID NO: 74);
d) DF I-4 (SEQ ID NO: 75);
e) DF I-11 (SEQ ID NO: 76);
f) DF I-12 (SEQ ID NO: 77);
g) DF I-5 (SEQ ID NO: 78);
h) DF I-13 (SEQ ID NO: 79);
i) DF I-14 (SEQ ID NO: 80);
j) DP I-15 (SEQ ID NO: 81);
k) DF I-6 (SEQ ID NO: 82);
l) DF I-7 (SEQ ID NO: 83);
m) DF I-8.1 (SEQ ID NO: 84);
n) DF I-8 (SEQ ID NO: 85);
o) DF I-9 (SEQ ID NO: 86);
p) DF I-16 (SEQ ID NO: 87);
q) DF I-10 (SEQ ID NO: 88);
r) DF I-17 (SEQ ID NO: 89);
s) DF I-21.1 (SEQ ID NO:90);
t) DF I-21.2 (SEQ ID NO: 91);
u) DF I-22.1 (SEQ ID NO: 92);
v) DF I-22.2 (SEQ ID NO: 93);
w) DF I-22.4 (SEQ ID NO: 94);
x) DF I-23. (SEQ ID NO: 95);
y) DF I-23.2 (SEQ ID NO: 96);
z) DF I-25.1 (SEQ ID NO: 97);
a') DF I.25.2 (SEQ ID NO: 98);
b') DF I-26.1 (SEQ ID NO: 99);
c') DF I-27.1 (SEQ ID NO:100);
d') DF I-28.1 (SEQ ID NO: 101);
e') DF I-28.2 (SEQ ID NO: 102);
f') DP II-20 (SEQ ID NO: 50);
g') DP II-20.1 (SEQ ID NO: 51);
h') DP II-20.2 (SEQ ID NO: 52);
i') DP II-20.3 (SEQ ID NO: 53);
j') DP II-20.4 (SEQ ID NO: 54);
k') DP II-20.5 (SEQ ID NO: 55);
l') DP II 20.6 (SEQ ID NO: 56);
m') DP II-1 (SEQ ID NO: 41);
o') DP II-2 (SEQ ID NO: 42);
p') DP II-3.1 (SEQ ID NO: 43);
q') DP II-4 (SEQ ID NO: 44);
r') DP II-5 (SEQ ID NO: 45);
s') DP II-6 (SEQ ID NO: 46);
t') DP II-7 (SEQ ID NO: 47):
u') DP II-8 (SEQ ID NO: 48);
v') DP II-9 (SEQ ID NO: 49);
w') DP II-1.1 (SEQ ID NO: 57);
x') DP II-1.2 (SEQ ID NO: 58);
y') DP II-2.1 (SEQ ID NO: 59):

z') DP II-2.2 (SEQ ID NO: 60);
a") DP II-2.3 (SEQ ID NO: 61):
b") DP II-21 (SEQ ID NO: 62);
c") DP II-22 (SEQ ID NO: 63);
d") DP II-26 (SEQ ID NO: 64);
e") DP II-26.1 (SEQ ID NO: 65);
f") DP II-23 (SEQ ID NO: 66);
g") DP II-23.1 (SEQ ID NO: 67);
h") DP 11-24 (SEQ ID NO: 68);
i") DP II-25 (SEQ ID NO: 69);
j") DP II-25.1 (SEQ ID NO: 70);
k") DP II-25.2 (SEQ ID NO: 71);
l") DF II-1 (SEQ ID NO: 103);
m") DF II-2 (SEQ ID NO: 104);
n") DF II-13.1 (SEQ ID NO: 105);
o") DF II-3.1 (SEQ ID NO: 106);
p") DF II-4.5 (SEQ ID NO: 107);
q") DF II-4.3 (SEQ ID NO: 108);
r") DF II-15 (SEQ ID NO: 109);
s") DF II-16 (SEQ ID NO: 110);
t") DF II-17 (SEQ ID NO: 111);
u") DF II-18 (SEQ ID NO: 112);
v") DF II-19 (SEQ ID NO: 113);
w") DF II-19.1 (SEQ ID NO: 114);
x") DF II-21 (SEQ ID NO: 115); and
y") DF II-22 (SEQ ID NO: 116).

13. A peptide of any one of claims 8 to 10 which either:

(a) does not bind immunoglobulin E specific for a protein allergen of the genus *Dermatophagoides* in at least 75% of individuals sensitive to the protein allergen, or if binding of the peptide to said immunoglobulin E occurs, such binding does not result in release of mediators from mast cells or basophils in a substantial percentage of individuals sensitive to the protein allergen; or
(b) binds immunoglobulin E to a lesser extent than the protein allergen from which the peptide is derived binds said immunoglobulin E.

14. A peptide of any one of claims 1 to 13, which modifies, in an individual sensitive to house dust mite to whom it is administered, the allergic response of the individual to a house dust mite allergen.

15. A peptide of any one of claims 1 to 14 wherein at least one of the amino acid residues which bind the MHC protein complex but is not essential for such binding is substituted, optionally said amino acid residue(s) being substituted by alanine, glutamic acid, or a methyl amino acid.

16. A peptide of claim 15 wherein at least one of the amino acid residues which is essential for binding to the MHC protein complex is substituted with a conservative amino acid residue.

17. A peptide of any one of claims 1 to 14 wherein at least one of the amino acid residues which binds to the T cell receptor, but is not essential for such binding, is substituted.

18. An isolated nucleic acid having a sequence encoding a peptide of any one of claims 1 to 17

19. An isolated peptide produced in a host cell transformed with the nucleic acid of claim 18.

20. A therapeutic composition comprising a peptide according to any one of claims 1 to 17, and a pharmaceutically acceptable carrier or diluent.

21. A therapeutic composition according to claim 20 comprising at least two peptides, said peptides each comprising at least one T cell epitope of a Dermatophagoides group I or II protein allergen, said peptides derived from the same or from different Dermatophagoides group I or II protein allergens.

**22.** A composition of claim 20 or 21 wherein either:

(a) said peptides are selected from:

a) DP I-21.1 (SEQ ID NO:27);
b) DP I-21.2 (SEQ ID NO:28);
c) DP I-22.1 (SEQ ID NO: 29);
d) DP 1.22.2 (SEQ ID NO: 30);
e) DP I-22.3 (SEQ ID NO: 31);
f) DP I-22.4 (SEQ ID NO: 32);
g) DP I-23.1 (SEQ ID NO: 33);
h) DP I-23.2 (SEQ ID NO: 34);
i) DP I-25.1 (SEQ ID NO: 35);
j) DP I-25.2 (SEQ ID NO: 36);
k) DP I-26.1 (SEQ ID NO: 37);
l) DP I-27.1 (SEQ ID NO: 38);
m) DP I-28.1 (SEQ ID NO: 39);
n) DP I-28.2 (SEQ ID NO:40);
o) DP I-1 (SEQ ID NO: 9);
p) DF I-.1 (SEQ ID NO: 72);
q) DF I-21.1 (SEQ ID NO: 90);
r) DF I-212 (SEQ ID NO: 91);
s) DF I-22.1 (SEQ ID NO: 92);
t) DF I22.2 (SEQ ID NO: 93);
u) DF 1-22.4 (SEQ ID NO: 94);
v) DF I-23.1 (SEQ ID NO-95);
w) DF 1-23.2 (SEQ ID NO: 96);
x) DF I-25.1 (SEQ ID NO: 97);
y) DF 1-25.2 (SEQ ID NO: 98);
z) DF I-26.1 (SEQ.ID NO: 99);
a') DF I-27.1 (SEQ ID NO: 100);
b') DF 1-28.1 (SEQ ID NO: 101);
c') DF I-28.2 (SEQ ID NO: 102);
d') DP II-20 (SEQ ID NO: 50);
e') DP II-20.1 (SEQ ID NO: 51);
f') DP II-20.2 (SEQ ID NO: 52);
g') DP II-20.3 (SEQ ID NO: 53);
h') DP II-20.4 (SEQ ID NO: 54);
i') DP II-20.5 (SEQ ID NO: 55);
j') DP II 20.6 (SEQ ID NO: 56);
k') DP II-1 (SEQ ID NO: 41);
l') DP II-1.1 (SEQ ID NO: 57);
m') DP II-1.2 (SEQ ID NO: 58);
n') DP II-2.1 (SEQ ID NO: 59);
o') DP II-2.2 (SEQ ID NO: 60):
p') DP II-2.3 (SEQ ID NO: 61);
q') DP II-21 (SEQ ID NO: 62);
r') DP II-22 (SEQ ID NO: 63);
s') DP II-26 (SEQ ID NO: 64);
t') DP II-26.1 (SEQ ID NO: 65):
u') DP II-23 (SEQ ID NO: 66);
v') DP II-23.1 (SEQ ID NO: 67);
w') DP II-24 (SEQ ID NO: 68);
x') DP II-25 (SEQ ID NO: 69);
y') DP II-25.1 (SEQ ID NO: 70);
z') DP II-25.2 (SEQ ID NO: 71);

a") DF II-1 (SEQ ID NO: 103)

b") DF n-2 (SEQ ID NO: 104);

c") DF II-13.1 (SEQ ID NO: 105);

d") DF II-3.1 (SEQ ID NO: 106):

c") DF II-4.5 (SEQ ID NO: 107);

f") DF II-4.3 (SEQ ID NO: 108):

g") DF II-15 (SEQ ID NO: 109);

h") DF II-16 (SEQ ID NO: 110):

i") DF II-17 (SEQ ID NO: 111);

j") DF II-18 (SEQ ID NO: 112);

k") DF II-19 (SEQ ID NO: 113);

l") DF II-19.1 (SEQ ID NO: 114)

m") DF II-21 (SEQ ID NO: 115); and

n") DF II-22 (SEQ ID NO: 116).

wherein said composition comprises a sufficient percentage of the T cell epitopes of at least one protein allergen such that upon administration of the composition to an individual sensitive to a house dust mite allergen, T cells of the individual are tolerized to said at least one protein allergen; or

(b) said composition comprises a combination of peptides selected from:

a) DP I-23.1 (SEQ ID NO: 29) and DP I-25.1 (SEQ ID NO: 35):

b) DP I-21.1 (SEQ ID NO: 27) and DP I-25.2 (SEQ ID NO: 36);

c) DP I-22.1 (SEQ ID NO: 29) and DP I-1 (SEQ ID NO: 9);

d) DP I-21.1 (SEQ ID NO: 27). DP I-22.1 (SEQ ID NO: 29), and DP I-25.2 (SEQ ID NO: 36):

e) DP I-21.2 (SEQ ID NO: 28). DP I-22.1 (SEQ ID NO: 29), and DP 1-23.1 (SEQ ID NO: 39):

f) DP I-1 (SEQ ID NO: 9), DP I-22.1 (SEQ ID NO: 29), and DP I-23.1 (SEQ ID NO: 33);

g) DP I-1 (SEQ ID NO: 9), DP I-22.1 (SEQ ID NO: 29), and DP I-25.2 (SEQ ID NO: 36);

h) DP I-21.1 (SEQ ID NO: 27). DP I-22.1 (SEQ ID NO: 29), DP I-23.1 (SEQ ID NO: 33), and DP I-25.2 (SEQ ID NO: 36);

i) DP I-21.2 (SEQ ID NO: 28), DP I-22.1 (SEQ ID NO: 29), and DP I-25.2 (SEQ ID NO: 36);

j) DP I-21.2 (SEQ ID NO: 27), DP I-22.1 (SEQ ID NO: 29), DP I-25.2 (SEQ ID NO: 36), and DP I-26.1 (SEQ ID NO: 37);

k) DF 1-21.2 (SEQ ID NO: 91) and DF I-22.1 (SEQ ID NO: 92):

l) DF I-21.1 (SEQ ID NO: 90), DF I-22.1 (SEQ ID NO: 92), and DF I-25.1 (SEQ ID NO: 97);

m) DF I-21.2 (SEQ ID NO: 91), DF I-22.1 (SEQ ID NO: 92), and DF I-25.1 (SEQ ID NO: 97);

n) DF I-1 (SEQ ID NO: 72) and DF I-22.1 (SEQ ID NO: 92);

o) DF I-1 (SEQ ID NO: 72), DF I-22.1 (SEQ ID NO: 92), and DF I-25.1 (SEQ ID NO: 97):

p) DF I-22.1 (SEQ ID NO: 29), and DF I-25.1 (SEQ ID NO: 35);

q) DF I-21.1 (SEQ ID NO: 90), DF I-22.1 (SEQ ID NO: 92), and DF I-23.1 (SEQ ID NO: 95);

r) DP I-21.1 (SEQ ID NO: 27), and DF I-22.1 (SEQ ID NO: 92);

s) DP I-1 (SEQ ID NO: 9). DP I-23.1 (SEQ ID NO: 33), DP I-25.1 (SEQ ID NO: 35), and DF I-1 (SEQ ID NO: 72):

t) DP I-1 (SEQ ID NO: 9). DP I-25.1 (SEQ ID NO: 35), DP I-23.1 (SEQ ID NO: 33), and DF I-21.2 (SEQ ID NO: 91);

u) DP I-1 (SEQ ID NO: 9), DP I-25.1 (SEQ ID NO: 35), DP I-23.1 (SEQ ID NO: 33), and DF I-21.1 (SEQ ID NO: 90);

v) DP II-22 (SEQ ID NO: 63), and DP II-25.2 (SEQ ID NO: 71);

w) DP II-22 (SEQ ID NO: 63), DP II-25.2 (SEQ ID NO: 71), and DP I-21.1 (SEQ ID NO: 27) and DP I-22.1 (SEQ ID NO: 29);

x) DP II-22 (SEQ ID NO: 63), DP II-25.2 (SEQ ID NO: 71), DP II-20.6 (SEQ ID NO: 56), DP I-22.1 (SEQ ID NO: 29), DP I-21.1. (SEQ ID NO: 27), and DP I-23.1 (SEQ ID NO: 33);

y) DP II-22 (SEQ ID NO: 63), DP II-25.2 (SEQ ID NO: 71), DP II.20.6 (SEQ ID NO: 56). DP I-21.1 (SEQ ID NO: 27), DP I-22.1 (SEQ ID NO: 29), and DP I-25.2 (SEQ ID NO:36);

z) DP II-22 (SEQ ID NO: 63), DP II-25.2 (SEQ ID NO: 71). DP I-21.1 (SEQ ID NO: 27). DP I-22.1 (SEQ ID NO: 29), and DP I-25.2 (SEQ ID NO: 36);

a') DP II-22 (SEQ ID NO: 63), DP II-25.2 (SEQ ID NO: 71), DP I-21.1 (SEQ ID NO: 27), DP I-22.1 (SEQ ID NO: 29), and DP I-23.1 (SEQ ID NO: 33);

b') DP II-22 (SEQ ID NO: 63). DP II-25.2 (SEQ ID NO: 71). DP I-1 (SEQ ID NO: 9). and DP I-33.1 (SEQ ID NO: 29);

c') DF II-4.5 (SEQ ID NO: 107) and DF II-2 (SEQ.ID NO: 104);

d') DF II-4.5 (SEQ ID NO: 107) and DF II-19.1 (SEQ ID NO: 114):

e') DF II-4.5 (SEQ ID NO: 107). DF II-2 (SEQ ID NO: 104), and DF II-19.1 (SEQ ID NO: 11.4):

f') DF II-4.5 (SEQ ID NO: 107), DF II-2 (SEQ ID NO: 104), and DF II-9 (SEQ ID NO: 86):

g') DF II-4.5 (SEQ ID NO: 107): and DF I-21.1 (SEQ ID NO:90);

h') DF II-4.5 (SEQ ID NO: 107), DP II.22

(SEQ ID NO: 63), and DP II-25.2 (SEQ ID NO: 71): and

i') DF II-4.5 (SEQ ID NO: 107). DF II.2 (SEQ ID NO: 104), and DP II-22 (SEQ ID NO: 63).

**23.** A composition of any one of claims 20 to 22 comprising the following combination of peptides: DP I-21.2 (SEQ ID NO: 27); DP I-23.1 (SEQ ID NO: 33); DP I-26.1 (SEQ ID NO: 37); DP II-20.6 (SEQ ID NO: 56); DP 11-22 (SEQ ID NO: 63); DP II-25.2 (SEQ ID NO: 71); and DF 1-22.2 (SEQ ID NO: 93).

**24.** The use of a peptide according to any one of claims 1 to 17 in the manufacture of a medicament for treating sensitivity to house dust mite.

**25.** The use of claim 24 in which said medicament is adapted for administering simultaneously or sequentially at least two different peptides/portions.

**26.** A method of detecting sensitivity to house dust mite in an individual, comprising combining a blood sample obtained from the individual with at least one of a peptide according to any one of claims 1 to 17 under conditions appropriate for binding of blood components therewith, and determining the extent to which such binding occurs as indicative of sensitivity in the individual to house dust mite.

**27.** A method of claim 26 wherein the extent to which binding occurs is determined by assessing T cell function, T cell proliferation or a combination thereof.

**Patentansprüche**

**1.** Isoliertes Peptid eines Proteinallergens der Dermatophagoides Gruppe I oder II, wobei dieses Peptid mindestens ein T-Zell-Epitop von diesem Proteinallergen umfasst, gegebenenfalls mindestens zwei T-Zell-Epitope, wobei diese Peptide einen Positivitätsindex von mindestens etwa 100 und einen mittleren T-Zell-Stimulationsindex von mindestens 2,0 aufweisen, bestimmt an einer Population von gegenüber dem Proteinallergen empfindlichen Individuen, wobei die Population gegebenenfalls aus mindestens 9 Individuen besteht, zum Beispiel mindestens 29 Individuen.

**2.** Isoliertes Peptid nach Anspruch 1, das einen Positivitätsindex von mindestens etwa 150 und einen mittleren T-Zell-Stimulationsindex von mindestens etwa 4,0 aufweist, bestimmt an einer Population von gegenüber dem Proteinallergen empfindlichen Individuen.

**3.** Isoliertes Peptid nach Anspruch 1 oder 2, ausgewählt aus entweder

(a) DP I-1 (SEQ ID NO: 9); DP I-21.1 (SEQ ID NO: 27); DP 1-21.2 (SEQ ID NO: 28); DP I-23.1 (SEQ ID NO: 33); DP 1-23.2 (SEQ ID NO: 34); DP 1-25.2 (SEQ ID NO: 36); und DP I-26.1 (SEQ ID NO: 37); oder

(b) DP 1-21.2, DP 1-23.1, DP 1-26.1, DP II-20.6, DP II-22, DP II-25.2 und DF 1-22.2 wie in Fig. 3 und 4 dargestellt.

**4.** Isoliertes Peptid nach einem der Ansprüche 1 bis 3, das entweder:

(a) in mindestens 75 % der gegenüber dem Proteinallergen empfindlichen Individuen kein für ein Proteinallergen der Gattung Dermatophagoides spezifisches Immunglobulin E bindet, oder wenn Bindung des Peptids an dieses Immunglobulin E auftritt, diese Bindung bei mindestens 75 % der gegenüber dem Proteinallergen empfindlichen Individuen nicht zu einer Freisetzung von Mediatoren aus Mastzellen oder Basophilen führt; oder

(b) Immunglobulin E in einem geringeren Ausmaß bindet als das Proteinallergen, aus dem das Peptid stammt, dieses Immunglobulin E bindet.

**5.** Isoliertes Peptid nach einem der Ansprüche 1 bis 4, wobei dieses Peptid ausgewählt wird aus:

a) DP I-1 (SEQ ID NO: 9);

b) DP 1-2 (SEQ ID NO: 10);
c) DP 1-3 (SEQ ID NO: 11);
d) DP 1-4 (SEQ ID NO: 12);
e) DP I-11.1 (SEQ ID NO: 13);
f) DP I-12.1 (SEQ ID NO: 14);
h) DP I-13 (SEQ ID NO: 17);
i) DP I-14 (SEQ ID NO: 18);
j) DP I-15 (SEQ ID NO: 19);
k) DP I-6.1 (SEQ ID NO: 20);
l) DP I-7.1 (SEQ ID NO: 21);
m) DP 1-8 (SEQ ID NO: 22);
n) DP 1-9 (SEQ ID NO: 23);
o) DP I-16 (SEQ ID NO: 24);
p) DP I-10 (SEQ ID NO: 25);
q) DP I-17 (SEQ ID NO: 26);
r) DP I-21.1 (SEQ ID NO: 27);
s) DP I-21.2 (SEQ ID NO: 28);
t) DP I-22.1 (SEQ ID NO: 29);
u) DP 1-22.2 (SEQ ID NO: 30);
v) DP 1-22.3 (SEQ ID NO: 31);
w) DP 1-22.4 (SEQ ID NO: 32);
x) DP I-23.1 (SEQ ID NO: 33);
y) DP 1-23.2 (SEQ ID NO: 34);
z) DP I-25.1 (SEQ ID NO: 35);
a') DP 1-25.2 (SEQ ID NO: 36);
b') DP I-26.1 (SEQ ID NO: 37);
c') DP I-27.1 (SEQ ID NO: 38);
d') DP I-28.1 (SEQ ID NO: 39); und
e') DP 1-28.2 (SEQ ID NO: 40),

wobei dieses Peptid einen mittleren T-Zell-Stimulationsindex gleich oder größer als den mittleren T-Zell-Stimulationsindex des genannten Peptids wie in Fig. 5 und Fig. 13 dargestellt aufweist, gegebenenfalls einen mittleren T-Zell-Stimulationsindex von mindestens 4,0.

6. Isoliertes Peptid nach einem der Ansprüche 1 bis 5 mit einer Aminosäuresequenz, die einer Aminosäuresequenz eines Peptids entspricht, das aus den folgenden ausgewählt wird

a) DP I-1 (SEQ ID NO: 9);
b) DP 1-2 (SEQ ID NO: 10);
c) DP 1-3 (SEQ ID NO: 11);
d) DP 1-4 (SEQ ID NO: 12);
e) DP I-11.1 (SEQ ID NO: 13);
f) DP I-12.1 (SEQ ID NO: 14);
h) DP I-13 (SEQ ID NO: 17);
i) DP I-14 (SEQ ID NO: 18);
j) DP I-15 (SEQ ID NO: 19);
k) DP I-6.1 (SEQ ID NO: 20);
l) DP I-7.1 (SEQ ID NO: 21);
m) DP 1-8 (SEQ ID NO: 22);
n) DP 1-9 (SEQ ID NO: 23);
o) DP I-16 (SEQ ID NO: 24);
p) DP I-10 (SEQ ID NO: 25);
q) DP I-17 (SEQ ID NO: 26);
r) DP I-21.1 (SEQ ID NO: 27);
s) DP I-21.2 (SEQ ID NO: 28);
t) DP I-22.1 (SEQ ID NO: 29);
u) DP 1-22.2 (SEQ ID NO: 30);
v) DP 1-22.3 (SEQ ID NO: 31);

w) DP 1-22.4 (SEQ ID NO: 32);
x) DP I-23.1 (SEQ ID NO: 33);
y) DP I-23.2 (SEQ ID NO: 34);
z) DP 1-25.1 (SEQ ID NO: 35);
a') DP 1-25.2 (SEQ ID NO: 36);
b') DP I-26.1 (SEQ ID NO: 37);
c') DP I-27.1 (SEQ ID NO: 38);
d') DP I-28.1 (SEQ ID NO: 39);
e') DP I-28.2 (SEQ ID NO: 40);
f) DP I-5.1 (SEQ ID NO: 16); und
g') DP I-23.1.1;
h') DP I-23.1.2);
i') DP I-223.1.3); und
j') DP I-23.1.4)

wie in Fig. 28 gezeigt.

7. Peptid nach einem der Ansprüche 1 bis 6, das in mindestens 75 % der gegenüber Der pI empfindlichen Individuen kein für Der pI spezifisches Immunglobulin E bindet, oder wenn Bindung des Peptids an dieses Immunglobulin E auftritt, diese Bindung bei mindestens 75 % der gegenüber Der pI empfindlichen Individuen nicht zu einer Freisetzung von Mediatoren aus Mastzellen oder Basophilen führt.

8. Isoliertes Peptid nach einem der Ansprüche 1 bis 7, enthaltend mindestens zwei Regionen, wobei jede Region mindestens ein T-Zell-Epitop eines Proteinallergens der Dermatophagoides Gruppe I oder II enthält, wobei diese Regionen vom gleichen oder von unterschiedlichen Proteinallergenen der Dermatophagoides Gruppe I oder II abstammen und wobei diese Regionen jeweils eine Aminosäuresequenz umfassen, die ausgewählt wird aus:

a) DP I-21.1 (SEQ ID NO: 27);
b) DP I-21.2 (SEQ ID NO: 28);
c) DP I-22.1 (SEQ ID NO: 29);
d) DP I-22.2 (SEQ ID NO: 30);
e) DP I-22.3 (SEQ ID NO: 31);
f) DP I-22.4 (SEQ ID NO: 32);
g) DP I-23.1 (SEQ ID NO: 33);
h) DP 1-23.2 (SEQ ID NO: 34);
i) DP I-25.1 (SEQ ID NO: 35);
j) DP I-25.2 (SEQ ID NO: 36);
k) DP I-26.1 (SEQ ID NO: 37);
l) DP I-27.1 (SEQ ID NO: 38);
m) DP I-28.1 (SEQ ID NO: 39);
n) DP 1-28.2 (SEQ ID NO: 40);
o) DP I-1 (SEQ ID NO: 9);
p) DF I-1 (SEQ ID NO: 72);
q) DF I-21.1 (SEQ ID NO: 90);
r) DF I-21.2 (SEQ ID NO: 91);
s) DF I-22.1 (SEQ ID NO: 92);
t) DF 1-22.2 (SEQ ID NO: 93);
u) DF 1-22.4 (SEQ ID NO: 94);
v) DF I-23.1 (SEQ ID NO: 95);
w) DF I-23.2 (SEQ ID NO: 96);
x) DF I-25.1 (SEQ ID NO: 97);
y) DF 1-25.2 (SEQ ID NO: 98);
z) DF I-26.1 (SEQ ID NO: 99);
a') DF I-27.1 (SEQ ID NO: 100);
b') DF I-28.1 (SEQ ID NO: 101);
c') DF 1-28.2 (SEQ ID NO: 102);
d') DP II-20 (SEQ ID NO: 50);
e') DP II-20.1 (SEQ ID NO: 51);

f) DP II-20.2 (SEQ ID NO: 52);
g') DP II-20.3 (SEQ ID NO: 53);
h') DP II-20.4 (SEQ ID NO: 54);
i') DP II-20.5 (SEQ ID NO: 55);
j') DP II-20.6 (SEQ ID NO: 56);
k') DP II-1 (SEQ ID NO: 41);
1') DP II-1.1 (SEQ ID NO: 57);
m') DP II-1.2 (SEQ ID NO: 58);
n') DP II-2.1 (SEQ ID NO: 59);
o') DP II-2.2 (SEQ ID NO: 60);
p') DP II-2.3 (SEQ ID NO: 61);
q') DP II-21 (SEQ ID NO: 62);
r') DP II-22 (SEQ ID NO: 63);
s') DP II-26 (SEQ ID NO: 64);
t') DP II-26.1 (SEQ ID NO: 65);
u') DP II-23 (SEQ ID NO: 66);
v') DP II-23.1 (SEQ ID NO: 67);
w') DP II-24 (SEQ ID NO: 68);
x') DP II-25 (SEQ ID NO: 69);
y') DP II-25.1 (SEQ ID NO: 70);
z') DP II-25.2 (SEQ ID NO: 71);
a") DF II-1 (SEQ ID NO: 103)
b") DF II-2 (SEQ ID NO: 104);
c") DF II-13.1 (SEQ ID NO: 105);
d") DF II-3.1 (SEQ ID NO: 106);
e") DF II-4.5 (SEQ ID NO: 107);
f') DF II-4.3 (SEQ ID NO: 108);
g") DF II-1 (SEQ ID NO: 109);
h") DF II-16 (SEQ ID NO: 110);
i") DF II-17 (SEQ ID NO: 111);
j") DF II-18 (SEQ ID NO: 112);
k") DF II-19 (SEQ ID NO: 113);
l") DF II-19.1 (SEQ ID NO: 114);
m") DF II-21 (SEQ ID NO: 115); und
n") DF II-22 (SEQ ID NO: 116).

**9.** Isoliertes Peptid nach Anspruch 8, wobei entweder

(i) diese Regionen eine Aminosäuresequenz enthalten, die ausgewählt wird aus:

a) DP I-21.2 (SEQ ID NO: 27);
b) DP I-23.1 (SEQ ID NO: 33);
c) DP I-26.1 (SEQ ID NO: 37);
d) DP II-20.6 (SEQ ID NO: 56);
e) DP II-22 (SEQ ID NO: 63);
f) DP II-25.2 (SEQ ID NO: 71); und
g) DP I-22.2 (SEQ ID NO: 93);

alle wie in Fig. 3 und 4 dargestellt; oder
(ii) dieses Peptid eine Kombination aus Regionen enthält, die ausgewählt werden aus:

a) DP I-22.1 (SEQ ID NO: 29) und DP I-25.1 (SEQ ID NO: 35);
b) DP I-21.1 (SEQ ID NO: 27) und DP I-25.2 (SEQ ID NO: 36);
c) DP I-22.1 (SEQ ID NO: 29) und DP I-1 (SEQ ID NO: 9);
d) DP I-21.1 (SEQ ID NO: 27), DP I-22.1 (SEQ ID NO: 29), und DP I-25.2 (SEQ ID NO: 36);
e) DP I-21.2 (SEQ ID NO: 28), DP I-22.1 (SEQ ID NO: 29), und-DP I-23.1 (SEQ ID NO: 39);
f) DP I-1 (SEQ ID NO: 9), DP I-22.1 (SEQ ID NO: 29), und DP I-23.1 (SEQ ID NO: 33);
g) DP I-1 (SEQ ID NO: 9), DP I-22.1 (SEQ ID NO: 29), und DP 1-25.2 (SEQ ID NO: 36);

h) DP I-21.1 (SEQ ID NO: 27), DP I-22.1 (SEQ ID NO: 29), DP I-23.1 (SEQ ID NO: 33), und DP 1-25.2 (SEQ ID NO: 36);

i) DP I-21.2 (SEQ ID NO: 28), DP I-22.1 (SEQ ID NO: 29), und DP 1-25.2 (SEQ ID NO: 36);

j) DP I-21.2 (SEQ ID NO: 27), DP I-22.1 (SEQ ID NO: 29), DP I-25.2 (SEQ ID NO: 36), und DP I-26.1 (SEQ ID NO: 37);

k) DF I-21.2 (SEQ ID NO: 91) und DF I-22.1 (SEQ ID NO: 92);

l) DF I-21.1 (SEQ ID NO: 90), DF I-22.1 (SEQ ID NO: 92), und DF I-25.1 (SEQ ID NO: 97);

m) DF I-21.2 (SEQ ID NO: 91), DF I-22.1 (SEQ ID NO: 92), und DF I-25.1 (SEQ ID NO: 97);

n) DF I-1 (SEQ ID NO: 72) und DF I-22.1 (SEQ ID NO: 92);

o) DF I-1 (SEQ ID NO: 72), DF I-22.1 (SEQ ID NO: 92), und DF I-25.1 (SEQ ID NO: 97);

p) DF I-22.1 (SEQ ID NO: 29), und DF I-25.1 (SEQ ID NO: 35);

q) DF I-21.1 (SEQ ID NO: 90), DF I-22.1 (SEQ ID NO: 92), und DF I-23.1 (SEQ ID NO: 95);

r) DF I-21.1 (SEQ ID NO: 27), und DF I-22.1 (SEQ ID NO: 92);

s) DP I-1 (SEQ ID NO: 9), DP I-23.1 (SEQ ID NO: 33), DP I-25.1 (SEQ ID NO: 35), und DF I-1 (SEQ ID NO: 72);

t) DP I-1 (SEQ ID NO: 9), DP I-25.1 (SEQ ID NO: 35), DP I-23.1 (SEQ ID NO: 33), und DF I-21.2 (SEQ ID NO: 91);

u) DP I-1 (SEQ ID NO: 9), DP I-25.1 (SEQ ID NO: 35), DP I-23.1 (SEQ ID NO: 33), und DF I-21.1 (SEQ ID NO: 90);

v) DP II-22 (SEQ ID NO: 63), und DP II-25.2 (SEQ ID NO: 71);

w) DP II-22 (SEQ ID NO: 63), DP II-25.2 (SEQ ID NO: 71), und DP I-21.1 1 (SEQ ID NO: 27) und DP I-22.1 (SEQ ID NO: 29);

x) DP II-22 (SEQ ID NO: 63), DP II-25.2 (SEQ ID NO: 71), DP II-20.6 (SEQ ID NO: 56), DP I-22.1 (SEQ ID NO: 29), DP I-21.1 (SEQ ID NO: 27), und DP I-23.1 (SEQ ID NO: 33);

y) DP II-22 (SEQ ID NO: 63), DP II-25.2 (SEQ ID NO: 71), DP II-20.6 (SEQ ID NO: 56), DP I-21.1 (SEQ ID NO: 27), DP I-22.1 (SEQ ID NO: 29), und DP 1-25.2 (SEQ ID NO: 36);

z) DP II-22 (SEQ ID NO: 63), DP II-25.2 (SEQ ID NO: 71), DP I-21.1 (SEQ ID NO: 27), DP I-22.1 (SEQ ID NO: 29), und DP I- 25.2 (SEQ ID NO: 36);

a') DP II-22 (SEQ ID NO: 63), DP II-25.2 (SEQ ID NO: 71), DP I-21.1 (SEQ ID NO: 27), DP I-22.1 (SEQ ID NO: 29), und DP I-23.1 (SEQ ID NO: 33);

b') DP II-22 (SEQ ID NO: 63), DP II-25.2 (SEQ ID NO: 71), DP I-1 (SEQ ID NO: 9), und DP I-22.1 (SEQ ID NO: 29);

c') DF II4.5 (SEQ ID NO: 107) und DF II-2 (SEQ ID NO: 104);

d') DF II-4.5 (SEQ ID NO: 107) und DF II-19.1 (SEQ ID NO: 114);

e') DF II-4.5 (SEQ ID NO: 107), DF II-2 (SEQ ID NO: 104), und DF II-19.1 (SEQ ID NO: 114);

f') DF II-4.5 (SEQ ID NO: 107), DF II-2 (SEQ ID NO: 104), und DF II-9 (SEQ ID NO: 86);

g') DF II-4.5 (SEQ ID NO: 107); und DF I-21.1 (SEQ ID NO: 90);

h') DF II-4.5 (SEQ ID NO: 107), DP II-22 (SEQ ID NO: 63), und DP II-25.2 (SEQ ID NO: 71); und

i') DF II-4.5 (SEQ ID NO: 107), DF II-2 (SEQ ID NO: 104), und DP II-22 (SEQ ID NO: 63);

(iii) genanntes Peptid die folgenden Kombinationen von Regionen enthält: DP I-21.2 (SEQ ID NO: 27); DP I-23.1 (SEQ ID NO: 33); DP I-26.1 (SEQ ID NO: 37); DP II-20.6 (SEQ ID NO: 56); DP II-22 (SEQ ID NO: 63); DP II-25.2 (SEQ ID NO: 71); und DF 1-22.2 (SEQ ID NO: 93); oder

(iv) dieses Peptid eine spezifische aufeinander folgende Anordnung von Aminosäuresequenzen aufweist, wobei diese Anordnung von Aminosäuresequenzen ausgewählt wird aus:

a) DP I-26.1, DP II-25.2, DP 1-22, DP II-20.6 bzw. DP I-21.2, wie in Fig. 25 dargestellt;

b) DP II-25.2, DP 1-22.2, DP I-23.1, DP II-22, DP I-26.1, DP I-21.2 bzw. DP 11-26.6, wie in Fig. 26 dargestellt; und

c) DP II-25.2, DP I-21.1, DP I-26.1, DP II-22, DP II-20.2 bzw. DF 1-22.2, wie in Fig. 27 dargestellt.

**10.** Isoliertes Peptid des Proteinallergens der Dermatophagoides Gruppe I oder II, wobei dieses Peptid mindestens ein T-Zell-Epitop dieses Proteinallergens enthält und wobei dieses Peptid eine Aminosäuresequenz enthält, die aus einer der folgenden ausgewählt wird:

a) DF I-1 (SEQ ID NO: 72);

b) DF I-2.1 (SEQ ID NO: 73);

c) DF 1-3 (SEQ ID NO: 74);

d) DF I-4 (SEQ ID NO: 75);

e) DF I-11 (SEQ ID NO: 76);
f) DF 1-12 (SEQ ID NO: 77);
g) DF 1-5 (SEQ ID NO: 78);
h) DF I-13 (SEQ ID NO: 79);
i) DF 1-14 (SEQ ID NO: 80);
j) DF I-15 (SEQ ID NO: 81);
k) DF 1-6 (SEQ ID NO: 82);
l) DF 1-7 (SEQ ID NO: 83);
m) DF I-8.1 (SEQ ID NO: 84);
n) DF 1-8 (SEQ ID NO: 85);
o) DF 1-9 (SEQ ID NO: 86);
p) DF 1-16 (SEQ ID NO: 87);
q) DF 1-10 (SEQ ID NO: 88);
r) DF 1-17 (SEQ ID NO: 89);
s) DF I-21.1 (SEQ ID NO: 90);
t) DF I-21.2 (SEQ ID NO: 91);
u) DF I-22.1 (SEQ ID NO: 92);
v) DF 1-22.2 (SEQ ID NO: 93);
w) DF 1-22.4 (SEQ ID NO: 94);
x) DF I-23.1 (SEQ ID NO: 95);
y) DF I-23.2 (SEQ ID NO: 96);
z) DF I-25.1 (SEQ ID NO: 97);
a') DF I-25.2 (SEQ ID NO: 98);
b') DF I-26.1 (SEQ ID NO: 99);
c') DF I-27.1 (SEQ ID NO: 100);
d') DF I-28.1 (SEQ ID NO: 101);
e') DF 1-28.2 (SEQ ID NO: 102);
f') DP II-20 (SEQ ID NO: 50);
g') DP II-20.1 (SEQ ID NO: 51);
h') DP II-20.2 (SEQ ID NO: 52);
i') DP II-20.3 (SEQ ID NO: 53);
j') DP II-20.4 (SEQ ID NO: 54);
k') DP II-20.5 (SEQ ID NO: 55);
l') DP II 20.6 (SEQ ID NO: 56);
m') DP II-1 (SEQ ID NO: 41);
o') DP II-2 (SEQ ID NO: 42);
p') DP II-3.1 (SEQ ID NO: 43);
q') DP II-4 (SEQ ID NO: 44);
r') DP II-5 (SEQ ID NO: 45);
s') DP II-6 (SEQ ID NO: 46);
t') DP II-7 (SEQ ID NO: 47);
u') DP II-8 (SEQ ID NO: 48);
v') DP II-9 (SEQ ID NO: 49);
w') DP II-1.1 (SEQ ID NO: 57);
x') DP II-1.2 (SEQ ID NO: 58);
y') DP II-2.1 (SEQ ID NO: 59);
z') DP II-2.2 (SEQ ID NO: 60);
a") DP II-2.3 (SEQ ID NO: 61);
b") DP II-21 (SEQ ID NO: 62);
c") DP II-22 (SEQ ID NO: 63);
d") DP II-26 (SEQ ID NO: 64);
e") DP II-26.1 (SEQ ID NO: 65);
f') DP II-23 (SEQ ID NO: 66);
g") DP II-23.1 (SEQ ID NO: 67);
h") DP II-24 (SEQ ID NO: 68);
i") DP II-25 (SEQ ID NO: 69);
j") DP II-25.1 (SEQ ID NO: 70);
k") DP II-25.2 (SEQ ID NO: 71);

l") DF II-1 (SEQ ID NO: 103);
m") DF II-2 (SEQ ID NO: 104);
n") DF II-13.1 (SEQ ID NO: 105);
o") DF II-3.1 (SEQ ID NO: 106);
p") DF II-4.5 (SEQ ID NO: 107);
q") DF II-4.3 (SEQ ID NO: 108);
r") DF II-15 (SEQ ID NO: 109);
s") DF II-16 (SEQ ID NO: 110);
t") DF II-17 (SEQ ID NO: 111);
u") DF II-18 (SEQ ID NO: 112);
v") DF II-19 (SEQ ID NO: 113);
w") DF II-19.1 (SEQ ID NO: 114);
x") DF II-21 (SEQ ID NO: 115); und
y") DF II-22 (SEQ ID NO: 116),

wobei dieses Peptid gegebenenfalls eine Immunglobulin-E-Produktion aufweist, die geringer ist als der Betrag an IgE-Produktion und/oder IL/4-Produktion, die durch das native Proteinallergen stimuliert wird.

11. Isoliertes Peptid, das immunologisch auf für ein Peptid nach Anspruch 10 spezifischen Antikörper reagiert oder T-Zellen, die auf dieses Peptid reagieren.

12. Isoliertes Peptid des Proteinallergens der Dermatophagoides Gruppe I oder II, wobei dieses Peptid ausgewählt wird aus:

(i)

a) DF I-1 (SEQ ID NO: 72);
b) DF I-21.1 (SEQ ID NO: 90);
c) DF I-22.1 (SEQ ID NO: 92);
d) DF I-25.1 (SEQ ID NO: 97);
e) DF I-23.1 (SEQ ID NO: 95);
f) DF 1-9 (SEQ ID NO: 86);
g) DF I-21.2 (SEQ ID NO: 91);
h) DP II-1 (SEQ ID NO: 41);
i) DP II-1.2 (SEQ ID NO: 58);
j) DP II-22 (SEQ ID NO: 63);
k) DP II-25.1 (SEQ ID NO: 70);
l) DP II-21 (SEQ ID NO: 62);
m) DP II-25 (SEQ ID NO: 69);
n) DP II-20.6 (SEQ ID NO: 56);
o) DP II-20 (SEQ ID NO: 50);
p) DF II-4.5 (SEQ ID NO: 107);
q) DF II-2 (SEQ ID NO: 104);
r) DF II-19.1 (SEQ ID NO: 114);
s) DF II-17 (SEQ ID NO: 111);
t) DF II-15 (SEQ ID NO: 109).
u) DP II-25.2 (SEQ ID NO: 71); und
v) DF 1-22.2 (SEQ ID NO: 92)

oder(ii)

a) DF I-1 (SEQ ID NO: 72);
b) DF I-2.1 (SEQ ID NO: 73);
c) DF 1-3 (SEQ ID NO: 74);
d) DF 1-4 (SEQ ID NO: 75);
e) DF I-11 (SEQ ID NO: 76);
f) DF 1-12 (SEQ ID NO: 77);
g) DF 1-5 (SEQ ID NO: 78);

h) DF I-13 (SEQ ID NO: 79);
i) DF I-14 (SEQ ID NO: 80);
j) DF I-15 (SEQ ID NO: 81);
k) DF 1-6 (SEQ ID NO: 82);
l) DF 1-7 (SEQ ID NO: 83);
m) DF I-8.1 (SEQ ID NO: 84);
n) DF 1-8 (SEQ ID NO: 85);
o) DF 1-9 (SEQ ID NO: 86);
p) DF I-16 (SEQ ID NO: 87);
q) DF I-10 (SEQ ID NO: 88);
r) DF I-17 (SEQ ID NO: 89);
s) DF I-21.1 (SEQ ID NO: 90);
t) DF I-21.2 (SEQ ID NO: 91);
u) DF I-22.1 (SEQ ID NO: 92);
v) DF 1-22.2 (SEQ ID NO: 93);
w) DF 1-22.4 (SEQ ID NO: 94);
x) DF I-23.1 (SEQ ID NO: 95);
y) DF I-23.2 (SEQ ID NO: 96);
z) DF I-25.1 (SEQ ID NO: 97);
a') DF I-25.2 (SEQ ID NO: 98);
b') DF I-26.1 (SEQ ID NO: 99);
c') DF I-27.1 (SEQ ID NO: 100);
d') DF I-28.1 (SEQ ID NO: 101);
e') DF 1-28.2 (SEQ ID NO: 102);
f') DP II-20 (SEQ ID NO50);
g') DP II-20.1 (SEQ ID NO: 51);
h') DP II-20.2 (SEQ ID NO: 52);
i') DP II-20.3 (SEQ ID NO: 53);
j') DP II-20.4 (SEQ ID NO: 54);
k') DP II-20.5 (SEQ ID NO: 55);
l') DP II 20.6 (SEQ ID NO: 56);
m') DP II-1 (SEQ ID NO: 41);
o') DP II-2 (SEQ ID NO: 42);
p') DP II-3.1 (SEQ ID NO: 43);
q') DP II-4 (SEQ ID NO: 44);
r') DP II-5 (SEQ ID NO: 45);
s') DP II-6 (SEQ ID NO: 46);
t') DP II-7 (SEQ ID NO: 47);
u') DP II-8 (SEQ ID NO: 48);
v') DP II-9 (SEQ ID NO: 49);
w') DP II-1.1 (SEQ ID NO: 57);
x') DP II-1.2 (SEQ ID NO: 58);
y') DP II-2.1 (SEQ ID NO: 59);
z') DP II-2.2 (SEQ ID NO: 60);
a") DP II-2.3 (SEQ ID NO: 61);
b") DP II-21 (SEQ ID NO: 62);
c") DP II-22 (SEQ ID NO: 63);
d") DP II-26 (SEQ ID NO: 64);
e") DP II-26.1 (SEQ ID NO: 65);
f") DP II-23 (SEQ ID NO: 66);
g") DP II-23.1 (SEQ ID NO: 67);
h") DP II-24 (SEQ ID NO: 68);
i") DP II-25 (SEQ ID NO: 69);
j") DP II-25.1 (SEQ ID NO: 70);
k") DP II-25.2 (SEQ ID NO: 71);
l") DF II-1 (SEQ ID NO: 103);
m") DF II-2 (SEQ ID NO: 104);
n") DF II-13.1 (SEQ ID NO: 105);

o") DF II-3.1 (SEQ ID NO: 106);
p") DF II-4.5 (SEQ ID NO: 107);
q") DF II-4.3 (SEQ ID NO: 108);
r") DF II-15 (SEQ ID NO: 109);
s") DF II-16 (SEQ ID NO: 110);
t") DF II-17 (SEQ ID NO: 111);
u") DF II-18 (SEQ ID NO: 112);
v") DF II-19 (SEQ ID NO: 113);
w") DF II-19.1 (SEQ ID NO: 114);
x") DF II-21 (SEQ ID NO: 115); und
y") DF II-22 (SEQ ID NO: 116).

13. Peptid nach einem der Ansprüche 8 bis 10, das entweder:

(a) in mindestens 75 % der gegenüber dem Proteinallergen empfindlichen Individuen kein für ein Proteinallergen der Gattung Dermatophagoides spezifisches Immunglobulin E bindet, oder wenn Bindung des Peptids an dieses Immunglobulin E auftritt, diese Bindung bei einem beträchtlichen Anteil der gegenüber dem Proteinallergen empfindlichen Individuen nicht zu einer Freisetzung von Mediatoren aus Mastzellen oder Basophilen führt; oder
(b) Immunglobulin E in einem geringeren Ausmaß bindet als das Proteinallergen, aus dem das Peptid stammt, dieses Immunglobulin E bindet.

14. Peptid nach einem der Ansprüche 1 bis 13, das in gegenüber Hausstaubmilben empfindlichen Individuen, denen es verabreicht wird, die allergische Reaktion des Individuums auf das Hausstaubmilbenallergen modifiziert.

15. Peptid nach einem der Ansprüche 1 bis 14, wobei mindestens einer der Aminosäurereste, die den MHC-Protein-Komplex binden, aber nicht für eine solche Bindung wesentlich sind, substituiert wird, wobei gegebenenfalls ein oder mehrere Aminosäurereste durch Alanin, Glutaminsäure oder eine Methylaminosäure substituiert werden.

16. Peptid nach Anspruch 15, wobei mindestens einer der Aminosäurereste, die für die Bindung des MHC-Protein-Komplexes wesentlich sind, durch einen konservativen Aminosäurerest substituiert wird.

17. Peptid nach einem der Ansprüche 1 bis 14, wobei mindestens einer der Aminosäurereste, die den T-Zell-Rezeptor binden, aber nicht für eine solche Bindung wesentlich sind, substituiert wird.

18. Isolierte Nucleinsäure mit einer Sequenz, die für ein Peptid nach einem der Ansprüche 1 bis 17 kodiert.

19. Isoliertes Peptid, hergestellt in einer mit der Nucleinsäure nach Anspruch 18 transformierten Wirtszelle.

20. Therapeutische Zusammensetzung, umfassend ein Peptid nach einem der Ansprüche 1 bis 17 und einen pharmazeutisch unbedenklichen Träger oder Verdünnungsmittel.

21. Therapeutische Zusammensetzung nach Anspruch 20, enthaltend mindestens zwei Peptide, wobei diese Peptide jeweils mindestens ein T-Zell-Epitop eines Proteinallergens der Dermatophagoides Gruppe I oder II enthalten, wobei diese Peptide vom gleichen oder von unterschiedlichen Proteinallergenen der Dermatophagoides Gruppe I oder II abstammen.

22. Zusammensetzung nach Anspruch 20 oder 21, wobei entweder:

(a) die Peptide ausgewählt werden aus:

a) DP I-21.1 (SEQ ID NO: 27);
b) DP I-21.2 (SEQ ID NO: 28);
c) DP I-22.1 (SEQ ID NO: 29);
d) DP 1-22.2 (SEQ ID NO: 30);
e) DP 1-22.3 (SEQ ID NO: 31);
f) DP 1-22.4 (SEQ ID NO: 32);
g) DP I-23.1 (SEQ ID NO: 33);
h) DP I-23.2 (SEQ ID NO: 34);

i) DP I-25.1 (SEQ ID NO: 35);
j) DP I-25.2 (SEQ ID NO: 36);
k) DP I-26.1 (SEQ ID NO: 37);
l) DP I-27.1 (SEQ ID NO: 38);
m) DP I-28.1 (SEQ ID NO: 39);
n) DP 1-28.2 (SEQ ID NO: 40);
o) DP I-1 (SEQ ID NO: 9);
p) DF I-1 (SEQ ID NO: 72);
q) DF I-21.1 (SEQ ID NO: 90);
r) DF I-21.2 (SEQ ID NO: 91);
s) DF I-22.1 (SEQ ID NO: 92);
t) DF 1-22.2 (SEQ ID NO: 93);
u) DF 1-22.4 (SEQ ID NO: 94);
v) DF 1-23.1 (SEQ ID NO: 95);
w) DF I-23.2 (SEQ ID NO: 96);
x) DF I-25.1 (SEQ ID NO: 97);
y) DF 1-25.2 (SEQ ID NO: 98);
z) DF I-26.1 (SEQ ID NO: 99);
a') DF I-27.1 (SEQ ID NO: 100);
b') DF I-28.1 (SEQ ID NO: 101);
c') DF 1-28.2 (SEQ ID NO: 102);
d') DP II-20 (SEQ ID NO: 50);
e') DP II-20.1 (SEQ ID NO: 51);
f') DP II-20.2 (SEQ ID NO: 52);
g') DP II-20.3 (SEQ ID NO: 53);
h') DP II-20.4 (SEQ ID NO: 54);
i') DP II-20.5 (SEQ ID NO: 55);
j') DP II 20.6 (SEQ ID NO: 56);
k') DP II-1 (SEQ ID NO: 41);
l') DP II-1.1 (SEQ ID NO: 57);
m') DP II-1.2 (SEQ ID NO: 58);
n') DP II-2.1 (SEQ ID NO: 59);
o') DP II-2.2 (SEQ ID NO: 60);
p') DP II-2.3 (SEQ ID NO: 61);
q') DP II-21 (SEQ ID NO: 62);
r') DP II-22 (SEQ ID NO: 63);
s') DP II-26 (SEQ ID NO: 64);
t') DP II-26.1 (SEQ ID NO: 65);
u') DP II-23 (SEQ ID NO: 66);
v') DP II-23.1 (SEQ ID NO: 67);
w') DP II-24 (SEQ ID NO: 68);
x') DP II-25 (SEQ ID NO: 69);
y') DP II-25.1 (SEQ ID NO: 70);
z') DP II-25.2 (SEQ ID NO: 71);
a") DF II-1 (SEQ ID NO: 103)
b") DF II-2 (SEQ ID NO: 104);
c") DF II-13.1 (SEQ ID NO: 105);
d") DF II-3.1 (SEQ ID NO: 106);
e") DF II-4.5 (SEQ ID NO: 107);
f') DF II-4.3 (SEQ ID NO: 108);
g") DF II-15 (SEQ ID NO: 109);
h") DF II-16 (SEQ ID NO: 110);
i") DF II-17 (SEQ ID NO: 111);
j") DF II-18 (SEQ ID NO: 112);
k") DF II-19 (SEQ ID NO: 113);
l" DF II-19.1 (SEQ ID NO: 114)
m") DF II-21 (SEQ ID NO: 115); und
n") DF II-22 (SEQ ID NO: 116),

wobei die Zusammensetzung einen ausreichenden Anteil an T-Zell-Epitopen von mindestens einem Proteinallergen enthält, so dass bei Verabreichung der Zusammensetzung an ein gegenüber einem Hausstaubmilbenallergen empfindliches Individuum, die T-Zellen des Individuums gegenüber dem mindestens einem Pro-teinallergen toleriert werden; oder

(b) diese Zusammensetzung eine Kombination aus Peptiden enthält, die ausgewählt werden aus:

a) DP I-22.1 (SEQ ID NO: 29) und DP I-25.1 (SEQ ID NO: 35);
b) DP I-21.1 (SEQ ID NO: 27) und DP I-25.2 (SEQ ID NO: 36);
c) DP I-22.1 (SEQ ID NO: 29) und DP I-1 (SEQ ID NO: 9);
d) DP I-21.1 (SEQ ID NO: 27), DP I-22.1 (SEQ ID NO: 29), und DP 1-25.2 (SEQ ID NO: 36);
e) DP I-21.2 (SEQ ID NO: 28), DP I-22.1 (SEQ ID NO: 29), und DP I-23.1 (SEQ ID NO: 39);
f) DP I-1 (SEQ ID NO: 9), DP I-22.1 (SEQ ID NO: 29), und DP I-23.1 (SEQ ID NO: 33);
g) DP I-1 (SEQ ID NO: 9), DP I-22.1 (SEQ ID NO: 29), und DP 1-25.2 (SEQ ID NO: 36);
h) DP I-21.1 (SEQ ID NO: 27), DP I-22.1 (SEQ ID NO: 29), DP I-23.1 (SEQ ID NO: 33), und DP 1-25.2 (SEQ ID NO: 36);
i) DP I-21.2 (SEQ ID NO: 28), DP I-22.1 (SEQ ID NO: 29), und DP I-25.2 (SEQ ID NO: 36);
j) DP I-21.2 (SEQ ID NO: 27), DP I-22.1 (SEQ ID NO: 29), DP I-25.2 (SEQ ID NO: 36), und DP I-26.1 (SEQ ID NO: 37);
k) DF I-21.2 (SEQ ID NO: 91) und DF I-22.1 (SEQ ID NO: 92);
l) DF I-21.1 (SEQ ID NO: 90), DF I-22.1 (SEQ ID NO: 92) und DF I-25.1 (SEQ ID NO: 97);
m) DF I-21.2 (SEQ ID NO: 91), DF I-22.1 (SEQ ID NO: 92), und DF I-25.1 (SEQ ID NO: 97);
n) DF I-1 (SEQ ID NO: 72) und DF I-22.1 (SEQ ID NO: 92);
o) DF I-1 (SEQ ID NO: 72), DF I-22.1 (SEQ ID NO: 92), und DF I-25.1 (SEQ ID NO: 97);
p) DF I-22.1 (SEQ ID NO: 29), und DF I-25.1 (SEQ ID NO: 35);
q) DF I-21.1 (SEQ ID NO: 90), DF I-22.1 (SEQ ID NO: 92), und DF I-23.1 (SEQ ID NO: 95);
r) DP I-21.1 (SEQ ID NO: 27), und DF I-22.1 (SEQ ID NO: 92);
s) DP I-1 (SEQ ID NO: 9), DP I-23.1 (SEQ ID NO: 33), DP 1-25.1 (SEQ ID NO: 35), und DF I-1(SEQ ID NO: 72);
t) DP I-1 (SEQ ID NO: 9), DP I-25.1 (SEQ ID NO: 35), DP I-23.1 (SEQ ID NO: 33), und DF I-21.2 (SEQ ID NO: 91);
u) DP I-1 (SEQ ID NO: 9), DP I-25.1 (SEQ ID NO: 35), DP I-23.1 (SEQ ID NO: 33), und DF I-21.1 (SEQ ID NO: 90);
v) DP II-22 (SEQ ID NO: 63), und DP II-25.2 (SEQ ID NO: 71);
w) DP II-22 (SEQ ID NO: 63), DP II-25.2 (SEQ ID NO: 71), und DP I-21.1 (SEQ ID NO: 27) und DP I-22.1 (SEQ ID NO: 29);
x) DP II-22 (SEQ ID NO: 63), DP II-25.2 (SEQ ID NO: 71), DP II-20.6 (SEQ ID NO: 56), DP I-22.1 (SEQ ID NO: 29), DP I-21.1 (SEQ ID NO: 27), und DP I-23.1 (SEQ ID NO: 33);
y) DP II-22 (SEQ ID NO: 63), DP II-25.2 (SEQ ID NO: 71), DP II-20.6 (SEQ ID NO: 56), DP I-21.1 (SEQ ID NO: 27), DP I-22.1 (SEQ ID NO: 29), und DP I-25.2 (SEQ ID NO: 36);
z) DP II-22 (SEQ ID NO: 63), DP II-25.2 (SEQ ID NO: 71), DP I-21.1 (SEQ ID NO: 27), DP I-22.1 (SEQ ID NO: 29), und DP I-25.2 (SEQ ID NO: 36);
a') DP II-22 (SEQ ID NO: 63), DP II-25.2 (SEQ ID NO: 71), DP I-21.1 (SEQ ID NO: 27), DP I-22.1 (SEQ ID NO: 29), und DP I-23.1 (SEQ ID NO: 33);
b') DP II-22 (SEQ ID NO: 63), DP II-25.2 (SEQ ID NO: 71), DP I-1 (SEQ ID NO: 9), und DP I-22.1 (SEQ ID NO: 29);
c') DF II-4.5 (SEQ ID NO: 107) und DF II-2 (SEQ ID NO: 104);
d') DF II-4.5 (SEQ ID NO: 107) und DF II-19.1 (SEQ ID NO: 114);
e') DF II-4.5 (SEQ ID NO: 107), DF II-2 (SEQ ID NO: 104), und DF II-19.1 (SEQ ID NO: 114);
f') DF II-4.5 (SEQ ID NO: 107), DF II-2 (SEQ ID NO: 104), und DF II-9 (SEQ ID NO: 86);
g') DF II-4.5 (SEQ ID NO: 107), und DF I-21.1 (SEQ ID NO: 90);
h') DF II-4.5 (SEQ ID NO: 107), DP II-22 (SEQ ID NO: 63), und DP II-25.2 (SEQ ID NO: 71); und
i') DF II-4.5 (SEQ ID NO: 107), DF II-2 (SEQ ID NO: 104), und DP II-22 (SEQ ID NO: 63).

**23.** Zusammensetzung nach einem der Ansprüche 20 bis 22, enthaltend die folgenden Peptidkombinationen:
DP I-21.2 (SEQ ID NO: 27); DP I-23.1 (SEQ ID NO: 33); DP I-26.1 (SEQ ID NO: 37); DP II-20.6 (SEQ ID NO: 56);
DP II-22 (SEQ ID NO: 63); DP II-25.2 (SEQ ID NO: 71); und DF 1-22.2 (SEQ ID NO: 93).

**24.** Verwendung eines Peptids nach einem der Ansprüche 1 bis 17 bei der Herstellung eines Arzneimittels zur Behandlung der Überempfindlichkeit gegenüber Hausstaubmilben.

**25.** Verwendung nach Anspruch 24, wobei dieses Arzneimittel für die gleichzeitige oder aufeinander folgende Verabreichung von mindestens zwei unterschiedlichen Peptiden/Teilen angepasst wird.

**26.** Verfahren zum Nachweis einer Überempfindlichkeit gegenüber Hausstaubmilben bei einem Individuum, umfassend das Kombinieren einer von dem Individuum erhaltenen Blutprobe mit mindestens einem Peptid nach einem der Ansprüche 1 bis 17 unter Bedingungen, die für eine Bindung der Blutbestandteile damit geeignet sind, und Bestimmen des Ausmaßes, mit dem eine solche Bindung auftritt, als Hinweis auf die Überempfindlichkeit des Individuums auf Hausstaubmilben.

**27.** Verfahren nach Anspruch 26, wobei das Ausmaß, mit dem Bindung auftritt, mittels Bewerten der T-Zell-Funktion, T-Zell-Proliferation oder einer Kombination daraus bestimmt wird.


**Revendications**

**1.** Peptide isolé d'un allergène de protéine *Dermatophagoïdes* groupe I ou II, ledit peptide comprenant au moins un épitope de lymphocyte T dudit allergène de protéine, en option au moins deux épitopes de lymphocyte T, ledit peptide ayant un indice de positivité d'au moins environ 100 et un indice de stimulation de lymphocyte T moyen d'au moins environ 2,0 comme déterminé dans une population d'individus sensibles audit allergène de protéine, en option la population étant d'au moins 9 individus, par exemple d'au moins 29 individus.

**2.** Peptide isolé selon la revendication 1 qui a un indice de positivité d'au moins environ 150 et un indice de stimulation de lymphocyte T moyen d'au moins environ 4,0 comme déterminé dans une population d'individus sensibles audit allergène de protéine.

**3.** Peptide isolé selon la revendication 1 ou 2 choisi parmi soit :

(a) DP I-1 (SEQ ID NO: 9); DP I-21.1 (SEQ ID NO: 27); DP I-21.2 (SEQ ID NO: 28); DP I-23.1 (SEQ ID NO: 33); DP I-23.2 (SEQ ID NO: 34); DP I-25.2 (SEQ ID NO: 36); et DP I-26.1 (SEQ ID NO: 37); ou
(b) DP 1-21.2, DP 1-23.1, DP 1-26.1, DP II-20.6, DP II-22, DP II-25.2 et DF 1-22.2 comme représenté sur les figures 3 et 4.

**4.** Peptide isolé selon l'une quelconque des revendications 1 à 3 qui soit :

(a) ne lie pas l'immunoglobuline E spécifique pour un allergène de protéine du genre *Dermatophagoïdes* dans au moins 75% d'individus sensibles à l'allergène de protéine, ou si il se produit une liaison du peptide à ladite immunoglobuline E, une telle liaison ne conduit pas à une libération de médiateurs des mastocytes ou basophiles dans au moins 75% d'individus sensibles à l'allergène de protéine; soit
(b) lie l'immunoglobuline E selon une moindre étendue que celle selon laquelle l'allergène de protéine duquel le peptide est dérivé lie l'immunoglobuline E.

**5.** Peptide isolé selon l'une quelconque des revendications 1 à 4, ledit peptide étant choisi parmi:

a) DP I-1 (SEQ ID NO: 9);
b) DP 1-2 (SEQ ID NO: 10);
c) DP 1-3 (SEQ ID NO: 11);
d) DP 1-4 (SEQ ID NO: 12);
e) DP I-11.1 (SEQ ID NO: 13);
f) DP I-12.1 (SEQ ID NO: 14);
h) DP I-13 (SEQ ID NO: 17);
i) DP I-14 (SEQ ID NO: 18);
j) DP I-15 (SEQ ID NO: 19);
k) DP I-6.1 (SEQ ID NO: 20);
l) DP I-7.1 (SEQ ID NO: 21);
m) DP 1-8 (SEQ ID NO: 22);
n) DP 1-9 (SEQ ID NO: 23);
o) DP I-16 (SEQ ID NO: 24);
p) DP I-10 (SEQ ID NO: 25);

q) DP I-17 (SEQ ID NO: 26);
r) DP I-21.1 (SEQ ID NO: 27);
s) DP I-21.2 (SEQ ID NO: 28);
t) DP I-22.1 (SEQ ID NO: 29);
u) DP I-22.2 (SEQ ID NO: 30);
v) DP I-22.3 (SEQ ID NO: 31);
w) DP I-22.4 (SEQ ID NO: 32);
x) DP I-23.1 (SEQ ID NO: 33);
y) DP I-23.2 (SEQ ID NO: 34);
z) DP I-25.1 (SEQ ID NO: 35);
a') DP I-25.2 (SEQ ID NO: 36);
b') DP I-26.1 (SEQ ID NO: 37);
c') DP I-27.1 (SEQ ID NO: 38);
d') DP I-28.1 (SEQ ID NO: 39); et
e') DP I-28.2 (SEQ ID NO: 40),

dans lequel ledit peptide a un indice de stimulation de lymphocyte T moyen équivalent ou supérieur à l'indice de stimulation de lymphocyte T moyen dudit peptide tel que représenté sur la figure 5 et la figure 15, en option un indice de stimulation de lymphocyte T moyen d'au moins 4,0.

**6.** Peptide isolé selon l'une quelconque des revendications 1 à 5, ayant une séquence d'acides aminés correspondant à une séquence d'acides aminés d'un peptide choisi parmi ce qui suit:

a) DP I-1 (SEQ ID NO: 9);
b) DP 1-2 (SEQ ID NO: 10);
c) DP 1-3 (SEQ ID NO: 11);
d) DP 1-4 (SEQ ID NO: 12);
e) DP I-11.1 (SEQ ID NO: 13);
f) DP I-12.1 (SEQ ID NO: 14);
h) DP I-13 (SEQ ID NO: 17);
i) DP I-14 (SEQ ID NO: 18);
j) DP I-15 (SEQ ID NO: 19);
k) DP I-6.1 (SEQ ID NO: 20);
l) DP I-7.1 (SEQ ID NO: 21);
m) DP 1-8 (SEQ ID NO: 22);
n) DP 1-9 (SEQ ID NO: 23);
o) DP 1-16 (SEQ ID NO: 24);
p) DP 1-10 (SEQ ID NO: 25);
q) DP I-17 (SEQ ID NO: 26);
r) DP I-21.1 (SEQ ID NO: 27);
s) DP I-21.2 (SEQ ID NO: 28);
t) DP I-22.1 (SEQ ID NO: 29);
u) DP I-22.2 (SEQ ID NO: 30);
v) DP I-22.3 (SEQ ID NO: 31);
w) DP I-22.4 (SEQ ID NO: 32);
x) DP I-23.1 (SEQ ID NO: 33);
y) DP I-23.2 (SEQ ID NO: 34);
z) DP I-25.1 (SEQ ID NO: 35);
a') DP I-25.2 (SEQ ID NO: 36);
b') DP I-26.1 (SEQ ID NO: 37);
c') DP I-27.1 (SEQ ID NO: 38);
d') DP I-28.1 (SEQ ID NO: 39);
e') DP I-28.2 (SEQ ID NO: 40);
f') DP I-5.1 (SEQ ID NO: 16); et
g') DP I-23.1.1;
h') DP I-23.1.2);
i') DP I-223.1.3); et
j') DP I-23.1.4)

comme représenté sur la figure 28

7. Peptide selon l'une quelconque des revendications 1 à 6 qui ne lie pas l'immunoglobuline E spécifique pour Der pI dans au moins 75% d'individus sensibles à Der pI, ou si il se produit une liaison du peptide à ladite immunoglobuline E, une telle liaison ne conduit pas à une libération de médiateurs des mastocytes ou basiphiles dans au moins 75% d'individus sensibles à Der pI.

8. Peptide isolé selon l'une quelconque des revendications 1 à 7 comprenant au moins deux régions, chaque région comprenant au moins deux épitopes de lymphocyte T d'un allergène de protéine *Dermatophagoïdes* groupe I ou II, lesdites régions étant dérivées du même allergène de protéine *Dermatophagoïdes* groupe I ou II ou d'un allergène de protéine *Dermatophagoïdes* groupe I ou II différent, lesdites régions comprenant chacune une séquence d'acides aminés parmi:

a) DP I-21.1 (SEQ ID NO: 27);
b) DP I-21.2 (SEQ ID NO: 28);
c) DP I-22.1 (SEQ ID NO: 29);
d) DP 1-22.2 (SEQ ID NO: 30);
e) DP 1-22.3 (SEQ ID NO: 31);
f) DP 1-22.4 (SEQ ID NO: 32);
g) DP I-23.1 (SEQ ID NO: 33);
h) DP I-23.2 (SEQ ID NO: 34);
i) DP I-25.1 (SEQ ID NO: 35);
j) DP I-25.2 (SEQ ID NO: 36);
k) DP I-26.1 (SEQ ID NO: 37);
l) DP I-27.1 (SEQ ID NO: 38);
m) DP I-28.1 (SEQ ID NO: 39);
n) DP 1-28.2 (SEQ ID NO: 40);
o) DP I-1 (SEQ ID NO: 9);
p) DF I-1 (SEQ ID NO: 72);
q) DF I-21.1 (SEQ ID NO: 90);
r) DF I-21.2 (SEQ ID NO: 91);
s) DF I-22.1 (SEQ ID NO: 92);
t) DF 1-22.2 (SEQ ID NO: 93);
u) DF 1-22.4 (SEQ ID NO: 94);
v) DF 1-23.1 (SEQ ID NO: 95);
w) DF I-23.2 (SEQ ID NO: 96);
x) DF I-25.1 (SEQ ID NO: 97);
y) DF I-25.2 (SEQ ID NO: 98);
z) DF I-26.1 (SEQ ID NO: 99);
a') DF I-27.1 (SEQ ID NO: 100);
b') DF 1-28.1 (SEQ ID NO: 101);
c') DF I-28.2 (SEQ ID NO: 102);
d') DP II-20 (SEQ ID NO: 50);
e') DP II-20.1 (SEQ ID NO: 51);
f) DP II-20.2 (SEQ ID NO: 52);
g') DP II-20.3 (SEQ ID NO: 53);
h') DP II-20.4 (SEQ ID NO: 54);
i') DP II-20.5 (SEQ ID NO: 55);
j') DP II-20.6 (SEQ ID NO: 56);
k') DP II-1 (SEQ ID NO: 41);
l') DP II-1.1 (SEQ ID NO: 57);
m') DP II-1.2 (SEQ ID NO: 58);
n') DP II-2.1 (SEQ ID NO: 59);
o') DP II-2.2 (SEQ ID NO: 60);
p') DP II-2.3 (SEQ ID NO: 61);
q') DP II-21 (SEQ ID NO: 62);
r') DP II-22 (SEQ ID NO: 63);
s') DP II-26 (SEQ ID NO: 64);

t') DP II-26.1 (SEQ ID NO: 65);
u') DP II-23 (SEQ ID NO: 66);
v') DP II-23.1 (SEQ ID NO: 67);
w') DP II-24 (SEQ ID NO: 68);
x') DP II-25 (SEQ ID NO: 69);
y') DP II-25.1 (SEQ ID NO: 70);
z') DP II-25.2 (SEQ ID NO: 71);
a") DF II-1 (SEQ ID NO: 103)
b") DF II-2 (SEQ ID NO: 104);
c") DF II-13.1 (SEQ ID NO: 105);
d") DF II-3.1 (SEQ ID NO: 106);
e") DF II-4.5 (SEQ ID NO: 107);
f') DF II-4.3 (SEQ ID NO: 108);
g") DF II-15 (SEQ ID NO: 109);
h") DF II-16 (SEQ ID NO: 110);
i") DF II-17 (SEQ ID NO: 111);
j") DF II-18 (SEQ ID NO: 112);
k") DF II-19 (SEQ ID NO: 113);
l") DF II-19.1 (SEQ ID NO: 114);
m") DF II-21 (SEQ ID NO: 115); et
n") DF II-22 (SEQ ID NO: 116).

9. Peptide isolé selon la revendication 8 dans lequel soit:

(i) lesdite régions comprenant une séquence d'acides aminés choisie parmi:

a) DP I-21.2 (SEQ ID NO: 27);
b) DP I-23.1 (SEQ ID NO: 33);
c) DP I-26.1 (SEQ ID NO: 37);
d) DP II-20.6 (SEQ ID NO: 56);
e) DP II-22 (SEQ ID NO: 63);
f) DP II-25.2 (SEQ ID NO: 71); et
g) DP 1-22.2 (SEQ ID NO: 93);

tous comme représentés sur les figures 3 et 4; soit
(ii) ledit peptide comprend une combinaison de régions choisies parmi:

a) DP I-22.1 (SEQ ID NO: 29) et DP I-25.1 (SEQ ID NO: 35);
b) DP I-21.1 (SEQ ID NO: 27) et DP I-25.2 (SEQ ID NO: 36);
c) DP I-22.1 (SEQ ID NO: 29) et DP I-1 (SEQ ID NO: 9);
d) DP I-21.1 (SEQ ID NO: 27), DP I-22.1 (SEQ ID NO: 29), et DP I-25.2 (SEQ ID NO: 36);
e) DP I-21.2 (SEQ ID NO: 28), DP I-22.1 (SEQ ID NO: 29), et DP I-23.1 (SEQ ID NO: 39);
f) DP I-1 (SEQ ID NO: 9), DP I-22.1 (SEQ ID NO: 29), et DP I-23.1 (SEQ ID NO: 33);
g) DP I-1 (SEQ ID NO: 9), DP I-22.1 (SEQ ID NO: 29), et DP I-25.2 (SEQ ID NO: 36);
h) DP I-21.1 (SEQ ID NO: 27), DP I-22.1 (SEQ ID NO: 29), DP I-23.1 (SEQ ID NO: 33), et DP 1-25.2 (SEQ ID NO: 36);
i) DP I-21.2 (SEQ ID NO: 28), DP I-22.1 (SEQ ID NO: 29), et DP I-25.2 (SEQ ID NO: 36);
j) DP I-21.2 (SEQ ID NO: 27), DP I-22.1 (SEQ ID NO: 29), DP I-25.2 (SEQ ID NO: 36), et DP I-26.1 (SEQ ID NO: 37);
k) DF I-21.2 (SEQ ID NO: 91) et DF I-22.1 (SEQ ID NO: 92);
l) DF I-21.1 (SEQ ID NO: 90), DF I-22.1 (SEQ ID NO: 92), et DF I-25.1 (SEQ ID NO: 97);
m) DF I-21.2 (SEQ ID NO: 91), DF I-22.1 (SEQ ID NO: 92), et DF I-25.1 (SEQ ID NO: 97);
n) DF I-1 (SEQ ID NO: 72) et DF I-22.1 (SEQ ID NO: 92);
o) DF I-1 (SEQ ID NO: 72), DF I-22.1 (SEQ ID NO: 92), et DF I-25.1 (SEQ ID NO: 97);
p) DF I-22.1 (SEQ ID NO: 29), et DF I-25.1 (SEQ ID NO: 35);
q) DF I-21.1 (SEQ ID NO: 90), DF I-22.1 (SEQ ID NO: 92), et DF I-23.1 (SEQ ID NO: 95);
r) DP I-21.1 (SEQ ID NO: 27), et DF I-22.1 (SEQ ID NO: 92);
s) DP I-1 (SEQ ID NO: 9), DP I-23.1 (SEQ ID NO: 33), DP I-25.1 (SEQ ID NO: 35), et DF I-1 (SEQ ID NO: 72);

t) DP I-1 (SEQ ID NO: 9), DP I-25.1 (SEQ ID NO: 35), DP I-23.1 (SEQ ID NO: 33), et DF I-21.2 (SEQ ID NO: 91);

u) DP I-1 (SEQ ID NO: 9), DP I-25.1 (SEQ ID NO: 35), DP I-23.1 (SEQ ID NO: 33), et DF I-21.1 (SEQ ID NO: 90);

v) DP II-22 (SEQ ID NO: 63), et DP II-25.2 (SEQ ID NO: 71);

w) DP II-22 (SEQ ID NO: 63), DP II-25.2 (SEQ ID NO: 71), et DP I-21.1 (SEQ ID NO: 27) et DP I-22.1 (SEQ ID NO: 29);

x) DP II-22 (SEQ ID NO: 63), DP II-25.2 (SEQ ID NO: 71), DP II-20.6 (SEQ ID NO: 56), DP I-22.1 (SEQ ID NO: 29), DP I-21.1 (SEQ ID NO: 27), et DP I-23.1 (SEQ ID NO: 33);

y) DP II-22 (SEQ ID NO: 63), DP II-25.2 (SEQ ID NO: 71), DP II-20.6 (SEQ ID NO: 56), DP I-21.1 (SEQ ID NO: 27), DP I-22.1 (SEQ ID NO: 29), et DP I-25.2 (SEQ ID NO: 36);

z) DP II-22 (SEQ ID NO: 63), DP II-25.2 (SEQ ID NO: 71), DP I-21.1 (SEQ ID NO: 27), DP I-22.1 (SEQ ID NO: 29), et DP I- 25.2 (SEQ ID NO: 36);

a') DP II-22 (SEQ ID NO: 63), DP II-25.2 (SEQ ID NO: 71), DP I-21.1 (SEQ ID NO: 27), DP I-22.1 (SEQ ID NO: 29), et DP I-23.1 (SEQ ID NO: 33);

b') DP II-22 (SEQ ID NO: 63), DP II-25.2 (SEQ ID NO: 71), DP I-1 (SEQ ID NO: 9), et DP I-22.1 (SEQ ID NO: 29);

c') DF II4.5 (SEQ ID NO: 107) et DF II-2 (SEQ ID NO: 104);

d') DF II-4.5 (SEQ ID NO: 107) et DF II-19.1 (SEQ ID NO: 114);

e') DF II-4.5 (SEQ ID NO: 107), DF II-2 (SEQ ID NO: 104), et DF II-19.1 (SEQ ID NO: 114);

f') DF II-4.5 (SEQ ID NO: 107), DF II-2 (SEQ ID NO: 104), et DF II-9 (SEQ ID NO: 86);

g') DF II-4.5 (SEQ ID NO: 107); et DF I-21.1 (SEQ ID NO: 90);

h') DF II-4.5 (SEQ ID NO: 107), DP II-22 (SEQ ID NO: 63), et DP II-25.2 (SEQ ID NO:71); et

i') DF II-4.5 (SEQ ID NO: 107), DF II-2 (SEQ ID NO: 104), et DP II-22 (SEQ ID NO: 63);

(iii) ledit peptide comprend la combinaison de régions qui suit: DP I-21.2 (SEQ ID NO: 27); DP I-23.1 (SEQ ID NO: 33); DP I-26.1 (SEQ ID NO: 37); DP II-20.6 (SEQ ID NO: 56); DP II-22 (SEQ ID NO: 63); DP II-25.2 (SEQ ID NO: 71); et DF 1-22.2 (SEQ ID NO: 93); soit

(iv) ledit peptide a un arrangement séquentiel spécifique de séquences d'acides aminés, ledit arrangement de séquences d'acides aminés étant choisi parmi:

a) DP I-26.1, DP II-25.2, DP 1-22, DP II-20.6 et DP I-21.2 respectivement comme représenté sur la figure 25;

b) DP II-25.2, DP 1-22.2, DP I-23.1, DP II-22, DP I-26.1, DP I-21.2 et DP II-26.6 respectivement comme représenté sur la figure 26; et

c) DP II-25.2, DP I-21.1, DP I-26.1, DP II-22, DP II-20.2 et DF 1-22.2 respectivement comme représenté sur la figure 27.

**10.** Peptide isolé d'un allergène de protéine *Dermatophagoïdes* groupe I ou II, ledit peptide comprenant au moins un épitope de lymphocyte T dudit allergène de protéine, ledit peptide comprenant une séquence d'acides aminés parmi l'un de ce sui suit:

a) DF I-1 (SEQ ID NO: 72);

b) DF I-2.1 (SEQ ID NO: 73);

c) DF 1-3 (SEQ ID NO: 74);

d) DF 1-4 (SEQ ID NO: 75);

e) DF I-11 (SEQ ID NO: 76);

f) DF 1-12 (SEQ ID NO: 77);

g) DF 1-5 (SEQ ID NO: 78);

h) DF I-13 (SEQ ID NO: 79);

i) DF I-14 (SEQ ID NO: 80);

j) DF I-15 (SEQ ID NO: 81);

k) DF 1-6 (SEQ ID NO: 82);

l) DF 1-7 (SEQ ID NO: 83);

m) DF I-8.1 (SEQ ID NO: 84);

n) DF 1-8 (SEQ ID NO: 85);

o) DF 1-9 (SEQ ID NO: 86);

p) DF I-16 (SEQ ID NO: 87);

q) DF I-10 (SEQ ID NO: 88);

r) DF I-17 (SEQ ID NO: 89);
s) DF I-21.1 (SEQ ID NO: 90);
t) DF I-21.2 (SEQ ID NO: 91);
u) DF I-22.1 (SEQ ID NO: 92);
v) DF 1-22.2 (SEQ ID NO: 93);
w) DF 1-22.4 (SEQ ID NO: 94);
x) DF I-23.1 (SEQ ID NO: 95);
y) DF I-23.2 (SEQ ID NO: 96);
z) DF I-25.1 (SEQ ID NO: 97);
a') DF 1-25.2 (SEQ ID NO: 98);
b') DF I-26.1 (SEQ ID NO: 99);
c') DF I-27.1 (SEQ ID NO: 100);
d') DF I-28.1 (SEQ ID NO: 101);
e') DF 1-28.2 (SEQ ID NO: 102);
f') DP II-20 (SEQ ID NO: 50);
g') DP II-20.1 (SEQ ID NO: 51);
h') DP II-20.2 (SEQ ID NO: 52);
i') DP II-20.3 (SEQ ID NO: 53);
j') DP II-20.4 (SEQ ID NO: 54);
k') DP II-20.5 (SEQ ID NO: 55);
l') DP II 20.6 (SEQ ID NO: 56);
m') DP II-1 (SEQ ID NO: 41);
o') DP II-2 (SEQ ID NO: 42);
p') DP II-3.1 (SEQ ID NO: 43);
q') DP II-4 (SEQ ID NO: 44);
r') DP II-5 (SEQ ID NO: 45);
s') DP II-6 (SEQ ID NO: 46);
t') DP II-7 (SEQ ID NO: 47);
u') DP II-8 (SEQ ID NO: 48);
v') DP II-9 (SEQ NO: 49);
w') DP II-1.1 (SEQ NO: 57);
x') DP II-1.2 (SEQ ID NO: 58);
y') DP II-2.1 (SEQ ID NO: 59);
z') DP II-2.2 (SEQ ID NO: 60);
a") DP II-2.3 (SEQ ID NO: 61);
b") DP II-21 (SEQ ID NO: 62);
c") DP II-22 (SEQ ID NO: 63);
d") DP II-26 (SEQ ID NO: 64);
e") DP II-26.1 (SEQ ID NO: 65);
f') DP II-23 (SEQ ID NO: 66);
g") DP II-23.1 (SEQ ID NO: 67);
h") DP II-24 (SEQ ID NO: 68);
i") DP II-25 (SEQ ID NO: 69);
j") DP II-25.1 (SEQ ID NO: 70);
k") DP II-25.2 (SEQ ID NO: 71);
l") DF II-1 (SEQ ID NO: 103);
m") DF II-2 (SEQ ID NO: 104);
n") DF II-13.1 (SEQ NO: 105);
o") DF II-3.1 (SEQ ID NO: 106);
p") DF II-4.5 (SEQ ID NO: 107);
q") DF II-4.3 (SEQ ID NO: 108);
r") DF II-15 (SEQ ID NO: 109);
s") DF II-16 (SEQ ID NO: 110);
t") DF II-17 (SEQ ID NO: 111);
u") DF II-18 (SEQ ID NO: 112);
v") DF II-19 (SEQ ID NO: 113);
w") DF II-19.1 (SEQ ID NO: 114);
x") DF II-21 (SEQ ID NO: 115); et

y") DF II-22 (SEQ ID NO: 116).

optionnellement ledit peptide ayant une production d'immunoglobuline E qui est inférieure à la valeur de production IgE et/ou production IL/4 stimulée par l'allergène de protéine natif.

**11.** Peptide isolé qui est immunologiquement réactif avec des anticorps spécifiques ou des lymphocytes T réactifs avec un peptide de la revendication 10.

**12.** Peptide isolé d'un allergène de protéine *Dermatophagoïdes* groupe I ou II, ledit peptide étant choisi parmi:

(i)

a) DF I-1 (SEQ ID NO: 72);
b) DF I-21.1 (SEQ ID NO: 90);
c) DF I-22.1 (SEQ ID NO: 92);
d) DF I-25.1 (SEQ ID NO: 97);
e) DF I-23.1 (SEQ ID NO: 95);
f) DF 1-9 (SEQ ID NO: 86);
g) DF 1-21.2 (SEQ ID NO: 91);
h) DP II-1 (SEQ ID NO: 41);
i) DP II-1.2 (SEQ ID NO: 58);
j) DP II-22 (SEQ ID NO: 63);
k) DP II-25.1 (SEQ ID NO: 70);
l) DP II-21 (SEQ ID NO: 62);
m) DP II-25 (SEQ ID NO: 69);
n) DP II-20.6 (SEQ ID NO: 56);
o) DP II-20 (SEQ ID NO: 50);
p) DF II-4.5 (SEQ ID NO: 107);
q) DF II-2 (SEQ ID NO: 104);
r) DF II-19.1 (SEQ ID NO: 114);
s) DF II-17 (SEQ ID NO: 111);
t) DF II-15 (SEQ ID NO: 109).
u) DP II-25.2 (SEQ ID NO: 71); et
v) DF 1-22.2 (SEQ ID NO: 92)

ou (ii)

a) DF I-1 (SEQ ID NO: 72);
b) DF I-2.1 (SEQ ID NO: 73);
c) DF 1-3 (SEQ ID NO: 74);
d) DF 1-4 (SEQ ID NO: 75);
e) DF I-11 (SEQ ID NO: 76);
f) DF 1-12 (SEQ ID NO: 77);
g) DF 1-5 (SEQ ID NO: 78);
h) DF I-13 (SEQ ID NO: 79);
i) DF I-14 (SEQ ID NO: 80);
j) DF 1-15 (SEQ ID NO: 81);
k) DF 1-6 (SEQ ID NO: 82);
l) DF 1-7 (SEQ ID NO: 83);
m) DF I-8.1 (SEQ ID NO: 84);
n) DF 1-8 (SEQ ID NO: 85);
o) DF 1-9 (SEQ ID NO: 86);
p) DF I-16 (SEQ ID NO: 87);
q) DF I-10 (SEQ ID NO: 88);
r) DF 1-17 (SEQ ID NO: 89);
s) DF I-21.1 (SEQ ID NO: 90);
t) DF I-21.2 (SEQ ID NO: 91);
u) DF I-22.1 (SEQ ID NO: 92);

v) DF 1-22.2 (SEQ ID NO: 93);

w) DF 1-22.4 (SEQ ID NO: 94);

x) DF I-23.1 (SEQ ID NO: 95);

y) DF I-23.2 (SEQ ID NO: 96);

z) DF I-25.1 (SEQ ID NO: 97);

a') DF I-25.2 (SEQ ID NO: 98);

b') DF I-26.1 (SEQ ID NO: 99);

c') DF I-27.1 (SEQ ID NO: 100);

d') DF I-28.1 (SEQ ID NO: 101);

e') DF I-28.2 (SEQ ID NO: 102);

f) DP II-20 (SEQ ID NO50);

g') DP II-20.1 (SEQ ID NO: 51);

h') DP II-20.2 (SEQ ID NO: 52);

i') DP II-20.3 (SEQ ID NO: 53);

j') DP II-20.4 (SEQ ID NO: 54);

k') DP II-20.5 (SEQ ID NO: 55);

l') DP II 20.6 (SEQ ID NO: 56);

m') DP II-1 (SEQ ID NO: 41);

o') DP II-2 (SEQ ID NO: 42);

p') DP II-3.1 (SEQ ID NO: 43);

q') DP II-4 (SEQ ID NO: 44);

r') DP II-5 (SEQ ID NO: 45);

s') DP II-6 (SEQ ID NO: 46);

t') DP II-7 (SEQ ID NO: 47);

u') DP II-8 (SEQ ID NO: 48);

v') DP II-9 (SEQ ID NO: 49);

w') DP II-1.1 (SEQ ID NO: 57);

x') DP II-1.2 (SEQ ID NO: 58);

y') DP II-2.1 (SEQ ID NO: 59);

z') DP II-2.2 (SEQ ID NO: 60);

a") DP II-2.3 (SEQ ID NO: 61);

b") DP II-21 (SEQ ID NO: 62);

c") DP II-22 (SEQ ID NO: 63);

d") DP II-26 (SEQ ID NO: 64);

e") DP II-26.1 (SEQ ID NO: 65);

f') DP II-23 (SEQ ID NO: 66);

g") DP II-23.1 (SEQ ID NO: 67);

h") DP II-24 (SEQ ID NO: 68);

i") DP II-25 (SEQ ID NO: 69);

j") DP II-25.1 (SEQ ID NO: 70);

k") DP II-25.2 (SEQ ID NO: 71);

l") DF II-1 (SEQ ID NO: 103);

m") DF II-2 (SEQ ID NO: 104);

n") DF II-13.1 (SEQ ID NO: 105);

o") DF II-3.1 (SEQ ID NO: 106);

p") DF II-4.5 (SEQ ID NO: 107);

q") DF II-4.3 (SEQ ID NO: 108);

r") DF II-1 (SEQ ID NO: 109);

s") DF II-16 (SEQ ID NO: 110);

t") DF II-17 (SEQ ID NO: 111);

u") DF II-18 (SEQ ID NO: 112);

v") DF II-19 (SEQ ID NO: 113);

w") DF II-19.1 (SEQ ID NO: 114);

x") DF II-21 (SEQ ID NO: 115); et

y") DF II-22 (SEQ ID NO: 116).

**13.** Peptide selon l'une quelconque des revendications 8 à 10 qui soit:

(a) ne lie pas l'immunoglobuline E spécifique pour un allergène de protéine du genre *Dermatophagoïdes* dans au moins 75% d'individus sensibles à l'allergène de protéine, ou si il se produit une liaison du peptide à ladite immunoglobuline E, une telle liaison ne conduit pas à une libération de médiateurs des mastocytes ou basophiles dans un pourcentage substantiel d'individus sensibles à l'allergène de protéine; soit

(b) lie l'immunoglobuline E selon une moindre étendue que celle selon laquelle l'allergène de protéine duquel le peptide est dérivé lie l'immunoglobuline E.

14. Peptide selon l'une quelconque des revendications 1 à 13 qui modifie, dans un individu sensible aux acariens de poussière de maison à qui il est administré, la réponse allergique de l'individu à un allergène d'acariens de poussière de maison.

15. Peptide selon l'une quelconque des revendications 1 à 14 dans lequel au moins l'un des résidus d'acides aminés qui lie le complexe de protéine MHC mais qui n'est pas essentiel pour une telle liaison est substitué, optionnellement ledit/lesdits résidu(s) d'acides aminés étant substitué(s) par alanine, acide glutamique ou acide aminé méthyle.

16. Peptide selon la revendication 15, dans lequel au moins l'un des résidus d'acides aminés qui est essentiel pour lier le complexe de protéine MHC est substitué avec un résidu d'acide aminé conservatif.

17. Peptide selon l'une quelconque des revendications 1 à 14 dans lequel au moins l'un des résidus d'acides aminés qui lie le récepteur de lymphocyte T mais qui n'est pas essentiel pour une telle liaison est substitué.

18. Acide nucléique isolé ayant une séquence codant un peptide selon l'une quelconque des revendications 1 à 17.

19. Peptide isolé produit dans une cellule hôte transformée avec l'acide nucléique de la revendication 18.

20. Composition thérapeutique comprenant un peptide selon l'une quelconque des revendications 1 à 17 et un support ou diluant acceptable pharmaceutiquement.

21. Composition thérapeutique selon la revendication 20 comprenant au moins deux peptides, lesdits peptides comprenant chacun au moins un épitope de lymphocyte T d'un allergène de protéine *Dermatophagoïdes* groupe I ou II, lesdits peptides étant dérivés du même allergène de protéine *Dermatophagoïdes* groupe I ou II ou d'un allergène de protéine *Dermatophagoïdes* groupe I ou II différent.

22. Composition selon la revendication 20 ou 21 dans laquelle soit:

(a) lesdits peptides sont choisis parmi:

a) DP I-21.1 (SEQ ID NO: 27);
b) DP I-21.2 (SEQ ID NO: 28);
c) DP I-22.1 (SEQ ID NO: 29);
d) DP 1-22.2 (SEQ ID NO: 30);
e) DP 1-22.3 (SEQ ID NO: 31);
f) DP 1-22.4 (SEQ ID NO: 32);
g) DP I-23.1 (SEQ ID NO: 33);
h) DP 1-23.2 (SEQ ID NO: 34);
i) DP I-25.1 (SEQ ID NO: 35);
j) DP I-25.2 (SEQ ID NO: 36);
k) DP I-26.1 (SEQ ID NO: 37);
l) DP I-27.1 (SEQ ID NO: 38);
m) DP I-28.1 (SEQ ID NO: 39);
n) DP 1-28.2 (SEQ ID NO: 40);
o) DP I-1 (SEQ ID NO: 9);
p) DF I-1 (SEQ ID NO: 72);
q) DF I-21.1 (SEQ ID NO: 90);
r) DF I-21.2 (SEQ ID NO: 91);
s) DF I-22.1 (SEQ ID NO: 92);
t) DF 1-22.2 (SEQ ID NO: 93);
u) DF 1-22.4 (SEQ ID NO: 94);

v) DF I-23.1 (SEQ ID NO: 95);

w) DF I-23.2 (SEQ ID NO: 96);

x) DF I-25.1 (SEQ ID NO: 97);

y) DF I-25.2 (SEQ ID NO: 98);

z) DF I-26.1 (SEQ ID NO: 99);

a') DF I-27.1 (SEQ ID NO: 100);

b') DF I-28.1 (SEQ ID NO: 101);

c') DF 1-28.2 (SEQ ID NO: 102);

d') DP II-20 (SEQ ID NO: 50);

e') DP II-20.1 (SEQ ID NO: 51);

f) DP II-20.2 (SEQ ID NO: 52);

g') DP II-20.3 (SEQ ID NO: 53);

h') DP II-20.4 (SEQ ID NO: 54);

i') DP II-20.5 (SEQ ID NO: 55);

j') DP II 20.6 (SEQ ID NO: 56);

k') DP II-1 (SEQ ID NO: 41);

l') DP II-1.1 (SEQ ID NO: 57);

m') DP II-1.2 (SEQ ID NO: 58);

n') DP II-2.1 (SEQ ID NO: 59);

o') DP II-2.2 (SEQ ID NO: 60);

p') DP II-2.3 (SEQ ID NO: 61);

q') DP II-21 (SEQ ID NO: 62);

r') DP II-22 (SEQ ID NO: 63);

s') DP II-26 (SEQ ID NO: 64);

t') DP II-26.1 (SEQ ID NO: 65);

u') DP II-23 (SEQ ID NO: 66);

v') DP II-23.1, (SEQ ID NO: 67);

w') DP II-24 (SEQ ID NO: 68);

x') DP II-25 (SEQ ID NO: 69);

y') DP II-25.1 (SEQ ID NO: 70);

z') DP II-25.2 (SEQ ID NO: 71);

a") DF II-1 (SEQ ID NO: 103)

b") DF II-2 (SEQ ID NO: 104);

c") DF II-13.1 (SEQ ID NO: 105);

d") DF II-3.1 (SEQ ID NO: 106);

e") DF II-4.5 (SEQ ID NO: 107);

f') DF II-4.3 (SEQ ID NO: 108);

g") DF II-15 (SEQ ID NO: 109);

h") DF II-16 (SEQ ID NO: 110);

i") DF II-17 (SEQ ID NO: 111);

j") DF II-18 (SEQ ID NO: 112);

k") DF II-19 (SEQ ID NO: 113);

l") DF II-19.1 (SEQ ID NO: 114)

m") DF II-21 (SEQ ID NO: 115); et

n") DF II-22 (SEQ ID NO: 116),

dans laquelle ladite composition comprend un pourcentage suffisant d'épitopes de lymphocyte T d'au moins un allergène de protéine tel que, suite à l'administration de la composition à un individu sensible à un allergène d'acarien de poussière de maison, des lymphocytes T de l'individu sont tolérés pour ledit au moins un allergène de protéine; soit

(b) ladite composition comprend une combinaison de peptides choisis parmi:

a) DP I-22.1 (SEQ ID NO: 29) et DP I-25.1 (SEQ ID NO: 35);

b) DP I-21.1 (SEQ ID NO: 27) et DP 1-25.2 (SEQ ID NO: 36);

c) DP I-22.1 (SEQ ID NO: 29) et DP I-1 (SEQ ID NO: 9);

d) DP I-21.1 (SEQ ID NO: 27), DP I-22.1 (SEQ ID NO: 29), et DP I-25.2 (SEQ ID NO: 36);

e) DP I-21.2 (SEQ ID NO: 28), DP I-22.1 (SEQ ID NO: 29), et DP I-23.1 (SEQ ID NO: 39);

f) DP I-1 (SEQ ID NO: 9), DP I-22.1 (SEQ ID NO: 29), et DP I-23.1 (SEQ ID NO: 33);

g) DP I-1 (SEQ ID NO: 9), DP I-22.1 (SEQ ID NO: 29), et DP I-25.2 (SEQ ID NO: 36);

h) DP I-21.1 (SEQ ID NO: 27), DP I-22.1 (SEQ ID NO: 29), DP I-23.1 (SEQ ID NO: 33), et DP I-25.2 (SEQ ID NO: 36);

i) DP I-21.2 (SEQ ID NO: 28), DP I-22.1 (SEQ ID NO: 29), et DP I-25.2 (SEQ ID NO: 36);

j) DP I-21.2 (SEQ ID NO: 27), DP I-22.1 (SEQ ID NO: 29), DP I-25.2 (SEQ ID NO: 36), et DP I-26.1 (SEQ ID NO: 37);

k) DF 1-21.2 (SEQ ID NO: 91) et DF I-22.1 (SEQ ID NO: 92);

l) DF I-21.1 (SEQ ID NO: 90), DF I-22.1 (SEQ ID NO: 92) et DF I-25.1 (SEQ ID NO: 97);

m) DF 1-21.2 (SEQ ID NO: 91), DF I-22.1 (SEQ ID NO: 92), et DF I-25.1 (SEQ ID NO: 97);

n) DF I-1 (SEQ ID NO: 72) et DF I-22.1 (SEQ ID NO: 92);

o) DF I-1 (SEQ ID NO: 72), DF I-22.1 (SEQ ID NO: 92), et DF I-25.1 (SEQ ID NO: 97);

p) DF I-22.1 (SEQ ID NO: 29), et DF I-25.1 (SEQ ID NO: 35);

q) DF I-21.1 (SEQ ID NO: 90), DF I-22.1 (SEQ ID NO: 92), et DF I-23.1 (SEQ ID NO: 95);

r) DP I-21.1 (SEQ ID NO: 27), et DF I-22.1 (SEQ ID NO: 92);

s) DP I-1 (SEQ ID NO: 9), DP I-23.1 (SEQ ID NO: 33), DP I-25.1 (SEQ ID NO: 35), et DF I-1 (SEQ ID NO: 72);

t) DP I-1 (SEQ ID NO: 9), DP I-25.1 (SEQ ID NO: 35), DP I-23.1 (SEQ ID NO: 33), et DF I-21.2 (SEQ ID NO: 91);

u) DP I-1 (SEQ ID NO: 9), DP I-25.1 (SEQ ID NO: 35), DP I-23.1 (SEQ ID NO: 33), et DF I-21.1 (SEQ ID NO: 90);

v) DP II-22 (SEQ ID NO: 63), et DP II-25.2 (SEQ ID NO: 71);

w) DP II-22 (SEQ ID NO: 63), DP II-25.2 (SEQ ID NO: 71), et DP I-21.1 (SEQ ID NO: 27) et DP I-22.1 (SEQ ID NO: 29);

x) DP II-22 (SEQ ID NO: 63), DP II-25.2 (SEQ ID NO: 71), DP II-20.6 (SEQ ID NO: 56), DP I-22.1 (SEQ ID NO: 29), DP I-21.1 (SEQ ID NO: 27), et DP I-23.1 (SEQ ID NO: 33);

y) DP II-22 (SEQ ID NO: 63), DP II-25.2 (SEQ ID NO: 71), DP II-20.6 (SEQ ID NO: 56), DP I-21.1 (SEQ ID NO: 27), DP I-22.1 (SEQ ID NO: 29), et DP 1-25.2 (SEQ ID NO: 36);

z) DP II-22 (SEQ ID NO: 63), DP II-25.2 (SEQ ID NO: 71), DP I-21.1 (SEQ ID NO: 27), DP I-22.1 (SEQ ID NO: 29), et DP I-25.2 (SEQ ID NO: 36);

a') DP II-22 (SEQ ID NO: 63), DP II-25.2 (SEQ ID NO: 71), DP I-21.1 (SEQ ID NO: 27), DP I-22.1 (SEQ ID NO: 29), et DP I-23.1 (SEQ ID NO: 33);

b') DP II-22 (SEQ ID NO: 63), DP II-25.2 (SEQ ID NO: 71), DP I-1 (SEQ ID NO: 9), et DP I-22.1 (SEQ ID NO: 29);

c') DF II-4.5 (SEQ ID NO: 107) et DF II-2 (SEQ ID NO: 104);

d') DF II-4.5 (SEQ ID NO: 107) et DF II-19.1 (SEQ ID NO: 114);

e') DF II-4.5 (SEQ ID NO: 107), DF II-2 (SEQ ID NO: 104), et DF II-19.1 (SEQ ID NO: 114);

f') DF II-4.5 (SEQ ID NO: 107), DF II-2 (SEQ ID NO: 104), et DF II-9 (SEQ ID NO: 86);

g') DF II-4.5 (SEQ ID NO: 107), et DF I-21.1 (SEQ ID NO: 90);

h') DF II-4.5 (SEQ ID NO: 107), DP II-22 (SEQ ID NO: 63), et DP II-25.2 (SEQ ID NO: 71); et

i') DF II-4.5 (SEQ ID NO: 107), DF II-2 (SEQ ID NO: 104), et DP II-22 (SEQ ID NO: 63).

23. Composition selon l'une quelconque des revendications 20 à 22 comprenant la combinaison de peptides qui suit: DP I-21.2 (SEQ ID NO: 27); DP I-23.1 (SEQ ID NO: 33); DP I-26.1 (SEQ ID NO: 37); DP II-20.6 (SEQ ID NO: 56); DP II-22 (SEQ ID NO: 63); DP II-25.2 (SEQ ID NO: 71); et DF 1-22.2 (SEQ ID NO: 93).

24. Utilisation d'un peptide selon l'une quelconque des revendications 1 à 17 dans la fabrication d'un médicament pour traiter la sensibilité aux acariens de poussière de maison.

25. Utilisation selon la revendication 24 dans laquelle ledit médicament est adapté pour administrer simultanément ou en séquence au moins deux peptides/parties différent(e)s.

26. Procédé de détection de la sensibilité aux acariens de poussière de maison dans un individu, comprenant la combinaison d'un échantillon sanguin obtenu à partir d'un individu avec au moins un peptide selon l'une quelconque des revendications 1 à 17 sous des conditions appropriées pour la liaison de composants sanguins avec, et la détermination de l'étendue selon laquelle une telle liaison se produit en tant qu'indicative de la sensibilité de l'individu aux acariens de poussière de maison.

27. Procédé selon la revendication 26 dans lequel l'étendue selon laquelle la liaison se produit est déterminée en évaluant une fonction de lymphocyte T, une prolifération de lymphocyte T ou une combinaison afférente.

Fig. 1

EP 0 623 168 B1

Der   fI   adaptor

```
                    T   S   A   C   R   I   N   S   V   N
        5'  -AATTTACCTCTGCTTGCCGTATCAACTCTGTTAACGCGGAATTCA-3'Hind III
φEcoR  I 3'-ATGGAGACGAACGGCATAGTTGAGACAATTGCGCCTTAAGTTCGA-5'
                                            Hpa I       EcoR I
```

Der   pI   adaptor

```
                T   N   A   C   S
        5'  -AATTTACCAACGCCTGCAGGCGAAGAATTCA-3'Hind III
φEcoR  I 3'-ATGGTTGCGGACGTCCGCTTCTTAAGTTCGA-5'
                      Pst I         EcoR I
```

## Fig. 2a

```
        5'  Der   fII/pII   primer
5'  -GGGAATTCGATCAAGTCGATGTCAAA-3'
```

```
        3'  fII/pII   primer
5'  -GGCTGCAGTTATATTTAATCGCGGATTTT-3'
```

```
        Der   fII  mutagenesis  primer
5'  -AATTGAAATCAAAGCCA-3'
```

## Fig. 2b

EP 0 623 168 B1

**Peptide Name**

| | |
|---|---|
| DPI-1(1-20) | TNACSING*NAPAEIDLRQMR |
| DPI-2(13-39) | EIDLRQMRTVTPIRMQGGCGSCWAFSG |
| DPI-3(21-49) | TVTPIRMQGGCGSCWAFSGVAATESAYLA |
| DPI-4(40-60) | VAATESAYLAHRNQSLDLAEQ |
| DPI-11.1(50-71) | HRNQSLDLAEQELVDCASQHGC |
| DPI-12.1(61-81) | ELVDCASQHGCHGDTIPRGIE |
| DPI-5(73-100) | GDTIPRGIEYIQHNGVVQESYYRYVARE |
| DPI-5.1(81-100) | EYIQHNGVVQESYYRYVARE |
| DPI-13(85-109) | HNGVVQESYYRYVAREQSCRRPNAQ |
| DPI-14(101-119) | QSCRRPNAQRFGISNYCQI |
| DPI-15(110-131) | RFGISNYCQIYPPNANKIREAL |
| DPI-6.1(120-143) | YPPNANKIREALAQTHSAIAVIIG |
| DPI-7.1(132-157) | AQTHSAIAVIIGIKDLDAFRHYDGRT |
| DPI-8(144-169) | IKDLDAFRHYDGRTIIQRDNGYQPNY |
| DPI-9(158-180) | IIQRDNGYQPNYHAVNIVGYSNA |
| DPI-16(170-191) | HAVNIVGYSNAQGVDYWIVRNS |
| DPI-10(181-204) | QGVDYWIVRNSWDTNWGDNGYGYF |
| DPI-17(197-222) | GDNGYGYFAANIDLMMIEEYPYVVIL |
| DPI-21.1(1-28) | TNACSING*NAPAEIDLRQMRTVTPIRMQ |
| DPI-21.2(5-28) | SING*NAPAEIDLRQMRTVTPIRMQ |
| DPI-22.1(36-64) | AFSGVAATESAYLAHRNQSLDLAEQELVD |
| DPI-22.2(40-64) | VAATESAYLAHRNQSLDLAEQELVD |
| DPI-22.3(36-60) | AFSGVAATESAYLAHRNQSLDLAEQ |
| DPI-22.4(40-68) | VAATESAYLAHRNQSLDLAEQELVDCASQ |
| DPI-23.1(81-109) | EYIQHNGVVQESYYRYVAREQSCRRPNAQ |
| DPI-23.2(74-102) | DTIPRGIEYIQHNGVVQESYYRYVAREQS |
| DPI-25.1(118-146) | QIYPPNANKIREALAQTHSAIAVIIGIKD |

Fig. 3

EP 0 623 168 B1

```
DPI-25.2(118-139)      QIYPPNANKIREALAQTHSAIA
DPI-26.1(141-166)      IIGIKDLDAFRHYDGRTIIQRDNGYQ
DPI-27.1(161-185)      RDNGYQPNYHAVNIVGYSNAQGVDY
DPI-28.1(173-201)      NIVGYSNAQGVDYWIVRNSWDTNWGDNGY
DPI-28.2(173-195)      NIVGYSNAQGVDYWIVRNSWDTN
DPII-1(1-20)           DQVDVKDCANHEIKKVLVPG
DPII-2(11-35)          HEIKKVLVPGCHGSEPCIIHRGKPF
DPII-3.1(22-50)        HGSEPCIIHRGKPFQLEAVFEANQNTKTA
DPII-4(36-60)          QLEAVFEANQNTKTAKIEIKASIDG
DPII-5(51-77)          KIEIKASIDGLEVDVPGIDPNACHYMK
DPII-6(61-86)          LEVDVPGIDPNACHYMKCPLVKGQQY
DPII-7(78-104)         CPLVKGQQYDIKYTWNVPKIAPKSENV
DPII-8(87-112)         DIKYTWNVPKIAPKSENVVVTVKVMG
DPII-9(105-129)        VVTVKVMGDDGVLACAIATHAKIRD
DPII-20(1-26)          DQVDVKDCANHEIKKVLVPGCHGSEP
DPII-20.1(1-26)E8      DQVDVKDEANHEIKKVLVPGCHGSEP
DPII-20.2(1-26)S8      DQVDVKDSANHEIKKVLVPGCHGSEP
DPII-20.3(1-26)E21     DQVDVKDCANHEIKKVLVPGEHGSEP
DPII-20.4(1-26)S21     DQVDVKDCANHEIKKVLVPGSHGSEP
DPII-20.5(1-26)E8E21   DQVDVKDEANHEIKKVLVPGEHGSEP
DPII-20.6(1-26)S8S21   DQVDVKDSANHEIKKVLVPGSHGSEP
```

## Fig. 3 cont.

EP 0 623 168 B1

```
DPII-1.1(1-20)E8              DQVDVKDEANHEIKKVLVPG
DPII-1.2(1-20)S8              DQVDVKDSANHEIKKVLVPG
DPII-2.1(11-26)               HEIKKVLVPGCHGSEP
DPII-2.2(11-26)E21            HEIKKVLVPGEHGSEP
DPII-2.3(11-26)S21            HEIKKVLVPGSHGSEP
DPII-21(33-60)               KPFQLEAVFEANQNTKTAKIEIKASIDG
DPII-22(36-63)               QLEAVFEANQNTKTAKIEIKASIDGLEV
DPII-26(41-67)               FEANQNTKTAKIEIKASIDGLEVDVPG
DPII-26.1(45-67)             QNTKTAKIEIKASIDGLEVDVPG
DPII-23(79-104)              PLVKGQQYDIKYTWNVPKIAPKSENV
DPII-23.1(79-104)Y92         PLVKGQQYDIKYTYNVPKIAPKSENV
DPII-24(100-112)             KSENVVVTVKVMG
DPII-25(107-129)             TVKVMGDDGVLACAIATHAKIRD
DPII-25.1(107-129)E119       TVKVMGDDGVLAEAIATHAKIRD
DPII-25.2(107-129)L111S119   TVKVLGDDGVLASAIATHAKIRD
```

## Fig . 3 cont.

EP 0 623 168 B1

## Peptide Name

| Peptide Name | Sequence |
|---|---|
| DFI-1(1-20) | TSACRINSVNVPSELDLRSLR |
| DFI-2.1(13-39) | ELDLRSLRTVTPIRMQGGCGSCWAFSG |
| DFI-3(21-49) | TVTPIRMQGGCGSCWAFSGVAATESAYLA |
| DFI-4(40-60) | VAATESAYLAYRNTSLDLSEQ |
| DFI-11(50-71) | YRNTSLDLSEQELVDCASQHGC |
| DFI-12(61-81) | ELVDCASQHGCHGDTIPRGIE |
| DFI-5(73-100) | GDTIPRGIEYIQQNGVVEERSYPYVARE |
| DFI-13(85-109) | QNGVVEERSYPYVAREQRCRRPNSQ |
| DFI-14(101-119) | QRCRRPNSQHYGISNYCQI |
| DFI-15(110-131) | HYGISNYCQIYPPDVKQIREAL |
| DFI-6(120-143) | YPPDVKQIREALTQTHTAIAVIIG |
| DFI-7(132-157) | TQTHTAIAVIIGIKDLRAFRHYDGRT |
| DFI-8.1(144-169) | IKDLRAFQHYDGRTIIQHDNGYQPNY |
| DFI-8(154-168) | DGRTIIQHDNGYQPN |
| DFI-9(158-180) | IIQHDNGYQPNYHAVNIVGYGST |
| DFI-16(170-191) | HAVNIVGYGSTQGDDYWIVRNS |
| DFI-10(181-204) | QGDDYWIVRNSWDTTWGDSGYGYF |
| DFI-17(197-222) | GDSGYGYFQAGNNLMMIEQYPYVVIM |
| DFI-21.1(1-28) | TSACRINSVNVPSELDLRSLRTVTPIRMQ |
| DFI-21.2(5-28) | RINSVNVPSELDLRSLRTVTPIRMQ |
| DFI-22.1(36-64) | AFSGVAATESAYLAYRNTSLDLSEQELVD |
| DFI-22.2(40-64) | VAATESAYLAYRNTSLDLSEQELVD |
| DFI-22.4(40-68) | VAATESAYLAYRNTSLDLSEQELVDCASQ |
| DFI-23.1(81-109) | EYIQQNGVVEERSYPYVAREQRCRRPNSQ |
| DFI-23.2(74-102) | DTIPRGIEYIQQNGVVEERSYPYVAREQR |
| DFI-25.1(118-146) | QIYPPDVKQIREALTQTHTAIAVIIGIKD |
| DFI-25.2(118-139) | QIYPPDVKQIREALTQTHTAIA |
| DFI-26.1(141-166) | IIGIKDLRAFQHYDGRTIIQHDNGYQ |

## Fig. 4

EP 0 623 168 B1

```
DFI-27.1(161-185)      HDNGYQPNYHAVNIVGYGSTQGDDY
DFI-28.1(173-201)      NIVGYGSTQGDDYWIVRNSWDTTWGDSGY
DFI-28.2(173-195)      NIVGYGSTQGDDYWIVRNSWDTT
DFII-1(1-20)           DQVDVKDCANNEIKKVMVDG
DFII-2(11-35)          NEIKKVMVDGCHGSDPCIIHRGKPF
DFII-13.1(22-50)       HGSDPCIIHRGKPFTLEALFDANQNTKTA
DFII-3.1(30-48)        HRGKPFTLEALFDANQNTK
DFII-4.5(36-60)        TLEALFDANQNTKTAKIEIKASLDG
DFII-4.3(45-70)        QNTKTAKIEIKASLDGLEIDVPGIDT
DFII-15(51-77)         KIEIKASLDGLEIDVPGIDTNACHFMK
DFII-16(61-86)         LEIDVPGIDTNACHFMKCPLVKGQQY
DFII-17(78-104)        CPLVKGQQYDAKYTWNVPKIAPKSENV
DFII-18(87-112)        DAKYTWNVPKIAPKSENVVVTVKLVG
DFII-19(95-129)        PKIAPKSENVVVTVKLVGDNGVLACAIATHAKIRD
DFII-19.1(105-129)     VVTVKLVGDNGVLACAIATHAKIRD
DFII-21(33-60)         KPFTLEALFDANQNTKTAKIEIKASLDG
DFII-22(36-63)         TLEALFDANQNTKTAKIEIKASLDGLEI
```

# Fig. 4 cont.

EP 0 623 168 B1

Fig. 5

Fig. 6

Fig. 7

DER f I PEPTIDES

DER p I PEPTIDES

| | |
|---|---|
| 38% (2.8) | DPI-17 |
| 63% (3.2) | DFI-17 |
| 38% (2.8) | DPI-10 |
| 38% (3.9) | DFI-10 |
| 25% (4.2) | DPI-16 |
| 75% (3.1) | DFI-16 |
| 50% (2.4) | DPI-9 |
| 75% (6.5) | DFI-9 |
| 88% (3.4) | DPI-8 |
| 63% (3.9) | DFI-8.1 |
| 0% (0.0) | DFI-7.1 |
| 50% (5.2) | DPI-7 |
| 50% (3.0) | DFI-6.1 |
| 38% (2.9) | DPI-6 |
| 50% (15.2) | DFI-15 |
| 50% (7.7) | DPI-15 |
| 50% (3.9) | DPI-14 |
| 38% (4.1) | DFI-14 |
| 25% (2.9) | DPI-13 |
| 63% (3.1) | DFI-13 |
| 75% (2.9) | DPI-5 |
| 50% (64.0) | DFI-5 |
| 13% (3.7) | DPI-12/12.1 |
| 25% (4.1) | DFI-12 |
| 75% (9.1) | DPI-11/11.1 |
| 63% (3.5) | DFI-11 |
| 75% (3.6) | DPI-4 |
| 50% (5.1) | DFI-4 |
| 50% (4.0) | DPI-3 |
| 50% (7.8) | DFI-3 |
| 13% (3.0) | DPI-2 |
| 50% (2.7) | DFI-2 |
| 75% (3.8) | DPI-1 |
| 75% (3.5) | DFI-1 |

25  20  15  10  5  0

RANKED SUM OF 5 HIGHEST PEPTIDE RESPONSES

Fig. 8

Fig. 9

Fig. 10

Fig. 11

Fig. 12

Fig. 13

Fig. 14

Fig. 15a

Fig. 15b

Fig. 16a

Fig. 16b

Fig. 17a

STIMULATION INDEX

Fig. 17b

Fig. 18a

STIMULATION INDEX

Fig. 18b

125

Fig. 18c

Fig. 18d

Fig. 19a

EP 0 623 168 B1

Fig. 19b

Fig. 20a

Fig. 20b

EP 0 623 168 B1

Fig. 21a

Fig. 21b

Fig. 21c

Fig. 21d

Fig. 21e

PEPTIDES

PMA
DP II-20.1
DP II-20.2
DP II-20.3
DP II-4S21
DP II-20.5
DP II-20.6
DP II-1

ABS 450 nm

0.5  0.4  0.3  0.2  0.1  0.0

2  6  18  54  162  BACKGROUND

1/ DILUTION

Fig. 21f

Fig. 21g

Fig. 21h

```
                    10         20         30         40         50         60
Der p I (a)  TNACSINGNA  PAEIDLRQMR  TVTPIRMQGG  CGSCWAFSGV  AATESAYLAH  RNQSLDLAEQ
Der P I (b)  ----------  ----------  ----------  ----------  --------Y   ----------
Der p I (c)  ----------  ----------  ----------  ----------  --------Y   ----------
Der p I (d)  ----------  ----------  ----------  ----------  ----------  ----------

                    70         80         90        100        110        120
Der p I (a)  ELVDCASQHG  CHGDTIPRGI  EYIQHNGVVQ  ESYYRYVARE  QSCRRPNAQR  FGISNYCQIY
Der P I (b)  ----------  ----------  ----------  ----------  ----------  ----------
Der p I (c)  ----------  ----------  K---------  ----------  ----------  ----------
Der p I (d)  ----------  ----------  ----------  ----------  ----------  ----------

                   130        140        150        160        170        180
Der p I (a)  PPNANKIREA  LAQTHSAIAV  IIGIKDLDAF  RHYDGRTIIQ  RDNGYQPNYH  AVNIVGYSNA
Der P I (b)  ---V------  ----------  ----------  ----------  ----------  ----------
Der p I (c)  ---V------  ----------  ----------  ----------  ----------  ----------
Der p I (d)  ----------  -----T----  ----------  ----------  ----------  ----------
Der p I (e)                                      ----------  ----------  ----------

                   190        200        210        220
Der p I (a)  QGVDYWIVRN  SWDTNWGDNG  YGYFAANIDL  MMIEEYPYVV  IL
Der P I (b)  ----------  ----------  ----------  ----------  --
Der p I (c)  ----------  ----------  ----------  ---Q-----   --
Der p I (d)  ----------  ----------  ----------  ----------  --
Der p I (e)  ----------  ----------  ----------  ----------  --
```

Fig. 22

EP 0 623 168 B1

```
                    10        20        30        40        50
Der p II (c)  DQVDVKDCANHEIKKVLVPGCHGSEPCIIHRGKPFQLEAVFEANQNTKTAK
        (1)   .........H.....L.P....E.........Q...V.E....T....
        (2)   .........H.....L.P....E.........Q...V.E....S....
Der f II      .........N.....M.D....D.........T...L.D....T....


                    60        70        80        90       100
Der p II (c)  IEIKASIDGLEVDVPGIDPNACHYMKCPLVKGQQYDIKYTWNVPKIAPKSE
        (1)   ......I............P....YM...........I..............
        (2)   ......I............P....YM...........I..............
Der f II      ......L............T....FM...........A..............
                                        V            I


                   110       120
Der p II (c)  NVVVTVKVMGDDGVLACAIATHAKIRD
        (1)   .......VM.DD.......A....I..
        (2)   .......VM.ND.......A....L..
Der f II      .......LV.DN.......A....I..
                      I         G
```

# Fig. 23

```
              10          20          30          40          50          60
pFL1        DQVDVKDCANNEIKKVMVPGCHGSEPCIIHRGKPFTLEALFDANQNTKTAKIEIKASLDGLE
pFL2        ...........N...........................................I.I........
MT  3       ...........N...........................................I.T........
MT  5 (1-92)...........S...........................................I.I........
MT18 (1-84) ...........N...........................................I.I........
MT16 (1-70) ...........N...........................................T.I........

              70          80          90         100         110         120         130
pFL1        IDVPGIDTNACHFVKCPLVKGQQYDIKYTWNVPKIAPKSENVVVTVKLIGDNGVLACAIATHAKIRD
pFL2        ...............M...........A.........................V.................
MT  3       ...............M...........A.........................V.................
MT  5       ...............M.........I....
MT18        ...............M........
```

# Fig. 24

EP 0 623 168 B1

EP 0 623 168 B1

```
         10        20        30        40        50        60
         |         |         |         |         |         |
3'- ATTATTGGCATCAAAGATTTAGACGCATTCCGTCATTATGATGGCCGAACAATCATTCAA

    I   I   G   I   K   D   L   D   A   F   R   H   Y   D   G   R   T   I   I   Q


         70        80        90       100       110       120
         |         |         |         |         |         |
   CGCGATAATGGTTACCAAACTGTTAAAGTTCTGGGTGATGATGGTGTTTTGGCCTCTGCT

    R   D   N   G   Y   Q   T   V   K   V   L   G   D   D   G   V   L   A   S   A


        130       140       150       160       170       180
         |         |         |         |         |         |
   ATTGCTACTCATGCTAAAATCCGCGATGTTGCCGCAACTGAATCAGCTTATTTGGCCTAC

    I   A   T   H   A   K   I   R   D   V   A   A   T   E   S   A   Y   L   A   Y


        190       200       210       220       230       240
         |         |         |         |         |         |
   CGTAACACGTCTTTGGATCTTTCTGAACAGGAACTCGTCGATCAATTGGAAGCCGTTTTC

    R   N   T   S   L   D   L   S   E   Q   E   L   V   D   Q   L   E   A   V   F


        250       260       270       280       290       300
         |         |         |         |         |         |
   GAAGCCAACCAAAACACAAAAACCGCTAAAATTGAAATCAAAGCCTCAATCGATGGTTTA

    E   A   N   Q   N   T   K   T   A   K   I   E   I   K   A   S   I   D   G   L
```

Fig. 25

```
             310         320         330         340         350         360
              |           |           |           |           |           |
GAAGTTGAATACATCCAACATAATGGTGTCGTCCAAGAAAGCTACTATCGATACGTTGCA

 E   V   E   Y   I   Q   H   N   G   V   V   Q   E   S   Y   Y   R   Y   V   A

             370         380         390         400         410         420
              |           |           |           |           |           |
CGAGAACAATCATGCCGACGACCAAATGCACAAGATCAAGTCGATGTCAAAGATTCTGCC

 R   E   Q   S   C   R   R   P   N   A   Q   D   Q   V   D   V   K   D   S   A

             430         440         450         460         470         480
              |           |           |           |           |           |
AATCATGAAATCAAAAAAGTTTTGGTACCAGGATCGCATGGTTCAGAACCAAGTATCAAT

 N   H   E   I   K   K   V   L   V   P   G   S   H   G   S   E   P   S   I   N

             490         500         510         520         530         540
              |           |           |           |           |           |
GGAAATGCTCCAGCTGAAATCGATTTGCGACAAATGCGAACTGTCACTCCCATTCGTATG

 G   N   A   P   A   E   I   D   L   R   Q   M   R   T   V   T   P   I   R   M

CAATAATGA-3'

 Q   -   -
```

## Fig. 25 cont.

EP 0 623 168 B1

```
                10           20           30           40           50           60
                |            |            |            |            |            |
5' - ACTGTTAAAGTTCTGGGTGATGATGGTGTTTTGGCCTCTGCTATTGCTACTCATGCTAAA

     T  V  K  V  L  G  D  D  G  V  L  A  S  A  I  A  T  H  A  K

                70           80           90          100          110          120
                |            |            |            |            |            |
     ATCCGCGATGTTGCCGCAACTGAATCAGCTTATTTGGCCTACCGTAACACGTCTTTGGAT

      I  R  D  V  A  A  T  E  S  A  Y  L  A  Y  R  N  T  S  L  D

               130          140          150          160          170          180
                |            |            |            |            |            |
     CTTTCTGAACAGGAACTCGTCGATGAATACATCCAACATAATGGTGTCGTCCAAGAAAGC

      L  S  E  Q  E  L  V  D  E  Y  I  Q  H  N  G  V  V  Q  E  S

               190          200          210          220          230          240
                |            |            |            |            |            |
     TACTATCGATACGTTGCACGAGAACAATCATGCCGACGACCAAATGCACAACAATTGGAA

      Y  Y  R  Y  V  A  R  E  Q  S  C  R  R  P  N  A  Q  Q  L  E

               250          260          270          280          290          300
                |            |            |            |            |            |
     GCCGTTTTCGAAGCCAACCAAAACACAAAAACGGCTAAAATTGAAATCAAAGCCTCAATC

      A  V  F  E  A  N  Q  N  T  K  T  A  K  I  E  I  K  A  S  I
```

## Fig. 26

EP 0 623 168 B1

```
          310         320         330         340         350         360
           |           |           |           |           |           |
GATGGTTTAGAAGTTATTATTGGCATCAAAGATTTAGACGCATTCCGTCATTATGATGGC

  D  G  L  E  V  I  I  G  I  K  D  L  D  A  F  R  H  Y  D  G

          370         380         390         400         410         420
           |           |           |           |           |           |
CGAACAATCATTCAACGCGATAATGGTTACCAAAGTATCAATGGAAATGCTCCAGCTGAA

  R  T  I  I  Q  R  D  N  G  Y  Q  S  I  N  G  N  A  P  A  E

          430         440         450         460         470         480
           |           |           |           |           |           |
ATCGATTTGCGACAAATGCGAACTGTCACTCCCATTCGTATGCAAGATCAAGTCGATGTC

  I  D  L  R  Q  M  R  T  V  T  P  I  R  M  Q  D  Q  V  D  V

          490         500         510         520         530         540
           |           |           |           |           |           |
AAAGATTCTGCCAATCATGAAATCAAAAAAGTTTTGGTACCAGGATCGCATGGTTCAGAA

  K  D  S  A  N  H  E  I  K  K  V  L  V  P  G  S  H  G  S  E
```

CCATAATGA-5'

  P  -  -

Fig. 26 cont.

EP 0 623 168 B1

```
              10         20         30         40         50         60
              |          |          |          |          |          |
5'-ACTGTTAAAGTTTTGGGTGATGATGGTGTTTTGGCCTCAGCTATTGCTACTCATGCTAAA

   T   V   K   V   L   G   D   D   G   V   L   A   S   A   I   A   T   H   A   K

              70         80         90        100        110        120
              |          |          |          |          |          |
  ATCCGCGATAGTATCAATGGAAATGCTCCAGCTGAAATCGATTTGCGACAAATGCGAACT

   I   R   D   S   I   N   G   N   A   P   A   E   I   D   L   R   Q   M   R   T

             130        140        150        160        170        180
              |          |          |          |          |          |
  GTCACTCCCATTCGTATGCAAGAATACATCCAACATAATGGTGTCGTCCAAGAAAGCTAC

   V   T   P   I   R   M   Q   E   Y   I   Q   H   N   G   V   V   Q   E   S   Y

             190        200        210        220        230        240
              |          |          |          |          |          |
  TATCGATACGTTGCACGAGAACAATCATGCCGACGACCAAATGCACAAATTATTGGCATC

   Y   R   Y   V   A   R   E   Q   S   C   R   R   P   N   A   Q   I   I   G   I

             250        260        270        280        290        300
              |          |          |          |          |          |
  AAAGATTTAGACGCATTCCGTCATTATGATGGCCGAACAATCATTCAACGCGATAATGGT

   K   D   L   D   A   F   R   H   Y   D   G   R   T   I   I   Q   R   D   N   G
```

## Fig. 27

```
        310         320         330         340         350         360
         |           |           |           |           |           |
TACCAACAATTGGAAGCCGTTTTCGAAGCCAACCAAAACACAAAAACGGCTAAAATTGAA

  Y   Q   Q   L   E   A   V   F   E   A   N   Q   N   T   K   T   A   K   I   E

        370         380         390         400         410         420
         |           |           |           |           |           |
ATCAAAGCCTCAATCGATGGTTTAGAAGTTGATCAAGTCGATGTCAAAGATTCAGCCAAT

  I   K   A   S   I   D   G   L   E   V   D   Q   V   D   V   K   D   S   A   N

        430         440         450         460         470         480
         |           |           |           |           |           |
CATGAAATCAAAAAGTTTTGGTACCAGGATCACATGGTTCAGAACCAGTTGCCGCAACT

  H   E   I   K   K   V   L   V   P   G   S   H   G   S   E   P   V   A   A   T

        490         500         510         520         530        540
         |           |           |           |           |           |
GAATCAGCTTATTTGGCCTACCGTAACACGTCTTTGGATCTTTCTGAACAGGAACTCGTC

  E   S   A   Y   L   A   Y   R   N   T   S   L   D   L   S   E   Q   E   L   V

GATTAGTAG-5'

  D   -   -
```

EP 0 623 168 B1

Fig. 27 cont.

```
DP I-23.1        EYIQHNGVVQESYYRYVAREQSCRRPNAQ
DP I-23.1.1    KKEYIQHNGVVQESYYRYVAREQSCRRPNAQ
DP I-23.1.2     KEYIQHNGVVQESYYRYVAREQSCRRPNAQ
DP I-23.1.3      EYIQHNGVVQESYYRYVAREQSSRRPNAQ
DP I-23.1.4      EYIQHNGVVQESYYRYVAREQSERRPNAQ


DP II-22         QLEAVFEANQNTKTAKIEIKASIDGLEV
DP II-22.1     KKQLEAVFEANQNTKTAKIEIKASIDGLEV
DP II-22.2      KQLEAVFEANQNTKTAKIEIKASIDGLEVK
```

Fig. 28

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US SN07881396 A
- US SN07777859 A

### Non-patent literature cited in the description

- **Lind, P. et al.** *Allergy.,* 1984, vol. 39, 259-274
- **van der Zee, J.S. et al.** *Journal Allergy and Clinical Immunology,* 1988, vol. 81, 884-896
- **Thompson, P.J. et al.** *Immunology,* 1988, vol. 64, 301-314
- **Thomas, W.R. et al.** *International Archives of Allergy and Applied Immunology,* 1988, vol. 85, 127-129
- **Chua, K.Y. et al.** *Journal of Experimental Medicine,* 1988, vol. 167, 175-182
- **Chua, K.Y. et al.** *International Archives of Allergy and Applied Immunology,* 1990, vol. 91, 118-123
- **Dilworth, R.J. et al.** *Clinical and Experimental Allergy,* vol. 21, 25-32
- **Yuuki, T. et al.** *Journal Allergol.,* 1990, vol. 39, 557-461
- **Trudinger, M. et al.** *Clinical and Experimental Allergy,* 1991, vol. 21, 33-37
- **Ovey, E.R. et al.** *Journal of Experimental Medicine,* 1989, vol. 170, 1457-1462
- **Chua, KY. et al.** *International Archives of Allergy and Applied Immunology,* 1990, vol. 91, 118-123
- **Green. W.K. et al.** *International Archives of Allergy and Applied Immunology,* 1990, vol. 93, 30-38
- **Chua, K.Y. et al.** *International Archives of Allergy and Applied Immunology,* 1990, vol. 91, 124-129
- **Thomas, W.R. et al.** *Epitopes of Atopic Allergens, Proceedings of Workshop from XIV Congress of the European Academy of Allergy and Clinical Immunology,* September 1989, 77-82
- **O'Hehir, R.E. et al.** *Annual Review Immunology,* 1991, vol. 9, 67-95
- **Stewart, G.A. et al.** *Epitopes of Atopic Allergens, Proceedings of Workshop from XIV Congress of the European Academy of Allergy and Clinical Immunology,* September 1989, 41-47
- **Yessel, H. et al.** *T cell Activation in Health and Disease: Discrimination Between Immunity and Tolerance,* 22 September 1990
- **Hessel, H. et al.** *Journal of Immunology,* 01 February 1992, vol. 148 (3), 738-745
- **Sambrook et al.** Molecular Cloning: A Laboratory Manual. Cold Spring Harbor Laboratory Press, 1989
- **Baldari et al.** *Embo J.,* 1987, vol. 6, 229-234
- **Kurjan ; Herskowitz.** *Cell,* 1982, vol. 30, 933-943
- **Schultz et al.** *Gene,* 1987, vol. 54, 113-123
- **Amann et al.** *Gene,* 1988, vol. 69, 301-315
- **Jameel et al.** *J. Virol.,* 1990, vol. 64, 3963-3966
- **Knapp et al.** *BioTechniques,* 1990, vol. 8, 280-281
- **Varney et al.** *British Medical Journal,* 1990, vol. 302, 265-269
- **Wie et al.** *International Archives of Allergy and Applied Immunology,* 1981, vol. 64, 84-99
- **Strejan et al.** *Journal of Neuroimmunology,* 1984, vol. 7, 27
- **Chapman et al.** *J. Allergy Clin. Immunol.,* 1987, vol. 80, 1479-1484
- **Heymann et al.** *J. Allergy Clin. Immunol.,* 1989, vol. 83, 1055-1067
- **Hochuli et al.** *Biotechnology,* 1988, vol. 86, 1321-1325
- **Studier et al.** *Methods In Enzymology,* 1990, vol. 185, 60-88
- **Chua et al.** *J. Exp. Med.,* 1988, vol. 167, 175-182
- **Dilworth et al.** *Clin. Exp. Allergy,* 1991, vol. 21, 25-32
- **Skoglund et al.** *Gene,* 1990, vol. 88, 1-5
- **Rafnar et al.** *J. Biol. Chem.,* 1991, vol. 226, 1229-1236
- **Chua et al.** *Int Arch. Appl. Immun.,* 1990, vol. 91, 118-123
- **Trudinger et al.** *Clin. Exp. Allergy,* 1991, vol. 21, 33-37
- **Kunkel.** *Proc. Natl. Acad. Sci. USA.,* 1985, vol. 82, 488-492

SEQUENCE LISTING

    (1) GENERAL INFORMATION:

        (i) APPLICANT: Kuo, Mei-chang
        Garman, Richard
        Greenstein, Julia

        (ii) TITLE OF INVENTION: T CELL EPITOPES OF THE MAJOR ALLERGENS FROM DERMATOPHAGOIDES (HOUSE DUST MITE)

(iii) NUMBER OF SEQUENCES: 119

(iv) CORRESPONDENCE ADDRESS:

(A) ADDRESSEE: LAHIVE & COCKFIELD
(B) STREET: 60 STATE STREET SUITE 510
(C) CITY: BOSTON
(D) STATE: MA
(E) COUNTRY: USA
(F) ZIP: 02109

(v) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: ASCII TEXT

(vi) CURRENT APPLICATION DATA:

(A) APPLICATION NUMBER: US 07/881,396

(B) FILING DATE: 08-MAY-1992

(C) CLASSIFICATION:

(viii) ATTORNEY/AGENT INFORMATION:

(A) NAME: MANDRAGOURAS, AMY E.
(B) REGISTRATION NUMBER: P36,207
(C) REFERENCE/DOCKET NUMBER: IMI-012/IPC-017C

(ix) TELECOMMUNICATION INFORMATION:

(A) TELEPHONE: (617) 227-7400
(B) TELEFAX: (617) 227-5941

(2) INFORMATION FOR SEQ ID NO:1:

(i) SEQUENCE CHARACTERISTICS:

(A) LENGTH: 834 base pairs
(B) TYPE: nucleic acid
(C) STRANDEDNESS: single
(D) TOPOLOGY: linear

(ii) MOLECULE TYPE: cDNA

(ix) FEATURE:

(A) NAME/KEY: CDS
(B) LOCATION: 1..738

(xi) SEQUENCE DESCRIPTION: SEQ ID NO:1:

```
AAA AAC CGA TTT TTG ATG AGT GCA GAA GCT TTT GAA CAC CTC AAA ACT
48
Lys Asn Arg Phe Leu Met Ser Ala Glu Ala Phe Glu His Leu Lys Thr
 1               5              10                  15

CAA TTC GAT TTG AAT GCT GAA ACT AAC GCC TGC AGT ATC AAT GGA AAT
96
Gln Phe Asp Leu Asn Ala Glu Thr Asn Ala Cys Ser Ile Asn Gly Asn
             20              25                  30

GCT CCA GCT GAA ATC GAT TTG CGA CAA ATG CGA ACT GTC ACT CCC ATT
144
Ala Pro Ala Glu Ile Asp Leu Arg Gln Met Arg Thr Val Thr Pro Ile
         35              40                  45

CGT ATG CAA GGA GGC TGT GGT TCA TGT TGG GCT TTC TCT GGT GTT GCC
192
Arg Met Gln Gly Gly Cys Gly Ser Cys Trp Ala Phe Ser Gly Val Ala
     50              55                  60

GCA ACT GAA TCA GCT TAT TTG GCT CAC CGT AAT CAA TCA TTG GAT CTT
240
Ala Thr Glu Ser Ala Tyr Leu Ala His Arg Asn Gln Ser Leu Asp Leu
65              70                  75                      80

GCT GAA CAA GAA TTA GTC GAT TGT GCT TCC CAA CAC GGT TGT CAT GGT
288
Ala Glu Gln Glu Leu Val Asp Cys Ala Ser Gln His Gly Cys His Gly
             85              90                  95

GAT ACC ATT CCA CGT GGT ATT GAA TAC ATC CAA CAT AAT GGT GTC GTC
336
Asp Thr Ile Pro Arg Gly Ile Glu Tyr Ile Gln His Asn Gly Val Val
             100             105                 110

CAA GAA AGC TAC TAT CGA TAC GTT GCA CGA GAA CAA TCA TGC CGA CGA
384
Gln Glu Ser Tyr Tyr Arg Tyr Val Ala Arg Glu Gln Ser Cys Arg Arg
         115             120                 125

CCA AAT GCA CAA CGT TTC GGT ATC TCA AAC TAT TGC CAA ATT TAC CCA
432
Pro Asn Ala Gln Arg Phe Gly Ile Ser Asn Tyr Cys Gln Ile Tyr Pro
     130             135                 140

CCA AAT GCA AAC AAA ATT CGT GAA GCT TTG GCT CAA ACC CAC AGC GCT
480
Pro Asn Ala Asn Lys Ile Arg Glu Ala Leu Ala Gln Thr His Ser Ala
145             150                 155                     160

ATT GCC GTC ATT ATT GGC ATC AAA GAT TTA GAC GCA TTC CGT CAT TAT
528
Ile Ala Val Ile Ile Gly Ile Lys Asp Leu Asp Ala Phe Arg His Tyr
             165             170                 175

GAT GGC CGA ACA ATC ATT CAA CGC GAT AAT GGT TAC CAA CCA AAC TAT
576
Asp Gly Arg Thr Ile Ile Gln Arg Asp Asn Gly Tyr Gln Pro Asn Tyr
             180             185                 190
```

21

```
CAC GCT GTC AAC ATT GTT GGT TAC AGT AAC GCA CAA GGT GTC GAT TAT
624
His Ala Val Asn Ile Val Gly Tyr Ser Asn Ala Gln Gly Val Asp Tyr
        195                 200                 205

TGG ATC GTA CGA AAC AGT TGG GAT ACC AAT TGG GGT GAT AAT GGT TAC
672
Trp Ile Val Arg Asn Ser Trp Asp Thr Asn Trp Gly Asp Asn Gly Tyr
    210                 215                 220

GGT TAT TTT GCT GCC AAC ATC GAT TTG ATG ATG ATT GAA GAA TAT CCA
720
Gly Tyr Phe Ala Ala Asn Ile Asp Leu Met Met Ile Glu Glu Tyr Pro
225                 230                 235                 240

TAT GTT GTC ATT CTC TAAACAAAAA GACAATTTCT TATATGATTG TCACTAATTT
775
Tyr Val Val Ile Leu
                245

ATTTAAAATC AAAATTTTTT AGAAAATGAA TAAATTCATT CACAAAAATT AAAAAAAAA
834
```

(2) INFORMATION FOR SEQ ID NO:2:

    (i) SEQUENCE CHARACTERISTICS:

        (A) LENGTH: 245 amino acids
        (B) TYPE: amino acid
        (D) TOPOLOGY: linear

    (ii) MOLECULE TYPE: protein
    (xi) SEQUENCE DESCRIPTION: SEQ ID NO:2:

```
Lys Asn Arg Phe Leu Met Ser Ala Glu Ala Phe Glu His Leu Lys Thr
 1               5                  10                  15

Gln Phe Asp Leu Asn Ala Glu Thr Asn Ala Cys Ser Ile Asn Gly Asn
            20                  25                  30

Ala Pro Ala Glu Ile Asp Leu Arg Gln Met Arg Thr Val Thr Pro Ile
        35                  40                  45

Arg Met Gln Gly Gly Cys Gly Ser Cys Trp Ala Phe Ser Gly Val Ala
        50                  55                  60

Ala Thr Glu Ser Ala Tyr Leu Ala His Arg Asn Gln Ser Leu Asp Leu
 65                  70                  75                  80

Ala Glu Gln Glu Leu Val Asp Cys Ala Ser Gln His Gly Cys His Gly
                85                  90                  95

Asp Thr Ile Pro Arg Gly Ile Glu Tyr Ile Gln His Asn Gly Val Val
            100                 105                 110

Gln Glu Ser Tyr Tyr Arg Tyr Val Ala Arg Glu Gln Ser Cys Arg Arg
            115                 120                 125

Pro Asn Ala Gln Arg Phe Gly Ile Ser Asn Tyr Cys Gln Ile Tyr Pro
    130                 135                 140

Pro Asn Ala Asn Lys Ile Arg Glu Ala Leu Ala Gln Thr His Ser Ala
145                 150                 155                 160

Ile Ala Val Ile Ile Gly Ile Lys Asp Leu Asp Ala Phe Arg His Tyr
                165                 170                 175

Asp Gly Arg Thr Ile Ile Gln Arg Asp Asn Gly Tyr Gln Pro Asn Tyr
            180                 185                 190

His Ala Val Asn Ile Val Gly Tyr Ser Asn Ala Gln Gly Val Asp Tyr
            195                 200                 205

Trp Ile Val Arg Asn Ser Trp Asp Thr Asn Trp Gly Asp Asn Gly Tyr
    210                 215                 220

Gly Tyr Phe Ala Ala Asn Ile Asp Leu Met Met Ile Glu Glu Tyr Pro
225                 230                 235                 240

Tyr Val Val Ile Leu
                245
```

(2) INFORMATION FOR SEQ ID NO:3:

   (i) SEQUENCE CHARACTERISTICS:

      (A) LENGTH: 588 base pairs
      (B) TYPE: nucleic acid
      (C) STRANDEDNESS: single
      (D) TOPOLOGY: linear

   (ii) MOLECULE TYPE: cDNA
   (ix) FEATURE:

(A) NAME/KEY: CDS
(B) LOCATION: 69..509

(xi) SEQUENCE DESCRIPTION: SEQ ID NO:3:


CACAAATTCT TCTTTCTTCC TTACTACTGA TCATTAATCT GAAAACAAAA CCAAACAAAC
60

CATTCAAA ATG ATG TAC AAA ATT TTG TGT CTT TCA TTG TTG GTC GCA GCC
110
         Met Met Tyr Lys Ile Leu Cys Leu Ser Leu Leu Val Ala Ala
         1           5                10

GTT GCT CGT GAT CAA GTC GAT GTC AAA GAT TGT GCC AAT CAT GAA ATC
158
Val Ala Arg Asp Gln Val Asp Val Lys Asp Cys Ala Asn His Glu Ile
 15                20             25                30

AAA AAA GTT TTG GTA CCA GGA TGC CAT GGT TCA GAA CCA TGT ATC ATT
206
Lys Lys Val Leu Val Pro Gly Cys His Gly Ser Glu Pro Cys Ile Ile
             35              40               45

CAT CGT GGT AAA CCA TTC CAA TTG GAA GCC GTT TTC GAA GCC AAC CAA
254
His Arg Gly Lys Pro Phe Gln Leu Glu Ala Val Phe Glu Ala Asn Gln
             50              55              60


AAC ACA AAA ACG GCT AAA ATT GAA ATC AAA GCC TCA ATC GAT GGT TTA
302
Asn Thr Lys Thr Ala Lys Ile Glu Ile Lys Ala Ser Ile Asp Gly Leu
        65             70              75

GAA GTT GAT GTT CCC GGT ATC GAT CCA AAT GCA TGC CAT TAC ATG AAA
350
Glu Val Asp Val Pro Gly Ile Asp Pro Asn Ala Cys His Tyr Met Lys
    80               85              90

TGC CCA TTG GTT AAA GGA CAA CAA TAT GAT ATT AAA TAT ACA TGG AAT
398
Cys Pro Leu Val Lys Gly Gln Gln Tyr Asp Ile Lys Tyr Thr Trp Asn
 95               100           105              110

GTT CCG AAA ATT GCA CCA AAA TCT GAA AAT GTT GTC GTC ACT GTT AAA
446
Val Pro Lys Ile Ala Pro Lys Ser Glu Asn Val Val Val Thr Val Lys
             115              120             125

GTT ATG GGT GAT GAT GGT GTT TTG GCC TGT GCT ATT GCT ACT CAT GCT
494
Val Met Gly Asp Asp Gly Val Leu Ala Cys Ala Ile Ala Thr His Ala
             130              135             140

AAA ATC CGC GAT TAAATAAACA AAATTTATTG ATTTTGTAAT CACAAATGAT
546
Lys Ile Arg Asp
         145


TGATTTTCTT TCCAAAAAAA AAATAAATAA AATTTTGGGA AT
588

(2) INFORMATION FOR SEQ ID NO:4:

    (i) SEQUENCE CHARACTERISTICS:

        (A) LENGTH: 146 amino acids
        (B) TYPE: amino acid
        (D) TOPOLOGY: linear

    (ii) MOLECULE TYPE: protein
    (xi) SEQUENCE DESCRIPTION: SEQ ID NO:4:

```
Met Met Tyr Lys Ile Leu Cys Leu Ser Leu Leu Val Ala Ala Val Ala
 1               5                  10                  15

Arg Asp Gln Val Asp Val Lys Asp Cys Ala Asn His Glu Ile Lys Lys
            20                  25                  30

Val Leu Val Pro Gly Cys His Gly Ser Glu Pro Cys Ile Ile His Arg
            35                  40                  45

Gly Lys Pro Phe Gln Leu Glu Ala Val Phe Glu Ala Asn Gln Asn Thr
        50                  55                  60

Lys Thr Ala Lys Ile Glu Ile Lys Ala Ser Ile Asp Gly Leu Glu Val
    65                  70                  75                  80

Asp Val Pro Gly Ile Asp Pro Asn Ala Cys His Tyr Met Lys Cys Pro
                85                  90                  95

Leu Val Lys Gly Gln Gln Tyr Asp Ile Lys Tyr Thr Trp Asn Val Pro
            100                 105                 110

Lys Ile Ala Pro Lys Ser Glu Asn Val Val Val Thr Val Lys Val Met
            115                 120                 125

Gly Asp Asp Gly Val Leu Ala Cys Ala Ile Ala Thr His Ala Lys Ile
        130                 135                 140

Arg Asp
145
```

(2) INFORMATION FOR SEQ ID NO:5:

    (i) SEQUENCE CHARACTERISTICS:

        (A) LENGTH: 1072 base pairs
        (B) TYPE: nucleic acid
        (C) STRANDEDNESS: single
        (D) TOPOLOGY: linear

    (ii) MOLECULE TYPE: cDNA
    (ix) FEATURE:

        (A) NAME/KEY: CDS
        (B) LOCATION: 36..1001

    (xi) SEQUENCE DESCRIPTION: SEQ ID NO:5:

```
CGTTTTCTTC CATCAAAATT AAAAATTCAT CAAAA ATG AAA TTC GTT TTG GCC
53
                                          Met Lys Phe Val Leu Ala
                                           1                   5

ATT GCC TCT TTG TTG GTA TTG AGC ACT GTT TAT GCT CGT CCA GCT TCA
101
Ile Ala Ser Leu Leu Val Leu Ser Thr Val Tyr Ala Arg Pro Ala Ser
                10              15                  20

ATC AAA ACT TTT GAA GAA TTC AAA AAA GCC TTC AAC AAA AAC TAT GCC
149
Ile Lys Thr Phe Glu Glu Phe Lys Lys Ala Phe Asn Lys Asn Tyr Ala
        25              30              35

ACC GTT GAA GAG GAA GAA GTT GCC CGT AAA AAC TTT TTG GAA TCA TTG
197
Thr Val Glu Glu Glu Glu Val Ala Arg Lys Asn Phe Leu Glu Ser Leu
    40                  45              50

AAA TAT GTT GAA GCT AAC AAA GGT GCC ATC AAC CAT TTG TCC GAT TTG
245
Lys Tyr Val Glu Ala Asn Lys Gly Ala Ile Asn His Leu Ser Asp Leu
55                  60              65                  70

TCA TTG GAT GAA TTC AAA AAC CGT TAT TTG ATG AGT GCT GAA GCT TTT
293
Ser Leu Asp Glu Phe Lys Asn Arg Tyr Leu Met Ser Ala Glu Ala Phe
                75              80                  85

GAA CAA CTC AAA ACT CAA TTC GAT TTG AAT GCC GAA ACA AGC GCT TGC
341
Glu Gln Leu Lys Thr Gln Phe Asp Leu Asn Ala Glu Thr Ser Ala Cys
                90              95                  100

CGT ATC AAT TCG GTT AAC GTT CCA TCG GAA TTG GAT TTA CGA TCA CTG
389
Arg Ile Asn Ser Val Asn Val Pro Ser Glu Leu Asp Leu Arg Ser Leu
            105             110                 115
```

```
CGA ACT GTC ACT CCA ATC CGT ATG CAA GGA GGC TGT GGT TCA TGT TGG
437
Arg Thr Val Thr Pro Ile Arg Met Gln Gly Gly Cys Gly Ser Cys Trp
    120                 125                 130

GCT TTC TCT GGT GTT GCC GCA ACT GAA TCA GCT TAT TTG GCC TAC CGT
485
Ala Phe Ser Gly Val Ala Ala Thr Glu Ser Ala Tyr Leu Ala Tyr Arg
135                 140                 145                     150

AAC ACG TCT TTG GAT CTT TCT GAA CAG GAA CTC GTC GAT TGC GCA TCT
533
Asn Thr Ser Leu Asp Leu Ser Glu Gln Glu Leu Val Asp Cys Ala Ser
                155                 160                 165

CAA CAC GGA TGT CAC GGC GAT ACA ATA CCA AGA GGC ATC GAA TAC ATC
581
Gln His Gly Cys His Gly Asp Thr Ile Pro Arg Gly Ile Glu Tyr Ile
                170                 175                 180

CAA CAA AAT GGT GTC GTT GAA GAA AGA AGC TAT CCA TAC GTT GCA CGA
629
Gln Gln Asn Gly Val Val Glu Glu Arg Ser Tyr Pro Tyr Val Ala Arg
            185                 190                 195

GAA CAA CGA TGC CGA CGA CCA AAT TCG CAA CAT TAC GGT ATC TCA AAC
677
Glu Gln Arg Cys Arg Arg Pro Asn Ser Gln His Tyr Gly Ile Ser Asn
    200                 205                 210

TAC TGC CAA ATT TAT CCA CCA GAT GTG AAA CAA ATC CGT GAA GCT TTG
725
Tyr Cys Gln Ile Tyr Pro Pro Asp Val Lys Gln Ile Arg Glu Ala Leu
215                 220                 225                     230

ACT CAA ACA CAC ACA GCT ATT GCC GTC ATT ATT GGC ATC AAA GAT TTG
773
Thr Gln Thr His Thr Ala Ile Ala Val Ile Ile Gly Ile Lys Asp Leu
                235                 240                 245

AGA GCT TTC CAA CAT TAT GAT GGA CGA ACA ATC ATT CAA CAT GAC AAT
821
Arg Ala Phe Gln His Tyr Asp Gly Arg Thr Ile Ile Gln His Asp Asn
                250                 255                 260

GGT TAT CAA CCA AAC TAT CAT GCC GTC AAC ATT GTC GGT TAC GGA AGT
869
Gly Tyr Gln Pro Asn Tyr His Ala Val Asn Ile Val Gly Tyr Gly Ser
            265                 270                 275

ACA CAA GGC GAC GAT TAT TGG ATC GTA CGA AAC AGT TGG GAT ACT ACC
917
Thr Gln Gly Asp Asp Tyr Trp Ile Val Arg Asn Ser Trp Asp Thr Thr
    280                 285                 290
```

27

```
TGG GGA GAT AGC GGA TAC GGA TAT TTC CAA GCC GGA AAC AAC CTC ATG
965
Trp Gly Asp Ser Gly Tyr Gly Tyr Phe Gln Ala Gly Asn Asn Leu Met
295               300           305                   310

ATG ATC GAA CAA TAT CCA TAT GTT GTA ATC ATG TGAACATTTG AAATTGAATA
1018
Met Ile Glu Gln Tyr Pro Tyr Val Val Ile Met
            315           320

TATTTATTTG TTTTCAAAAT AAAAACAACT ACTCTTGCGA GTATTTTTTA CTCG
1072
```

(2) INFORMATION FOR SEQ ID NO:6:

   (i) SEQUENCE CHARACTERISTICS:

      (A) LENGTH: 321 amino acids
      (B) TYPE: amino acid
      (D) TOPOLOGY: linear

   (ii) MOLECULE TYPE: protein
   (xi) SEQUENCE DESCRIPTION: SEQ ID NO:6:

```
Met Lys Phe Val Leu Ala Ile Ala Ser Leu Leu Val Leu Ser Thr Val
 1               5                  10                  15

Tyr Ala Arg Pro Ala Ser Ile Lys Thr Phe Glu Glu Phe Lys Lys Ala
            20              25                  30

Phe Asn Lys Asn Tyr Ala Thr Val Glu Glu Glu Glu Val Ala Arg Lys
        35              40                  45

Asn Phe Leu Glu Ser Leu Lys Tyr Val Glu Ala Asn Lys Gly Ala Ile
    50              55                  60

Asn His Leu Ser Asp Leu Ser Leu Asp Glu Phe Lys Asn Arg Tyr Leu
65              70                  75                      80

Met Ser Ala Glu Ala Phe Glu Gln Leu Lys Thr Gln Phe Asp Leu Asn
            85                  90                      95

Ala Glu Thr Ser Ala Cys Arg Ile Asn Ser Val Asn Val Pro Ser Glu
        100             105                 110

Leu Asp Leu Arg Ser Leu Arg Thr Val Thr Pro Ile Arg Met Gln Gly
        115                 120                 125

Gly Cys Gly Ser Cys Trp Ala Phe Ser Gly Val Ala Ala Thr Glu Ser
    130             135                 140

Ala Tyr Leu Ala Tyr Arg Asn Thr Ser Leu Asp Leu Ser Glu Gln Glu
145             150                 155                 160

Leu Val Asp Cys Ala Ser Gln His Gly Cys His Gly Asp Thr Ile Pro
            165             170                 175

Arg Gly Ile Glu Tyr Ile Gln Gln Asn Gly Val Val Glu Glu Arg Ser
        180             185                 190

Tyr Pro Tyr Val Ala Arg Glu Gln Arg Cys Arg Arg Pro Asn Ser Gln
        195             200             205

His Tyr Gly Ile Ser Asn Tyr Cys Gln Ile Tyr Pro Pro Asp Val Lys
    210             215                 220


Gln Ile Arg Glu Ala Leu Thr Gln Thr His Thr Ala Ile Ala Val Ile
225             230                 235                 240

Ile Gly Ile Lys Asp Leu Arg Ala Phe Gln His Tyr Asp Gly Arg Thr
            245             250                 255

Ile Ile Gln His Asp Asn Gly Tyr Gln Pro Asn Tyr His Ala Val Asn
        260             265                 270

Ile Val Gly Tyr Gly Ser Thr Gln Gly Asp Asp Tyr Trp Ile Val Arg
        275             280                 285

Asn Ser Trp Asp Thr Thr Trp Gly Asp Ser Gly Tyr Gly Tyr Phe Gln
    290             295                 300

Ala Gly Asn Asn Leu Met Met Ile Glu Gln Tyr Pro Tyr Val Val Ile
305             310                 315                 320

Met
```

29

(2) INFORMATION FOR SEQ ID NO:7:

    (i) SEQUENCE CHARACTERISTICS:

        (A) LENGTH: 491 base pairs
        (B) TYPE: nucleic acid
        (C) STRANDEDNESS: single
        (D) TOPOLOGY: linear

    (ii) MOLECULE TYPE: cDNA
    (ix) FEATURE:

        (A) NAME/KEY: CDS
        (B) LOCATION: 1..390

    (xi) SEQUENCE DESCRIPTION: SEQ ID NO:7:

```
GAT CAA GTC GAT GTT AAA GAT TGT GCC AAC AAT GAA ATC AAA AAA GTA
48
Asp Gln Val Asp Val Lys Asp Cys Ala Asn Asn Glu Ile Lys Lys Val
  1               5                  10                  15

ATG GTC GAT GGT TGC CAT GGT TCT GAT CCA TGC ATA ATC CAT CGT GGT
96
Met Val Asp Gly Cys His Gly Ser Asp Pro Cys Ile Ile His Arg Gly
               20                  25                  30

AAA CCA TTC ACT TTG GAA GCC TTA TTC GAT GCC AAC CAA AAC ACT AAA
144
Lys Pro Phe Thr Leu Glu Ala Leu Phe Asp Ala Asn Gln Asn Thr Lys
               35                  40                  45

ACC GCT AAA ACT GAA ATC AAA GCC AGC CTC GAT GGT CTT GAA ATT GAT
192
Thr Ala Lys Thr Glu Ile Lys Ala Ser Leu Asp Gly Leu Glu Ile Asp
       50                  55                  60
```

.

```
GTT CCC GGT ATT GAT ACC AAT GCT TGC CAT TTT ATG AAA TGT CCA TTG
240
Val Pro Gly Ile Asp Thr Asn Ala Cys His Phe Met Lys Cys Pro Leu
 65                  70                  75                  80

GTT AAA GGT CAA CAA TAT GAT GCC AAA TAT ACA TGG AAT GTG CCC AAA
288
Val Lys Gly Gln Gln Tyr Asp Ala Lys Tyr Thr Trp Asn Val Pro Lys
                85                  90                  95

ATT GCA CCA AAA TCT GAA AAC GTT GTC GTT ACA GTC AAA CTT GTT GGT
336
Ile Ala Pro Lys Ser Glu Asn Val Val Val Thr Val Lys Leu Val Gly
            100                 105                 110

GAT AAT GGT GTT TTG GCT TGC GCT ATT GCT ACC CAC GCT AAA ATC CGT
384
Asp Asn Gly Val Leu Ala Cys Ala Ile Ala Thr His Ala Lys Ile Arg
        115                 120                 125

GAT TAAAAAAAAA AAATAAATAT GAAAATTTTC ACCAACATCG AACAAAATTC
437
Asp
    130

AATAACCAAA ATTTGAATCA AAAACGGAAT TCCAAGCTGA GCGCCGGTCG CTAC
491
```

(2) INFORMATION FOR SEQ ID NO:8:

    (i) SEQUENCE CHARACTERISTICS:

        (A) LENGTH: 129 amino acids
        (B) TYPE: amino acid
        (D) TOPOLOGY: linear

    (ii) MOLECULE TYPE: protein
    (xi) SEQUENCE DESCRIPTION: SEQ ID NO:8:

```
Asp Gln Val Asp Val Lys Asp Cys Ala Asn Asn Glu Ile Lys Lys Val
  1               5                  10                  15

Met Val Asp Gly Cys His Gly Ser Asp Pro Cys Ile Ile His Arg Gly
            20                  25                  30

Lys Pro Phe Thr Leu Glu Ala Leu Phe Asp Ala Asn Gln Asn Thr Lys
        35                  40                  45

Thr Ala Lys Thr Glu Ile Lys Ala Ser Leu Asp Gly Leu Glu Ile Asp
    50                  55                  60

Val Pro Gly Ile Asp Thr Asn Ala Cys His Phe Met Lys Cys Pro Leu
 65                  70                  75                  80

Val Lys Gly Gln Gln Tyr Asp Ala Lys Tyr Thr Trp Asn Val Pro Lys
                85                  90                  95

Ile Ala Pro Lys Ser Glu Asn Val Val Val Thr Val Lys Leu Val Gly
            100                 105                 110
```

```
        Asp Asn Gly Val Leu Ala Cys Ala Ile Ala Thr His Ala Lys Ile Arg
                115                     120             125

        Asp
```

(2) INFORMATION FOR SEQ ID NO:9:

    (i) SEQUENCE CHARACTERISTICS:

        (A) LENGTH: 20 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:9:

```
        Thr Asn Ala Cys Ser Ile Asn Gly Asn Ala Pro Ala Glu Ile Asp Leu
        1                 5                 10                  15

        Arg Gln Met Arg
                    20
```

(2) INFORMATION FOR SEQ ID NO:10:

    (i) SEQUENCE CHARACTERISTICS:

        (A) LENGTH: 27 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:10:

```
        Glu Ile Asp Leu Arg Gln Met Arg Thr Val Thr Pro Ile Arg Met Gln
        1                 5                 10                  15

        Gly Gly Cys Gly Ser Cys Trp Ala Phe Ser Gly
                    20              25
```

(2) INFORMATION FOR SEQ ID NO:11:

    (i) SEQUENCE CHARACTERISTICS:

        (A) LENGTH: 29 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:11:

```
Thr Val Thr Pro Ile Arg Met Gln Gly Gly Cys Gly Ser Cys Trp Ala
1               5               10              15
```

```
Phe Ser Gly Val Ala Ala Thr Glu Ser Ala Tyr Leu Ala
            20              25
```

(2) INFORMATION FOR SEQ ID NO:12:

    (i) SEQUENCE CHARACTERISTICS:

        (A) LENGTH: 21 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:12:

```
Val Ala Ala Thr Glu Ser Ala Tyr Leu Ala His Arg Asn Gln Ser Leu
1               5               10              15
```

```
Asp Leu Ala Glu Gln
            20
```

(2) INFORMATION FOR SEQ ID NO:13:

    (i) SEQUENCE CHARACTERISTICS:

        (A) LENGTH: 22 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:13:

```
His Arg Asn Gln Ser Leu Asp Leu Ala Glu Gln Glu Leu Val Asp Cys
1               5               10              15
```

```
Ala Ser Gln His Gly Cys
            20
```

(2) INFORMATION FOR SEQ ID NO:14:

    (i) SEQUENCE CHARACTERISTICS:

        (A) LENGTH: 21 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:14:

```
Glu Leu Val Asp Cys Ala Ser Gln His Gly Cys His Gly Asp Thr Ile
1               5               10              15

Pro Arg Gly Ile Glu
            20
```

(2) INFORMATION FOR SEQ ID NO:15:

   (i) SEQUENCE CHARACTERISTICS:

      (A) LENGTH: 28 amino acids
      (B) TYPE: amino acid
      (D) TOPOLOGY: linear

   (ii) MOLECULE TYPE: peptide
   (v) FRAGMENT TYPE: N-terminal
   (xi) SEQUENCE DESCRIPTION: SEQ ID NO:15:

```
Gly Asp Thr Ile Pro Arg Gly Ile Glu Tyr Ile Gln His Asn Gly Val
1               5               10              15

Val Gln Glu Ser Tyr Tyr Arg Tyr Val Ala Arg Glu
            20              25
```

(2) INFORMATION FOR SEQ ID NO:16:

   (i) SEQUENCE CHARACTERISTICS:

      (A) LENGTH: 20 amino acids
      (B) TYPE: amino acid
      (D) TOPOLOGY: linear

   (ii) MOLECULE TYPE: peptide
   (v) FRAGMENT TYPE: N-terminal
   (xi) SEQUENCE DESCRIPTION: SEQ ID NO:16:

```
Glu Tyr Ile Gln His Asn Gly Val Val Gln Glu Ser Tyr Tyr Arg Tyr
1               5               10              15

Val Ala Arg Glu
            20
```

(2) INFORMATION FOR SEQ ID NO:17:

   (i) SEQUENCE CHARACTERISTICS:

      (A) LENGTH: 25 amino acids
      (B) TYPE: amino acid
      (D) TOPOLOGY: linear

   (ii) MOLECULE TYPE: peptide
   (v) FRAGMENT TYPE: N-terminal
   (xi) SEQUENCE DESCRIPTION: SEQ ID NO:17:

```
        His Asn Gly Val Val Gln Glu Ser Tyr Tyr Arg Tyr Val Ala Arg Glu
        1                   5                   10                  15

        Gln Ser Cys Arg Arg Pro Asn Ala Gln
                        20              25
```

(2) INFORMATION FOR SEQ ID NO:18:

  (i) SEQUENCE CHARACTERISTICS:

    (A) LENGTH: 19 amino acids
    (B) TYPE: amino acid
    (D) TOPOLOGY: linear

  (ii) MOLECULE TYPE: peptide
  (v) FRAGMENT TYPE: N-terminal
  (xi) SEQUENCE DESCRIPTION: SEQ ID NO:18:

```
        Gln Ser Cys Arg Arg Pro Asn Ala Gln Arg Phe Gly Ile Ser Asn Tyr
        1                   5                   10                  15

        Cys Gln Ile
```

(2) INFORMATION FOR SEQ ID NO:19:

  (i) SEQUENCE CHARACTERISTICS:

    (A) LENGTH: 22 amino acids
    (B) TYPE: amino acid
    (D) TOPOLOGY: linear

  (ii) MOLECULE TYPE: peptide
  (v) FRAGMENT TYPE: N-terminal
  (xi) SEQUENCE DESCRIPTION: SEQ ID NO:19:

```
        Arg Phe Gly Ile Ser Asn Tyr Cys Gln Ile Tyr Pro Pro Asn Ala Asn
        1                   5                   10                  15

        Lys Ile Arg Glu Ala Leu
                        20
```

(2) INFORMATION FOR SEQ ID NO:20:

  (i) SEQUENCE CHARACTERISTICS:

    (A) LENGTH: 24 amino acids
    (B) TYPE: amino acid
    (D) TOPOLOGY: linear

  (ii) MOLECULE TYPE: peptide
  (v) FRAGMENT TYPE: N-terminal
  (xi) SEQUENCE DESCRIPTION: SEQ ID NO:20:

```
Tyr Pro Pro Asn Ala Asn Lys Ile Arg Glu Ala Leu Ala Gln Thr His
1               5                   10                  15

Ser Ala Ile Ala Val Ile Ile Gly
            20
```

(2) INFORMATION FOR SEQ ID NO:21:

(i) SEQUENCE CHARACTERISTICS:

(A) LENGTH: 26 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:21:

```
Ala Gln Thr His Ser Ala Ile Ala Val Ile Ile Gly Ile Lys Asp Leu
1               5                   10                  15

Asp Ala Phe Arg His Tyr Asp Gly Arg Thr
            20              25
```

(2) INFORMATION FOR SEQ ID NO:22:

(i) SEQUENCE CHARACTERISTICS:

(A) LENGTH: 26 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:22:

```
Ile Lys Asp Leu Asp Ala Phe Arg His Tyr Asp Gly Thr Arg Ile Ile
1               5                   10                  15

Gln Arg Asp Asn Gly Tyr Gln Pro Asn Tyr
            20              25
```

(2) INFORMATION FOR SEQ ID NO:23:

(i) SEQUENCE CHARACTERISTICS:

(A) LENGTH: 23 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:23:

```
Ile Ile Gln Arg Asp Asn Gly Tyr Gln Pro Asn Tyr His Ala Val Asn
1                   5                   10                  15

Ile Val Gly Tyr Ser Asn Ala
                20
```

(2) INFORMATION FOR SEQ ID NO:24:

   (i) SEQUENCE CHARACTERISTICS:

      (A) LENGTH: 22 amino acids
      (B) TYPE: amino acid
      (D) TOPOLOGY: linear

   (ii) MOLECULE TYPE: peptide
   (v) FRAGMENT TYPE: N-terminal
   (xi) SEQUENCE DESCRIPTION: SEQ ID NO:24:

```
His Ala Val Asn Ile Val Gly Tyr Ser Asn Ala Gln Gly Val Asp Tyr
1                   5                   10                  15

Trp Ile Val Arg Asn Ser
                20
```

(2) INFORMATION FOR SEQ ID NO:25:

   (i) SEQUENCE CHARACTERISTICS:

      (A) LENGTH: 24 amino acids
      (B) TYPE: amino acid
      (D) TOPOLOGY: linear

   (ii) MOLECULE TYPE: peptide
   (v) FRAGMENT TYPE: N-terminal
   (xi) SEQUENCE DESCRIPTION: SEQ ID NO:25:

```
Gln Gly Val Asp Tyr Trp Ile Val Arg Asn Ser Trp Asp Thr Asn Trp
1                   5                   10                  15

Gly Asp Asn Gly Tyr Gly Tyr Phe
                20
```

(2) INFORMATION FOR SEQ ID NO:26:

   (i) SEQUENCE CHARACTERISTICS:

      (A) LENGTH: 26 amino acids
      (B) TYPE: amino acid
      (D) TOPOLOGY: linear

   (ii) MOLECULE TYPE: peptide
   (v) FRAGMENT TYPE: N-terminal

(xi) SEQUENCE DESCRIPTION: SEQ ID NO:26:

```
Gly Asp Asn Gly Tyr Gly Tyr Phe Ala Ala Asn Ile Asp Leu Met Met
1               5                   10                  15

Ile Glu Glu Tyr Pro Tyr Val Val Ile Leu
            20                  25
```

(2) INFORMATION FOR SEQ ID NO:27:

(i) SEQUENCE CHARACTERISTICS:

(A) LENGTH: 28 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:27:

```
Thr Asn Ala Cys Ser Ile Asn Gly Asn Ala Pro Ala Glu Ile Asp Leu
1               5                   10                  15

Arg Gln Met Arg Thr Val Thr Pro Ile Arg Met Gln
            20                  25
```

(2) INFORMATION FOR SEQ ID NO:28:

(i) SEQUENCE CHARACTERISTICS:

(A) LENGTH: 24 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:28:

```
Ser Ile Asn Gly Asn Ala Pro Ala Glu Ile Asp Leu Arg Gln Met Arg
1               5                   10                  15

Thr Val Thr Pro Ile Arg Met Gln
            20
```

(2) INFORMATION FOR SEQ ID NO:29:

(i) SEQUENCE CHARACTERISTICS:

(A) LENGTH: 29 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:29:

```
        Ala Phe Ser Gly Val Ala Ala Thr Glu Ser Ala Tyr Leu Ala His Arg
        1               5                   10                  15

        Asn Gln Ser Leu Asp Leu Ala Glu Gln Glu Leu Val Asp
                    20                  25
```

(2) INFORMATION FOR SEQ ID NO:30:

    (i) SEQUENCE CHARACTERISTICS:

        (A) LENGTH: 25 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:30:

```
        Val Ala Ala Thr Glu Ser Ala Tyr Leu Ala His Arg Asn Gln Ser Leu
        1               5                   10                  15

        Asp Leu Ala Glu Gln Glu Leu Val Asp
                    20                  25
```

(2) INFORMATION FOR SEQ ID NO:31:

    (i) SEQUENCE CHARACTERISTICS:

        (A) LENGTH:- 25 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:31:

```
        Ala Phe Ser Gly Val Ala Ala Thr Glu Ser Ala Tyr Leu Ala His Arg
        1               5                   10                  15

        Asn Gln Ser Leu Asp Leu Ala Glu Gln
                    20                  25
```

(2) INFORMATION FOR SEQ ID NO:32:

    (i) SEQUENCE CHARACTERISTICS:

        (A) LENGTH: 29 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:32:

```
Val Ala Ala Thr Glu Ser Ala Tyr Leu Ala His Arg Asn Gln Ser Leu
1               5               10                  15

Asp Leu Ala Glu Gln Glu Leu Val Asp Cys Ala Ser Gln
            20              25
```

(2) INFORMATION FOR SEQ ID NO:33:

    (i) SEQUENCE CHARACTERISTICS:

        (A) LENGTH: 28 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:33:

```
Glu Tyr Ile Gln His Asn Gly Val Val Gln Glu Ser Tyr Tyr Arg Tyr
1               5               10                  15

Val Ala Arg Glu Gln Cys Arg Arg Pro Asn Ala Gln
            20              25
```

(2) INFORMATION FOR SEQ ID NO:34:

    (i) SEQUENCE CHARACTERISTICS:

        (A) LENGTH: 29 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:34:

```
Asp Thr Ile Pro Arg Gly Ile Glu Tyr Ile Gln His Asn Gly Val Val
1               5               10                  15

Gln Glu Ser Tyr Tyr Arg Tyr Val Ala Arg Glu Gln Ser
            20              25
```

(2) INFORMATION FOR SEQ ID NO:35:

    (i) SEQUENCE CHARACTERISTICS:

        (A) LENGTH: 29 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:35:

```
Gln Ile Tyr Pro Pro Asn Ala Asn Lys Ile Arg Glu Ala Leu Ala Gln
1               5                   10              15

Thr His Ser Ala Ile Ala Val Ile Ile Gly Ile Lys Asp
            20              25
```

(2) INFORMATION FOR SEQ ID NO:36:

(i) SEQUENCE CHARACTERISTICS:

(A) LENGTH: 22 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:36:

```
Gln Ile Tyr Pro Pro Asn Ala Asn Lys Ile Arg Glu Ala Leu Ala Gln
1               5                   10              15

Thr His Ser Ala Ile Ala
            20
```

(2) INFORMATION FOR SEQ ID NO:37:

(i) SEQUENCE CHARACTERISTICS:

(A) LENGTH: 26 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:37:

```
Ile Ile Gly Ile Lys Asp Leu Asp Ala Phe Arg His Tyr Asp Gly Arg
1               5                   10              15

Thr Ile Ile Gln Arg Asp Asn Gly Tyr Gln
            20              25
```

(2) INFORMATION FOR SEQ ID NO:38:

(i) SEQUENCE CHARACTERISTICS:

(A) LENGTH: 25 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:38:

```
Arg Asp Asn Gly Tyr Gln Phe Asn Tyr His Ala Val Asn Ile Val Gly
1               5                   10                  15

Tyr Ser Asn Ala Gln Gly Val Asp Tyr
            20              25
```

(2) INFORMATION FOR SEQ ID NO:39:

(i) SEQUENCE CHARACTERISTICS:

(A) LENGTH: 29 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:39:

```
Asn Ile Val Gly Tyr Ser Asn Ala Gln Gly Val Asp Tyr Trp Ile Val
1               5                   10                  15

Arg Asn Ser Trp Asp Thr Asn Trp Gly Asp Asn Gly Tyr
            20                  25
```

(2) INFORMATION FOR SEQ ID NO:40:

(i) SEQUENCE CHARACTERISTICS:

(A) LENGTH: 23 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:40:

```
Asn Ile Val Gly Tyr Ser Asn Ala Gln Gly Val Asp Tyr Trp Ile Val
1               5                   10                  15

Arg Asn Ser Trp Asp Thr Asn
            20
```

(2) INFORMATION FOR SEQ ID NO:41:

(i) SEQUENCE CHARACTERISTICS:

(A) LENGTH: 20 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:41:

```
          Asp Gln Val Asp Val Lys Asp Cys Ala Asn His Glu Ile Lys Lys Val
          1               5                   10                  15

          Leu Val Pro Gly
                      20
```

(2) INFORMATION FOR SEQ ID NO:42:

    (i) SEQUENCE CHARACTERISTICS:

        (A) LENGTH: 25 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:42:

```
          His Glu Ile Lys Lys Val Leu Val Pro Gly Cys His Gly Ser Glu Pro
          1               5                   10                  15

          Cys Ile Ile His Arg Gly Lys Pro Phe
                      20                  25
```

(2) INFORMATION FOR SEQ ID NO:43:

    (i) SEQUENCE CHARACTERISTICS:

        (A) LENGTH: 29 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:43:

```
          His Gly Ser Glu Pro Cys Ile Ile His Arg Gly Lys Pro Phe Gln Leu
          1               5                   10                  15

          Glu Ala Val Phe Glu Ala Asn Gln Asn Thr Lys Thr Ala
                      20                  25
```

(2) INFORMATION FOR SEQ ID NO:44:

    (i) SEQUENCE CHARACTERISTICS:

        (A) LENGTH: 25 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:44:

```
Gln Leu Glu Ala Val Phe Glu Ala Asn Gln Asn Thr Lys Thr Ala Lys
1               5                   10                  15

Ile Glu Ile Lys Ala Ser Ile Asp Gly
                20                  25
```

(2) INFORMATION FOR SEQ ID NO:45:

(i) SEQUENCE CHARACTERISTICS:

(A) LENGTH: 27 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:45:

```
Lys Ile Glu Ile Lys Ala Ser Ile Asp Gly Leu Glu Val Asp Val Pro
1               5                   10                  15

Gly Ile Asp Pro Asn Ala Cys His Tyr Met Lys
                20                  25
```

(2) INFORMATION FOR SEQ ID NO:46:

(i) SEQUENCE CHARACTERISTICS:

(A) LENGTH: 26 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:46:

```
Leu Glu Val Asp Val Pro Gly Ile Asp Pro Asn Ala Cys His Tyr Met
1               5                   10                  15

Lys Cys Pro Leu Val Lys Gly Gln Gln Tyr
                20                  25
```

(2) INFORMATION FOR SEQ ID NO:47:

(i) SEQUENCE CHARACTERISTICS:

(A) LENGTH: 27 amino acids
(B) TYPE: amino acid
(D) TOPOLOGY: linear '

(ii) MOLECULE TYPE: peptide
(v) FRAGMENT TYPE: N-terminal
(xi) SEQUENCE DESCRIPTION: SEQ ID NO:47:

Cys Pro Leu Val Lys Gly Gln Gln Tyr Asp Ile Lys Tyr Thr Trp Asn
1                   5                   10                  15

Val Pro Lys Ile Ala Pro Lys Ser Glu Asn Val
                20                  25

(2) INFORMATION FOR SEQ ID NO:48:

    (i) SEQUENCE CHARACTERISTICS:

        (A) LENGTH: 26 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:48:

Asp Ile Lys Tyr Thr Trp Asn Val Pro Lys Ile Ala Pro Lys Ser Glu
1                   5                   10                  15

Asn Val Val Val Thr Val Lys Val Met Gly
                20                  25

(2) INFORMATION FOR SEQ ID NO:49:

    (i) SEQUENCE CHARACTERISTICS:

        (A) LENGTH: 25 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:49:

Val Val Thr Val Lys Val Met Gly Asp Asp Gly Val Leu Ala Cys Ala
1                   5                   10                  15

Ile Ala Thr His Ala Lys Ile Arg Asp
                20                  25

(2) INFORMATION FOR SEQ ID NO:50:

    (i) SEQUENCE CHARACTERISTICS:

        (A) LENGTH: 26 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:50:

```
Asp Gln Val Asp Val Lys Asp Cys Ala Asn His Glu Ile Lys Lys Val
1               5                   10                  15

Leu Val Pro Gly Cys His Gly Ser Glu Pro
            20                  25
```

(2) INFORMATION FOR SEQ ID NO:51:

   (i) SEQUENCE CHARACTERISTICS:

      (A) LENGTH: 26 amino acids
      (B) TYPE: amino acid
      (D) TOPOLOGY: linear

   (ii) MOLECULE TYPE: peptide
   (v) FRAGMENT TYPE: N-terminal
   (xi) SEQUENCE DESCRIPTION: SEQ ID NO:51:

```
Asp Gln Val Asp Val Lys Asp Glu Ala Asn His Glu Ile Lys Lys Val
1               5                   10                  15

Leu Val Pro Gly Cys His Gly Ser Glu Pro
            20                  25
```

(2) INFORMATION FOR SEQ ID NO:52:

   (i) SEQUENCE CHARACTERISTICS:

      (A) LENGTH: 26 amino acids
      (B) TYPE: amino acid
      (D) TOPOLOGY: linear

   (ii) MOLECULE TYPE: peptide
   (v) FRAGMENT TYPE: N-terminal
   (xi) SEQUENCE DESCRIPTION: SEQ ID NO:52:

```
Asp Gln Val Asp Val Lys Asp Ser Ala Asn His Glu Ile Lys Lys Val
1               5                   10                  15

Leu Val Pro Gly Cys His Gly Ser Glu Pro
            20                  25
```

(2) INFORMATION FOR SEQ ID NO:53:

   (i) SEQUENCE CHARACTERISTICS:

      (A) LENGTH: 26 amino acids
      (B) TYPE: amino acid
      (D) TOPOLOGY: linear

   (ii) MOLECULE TYPE: peptide
   (v) FRAGMENT TYPE: N-terminal
   (xi) SEQUENCE DESCRIPTION: SEQ ID NO:53:

```
Asp Gln Val Asp Val Lys Asp Cys Ala Asn His Glu Ile Lys Lys Val
1               5               10                  15

Leu Val Pro Gly Glu His Gly Ser Glu Pro
            20              25
```

(2) INFORMATION FOR SEQ ID NO:54:

(i) SEQUENCE CHARACTERISTICS:

(A) LENGTH: 26 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:54:

```
Asp Gln Val Asp Val Lys Asp Cys Ala Asn His Glu Ile Lys Lys Val
1               5               10                  15

Leu Val Pro Gly Ser His Gly Ser Glu Pro
            20              25
```

(2) INFORMATION FOR SEQ ID NO:55:

(i) SEQUENCE CHARACTERISTICS:

(A) LENGTH: 26 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:55:

```
Asp Gln Val Asp Val Lys Asp Glu Ala Asn His Glu Ile Lys Lys Val
1               5               10                  15

Leu Val Pro Gly Glu His Gly Ser Glu Pro
            20              25
```

(2) INFORMATION FOR SEQ ID NO:56:

(i) SEQUENCE CHARACTERISTICS:

(A) LENGTH: 26 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:56:

```
Asp Gln Val Asp Val Lys Asp Ser Ala Asn His Glu Ile Lys Lys Val
1               5                   10                  15

Leu Val Pro Gly Ser His Gly Ser Glu Pro
            20                  25
```

(2) INFORMATION FOR SEQ ID NO:57:

    (i) SEQUENCE CHARACTERISTICS:

        (A) LENGTH: 20 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:57:

```
Asp Gln Val Asp Val Lys Asp Glu Ala Asn His Glu Ile Lys Lys Val
1               5                   10                  15

Leu Val Pro Gly
            20
```

(2) INFORMATION FOR SEQ ID NO:58:

    (i) SEQUENCE CHARACTERISTICS:

        (A) LENGTH: 20 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:58:

```
Asp Gln Val Asp Val Lys Asp Ser Ala Asn His Glu Ile Lys Lys Val
1               5                   10                  15

Leu Val Pro Gly
            20
```

(2) INFORMATION FOR SEQ ID NO:59:

    (i) SEQUENCE CHARACTERISTICS:

        (A) LENGTH: 16 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:59:

```
        His Glu Ile Lys Lys Val Leu Val Pro Gly Cys His Gly Ser Glu Pro
        1               5                   10                  15
```

(2) INFORMATION FOR SEQ ID NO:60:

    (i) SEQUENCE CHARACTERISTICS:

        (A) LENGTH: 16 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:60:

```
        His Glu Ile Lys Lys Val Leu Val Pro Gly Glu His Gly Ser Glu Pro
        1               5                   10                  15
```

(2) INFORMATION FOR SEQ ID NO:61:

    (i) SEQUENCE CHARACTERISTICS:

        (A) LENGTH: 16 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:61:

```
        His Glu Ile Lys Lys Val Leu Val Pro Gly Ser His Gly Ser Glu Pro
        1               5                   10                  15
```

(2) INFORMATION FOR SEQ ID NO:62:

    (i) SEQUENCE CHARACTERISTICS:

        (A) LENGTH: 28 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:62:

```
        Lys Pro Phe Gln Leu Glu Ala Val Phe Glu Ala Asn Gln Asn Thr Lys
        1               5                   10                  15

        Thr Ala Lys Ile Glu Ile Lys Ala Ser Thr Asp Gly
                    20                  25
```

(2) INFORMATION FOR SEQ ID NO:63:

(i) SEQUENCE CHARACTERISTICS:

    (A) LENGTH: 28 amino acids
    (B) TYPE: amino acid
    (D) TOPOLOGY: linear

(ii) MOLECULE TYPE: peptide
(v) FRAGMENT TYPE: N-terminal
(xi) SEQUENCE DESCRIPTION: SEQ ID NO:63:

```
Gln Leu Glu Ala Val Phe Glu Ala Asn Gln Asn Thr Lys Thr Ala Lys
1               5                   10                  15

Ile Glu Ile Lys Ala Ser Ile Asp Gly Leu Glu Val
                20                  25
```

(2) INFORMATION FOR SEQ ID NO:64:

(i) SEQUENCE CHARACTERISTICS:

    (A) LENGTH: 27 amino acids
    (B) TYPE: amino acid
    (D) TOPOLOGY: linear

(ii) MOLECULE TYPE: peptide
(v) FRAGMENT TYPE: N-terminal
(xi) SEQUENCE DESCRIPTION: SEQ ID NO:64:

```
Phe Glu Ala Asn Gln Asn Thr Lys Thr Ala Lys Ile Glu Ile Lys Ala
1               5                   10                  15

Ser Ile Asp Gly Leu Glu Val Asp Val Pro Gly
                20                  25
```

(2) INFORMATION FOR SEQ ID NO:65:

(i) SEQUENCE CHARACTERISTICS:

    (A) LENGTH: 23 amino acids
    (B) TYPE: amino acid
    (D) TOPOLOGY: linear

(ii) MOLECULE TYPE: peptide
(v) FRAGMENT TYPE: N-terminal
(xi) SEQUENCE DESCRIPTION: SEQ ID NO:65:

```
Gln Asn Thr Lys Thr Ala Lys Ile Glu Ile Lys Ala Ser Ile Asp Gly
1               5                   10                  15

Leu Glu Val Asp Val Pro Gly
                20
```

(2) INFORMATION FOR SEQ ID NO:66:

(i) SEQUENCE CHARACTERISTICS:

(A) LENGTH: 26 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:66:

```
Pro Leu Val Lys Gly Gln Gln Tyr Asp Ile Lys Tyr Thr Trp Asn Val
1                   5                   10                  15

Pro Lys Ile Ala Pro Lys Ser Glu Asn Val
            20                  25
```

(2) INFORMATION FOR SEQ ID NO:67:

(i) SEQUENCE CHARACTERISTICS:

(A) LENGTH: 26 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:67:

```
Pro Leu Val Lys Gly Gln Gln Tyr Asp Ile Lys Tyr Thr Tyr Asn Val
1                   5                   10                  15

Pro Lys Ile Ala Pro Lys Ser Glu Asn Val
            20                  25
```

(2) INFORMATION FOR SEQ ID NO:68:

(i) SEQUENCE CHARACTERISTICS:

(A) LENGTH: 13 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:68:

```
Lys Ser Glu Asn Val Val Val Thr Val Lys Val Met Gly
1                   5                   10
```

(2) INFORMATION FOR SEQ ID NO:69:

(i) SEQUENCE CHARACTERISTICS:

(A) LENGTH: 23 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:69:

```
Thr Val Lys Val Met Gly Asp Asp Gly Val Leu Ala Cys Ala Ile Ala
1               5                   10                  15

Thr His Ala Lys Ile Arg Asp
                20
```

(2) INFORMATION FOR SEQ ID NO:70:

(i) SEQUENCE CHARACTERISTICS:

(A) LENGTH: 23 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:70:

```
Thr Val Lys Val Met Gly Asp Asp Gly Val Leu Ala Glu Ala Ile Ala
1               5                   10                  15

Thr His Ala Lys Ile Arg Asp
                20
```

(2) INFORMATION FOR SEQ ID NO:71:

(i) SEQUENCE CHARACTERISTICS:

(A) LENGTH: 23 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:71:

```
Thr Val Lys Val Leu Gly Asp Asp Gly Val Leu Ala Ser Ala Ile Ala
1               5                   10                  15

Thr His Ala Lys Ile Arg Asp
                20
```

(2) INFORMATION FOR SEQ ID NO:72:

(i) SEQUENCE CHARACTERISTICS:

(A) LENGTH: 21 amino acids
(B) TYPE: amino acid
(D) TOPOLOGY: linear

(ii) MOLECULE TYPE: peptide

(v) FRAGMENT TYPE: N-terminal
(xi) SEQUENCE DESCRIPTION: SEQ ID NO:72:

```
Thr Ser Ala Cys Arg Ile Asn Ser Val Asn Val Pro Ser Glu Leu Asp
1               5                   10                  15

Leu Arg Ser Leu Arg
            20
```

(2) INFORMATION FOR SEQ ID NO:73:

(i) SEQUENCE CHARACTERISTICS:

(A) LENGTH: 27 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:73:

```
Glu Leu Asp Leu Arg Ser Leu Arg Thr Val Thr Pro Ile Arg Met Gln
1               5                   10                  15

Gly Gly Cys Gly Ser Cys Trp Ala Phe Ser Gly
            20              25
```

(2) INFORMATION FOR SEQ ID NO:74:

(i) SEQUENCE CHARACTERISTICS:

(A) LENGTH: 29 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:74:

```
Thr Val Thr Pro Ile Arg Met Gln Gly Gly Cys Gly Ser Cys Trp Ala
1               5                   10                  15

Phe Ser Gly Val Ala Ala Thr Glu Ser Ala Tyr Leu Ala
            20              25
```

(2) INFORMATION FOR SEQ ID NO:75:

(i) SEQUENCE CHARACTERISTICS:

(A) LENGTH: 21 amino acids
(B) TYPE: amino acid
(D) TOPOLOGY: linear

(ii) MOLECULE TYPE: peptide
(v) FRAGMENT TYPE: N-terminal

(xi) SEQUENCE DESCRIPTION: SEQ ID NO:75:

```
Val Ala Ala Thr Glu Ser Ala Tyr Leu Ala Tyr Arg Asn Thr Ser Leu
1               5                   10                  15

Asp Leu Ser Glu Gln
            20
```

(2) INFORMATION FOR SEQ ID NO:76:

(i) SEQUENCE CHARACTERISTICS:

(A) LENGTH: 22 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:76:

```
Tyr Arg Asn Thr Ser Leu Asp Leu Ser Glu Gln Glu Leu Val Asp Cys
1               5                   10                  15

Ala Ser Gln His Gly Cys
            20
```

(2) INFORMATION FOR SEQ ID NO:77:

(i) SEQUENCE CHARACTERISTICS:

(A) LENGTH: 21 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:77:

```
Glu Leu Val Asp Cys Ala Ser Gln His Gly Cys His Gly Asp Thr Ile
1               5                   10                  15

Pro Arg Gly Ile Glu
            20
```

(2) INFORMATION FOR SEQ ID NO:78:

(i) SEQUENCE CHARACTERISTICS:

(A) LENGTH: 28 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:78:

```
Gly Asp Thr Ile Pro Arg Gly Ile Glu Tyr Ile Gln Gln Asn Gly Val
1               5                   10                  15

Val Glu Glu Arg Ser Tyr Pro Tyr Val Ala Arg Glu
                20              25
```

(2) INFORMATION FOR SEQ ID NO:79:

   (i) SEQUENCE CHARACTERISTICS:

      (A) LENGTH: 25 amino acids
      (B) TYPE: amino acid
      (D) TOPOLOGY: linear

   (ii) MOLECULE TYPE: peptide
   (v) FRAGMENT TYPE: N-terminal
   (xi) SEQUENCE DESCRIPTION: SEQ ID NO:79:

```
Gln Asn Gly Val Val Glu Glu Arg Ser Tyr Pro Tyr Val Ala Arg Glu
1               5                   10                  15

Gln Arg Cys Arg Arg Pro Asn Ser Gln
                20              25
```

(2) INFORMATION FOR SEQ ID NO:80:

   (i) SEQUENCE CHARACTERISTICS:

      (A) LENGTH: 19 amino acids
      (B) TYPE: amino acid
      (D) TOPOLOGY: linear

   (ii) MOLECULE TYPE: peptide
   (v) FRAGMENT TYPE: N-terminal
   (xi) SEQUENCE DESCRIPTION: SEQ ID NO:80:

```
Gln Arg Cys Arg Arg Pro Asn Ser Gln His Tyr Gly Ile Ser Asn Tyr
1               5                   10                  15

Cys Gln Ile
```

(2) INFORMATION FOR SEQ ID NO:81:

   (i) SEQUENCE CHARACTERISTICS:

      (A) LENGTH: 22 amino acids
      (B) TYPE: amino acid
      (D) TOPOLOGY: linear

   (ii) MOLECULE TYPE: peptide
   (v) FRAGMENT TYPE: N-terminal
   (xi) SEQUENCE DESCRIPTION: SEQ ID NO:81:

```
His Tyr Gly Ile Ser Asn Tyr Cys Gln Ile Tyr Pro Pro Asp Val Lys
1               5                   10                  15

Gln Ile Arg Glu Ala Leu
                20
```

(2) INFORMATION FOR SEQ ID NO:82:

    (i) SEQUENCE CHARACTERISTICS:

      (A) LENGTH: 24 amino acids
      (B) TYPE: amino acid
      (D) TOPOLOGY: linear

    (ii) MOLECULE TYPE: peptide
    (v) FRAGMENT TYPE: N-terminal
    (xi) SEQUENCE DESCRIPTION: SEQ ID NO:82:

```
Tyr Pro Pro Asp Val Lys Gln Ile Arg Glu Ala Leu Phe Gln Thr His
1               5                   10                  15

Thr Ala Ile Ala Val Ile Ile Gly
                20
```

(2) INFORMATION FOR SEQ ID NO:83:

    (i) SEQUENCE CHARACTERISTICS:

      (A) LENGTH: 26 amino acids
      (B) TYPE: amino acid
      (D) TOPOLOGY: linear

    (ii) MOLECULE TYPE: peptide
    (v) FRAGMENT TYPE: N-terminal
    (xi) SEQUENCE DESCRIPTION: SEQ ID NO:83:

```
Thr Gln Thr His Thr Ala Ile Ala Val Ile Ile Gly Ile Lys Asp Leu
1               5                   10                  15

Arg Ala Phe Gln His Tyr Asp Gly Arg Thr
                20                  25
```

(2) INFORMATION FOR SEQ ID NO:84:

    (i) SEQUENCE CHARACTERISTICS:

      (A) LENGTH: 26 amino acids
      (B) TYPE: amino acid
      (D) TOPOLOGY: linear

    (ii) MOLECULE TYPE: peptide
    (v) FRAGMENT TYPE: N-terminal
    (xi) SEQUENCE DESCRIPTION: SEQ ID NO:84:

```
Ile Lys Asp Leu Arg Ala Phe Gln His Tyr Asp Gly Arg Thr Ile Ile
1                   5                   10                      15

Gln His Asp Asn Gly Tyr Gln Pro Asn Tyr
                20                  25
```

(2) INFORMATION FOR SEQ ID NO:85:

    (i) SEQUENCE CHARACTERISTICS:

        (A) LENGTH: 15 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:85:

```
Asp Gly Arg Thr Ile Ile Gln His Asp Asn Gly Tyr Gln Pro Asn
1               5                   10                  15
```

(2) INFORMATION FOR SEQ ID NO:86:

    (i) SEQUENCE CHARACTERISTICS:

        (A) LENGTH: 23 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:86:

```
Ile Ile Gln His Asp Asn Gly Tyr Gln Pro Asn Tyr His Ala Val Asn
1               5                   10                      15

Ile Val Gly Tyr Gly Ser Thr
                20
```

(2) INFORMATION FOR SEQ ID NO:87:

    (i) SEQUENCE CHARACTERISTICS:

        (A) LENGTH: 22 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:87:

```
His Ala Val Asn Ile Val Gly Tyr Gly Ser Thr Gln Gly Asp Asp Tyr
1                   5               10                  15

Trp Ile Val Arg Asn Ser
                20
```

(2) INFORMATION FOR SEQ ID NO:88:

    (i) SEQUENCE CHARACTERISTICS:

        (A) LENGTH: 24 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:88:

```
Gln Gly Asp Asp Tyr Trp Ile Val Arg Asn Ser Trp Asp Thr Thr Trp
1                   5               10                  15

Gly Asp Ser Gly Tyr Gly Tyr Phe
                20
```

(2) INFORMATION FOR SEQ ID NO:89:

    (i) SEQUENCE CHARACTERISTICS:

        (A) LENGTH: 26 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:89:

```
Gly Asp Ser Gly Tyr Gly Tyr Phe Gln Ala Gly Asn Asn Leu Met Met
1                   5               10                  15

Ile Glu Gln Tyr Pro Tyr Val Val Ile Met
                20              25
```

(2) INFORMATION FOR SEQ ID NO:90:

    (i) SEQUENCE CHARACTERISTICS:

        (A) LENGTH: 29 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:90:

```
Thr Ser Ala Cys Arg Ile Asn Ser Val Asn Val Pro Ser Glu Leu Asp
1               5               10              15

Leu Arg Ser Leu Arg Thr Val Thr Pro Ile Arg Met Gln
            20              25
```

(2) INFORMATION FOR SEQ ID NO:91:

(i) SEQUENCE CHARACTERISTICS:

(A) LENGTH: 25 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:91:

```
Arg Ile Asn Ser Val Asn Val Pro Ser Glu Leu Asp Leu Arg Ser Leu
1               5               10              15

Arg Thr Val Thr Pro Ile Arg Met Gln
            20              25
```

(2) INFORMATION FOR SEQ ID NO:92:

(i) SEQUENCE CHARACTERISTICS:

(A) LENGTH: 29 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:92:

```
Ala Phe Ser Gly Val Ala Ala Thr Glu Ser Ala Tyr Leu Ala Tyr Arg
1               5               10              15

Asn Thr Ser Leu Asp Leu Ser Glu Gln Glu Leu Val Asp
            20              25
```

(2) INFORMATION FOR SEQ ID NO:93:

(i) SEQUENCE CHARACTERISTICS:

(A) LENGTH: 25 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:93:

```
        Val Ala Ala Thr Glu Ser Ala Tyr Leu Ala Tyr Arg Asn Thr Ser Leu
        1                   5                   10                  15

        Asp Leu Ser Glu Gln Glu Leu Val Asp
                        20                  25
```

(2) INFORMATION FOR SEQ ID NO:94:

(i) SEQUENCE CHARACTERISTICS:

(A) LENGTH: 29 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:94:

```
        Val Ala Ala Thr Glu Ser Ala Tyr Leu Ala Tyr Arg Asn Thr Ser Leu
        1                   5                   10                  15

        Asp Leu Ser Glu Gln Glu Leu Val Asp Cys Ala Ser Gln
                        20                  25
```

(2) INFORMATION FOR SEQ ID NO:95:

(i) SEQUENCE CHARACTERISTICS:

(A) LENGTH: 29 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:95:

```
        Glu Tyr Ile Gln Gln Asn Gly Val Val Glu Glu Arg Ser Tyr Pro Tyr
        1                   5                   10                  15

        Val Ala Arg Glu Gln Arg Cys Arg Arg Pro Asn Ser Gln
                        20                  25
```

(2) INFORMATION FOR SEQ ID NO:96:

(i) SEQUENCE CHARACTERISTICS:

(A) LENGTH: 29 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:96:

```
Asp Thr Ile Pro Arg Gly Ile Glu Tyr Ile Gln Gln Asn Gly Val Val
1               5               10                  15

Glu Glu Arg Ser Tyr Pro Tyr Val Ala Arg Glu Gln Arg
            20              25
```

(2) INFORMATION FOR SEQ ID NO:97:

(i) SEQUENCE CHARACTERISTICS:

(A) LENGTH: 29 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:97:

```
Gln Ile Tyr Pro Pro Asp Val Lys Gln Ile Arg Glu Ala Leu Thr Gln
1               5               10                  15

Thr His Thr Ala Ile Ala Val Ile Ile Gly Ile Lys Asp
            20              25
```

(2) INFORMATION FOR SEQ ID NO:98:

(i) SEQUENCE CHARACTERISTICS:

(A) LENGTH: 22 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:98:

```
Gln Ile Tyr Pro Pro Asp Val Lys Gln Ile Arg Glu Ala Leu Thr Gln
1               5               10                  15

Thr His Thr Ala Ile Ala
            20
```

(2) INFORMATION FOR SEQ ID NO:99:

(i) SEQUENCE CHARACTERISTICS:

(A) LENGTH: 26 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:99:

```
    Ile Ile Gly Ile Lys Asp Leu Arg Ala Phe Gln His Tyr Asp Gly Arg
    1               5                   10              15

    Thr Ile Ile Gln His Asp Asn Gly Tyr Gln
                20              25
```

(2) INFORMATION FOR SEQ ID NO:100:

   (i) SEQUENCE CHARACTERISTICS:

      (A) LENGTH: 25 amino acids
      (B) TYPE: amino acid
      (D) TOPOLOGY: linear

   (ii) MOLECULE TYPE: peptide
   (v) FRAGMENT TYPE: N-terminal
   (xi) SEQUENCE DESCRIPTION: SEQ ID NO:100:

```
    His Asp Asn Gly Tyr Gln Pro Asn Tyr His Ala Val Asn Ile Val Gly
    1               5                   10              15

    Tyr Gly Ser Thr Gln Gly Asp Asp Tyr
                20              25
```

(2) INFORMATION FOR SEQ ID NO:101:

   (i) SEQUENCE CHARACTERISTICS:

      (A) LENGTH: 29 amino acids
      (B) TYPE: amino acid
      (D) TOPOLOGY: linear

   (ii) MOLECULE TYPE: peptide
   (v) FRAGMENT TYPE: N-terminal
   (xi) SEQUENCE DESCRIPTION: SEQ ID NO:101:

```
    Asn Ile Val Gly Tyr Gly Ser Thr Gln Gly Asp Asp Tyr Trp Ile Val
    1               5                   10              15

    Arg Asn Ser Trp Asp Thr Thr Trp Gly Asp Ser Gly Tyr
                20              25
```

(2) INFORMATION FOR SEQ ID NO:102:

   (i) SEQUENCE CHARACTERISTICS:

      (A) LENGTH: 23 amino acids
      (B) TYPE: amino acid
      (D) TOPOLOGY: linear

   (ii) MOLECULE TYPE: peptide
   (v) FRAGMENT TYPE: N-terminal
   (xi) SEQUENCE DESCRIPTION: SEQ ID NO:102:

```
        Asn Ile Val Gly Tyr Gly Ser Thr Gln Gly Asp Asp Tyr Trp Ile Val
        1                   5                   10                  15

        Arg Asn Ser Trp Asp Thr Thr
                        20
```

(2) INFORMATION FOR SEQ ID NO:103:

    (i) SEQUENCE CHARACTERISTICS:

        (A) LENGTH: 20 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:103:

```
        Asp Gln Val Asp Val Lys Asp Cys Ala Asn Asn Glu Ile Lys Lys Val
        1                   5                   10                  15

        Met Val Asp Gly
                    20
```

(2) INFORMATION FOR SEQ ID NO:104:

    (i) SEQUENCE CHARACTERISTICS:

        (A) LENGTH: 25 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:104:

```
        Asn Glu Ile Lys Lys Val Met Val Asp Gly Cys His Gly Ser Asp Pro
        1                   5                   10                  15

        Cys Ile Ile His Arg Gly Lys Pro Phe
                        20                  25
```

(2) INFORMATION FOR SEQ ID NO:105:

    (i) SEQUENCE CHARACTERISTICS:

        (A) LENGTH: 29 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:105:

```
His Gly Ser Asp Pro Cys Ile Ile His Arg Gly Lys Pro Phe Thr Leu
 1               5                  10                      15

Glu Ala Leu Phe Asp Ala Asn Gln Asn Thr Lys Thr Ala
              20                  25
```

(2) INFORMATION FOR SEQ ID NO:106:

(i) SEQUENCE CHARACTERISTICS:

(A) LENGTH: 19 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:106:

```
His Arg Gly Lys Pro Phe Thr Leu Glu Ala Leu Phe Asp Ala Asn Gln
 1               5                  10                      15

Asn Thr Lys
```

(2) INFORMATION FOR SEQ ID NO:107:

(i) SEQUENCE CHARACTERISTICS:

(A) LENGTH: 25 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:107:

```
Thr Leu Glu Ala Leu Phe Asp Ala Asn Gln Asn Thr Lys Thr Ala Lys
 1               5                  10                      15

Ile Glu Ile Lys Ala Ser Leu Asp Gly
              20                  25
```

(2) INFORMATION FOR SEQ ID NO:108:

(i) SEQUENCE CHARACTERISTICS:

(A) LENGTH: 26 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:108:

```
Gln Asn Thr Lys Thr Ala Lys Ile Glu Ile Lys Ala Ser Leu Asp Gly
1               5                   10                  15

Leu Glu Ile Asp Val Pro Gly Ile Asp Thr
                20                  25
```

(2) INFORMATION FOR SEQ ID NO:109:

    (i) SEQUENCE CHARACTERISTICS:

        (A) LENGTH: 27 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:109:

```
Lys Ile Glu Ile Lys Ala Ser Leu Asp Gly Leu Glu Ile Asp Val Pro
1               5                   10                  15

Gly Ile Asp Thr Asn Ala Cys His Phe Met Lys
                20                  25
```

(2) INFORMATION FOR SEQ ID NO:110:

    (i) SEQUENCE CHARACTERISTICS:

        (A) LENGTH: 26 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:110:

```
Leu Glu Ile Asp Val Pro Gly Ile Asp Thr Asn Ala Cys His Phe Met
1               5                   10                  15

Lys Cys Pro Leu Val Lys Gly Gln Gln Tyr
                20                  25
```

(2) INFORMATION FOR SEQ ID NO:111:

    (i) SEQUENCE CHARACTERISTICS:

        (A) LENGTH: 27 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:111:

```
Cys Pro Leu Val Lys Gly Gln Gln Tyr Asp Ala Lys Tyr Thr Trp Asn
1               5                   10                  15

Val Pro Lys Ile Ala Pro Lys Ser Glu Asn Val
            20                  25
```

(2) INFORMATION FOR SEQ ID NO:112:

(i) SEQUENCE CHARACTERISTICS:

(A) LENGTH: 26 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:112:

```
Asp Ala Lys Tyr Thr Trp Asn Val Pro Lys Ile Ala Pro Lys Ser Glu
1               5                   10                  15

Asn Val Val Val Thr Val Lys Leu Val Gly
            20                  25
```

(2) INFORMATION FOR SEQ ID NO:113:

(i) SEQUENCE CHARACTERISTICS:

(A) LENGTH: 35 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:113:

```
Pro Lys Ile Ala Pro Lys Ser Glu Asn Val Val Val Thr Val Lys Leu
1               5                   10                  15

Val Gly Asp Asn Gly Val Leu Ala Cys Ala Ile Ala Thr His Ala Lys
            20                  25                  30

Ile Arg Asp
        35
```

(2) INFORMATION FOR SEQ ID NO:114:

(i) SEQUENCE CHARACTERISTICS:

(A) LENGTH: 25 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:114:

```
Val Val Thr Val Lys Leu Val Gly Asp Asn Gly Val Leu Ala Cys Ala
1               5                   10                  15

Ile Ala Thr His Ala Lys Ile Arg Asp
            20          25
```

(2) INFORMATION FOR SEQ ID NO:115:

    (i) SEQUENCE CHARACTERISTICS:

        (A) LENGTH: 28 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:115:

```
Lys Pro Phe Thr Leu Glu Ala Leu Phe Asp Ala Asn Gln Asn Thr Lys
1               5                   10                  15

Thr Ala Lys Ile Glu Ile Lys Ala Ser Leu Asp Gly
            20          25
```

(2) INFORMATION FOR SEQ ID NO:116:

    (i) SEQUENCE CHARACTERISTICS:

        (A) LENGTH: 28 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:116:

```
Thr Leu Glu Ala Leu Phe Asp Ala Asn Gln Asn Thr Lys Thr Ala Lys
1               5                   10                  15

Ile Glu Ile Lys Ala Ser Leu Asp Gly Leu Glu Ile
            20          25
```

(2) INFORMATION FOR SEQ ID NO:117:

    (i) SEQUENCE CHARACTERISTICS:

        (A) LENGTH: 22 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:117:

```
His Arg Asn Gln Ser Leu Asp Leu Ala Glu Gln Asp Leu Val Asp Cys
1               5                   10                  15

Ala Ser Gln His Gly Cys
                20
```

(2) INFORMATION FOR SEQ ID NO:118:

    (i) SEQUENCE CHARACTERISTICS:

        (A) LENGTH: 21 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:118:

```
Asp Leu Val Asp Cys Ala Ser Gln His Gly Cys His Gly Asp Thr Ile
1               5                   10                  15

Pro Arg Gly Ile Glu
                20
```

(2) INFORMATION FOR SEQ ID NO:119:

    (i) SEQUENCE CHARACTERISTICS:

        (A) LENGTH: 29 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:119:

```
His Gly Ser Glu Pro Cys Ile Ile His Arg Gly Lys Pro Phe Gln Leu
1               5                   10                  15

Glu Ala Val Phe Glu Ala Val Gln Asn Thr Lys Thr Ala
                20                  25
```